# EUROPEAN PATENT APPLICATION

(11) **EP 4 559 910 A1**
(43) Date of publication of application: **28.05.2025**
(21) Application number: 23842329.7
(22) Date of filing: 18.07.2023
(51) Int. Cl.: C07D 405/14, A61K 31/44, A61P 3/00, A61P 3/10, A61P 35/00, A61P 37/00

(54) **POLYMORPHIC FORM OF GLP-1R AGONIST, PREPARATION METHOD THEREFOR AND USE THEREOF**

(30) Priority: 18.07.2022 CN 202210845410; 18.07.2022 CN 202210843897
(71) Applicant: Minidrank Therapeutics (Suzhou) New Drug Research and Development Co., Ltd, Suzhou, Jiangsu 215127 (CN)
(72) Inventor: ZHANG, Long, Suzhou, Jiangsu 215127 (CN); NIU, Zhangming, Suzhou, Jiangsu 215127 (CN); HU, Yang, Suzhou, Jiangsu 215127 (CN)
(74) Representative: Gille Hrabal Partnerschaftsgesellschaft mbB Patentanwälte
(86) International application number: PCT/CN2023/107997
(87) International publication number: WO 2024/017266

(57) **Abstract**

The present invention provides a polymorphic form of a GLP-1R agonist compound (III), a preparation method therefor and a use thereof. Compared with an amorphous form of compound (III), the polymorphic form of the present invention has higher stability and better processability, and is more suitable for preparing a drug for preventing or treating diseases related to a GLP-1R target and a signaling pathway thereof, such as type 2 diabetes, prediabetes, obesity, non-alcoholic fatty liver disease, non-alcoholic steatohepatitis, kidney disease, gout, hyperhematuria, cardiovascular disease, etc.

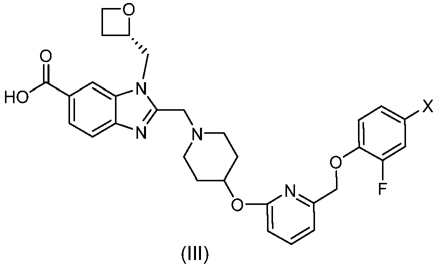

## Description

The present disclosure claims priority to:
the prior Patent Application No. 2022108454100 entitled "POLYMORPHIC FORM OF GLP-1R AGONIST COMPOUND, PREPARATION METHOD THEREFOR AND USE THEREOF" and filed with the China National Intellectual Property Administration on July 18, 2022; and
the prior Patent Application No. 2022108438979 entitled "POLYMORPHIC FORM OF GLP-1R AGONIST, PREPARATION METHOD THEREFOR AND USE THEREOF" and filed with the China National Intellectual Property Administration on July 18, 2022,
the contents of which are incorporated herein by reference in their entireties.

### TECHNICAL FIELD

The present disclosure relates to the field of pharmaceutical chemistry, and particularly, to a polymorph of a GLP-1R agonist, a method for preparing same, and use thereof.

### BACKGROUND

Diabetes is a chronic disease characterized by high blood glucose levels and is caused by (relatively or absolutely) insufficient insulin secretion or insulin action disorders. According to the ninth edition (the latest edition) of the International Diabetes Federation (IDF) Diabetes Atlas, approximately 463 million adults (aged 20-79) worldwide were living with diabetes in 2019, and the number of patients with diabetes is projected to be 578 million by 2030. At this rate, 700 million people worldwide will be living with diabetes in 2045. Therefore, diabetes has become one of the most pressing global social health problems of the 21st century.

Currently, various pharmacological methods are available to treat hyperglycemia and the attendant T2DM (Hampp et al., Use of Antidiabetic Drugs in the U.S., 2003-2012, Diabetes Care, 37:1367-1374, 2014). These methods can be classified into six major categories, each acting through a different major mechanism.

Insulin secretagogues include sulfonylureas, dipeptidyl peptidase IV (PP-IV) inhibitors, and glucagon-like peptide-1 receptor (GLP-1R) agonists, which enhance insulin secretion by acting on pancreatic β cells. Sulfonylureas have limited efficacy and tolerability, cause weight gain, and often induce hypoglycemia. DP-IV inhibitors have limited efficacy. Commercially available GLP-1R agonists are peptides that are administered by subcutaneous injection, and liraglutide is approved for the treatment of obesity.

Biguanides, such as metformin, are believed to act mainly by reducing hepatic glucose production. Biguanides often cause gastrointestinal discomfort and lactic acidosis, which further limit their use.

α-Glucosidase inhibitors, such as acarbose, can reduce intestinal glucose absorption. These medicaments often cause gastrointestinal discomfort.

Thiazolidinediones, such as pioglitazone and rosiglitazone, act on specific receptors in the liver, muscle, and adipose tissue. They regulate lipid metabolism and subsequently enhance the responses of these tissues to insulin action. Frequent use of these drugs may cause weight gain and may induce edema and anemia.

Insulin, either alone or in combination with the medicaments described above, is used for more severe cases, and frequent use of insulin may also cause weight gain and involve a risk of hypoglycemia.

Sodium-glucose cotransporter 2 (SGLT2) inhibitors (such as dapagliflozin, empagliflozin, canagliflozin, and ertugliflozin) inhibit glucose reabsorption in the kidneys, thereby lowering blood glucose levels. This emerging drug category may be associated with ketoacidosis and urinary tract infections.

However, except for GLP-1R agonists and SGLT2 inhibitors, these drugs have limited efficacy and fail to address the most important issues: the functional decline of β cells and related obesity. Therefore, there is a need for more effective pharmaceutical interventions with relatively few side effects and easy administration.

GLP-1 is a 30-amino-acid-long incretin hormone secreted by intestinal L cells in response to food intake. GLP-1 has been shown to stimulate insulin secretion in a physiological and glucose-dependent manner, reduce glucagon secretion, inhibit gastric emptying, reduce appetite, and stimulate β cell proliferation. In non-clinical trials, GLP-1 promotes continued β cell competence by stimulating the transcription of genes important for glucose-dependent insulin secretion and by promoting β cell neogenesis (Meier, et al., Biodrugs, 17(2):93-102, 2013).

In healthy individuals, GLP-1 plays an important role in regulating postprandial blood glucose levels by stimulating glucose-dependent insulin secretion by the pancreas to increase peripheral glucose absorption. GLP-1 also inhibits glucagon secretion, resulting in reduced hepatic glucose output. In addition, GLP-1 delays gastric emptying and slows small bowel movements to delay food absorption. In people with T2DM, the normal postprandial rise in GLP-1 is absent or reduced (Vilsbøll et al., Diabetes, 50: 609-613, 2001).

The structure of GLP-1 has been correspondingly engineered and modified in scientific research to increase its half-life and thus prolong its *in vivo* biological effect. However, currently, clinically used long-acting GLP-1 analogs, such as liraglutide, exenatide, etc., are all polypeptides and require frequent injections, which lead to relatively poor patient compliance. Therefore, the development of small-molecule GLP-1R agonists will focus on improving patient compliance, simplifying administration, and reducing side effects of drugs; these agonists have a wide clinical market prospect.

Hangzhou Mindrank AI Ltd. has developed a class of small-molecule compounds with novel structures and potent GLP-1R agonistic activity. Among them, a compound with the chemical name (*S*)-2-((4-((6-((4-chloro-2-fluorophenoxy)methyl)pyridin-2-yl)oxy)piperidin-1-yl)methyl)-1-(oxetan-2-ylmethyl)-1 *H*-benzo[*d*]imidazole-6-carboxylic acid and a compound with the chemical name (*S*)-2-((4-((6-((4-cyano-2-fluorophenoxy)methyl)pyridin-2-yl)oxy)piperidin-1-yl)methyl)-1-(oxetan-2-ylmethyl)-1 *H*-benzo[*d*]imidazole-6-carboxylic acid exhibit potent agonistic activity and good druggability, and the structures of the two compounds are as follows, respectively:

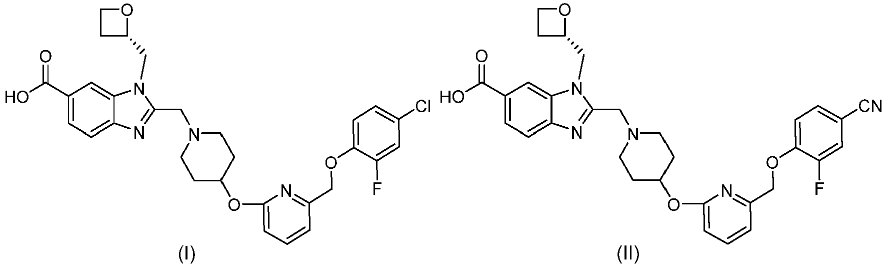

The free compounds of formula (I) and formula (II) have been identified by the inventor as amorphous compounds, which are prone to water absorption or softening and to crystal form transformation during long-term storage, and are therefore not suitable for the development of clinical formulations. To meet the needs of clinical research and the approval of pharmaceutical formulations, it is imperative to develop an aggregation form suitable for drug development, which can overcome the defects present in the prior art.

The successful development of solid forms of a drug often requires such properties as a solid form desirable for separation and purification after synthesis and a dosage form suitable for industrial production, for long-term preservation with minimized water absorption, decomposition or conversion to other solid forms and for rapid absorption in the human body after administration (e.g., soluble in water and gastric juice). To meet the needs of clinical research and the approval of pharmaceutical formulations, it is imperative to develop a solid drug form that is easy to separate and purify, suitable for industrial production, and physicochemically stable.

### SUMMARY

To solve the problems with the prior art, in a first aspect of the present disclosure, provided is a polymorph of compound III shown as follows, 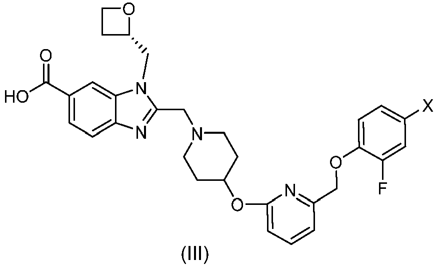 wherein, X is Cl or CN.

According to the embodiments of the present disclosure, the polymorph may be a crystal form of a non-solvate, a hydrate, or a solvate of compound III.

According to the embodiments of the present disclosure, compound III has a structure of compound I or compound II:

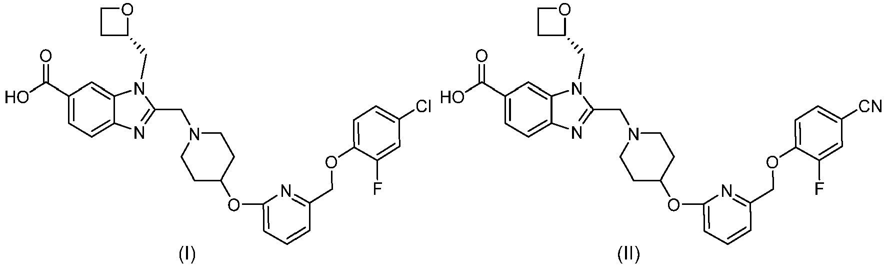

The crystal form of a non-solvate of compound I may be the following crystal form A, B, C, D, E, or F; the crystal form of a hydrate of compound I may be the following crystal form G; the crystal form of a solvate of compound I may be the following crystal form H, I-1, I-2, J, K, L, M, N, O, P, Q, R, S, or T.

The crystal form of a non-solvate of compound II may be the following crystal form 2A, 2B, 2C, 2D, or 2E; the crystal form of a hydrate of compound II may be the following crystal form 2F or 2G; the crystal form of a solvate of compound II may be the following crystal form 2H, 2I, 2J-1, 2J-2, 2K, 2L-1, 2L-2, 2M-1, 2M-2, 2N, 2O, 2P, 2Q-1, 2Q-2, 2R, 2S, 2T, or 2U.

Crystal form A of compound I provided by the present disclosure has an X-ray powder diffraction pattern comprising peaks at diffraction angles (2θ) of 19.79 ± 0.2°, 13.13 ± 0.2°, 22.07 ± 0.2°, and 9.48 ± 0.2°;
preferably, further comprising peaks at diffraction angles (2θ) of 7.80 ± 0.2°, 13.59 ± 0.2°, 11.43 ± 0.2°, 18.07 ± 0.2°, and 12.45 ± 0.2°;
and more preferably, further comprising peaks at diffraction angles (2θ) of 14.58 ± 0.2°, 24.66 ± 0.2°, 14.24 ± 0.2°, 4.85 ± 0.2°, 23.70 ± 0.2°, and 26.51 ± 0.2°.

Preferably, crystal form A has an X-ray powder diffraction pattern comprising diffraction angles (2θ) shown in Table 1, wherein the 2θ angles have a margin of error of ±0.20°:

**Table 1**

| 2θ (°) | Intensity% | 2θ (°) | Intensity% |
|---|---|---|---|
| 4.85 | 13.1 | 18.07 | 20.8 |
| 7.80 | 37.6 | 19.79 | 100.0 |
| 9.48 | 42.2 | 22.07 | 44.2 |
| 11.43 | 29.1 | 23.70 | 12.5 |
| 12.45 | 18.6 | 24.66 | 14.7 |
| 13.13 | 51.3 | 25.79 | 8.3 |
| 13.59 | 31.1 | 26.51 | 12.5 |
| 14.24 | 13.3 | 31.12 | 8.3 |
| 14.58 | 17.8 | | |

Preferably, crystal form A has X-ray powder diffraction intensities shown in Table 1.

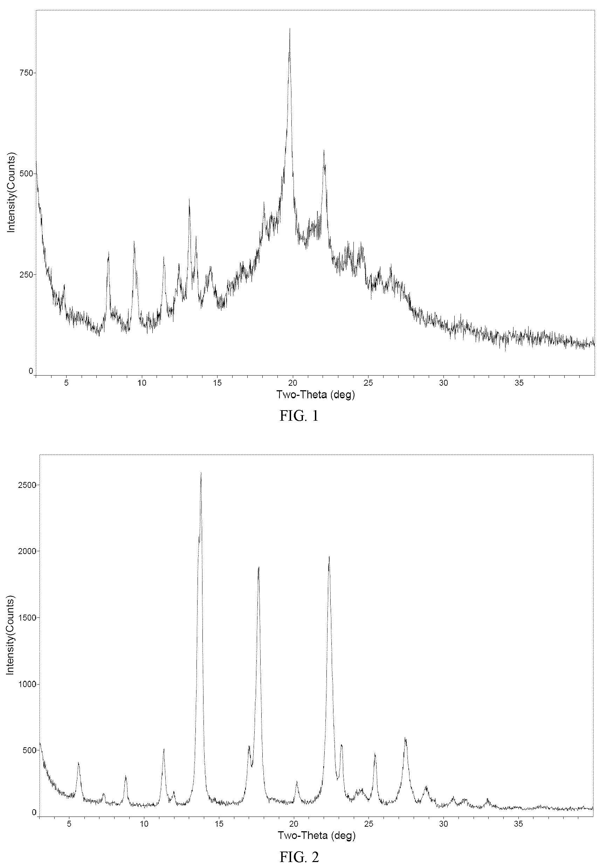

Preferably, crystal form A has an X-ray powder diffraction pattern substantially shown in FIG. 1.

Preferably, crystal form A exhibits an endothermic peak in a DSC analysis when heated to a peak temperature of about 99.81 °C.

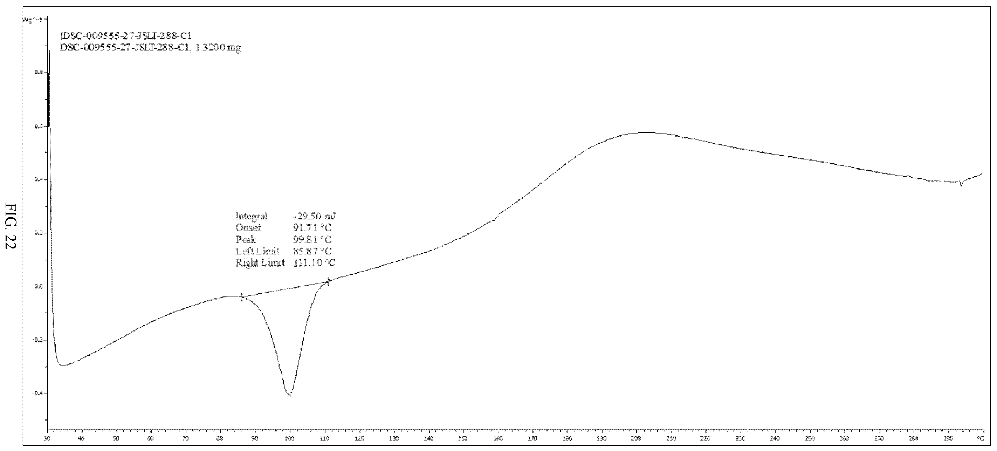

Preferably, crystal form A has a DSC profile substantially shown in FIG. 22.

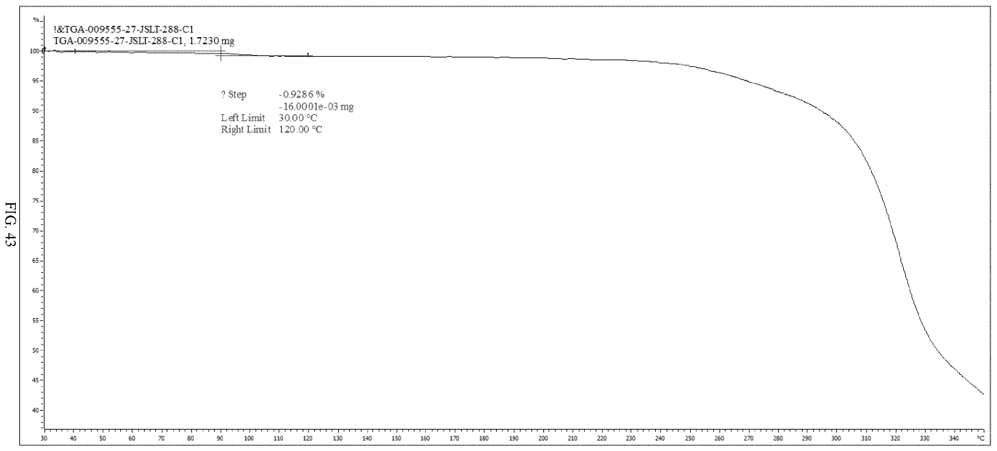

Preferably, crystal form A has a TGA profile substantially shown in FIG. 43.

Crystal form B of compound I provided by the present disclosure has an X-ray powder diffraction pattern comprising peaks at diffraction angles (2θ) of 13.79 ± 0.2°, 22.36 ± 0.2°, 17.66 ± 0.2°, and 27.41 ± 0.2°;
preferably, further comprising peaks at diffraction angles (2θ) of 11.31 ± 0.2°, 23.16 ± 0.2°, 25.40 ± 0.2°, 5.59 ± 0.2°, and 8.74 ± 0.2°;
and more preferably, further comprising peaks at diffraction angles (2θ) of 20.20 ± 0.2°, 28.85 ± 0.2°, 11.96 ± 0.2°, 24.19 ± 0.2°, 24.39 ± 0.2°, and 7.28 ± 0.2°.

Preferably, crystal form B has an X-ray powder diffraction pattern comprising diffraction angles (2θ) shown in Table 2, wherein the 2θ angles have a margin of error of ±0.20°:

**Table 2**

| 2θ (°) | Intensity% | 2θ (°) | Intensity% |
|---|---|---|---|
| 5.59 | 11.3 | 23.16 | 17.0 |
| 7.28 | 2.9 | 24.19 | 3.3 |
| 8.74 | 8.8 | 24.39 | 3.0 |
| 11.31 | 17.0 | 25.40 | 14.7 |
| 11.96 | 3.7 | 27.41 | 19.7 |
| 13.79 | 100.0 | 28.85 | 5.3 |
| 16.98 | 17.4 | 29.42 | 2.2 |
| 17.66 | 70.5 | 30.66 | 2.7 |
| 20.20 | 6.7 | 31.42 | 2.0 |
| 22.36 | 71.9 | 32.93 | 2.9 |

Preferably, crystal form B has X-ray powder diffraction intensities shown in Table 2.

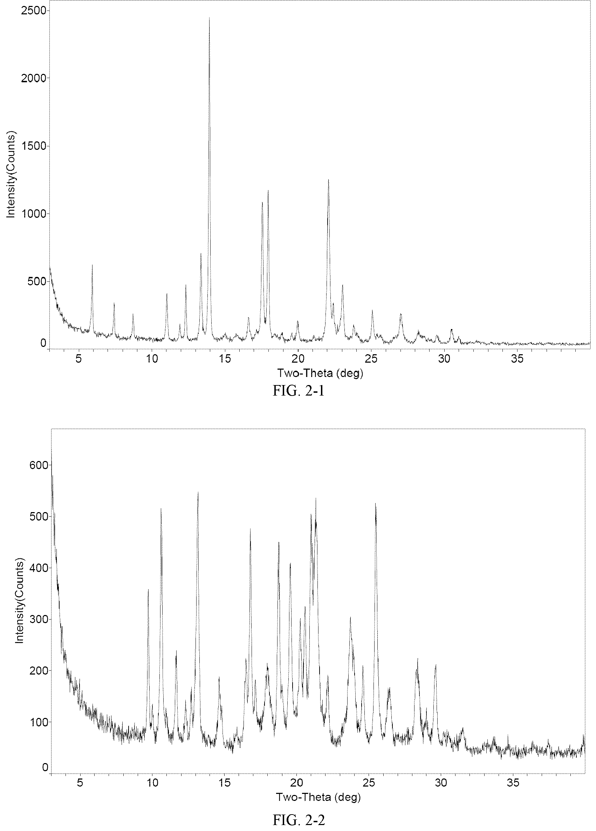

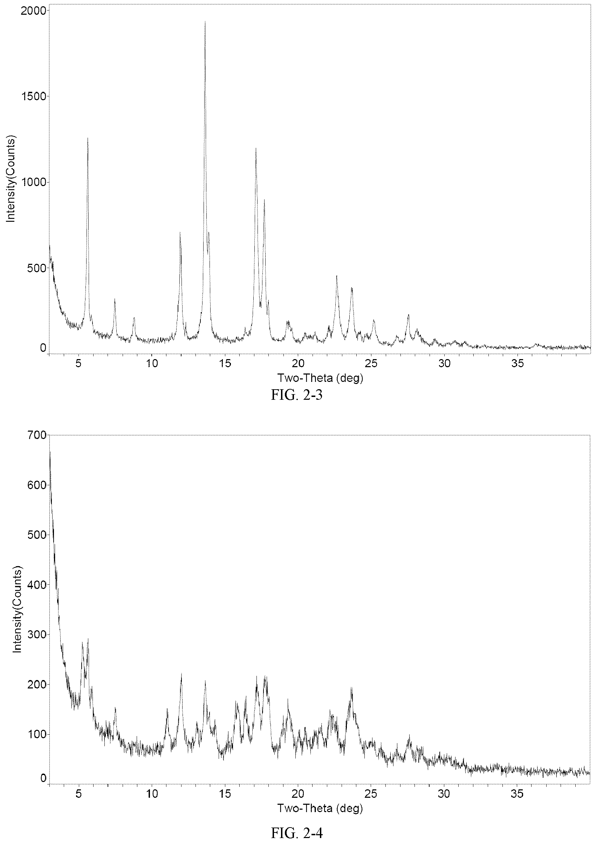

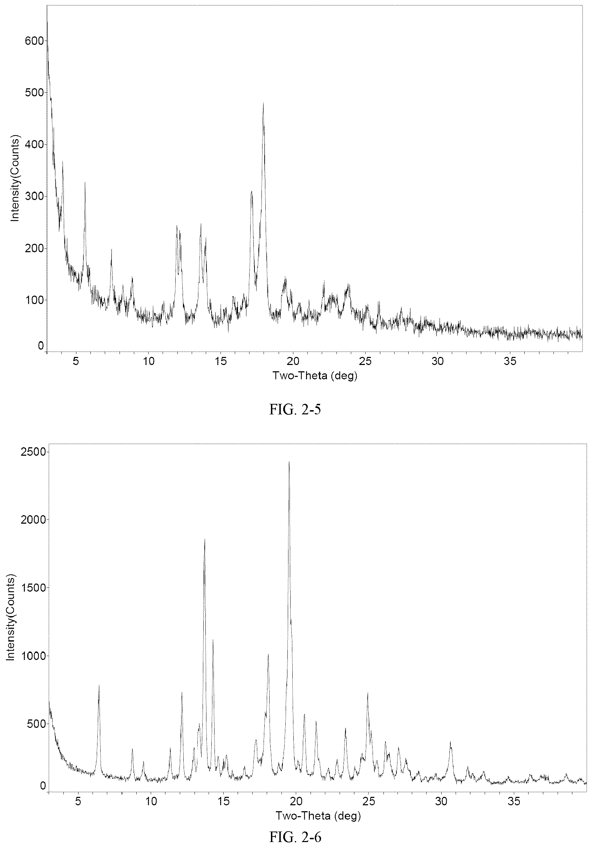

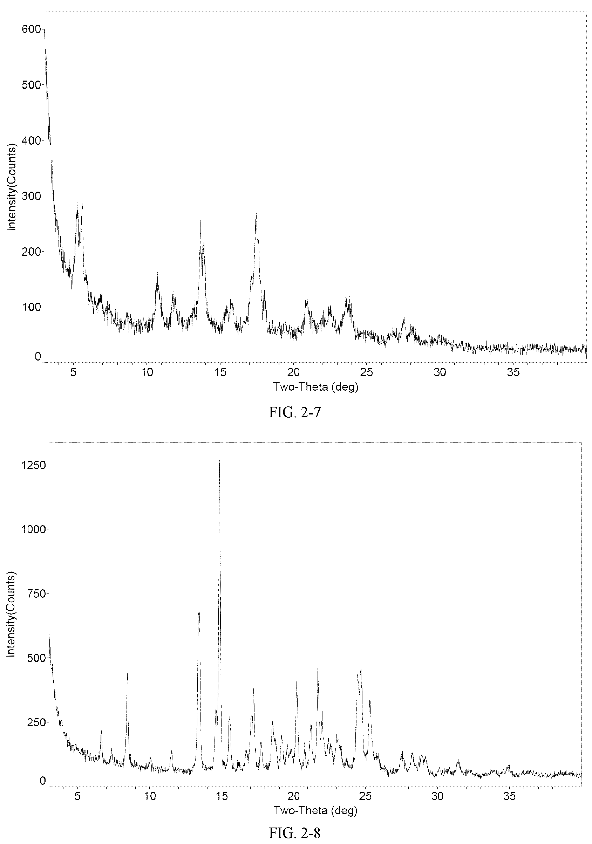

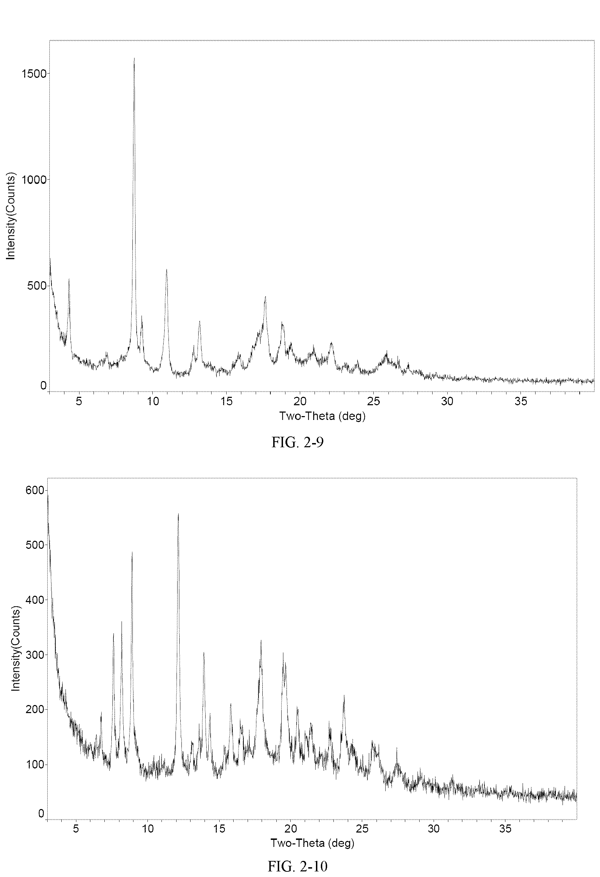

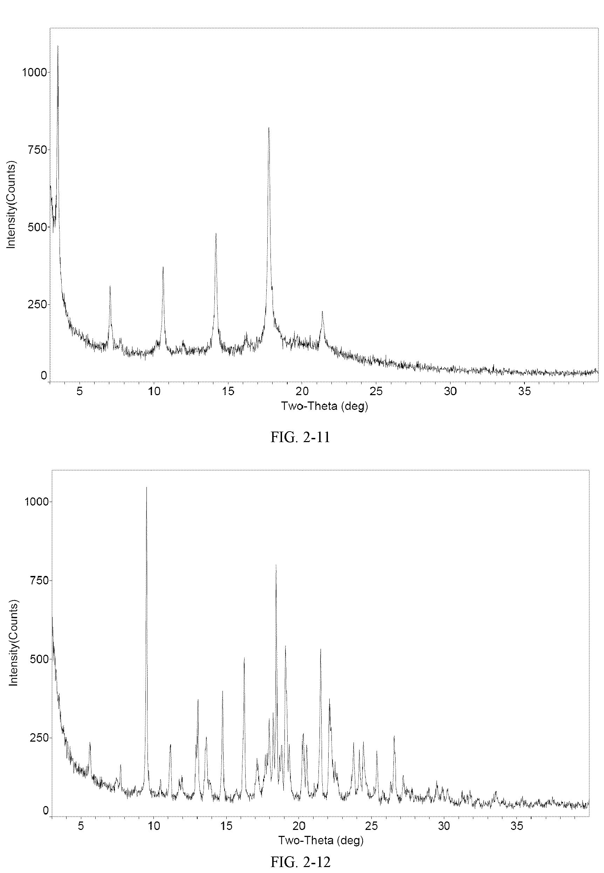

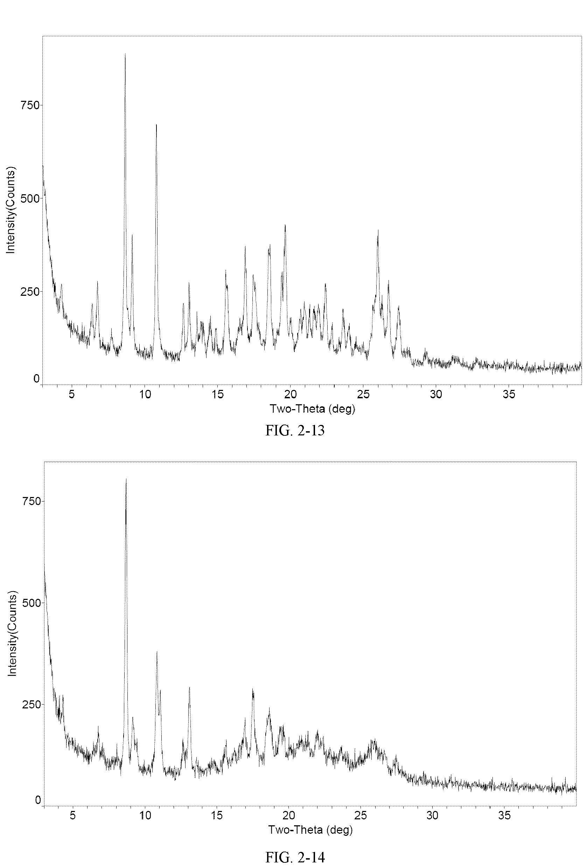

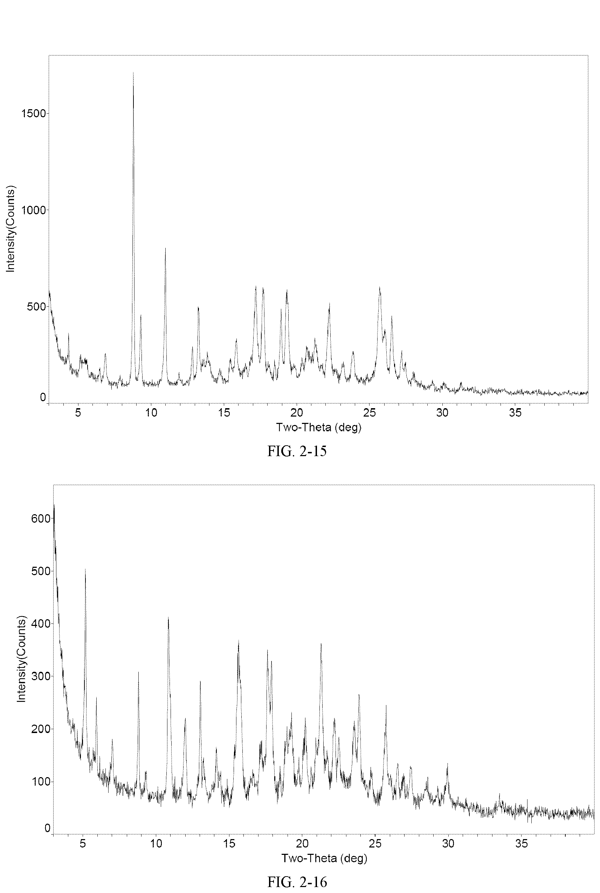

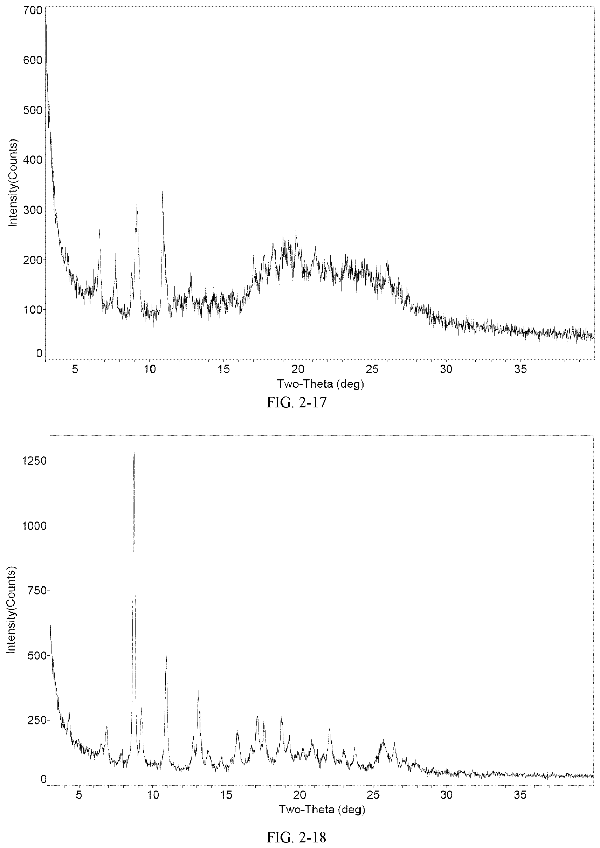

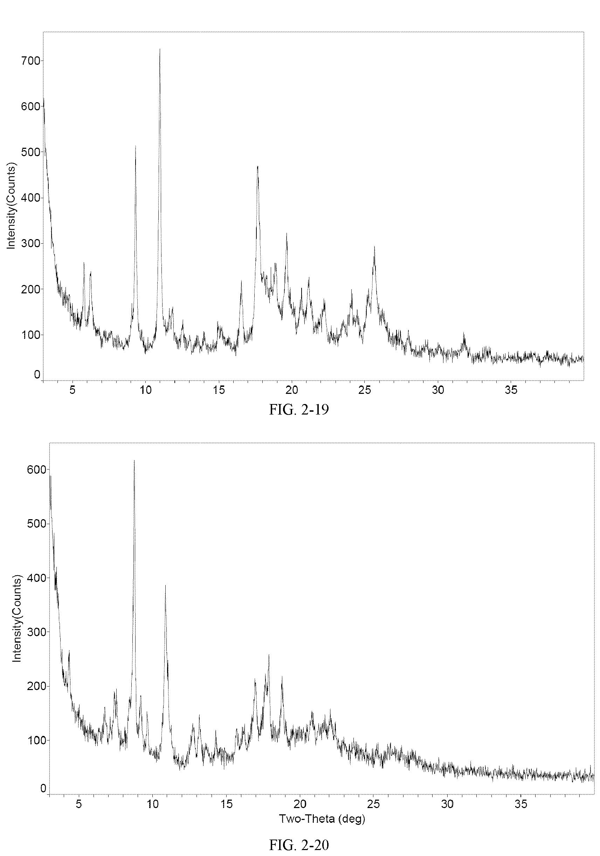

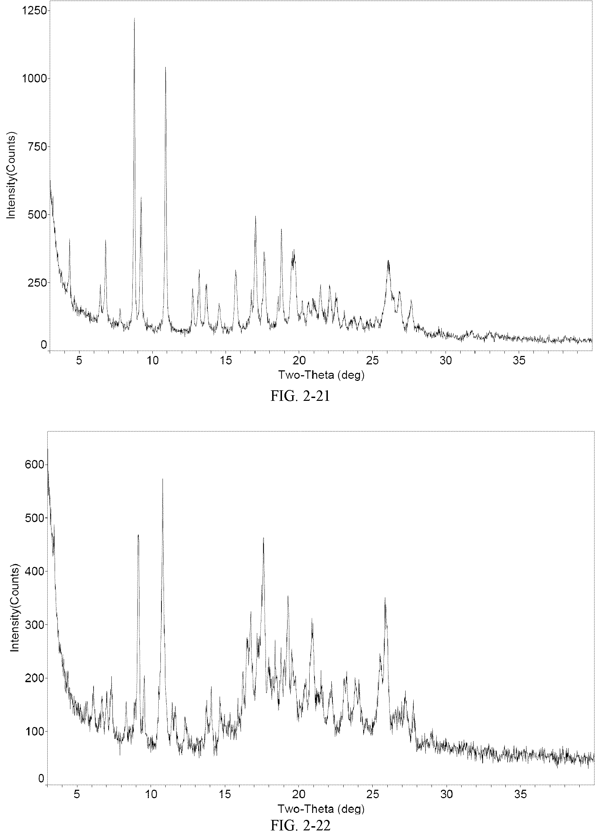

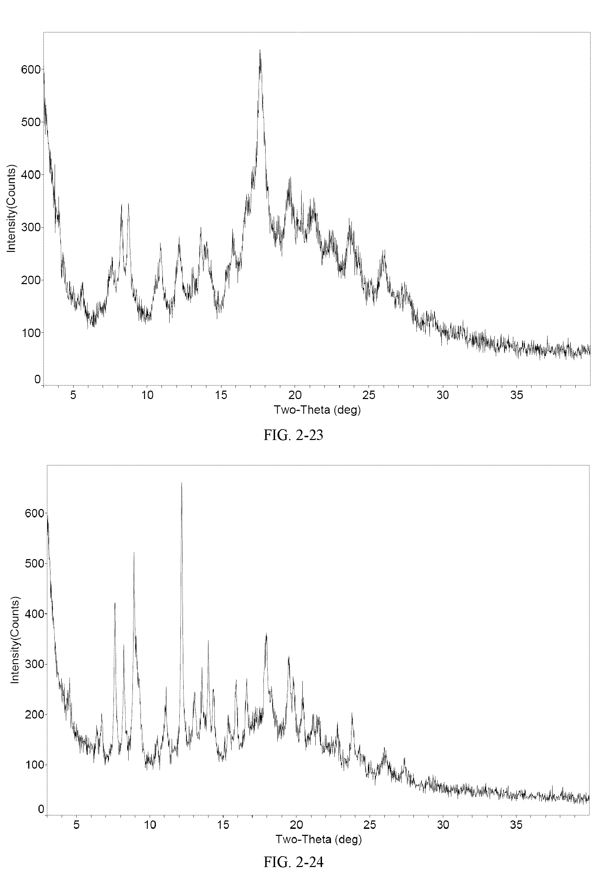

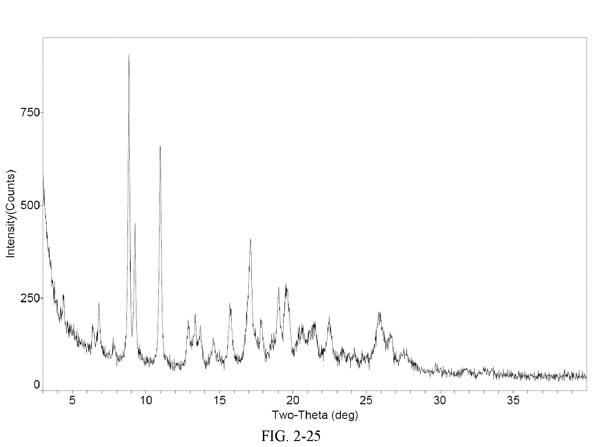

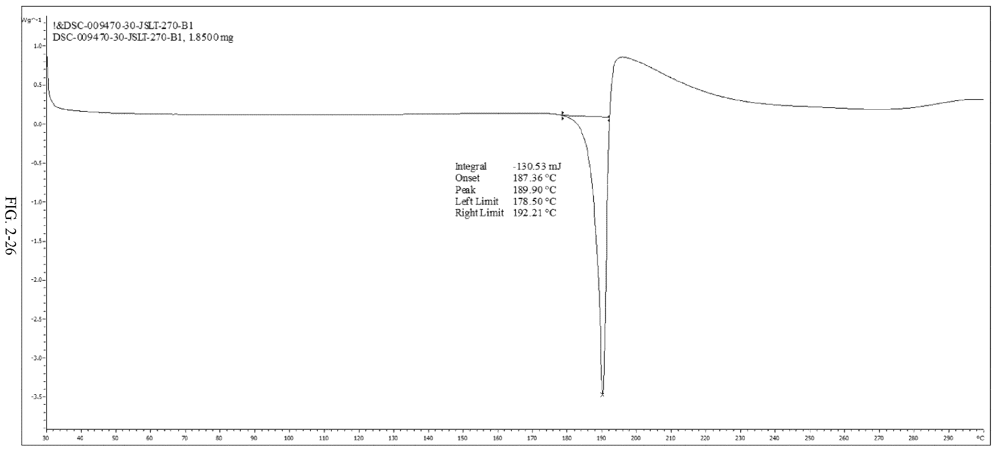

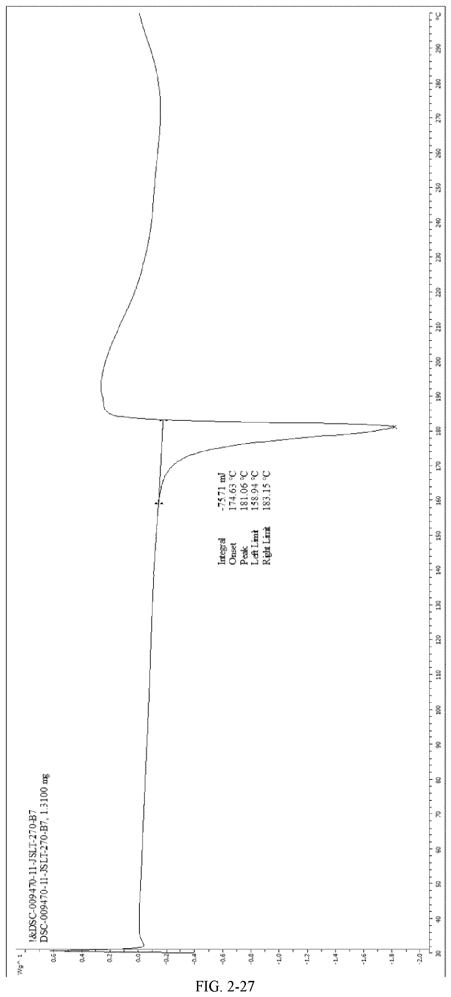

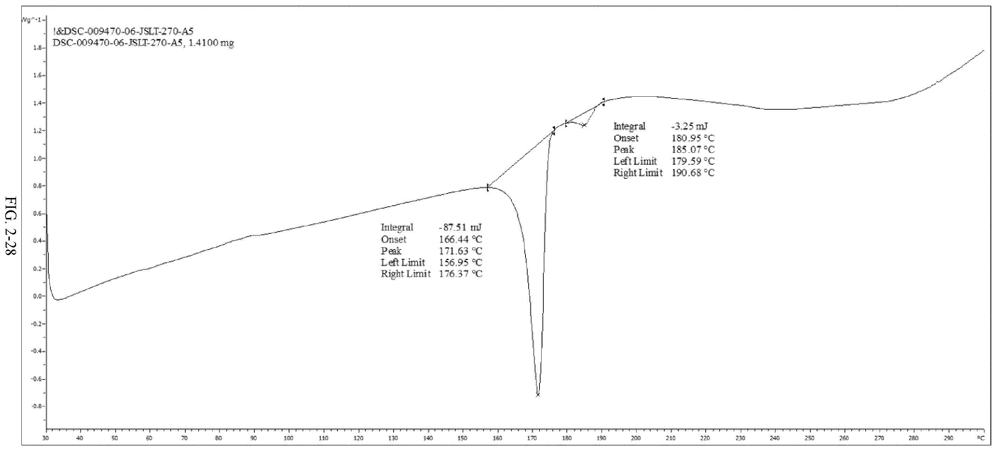

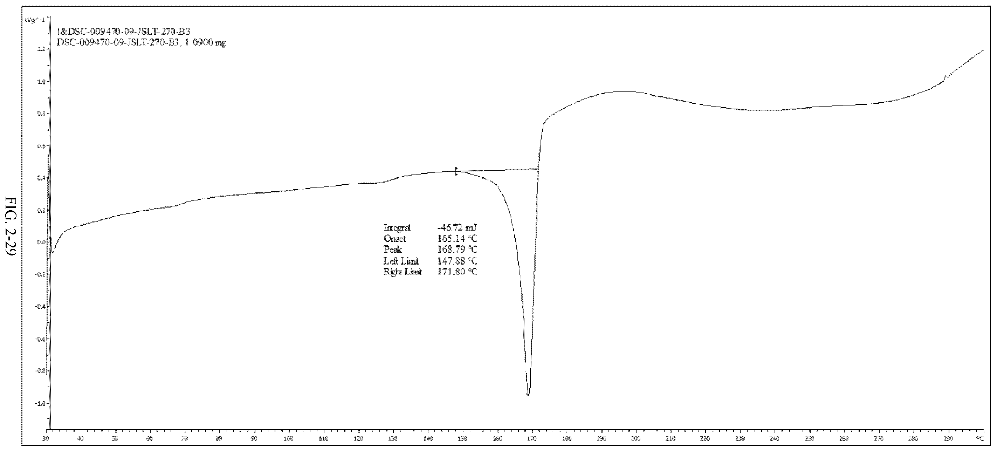

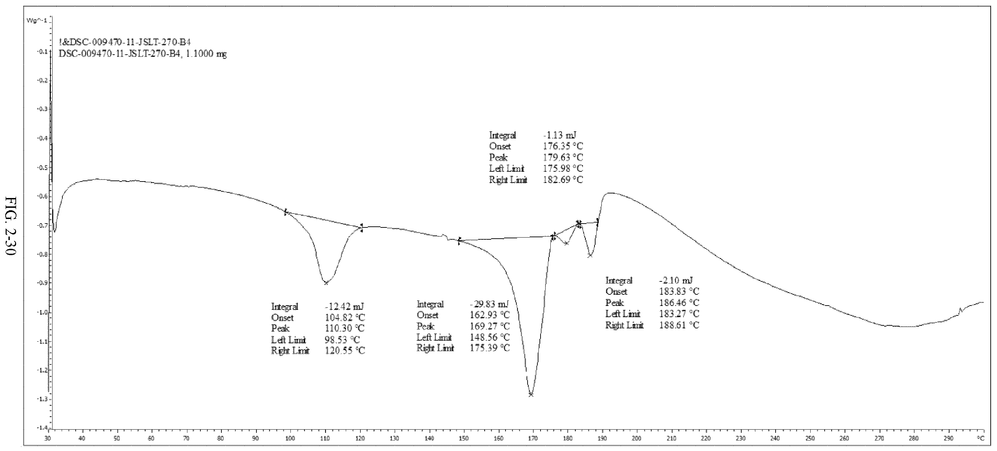

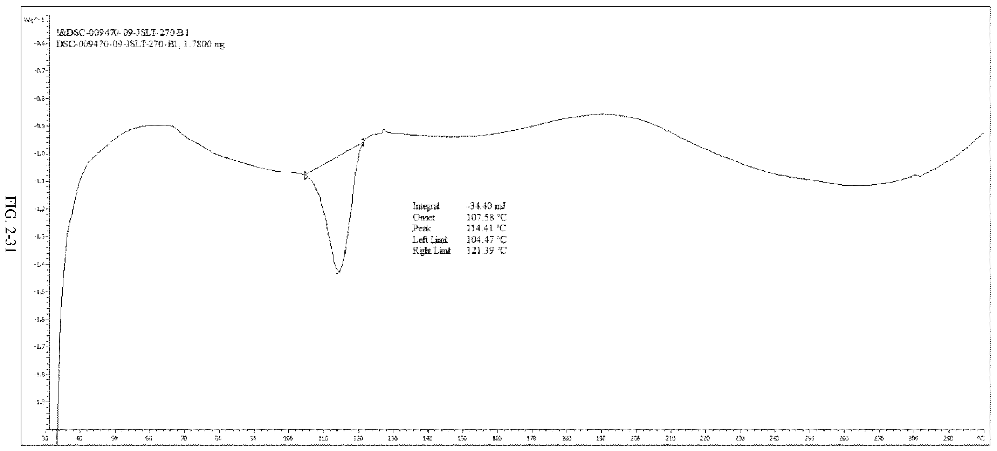

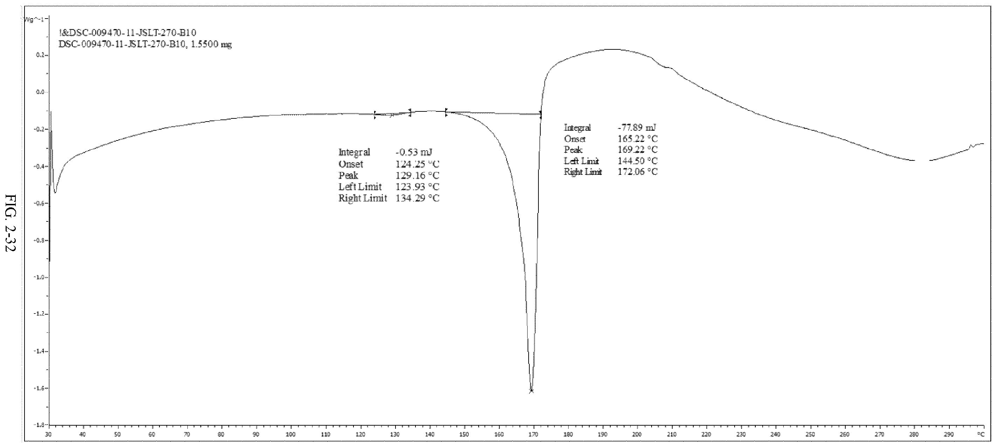

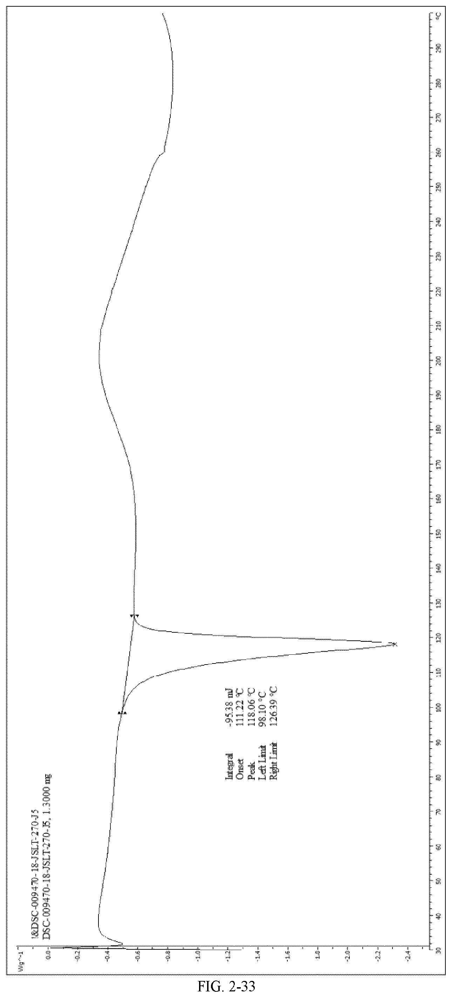

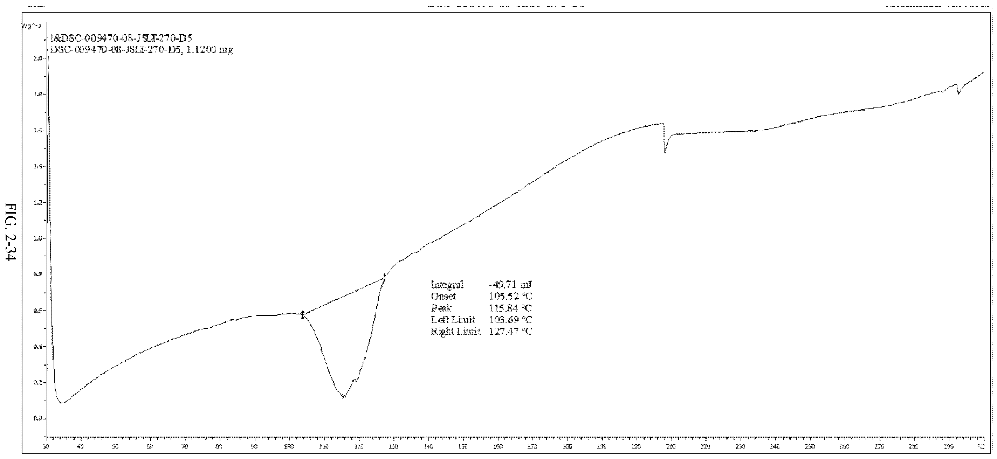

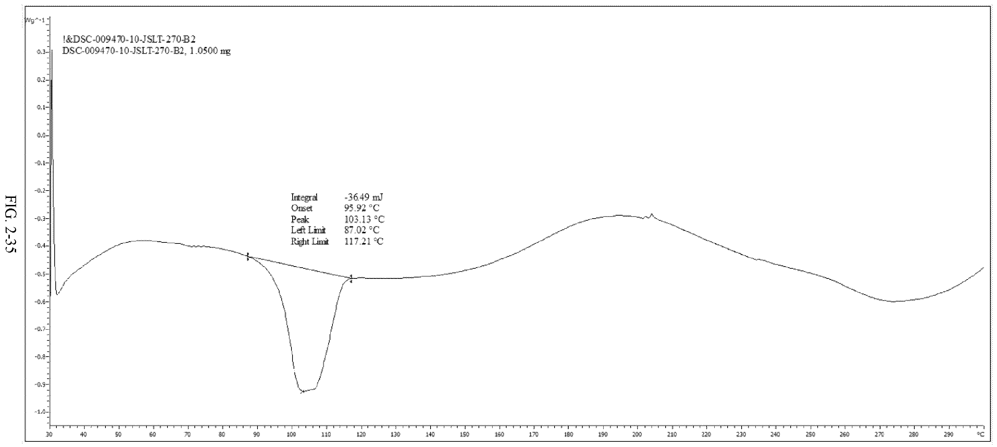

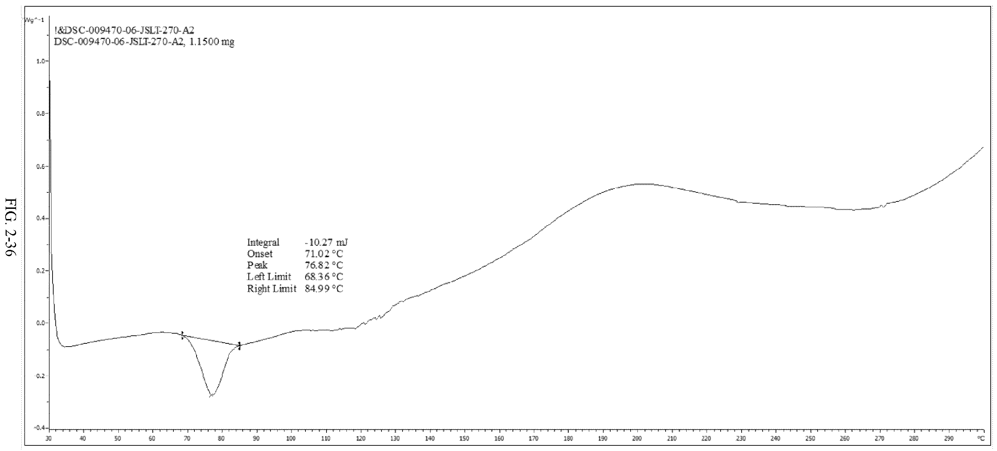

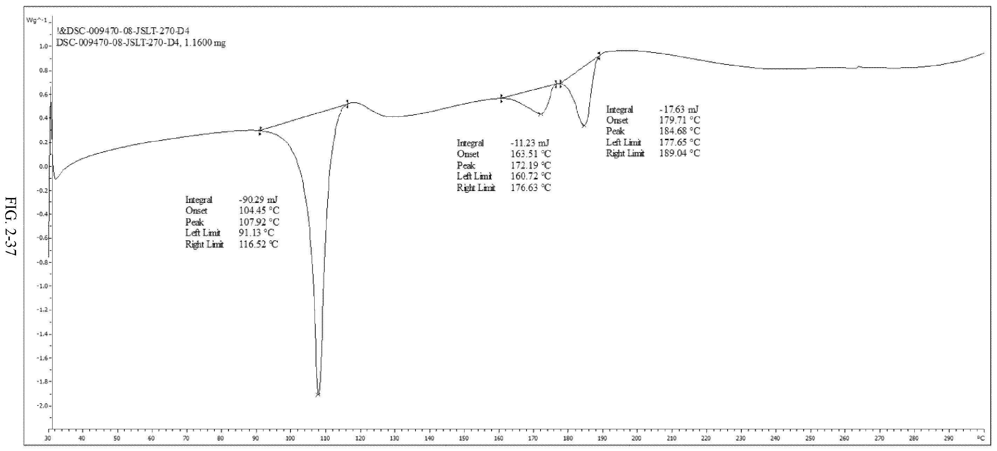

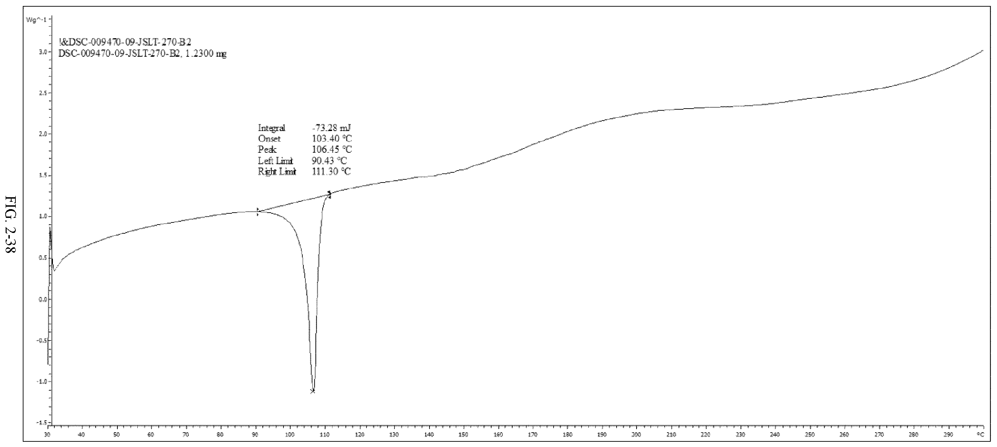

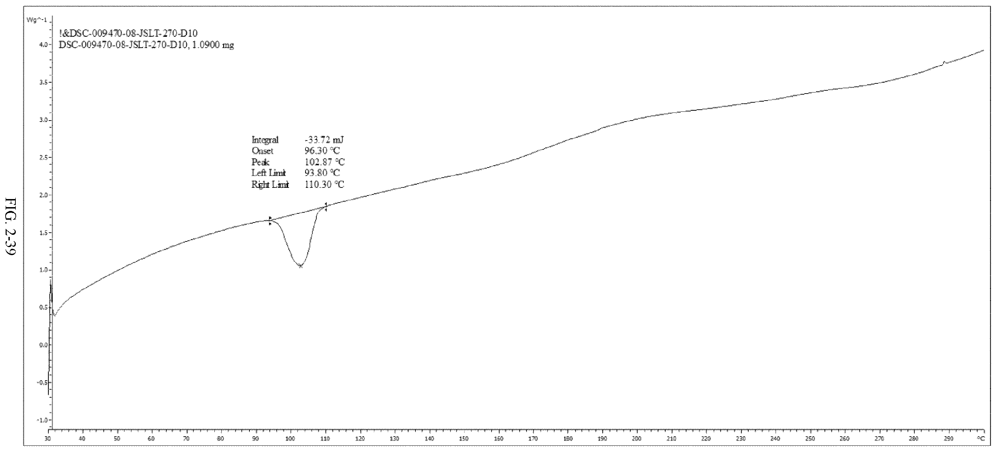

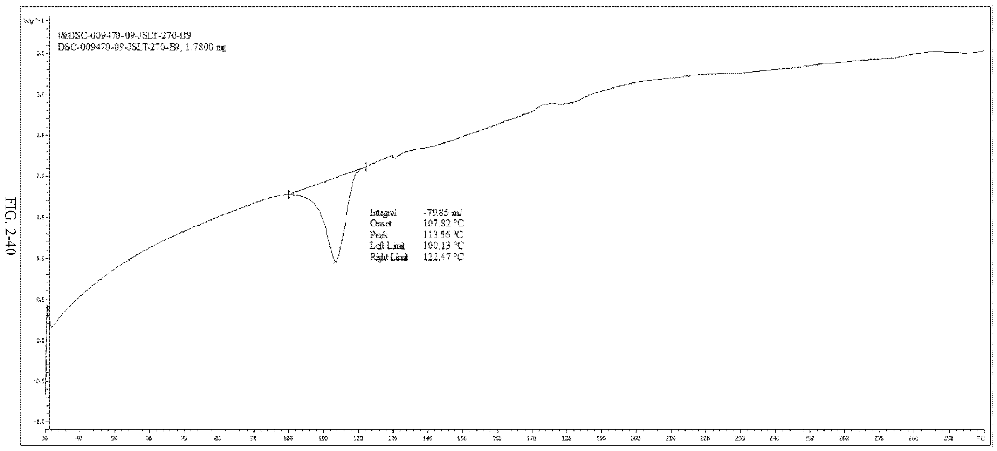

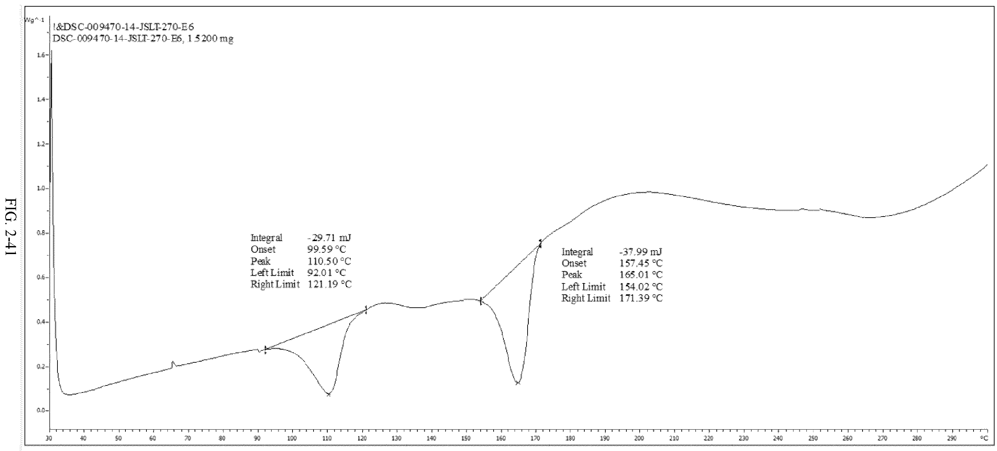

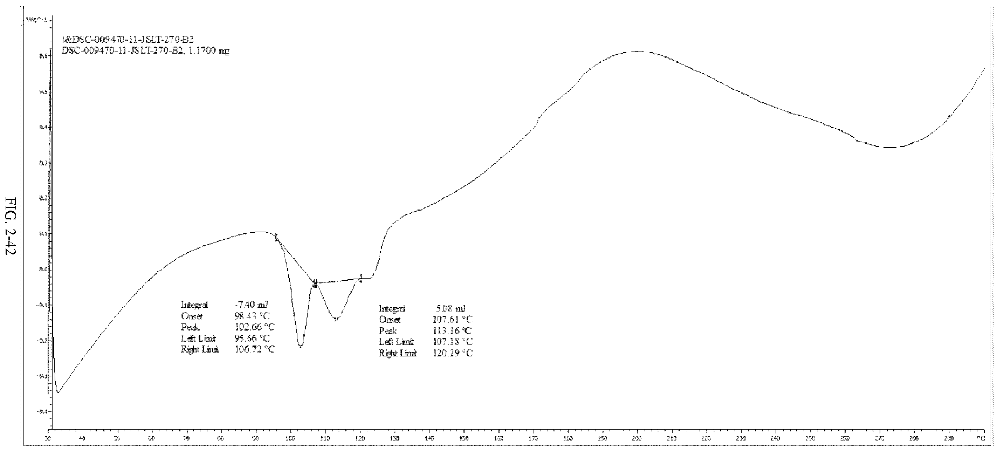

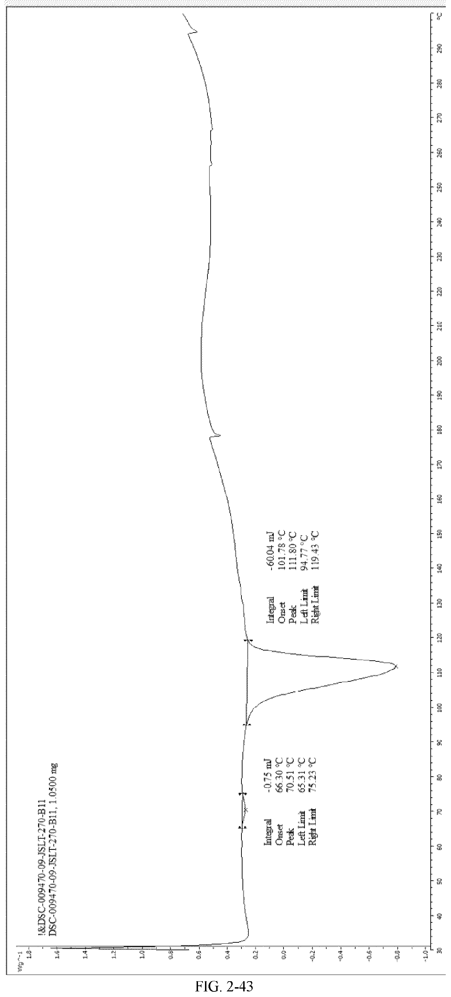

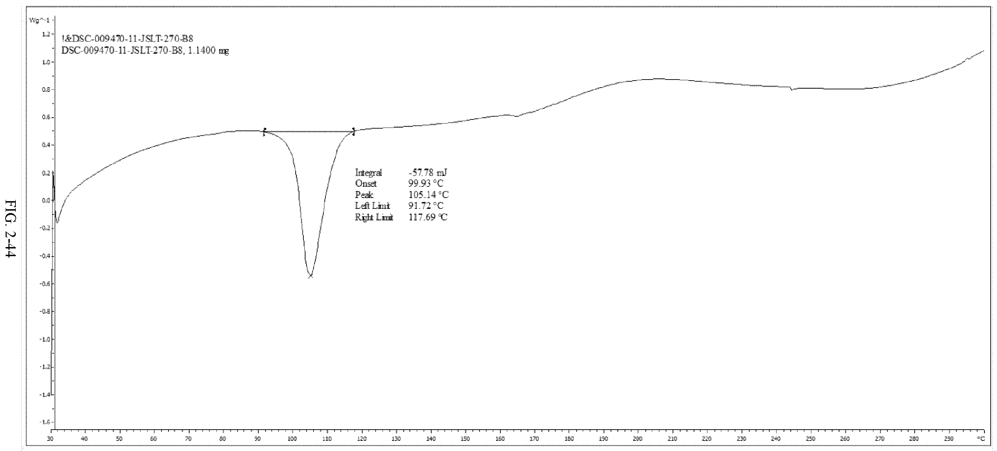

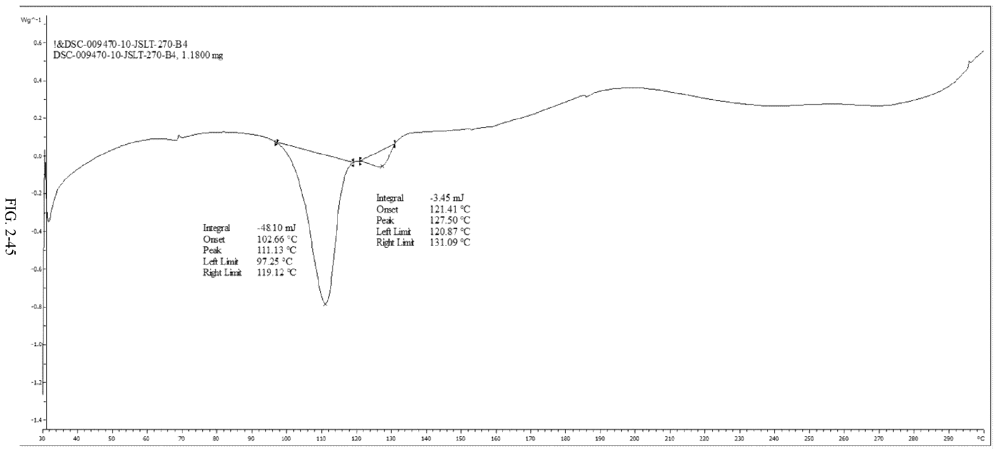

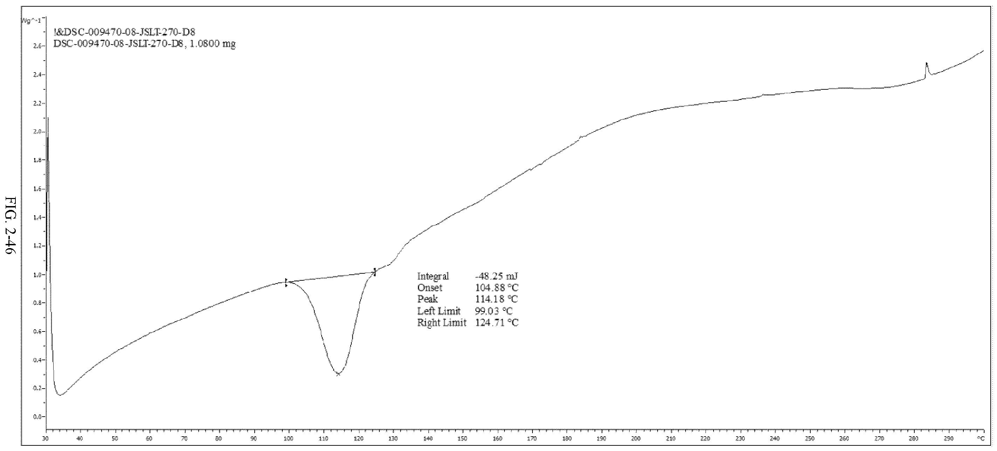

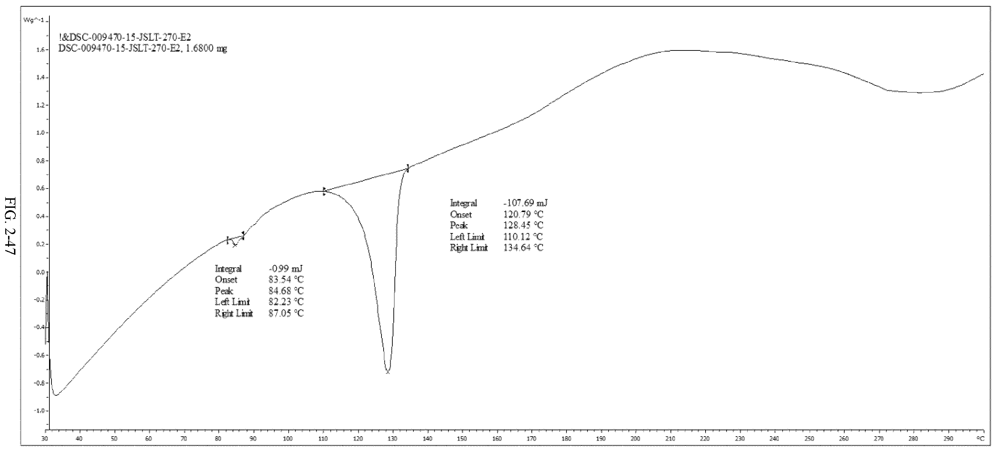

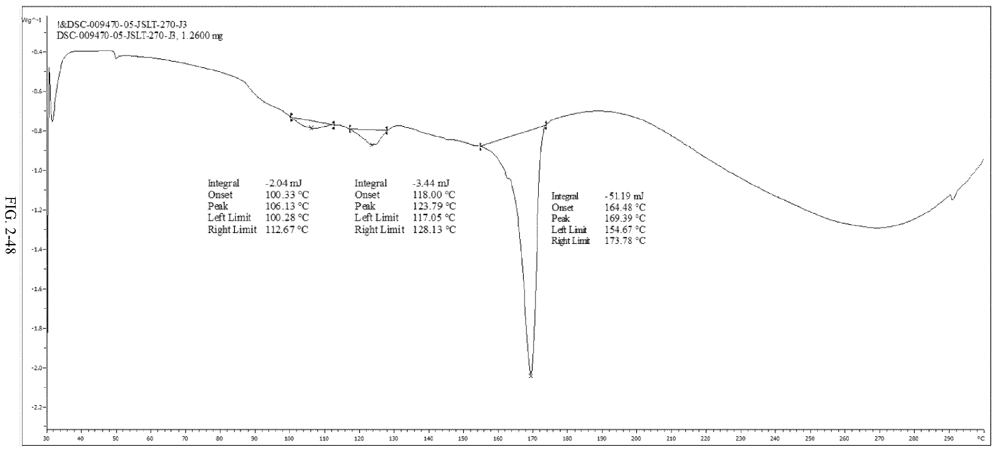

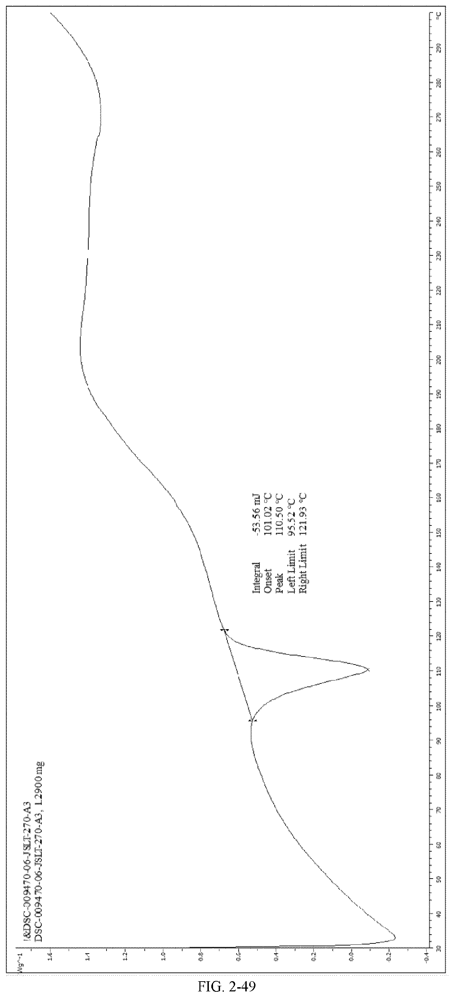

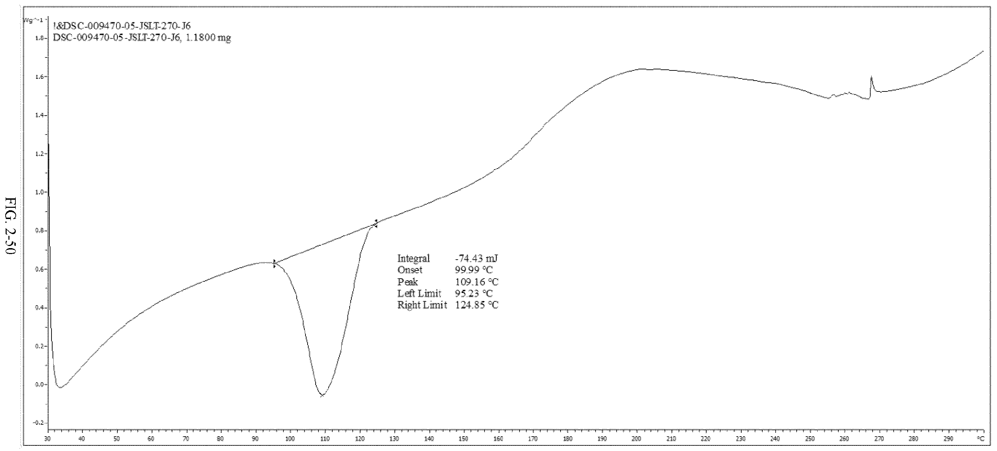

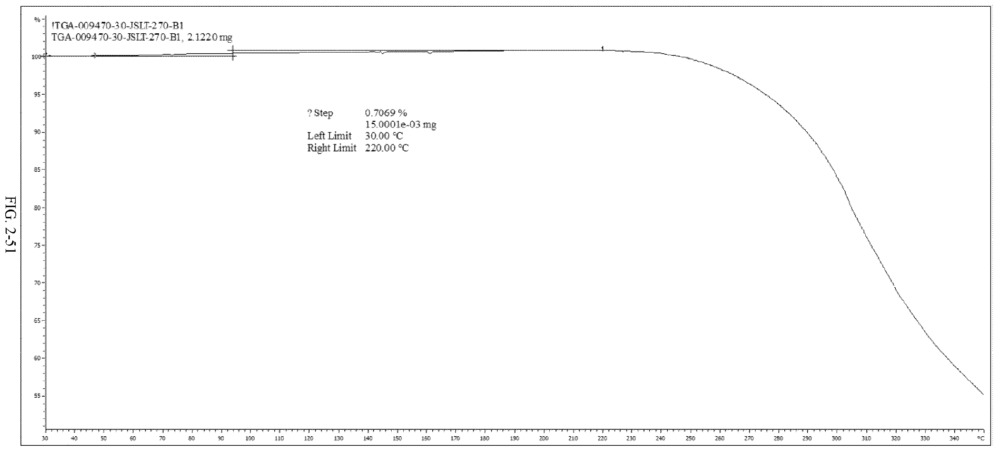

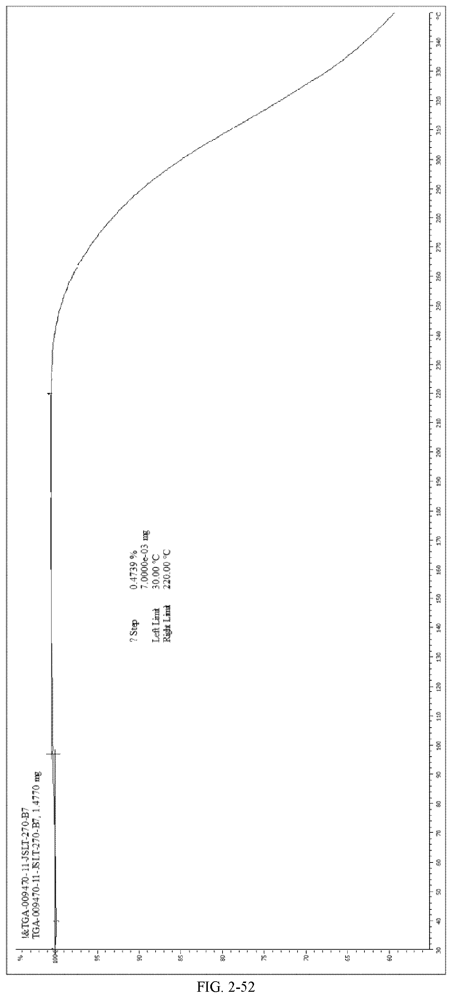

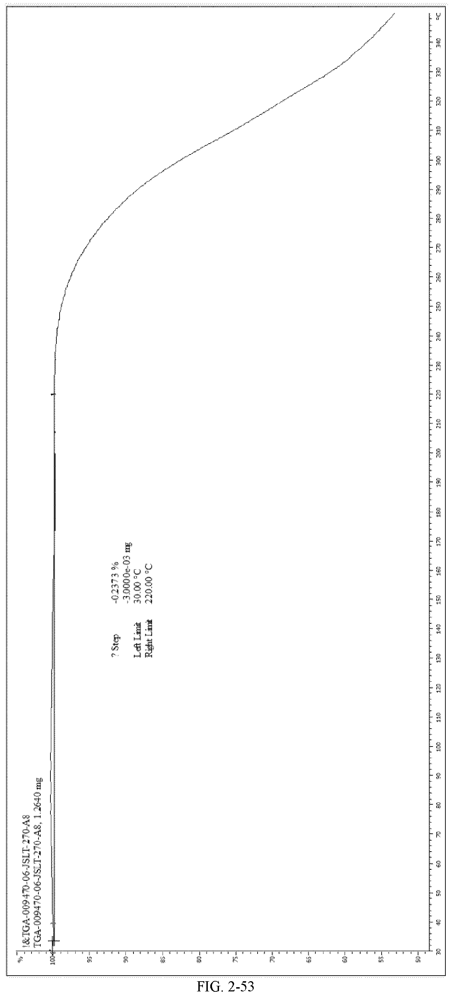

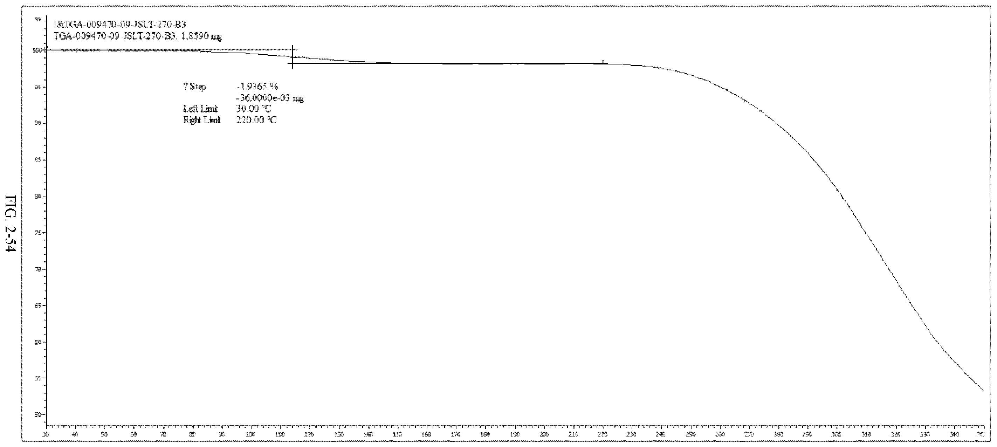

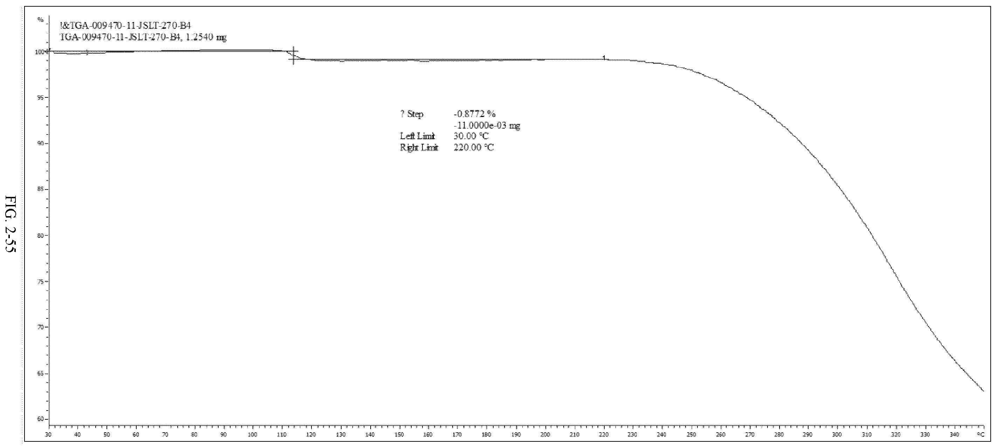

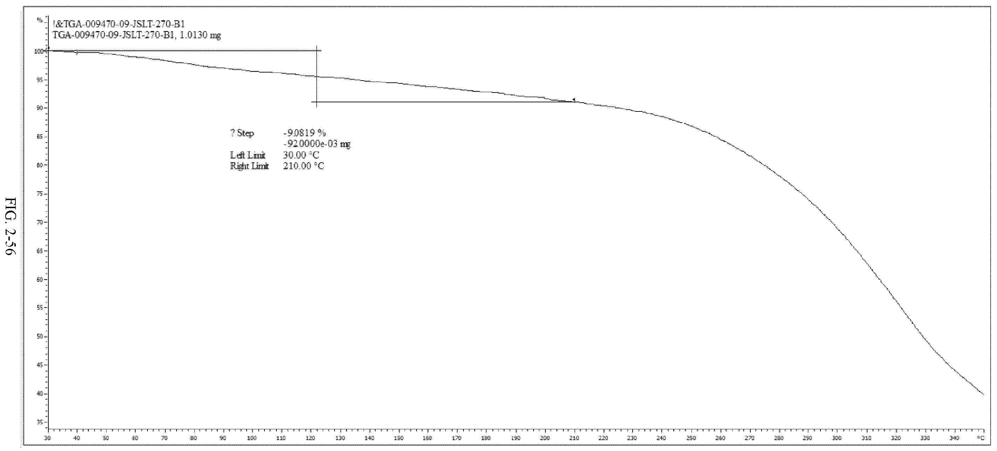

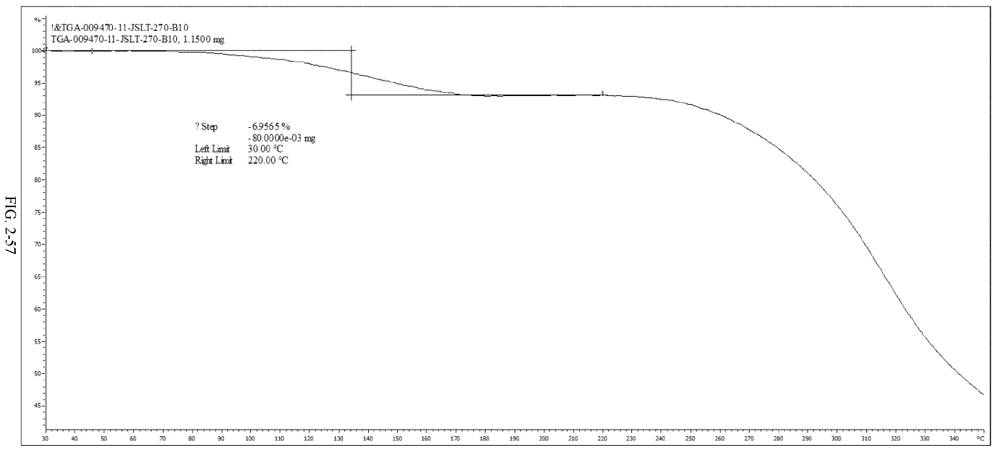

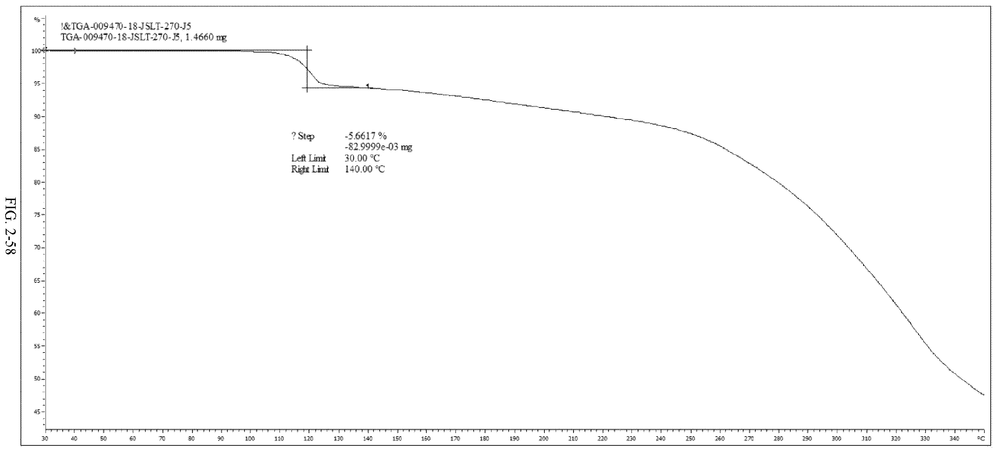

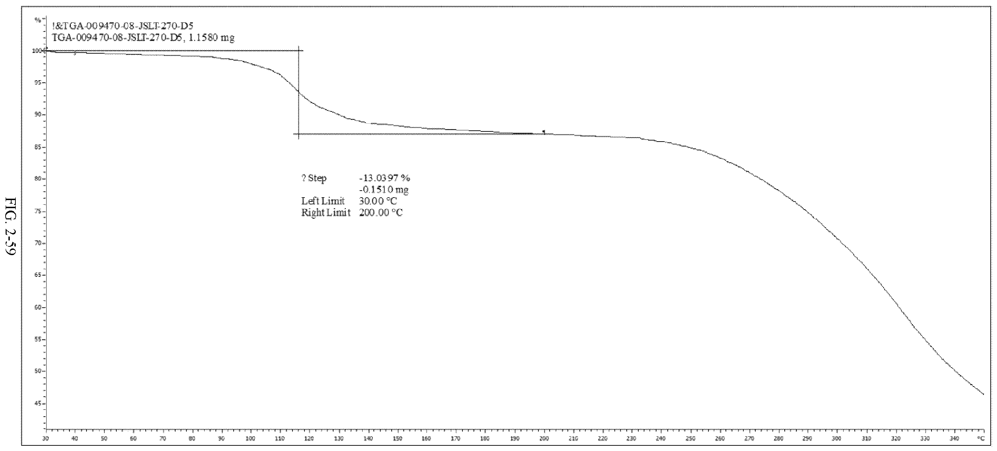

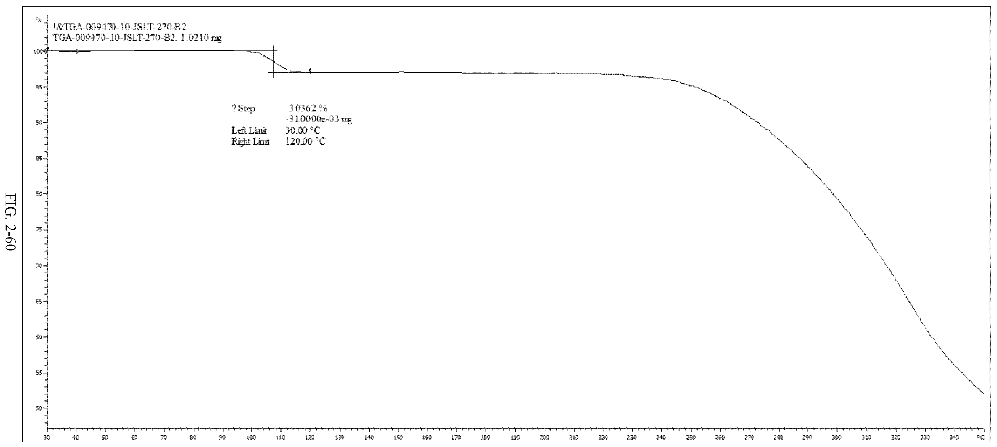

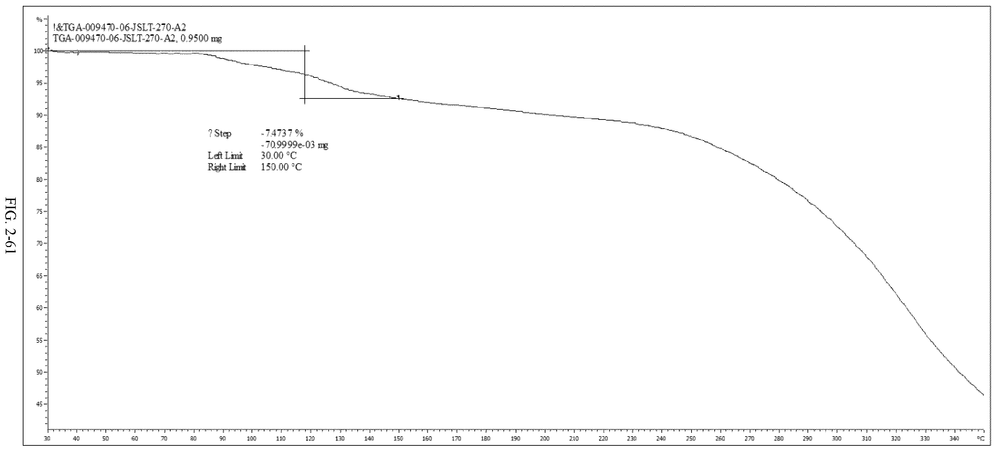

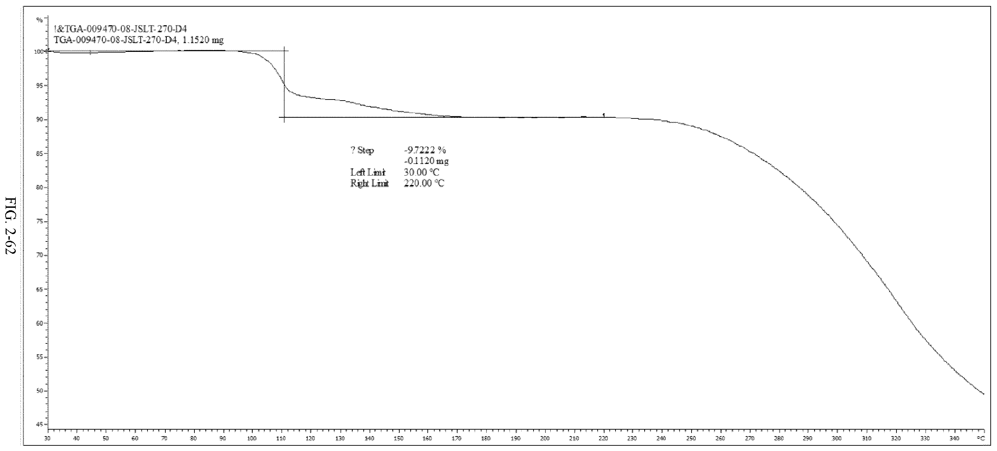

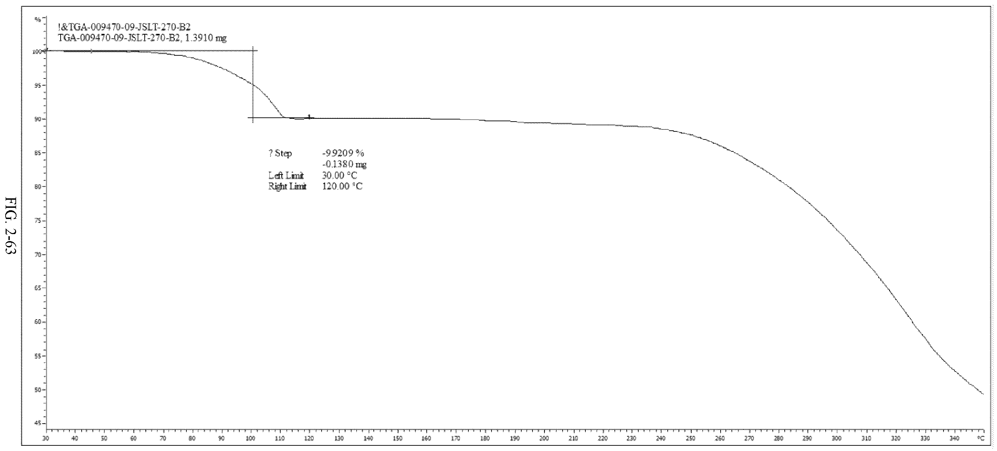

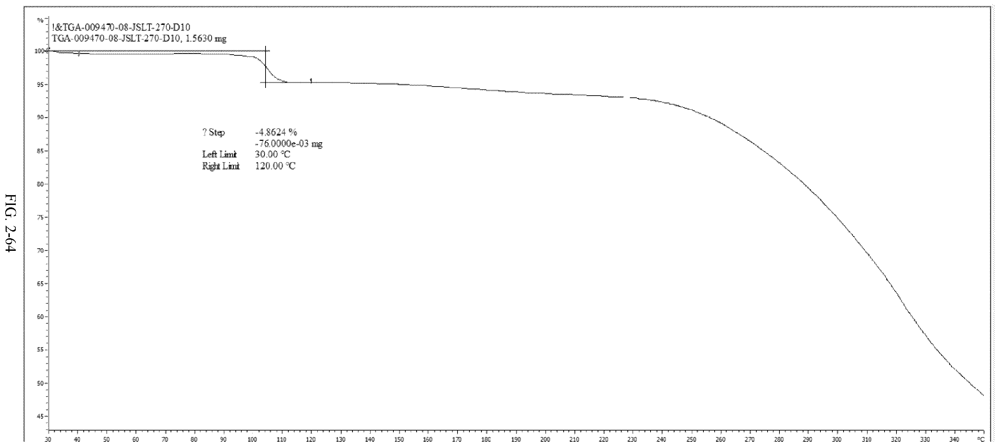

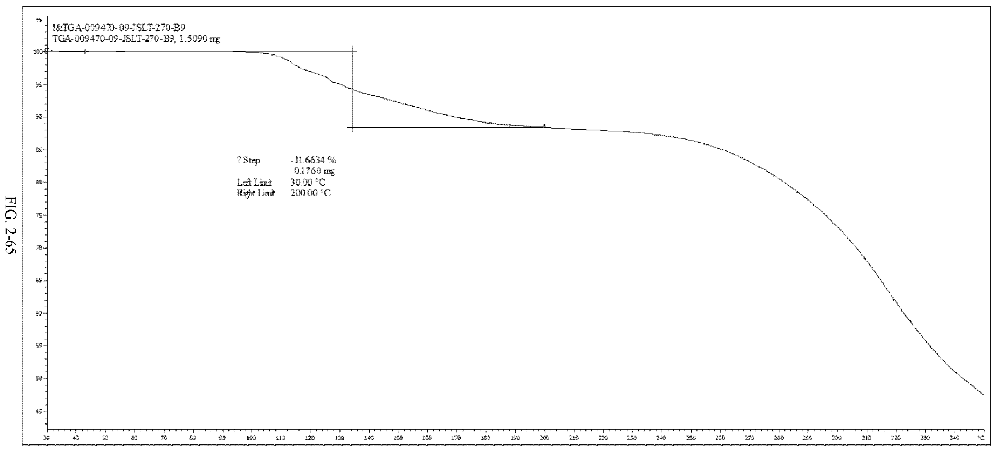

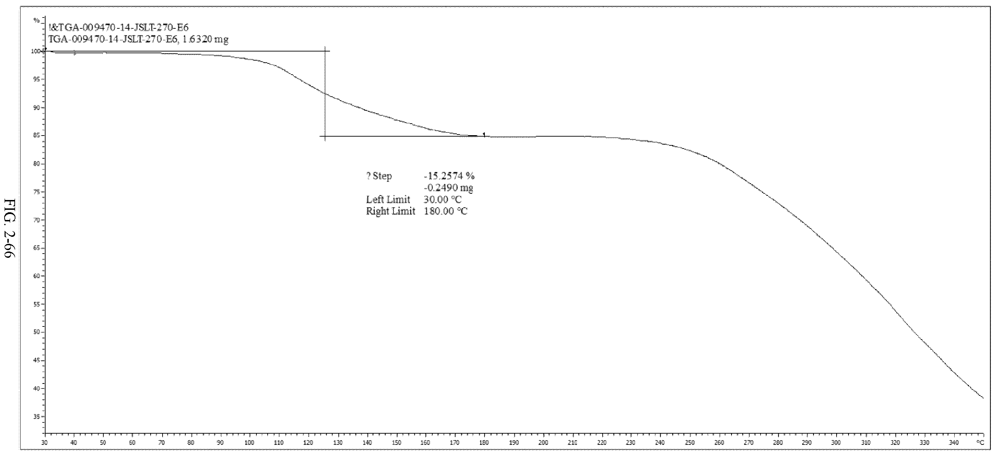

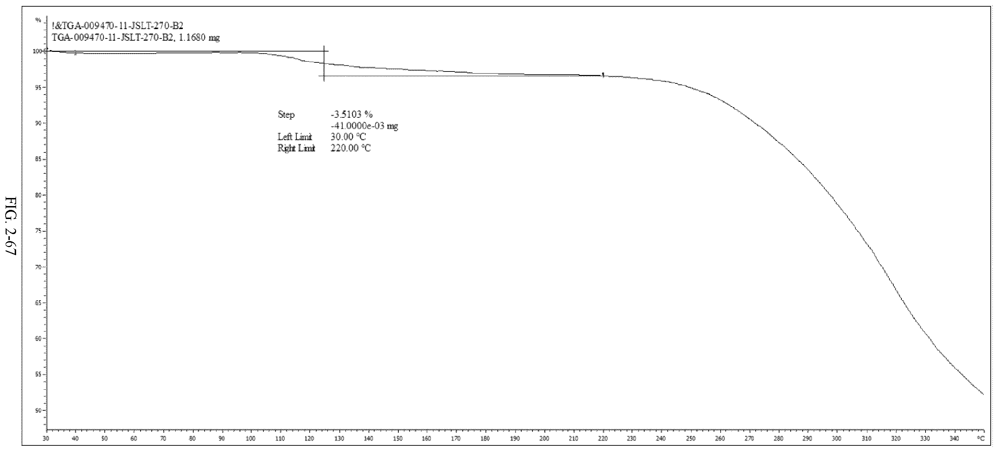

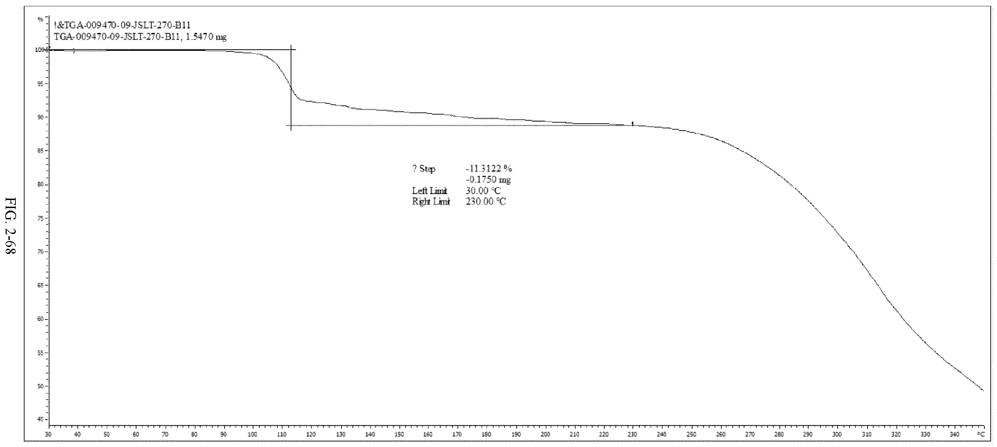

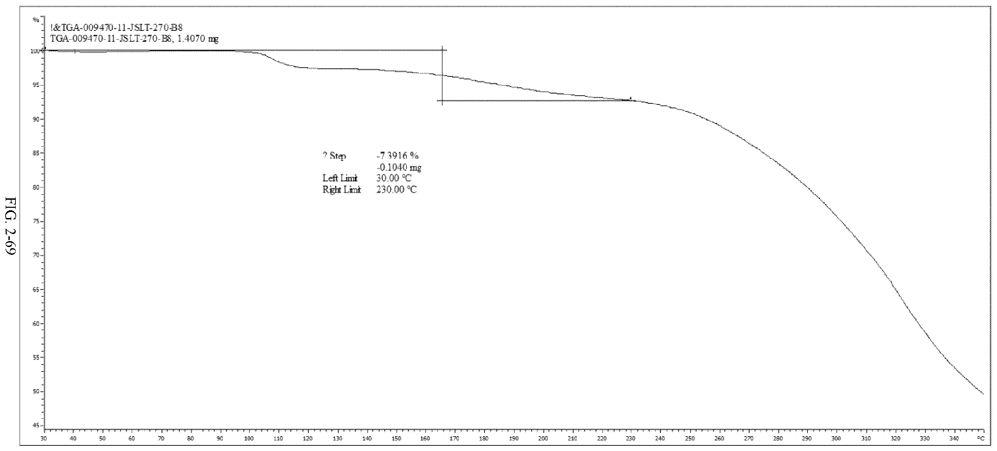

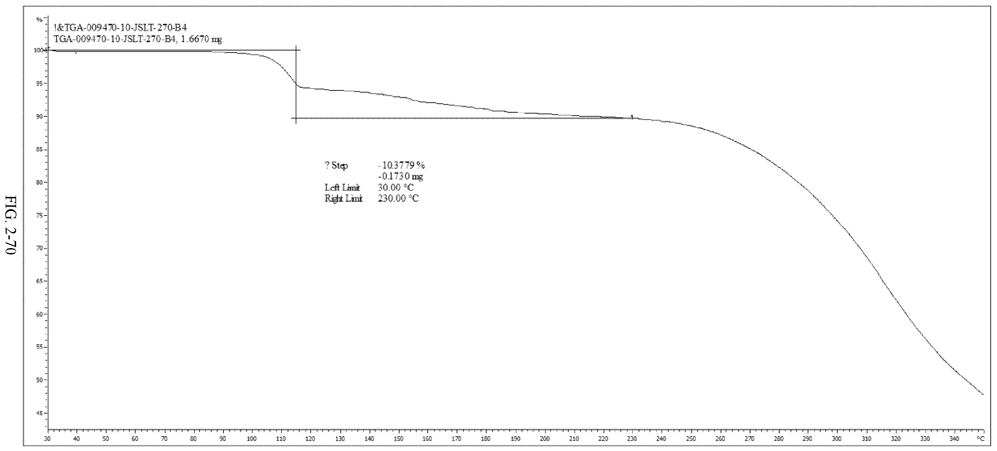

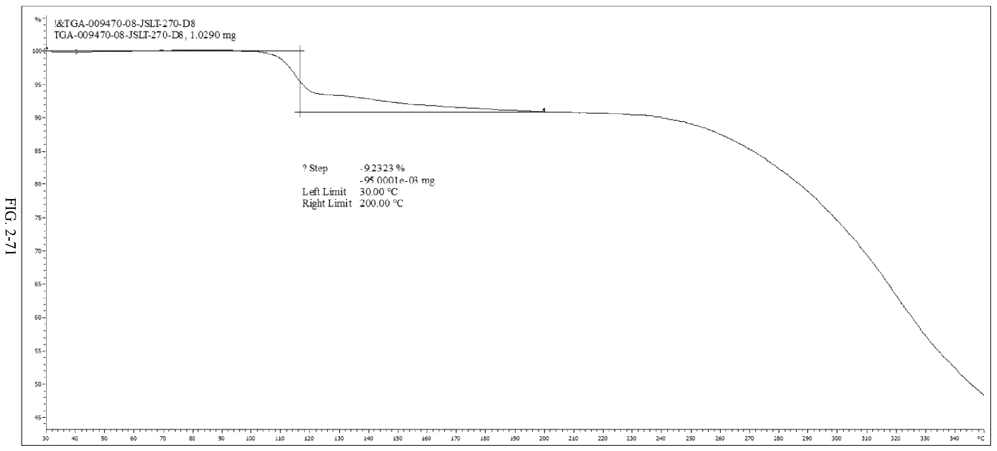

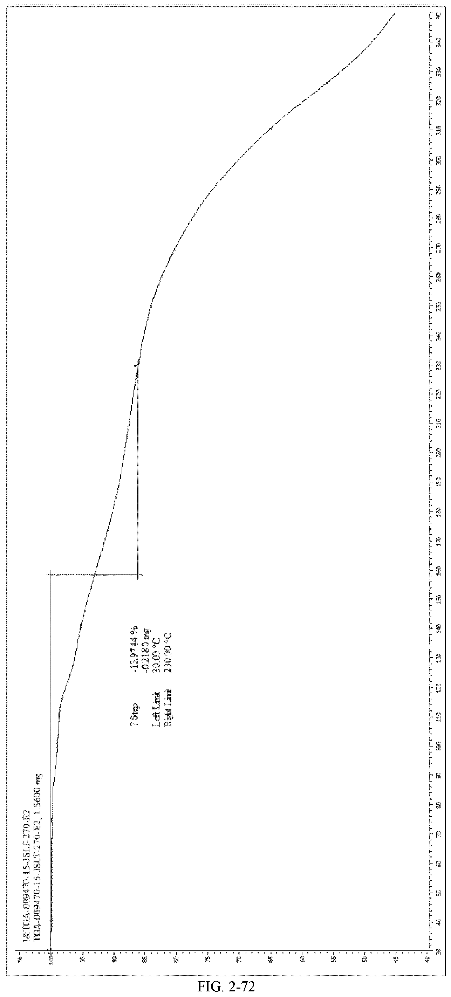

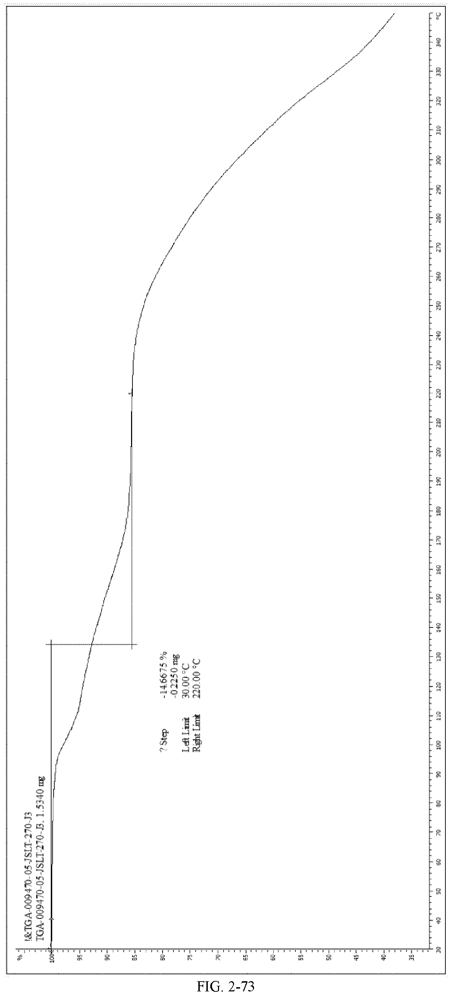

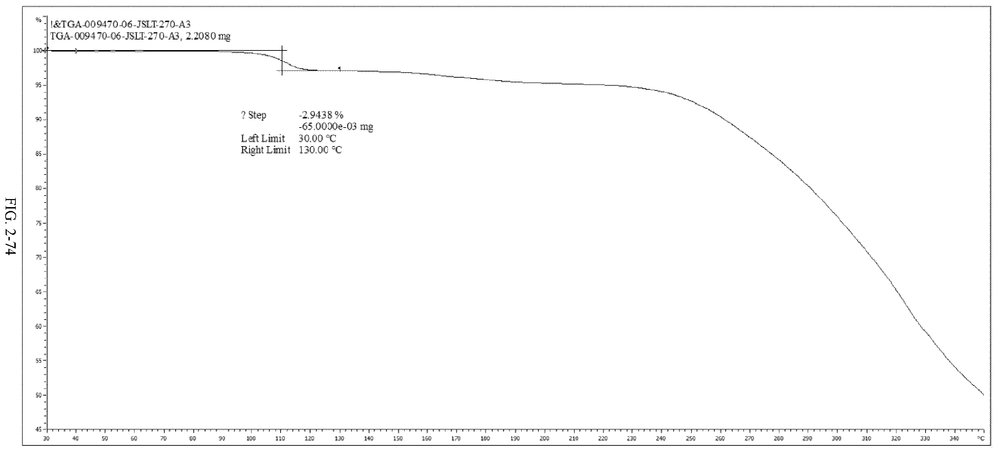

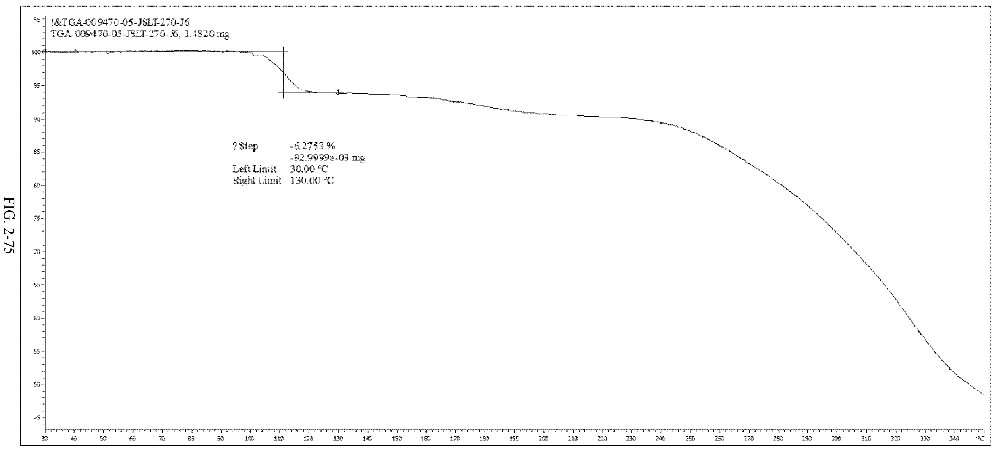

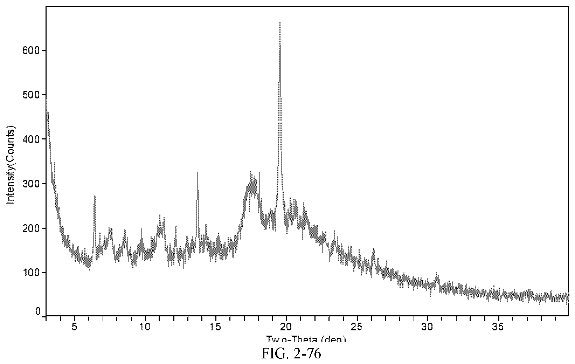

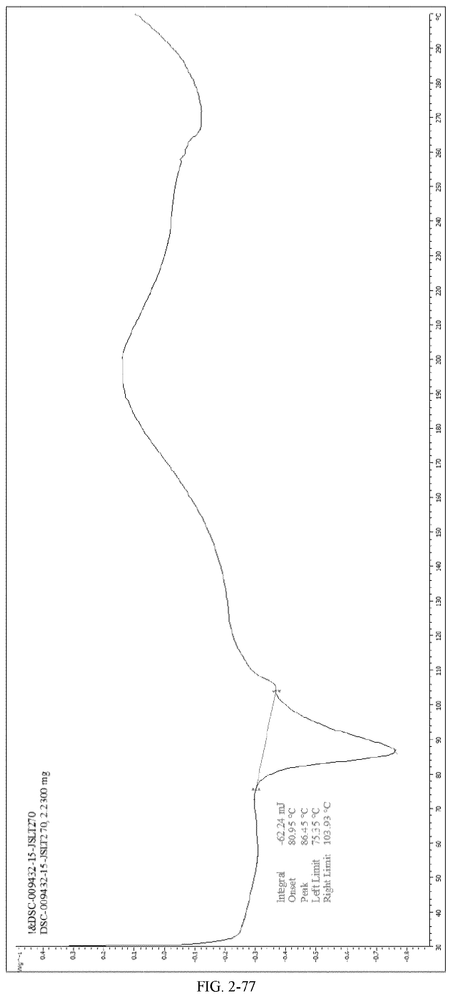

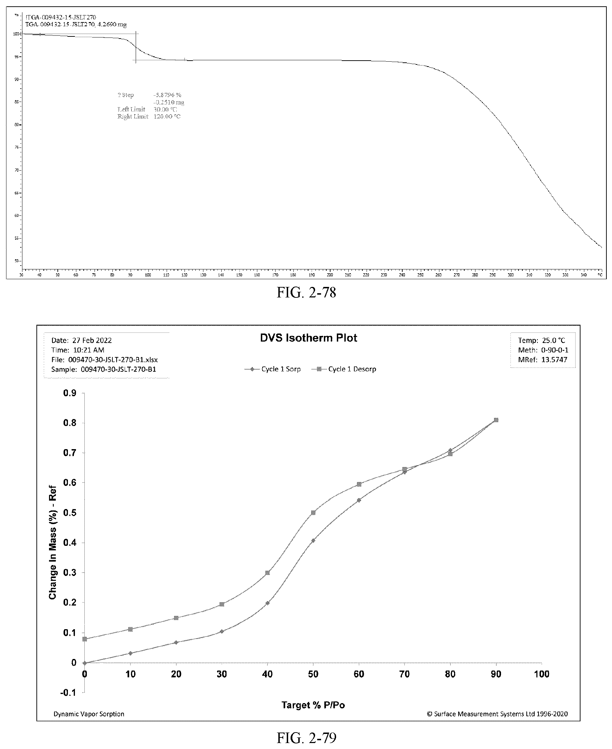

Preferably, crystal form B has an X-ray powder diffraction pattern substantially shown in FIG. 2.

Preferably, crystal form B exhibits an endothermic peak in a DSC analysis when heated to a peak temperature of about 177.71 °C.

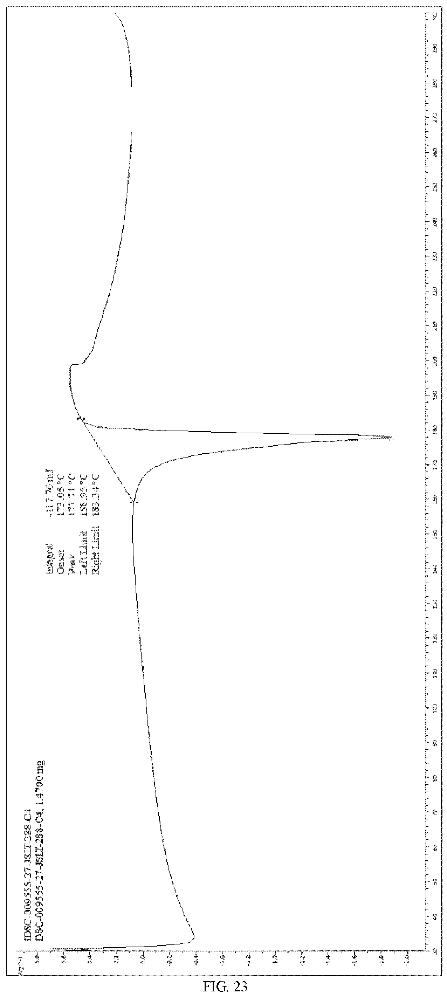

Preferably, crystal form B has a DSC profile substantially shown in FIG. 23.

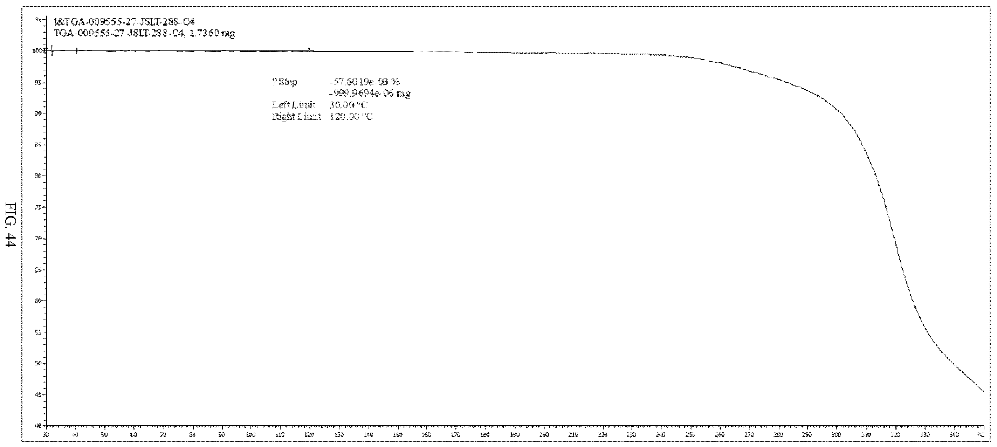

Preferably, crystal form B has a TGA profile substantially shown in FIG. 44.

Crystal form C of compound I provided by the present disclosure has an X-ray powder diffraction pattern comprising peaks at diffraction angles (2θ) of 11.27 ± 0.2°, 19.30 ± 0.2°, 17.92 ± 0.2°, and 20.04 ± 0.2°;
preferably, further comprising peaks at diffraction angles (2θ) of 14.70 ± 0.2°, 22.75 ± 0.2°, 21.21 ± 0.2°, 10.48 ± 0.2°, and 18.83 ± 0.2°;
and more preferably, further comprising peaks at diffraction angles (2θ) of 4.84 ± 0.2°, 10.20 ± 0.2°, 14.16 ± 0.2°, 22.15 ± 0.2°, 20.41 ± 0.2°, and 10.98 ± 0.2°.

Preferably, crystal form C has an X-ray powder diffraction pattern comprising diffraction angles (2θ) shown in Table 3, wherein the 2θ angles have a margin of error of ±0.20°:

**Table 3**

| 2θ (°) | Intensity% | 2θ (°) | Intensity% |
|---|---|---|---|
| 4.84 | 36.3 | 20.41 | 18.0 |
| 9.74 | 5.0 | 20.65 | 15.0 |
| 10.20 | 27.6 | 21.21 | 46.9 |
| 10.48 | 46.1 | 22.15 | 23.2 |
| 10.98 | 16.3 | 22.75 | 48.7 |
| 11.27 | 100.0 | 24.41 | 12.2 |
| 12.03 | 6.7 | 25.33 | 8.5 |
| 12.54 | 7.8 | 26.26 | 8.9 |
| 14.16 | 23.4 | 26.88 | 14.3 |
| 14.70 | 53.2 | 28.22 | 12.5 |
| 16.45 | 12.6 | 29.01 | 8.5 |
| 17.27 | 6.1 | 30.74 | 6.5 |
| 17.92 | 63.1 | 31.40 | 7.6 |
| 18.83 | 42.1 | 34.65 | 4.0 |
| 19.30 | 74.2 | 36.35 | 4.8 |
| 20.04 | 56.3 | | |

Preferably, crystal form C has X-ray powder diffraction intensities shown in Table 3.

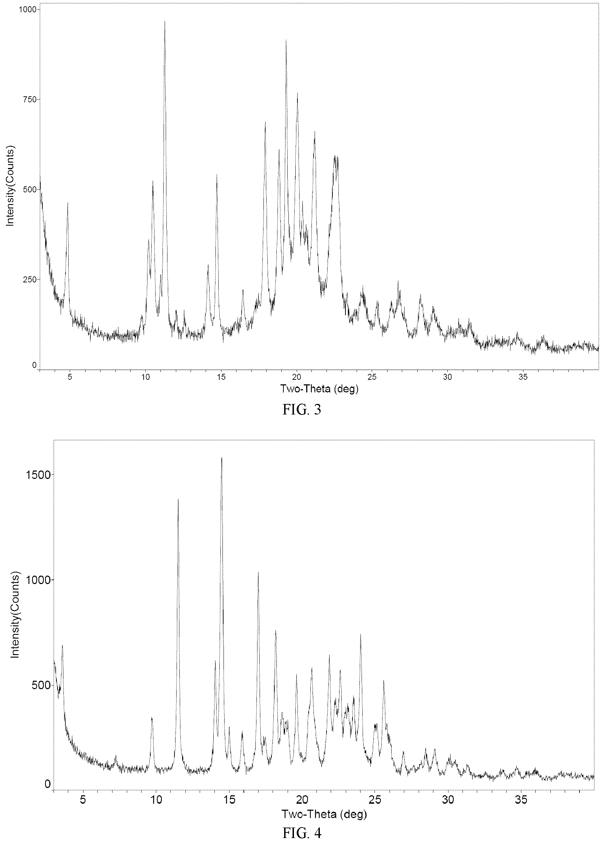

Preferably, crystal form C has an X-ray powder diffraction pattern substantially shown in FIG. 3.

Preferably, crystal form C exhibits an endothermic peak in a DSC analysis when heated to a peak temperature of about 104.51 °C.

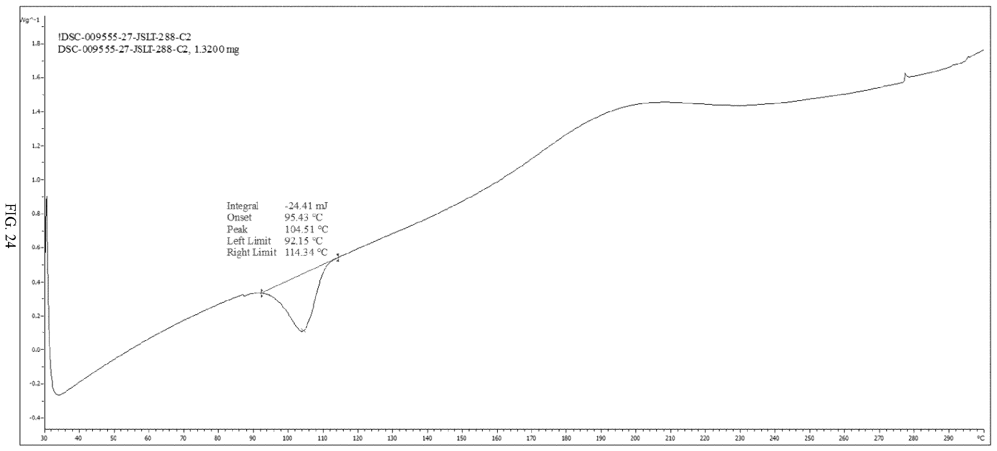

Preferably, crystal form C has a DSC profile substantially shown in FIG. 24.

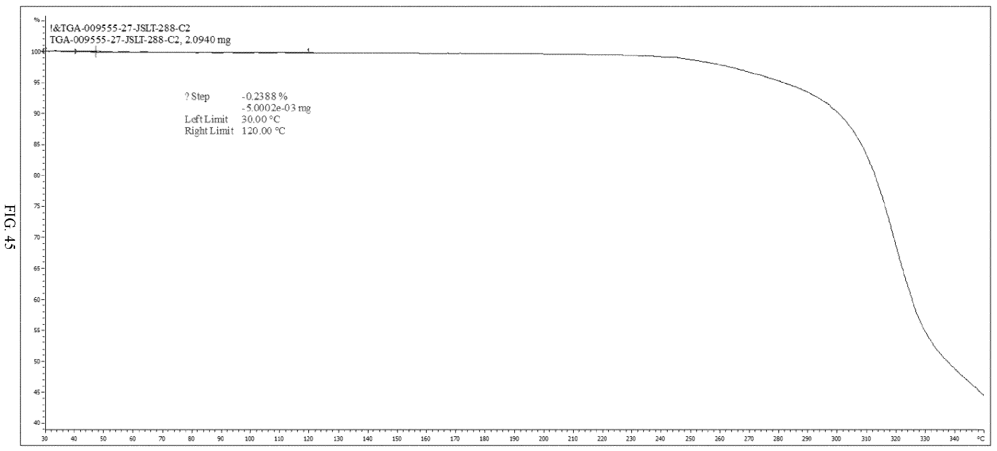

Preferably, crystal form C has a TGA profile substantially shown in FIG. 45.

Crystal form D of compound I provided by the present disclosure has an X-ray powder diffraction pattern comprising peaks at diffraction angles (2θ) of 14.49 ± 0.2°, 16.98 ± 0.2°, 11.51 ± 0.2°, and 18.17 ± 0.2°;
preferably, further comprising peaks at diffraction angles (2θ) of 24.02 ± 0.2°, 21.87 ± 0.2°, 3.58 ± 0.2°, 14.04 ± 0.2°, and 20.68 ± 0.2°;
and more preferably, further comprising peaks at diffraction angles (2θ) of 19.59 ± 0.2°, 25.60 ± 0.2°, 22.30 ± 0.2°, 22.61 ± 0.2°, 23.53 ± 0.2°, and 9.70 ± 0.2°.

Preferably, crystal form D has an X-ray powder diffraction pattern comprising diffraction angles (2θ) shown in Table 4, wherein the 2θ angles have a margin of error of ±0.20°:

**Table 4**

| 2θ (°) | Intensity% | 2θ (°) | Intensity% |
|---|---|---|---|
| 3.58 | 33.8 | 22.30 | 20.8 |
| 6.69 | 2.5 | 22.61 | 20.5 |
| 7.24 | 4.3 | 23.16 | 8.2 |
| 9.70 | 17.4 | 23.53 | 20.4 |
| 11.51 | 86.8 | 24.02 | 34.8 |
| 14.04 | 32.8 | 25.11 | 10.2 |
| 14.49 | 100.0 | 25.60 | 27.5 |
| 15.00 | 10.5 | 26.00 | 8.2 |
| 15.91 | 12.1 | 26.92 | 6.4 |
| 16.98 | 61.5 | 28.17 | 3.1 |
| 17.47 | 8.3 | 28.47 | 7.5 |
| 18.17 | 41.4 | 29.12 | 7.2 |
| 18.62 | 15.6 | 30.15 | 6.4 |
| 19.01 | 13.0 | 30.50 | 4.6 |
| 19.59 | 27.7 | 31.36 | 3.4 |
| 20.45 | 16.0 | 33.81 | 2.4 |
| 20.68 | 29.9 | 34.69 | 3.5 |
| 21.87 | 34.4 | | |

Preferably, crystal form D has X-ray powder diffraction intensities shown in Table 4.

Preferably, crystal form D has an X-ray powder diffraction pattern substantially shown in FIG. 4.

Preferably, crystal form D exhibits an endothermic peak in a DSC analysis when heated to a peak temperature of about 167.48 °C.

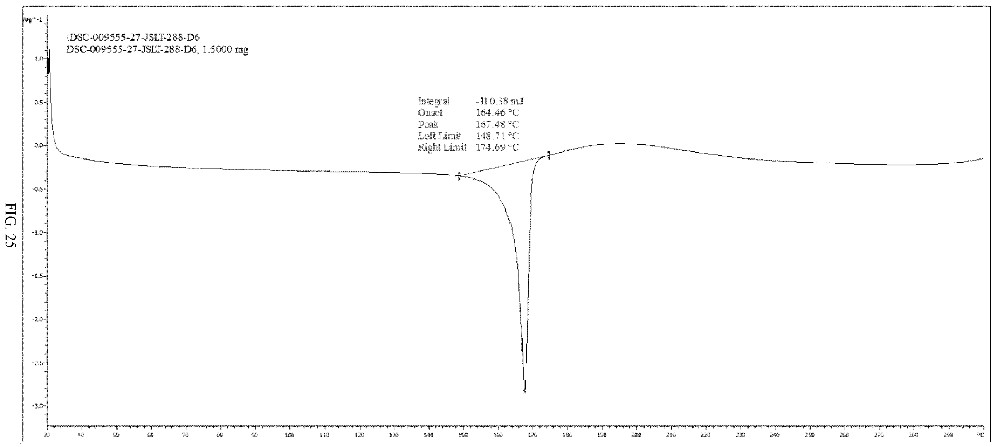

Preferably, crystal form D has a DSC profile substantially shown in FIG. 25.

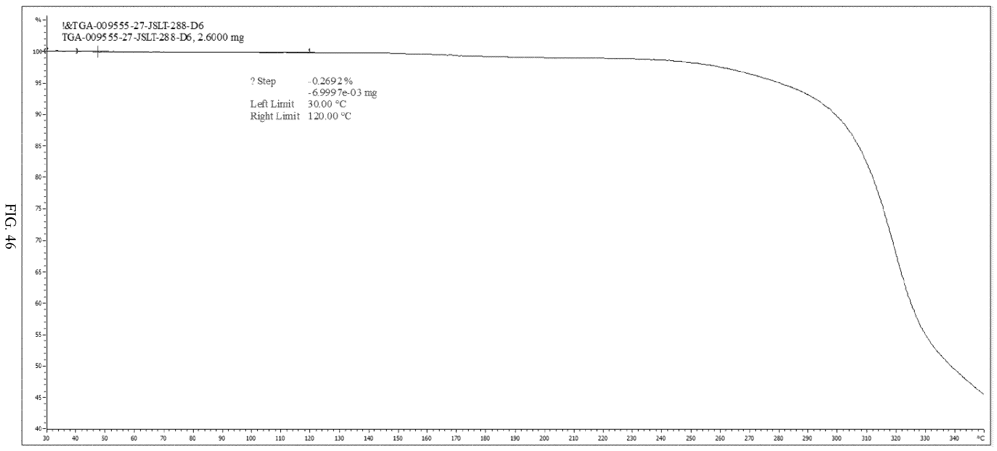

Preferably, crystal form D has a TGA profile substantially shown in FIG. 46.

Crystal form E of compound I provided by the present disclosure has an X-ray powder diffraction pattern comprising peaks at diffraction angles (2θ) of 11.49 ± 0.2°, 12.17 ± 0.2°, 21.15 ± 0.2°, and 20.16 ± 0.2°;
preferably, further comprising peaks at diffraction angles (2θ) of 14.35 ± 0.2°, 26.49 ± 0.2°, 19.40 ± 0.2°, 4.32 ± 0.2°, and 17.51 ± 0.2°;
and more preferably, further comprising peaks at diffraction angles (2θ) of 10.87 ± 0.2°, 25.13 ± 0.2°, 24.68 ± 0.2°, 18.01 ± 0.2°, 16.83 ± 0.2°, and 15.19 ± 0.2°.

Preferably, crystal form E has an X-ray powder diffraction pattern comprising diffraction angles (2θ) shown in Table 5, wherein the 2θ angles have a margin of error of ±0.20°:

**Table 5**

| 2θ (°) | Intensity% | 2θ (°) | Intensity% |
|---|---|---|---|
| 4.32 | 32.3 | 21.87 | 7.1 |
| 10.87 | 26.9 | 22.95 | 4.7 |
| 11.49 | 100.0 | 23.28 | 7.9 |
| 12.17 | 92.0 | 23.92 | 7.6 |
| 13.14 | 15.6 | 24.24 | 5.8 |
| 13.61 | 6.2 | 24.68 | 20.8 |
| 14.35 | 46.1 | 25.13 | 25.5 |
| 15.19 | 16.2 | 26.08 | 11.3 |
| 15.48 | 6.2 | 26.49 | 44.1 |
| 16.83 | 17.2 | 28.30 | 3.1 |
| 17.51 | 30.3 | 28.79 | 4.8 |
| 18.01 | 18.4 | 29.96 | 3.6 |
| 18.85 | 10.4 | 30.21 | 6.1 |
| 19.40 | 43.1 | 31.69 | 4.5 |
| 20.16 | 66.7 | 34.99 | 3.0 |
| 21.15 | 98.3 | 36.05 | 2.8 |

Preferably, crystal form E has X-ray powder diffraction intensities shown in Table 5.

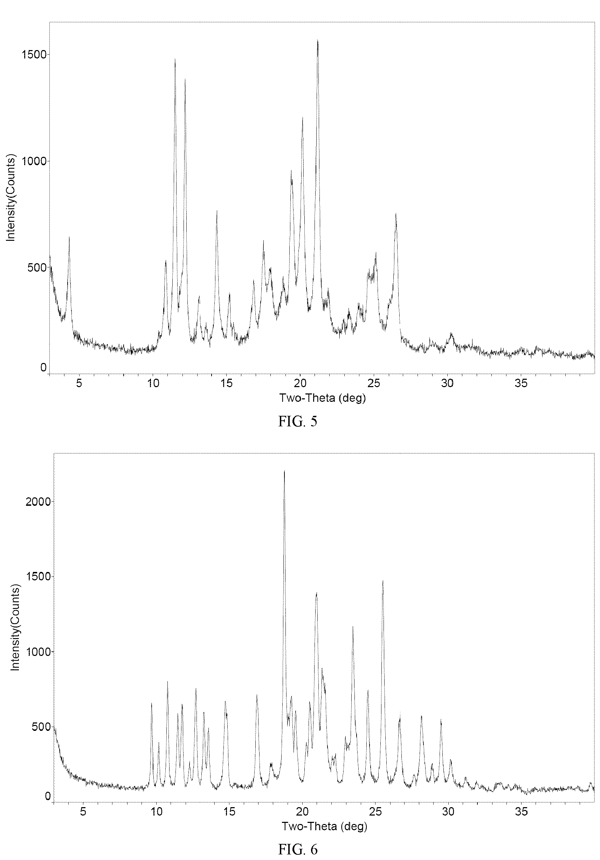

Preferably, crystal form E has an X-ray powder diffraction pattern substantially shown in FIG. 5.

Preferably, crystal form E exhibits an endothermic peak in a DSC analysis when heated to a peak temperature of about 112.00 °C.

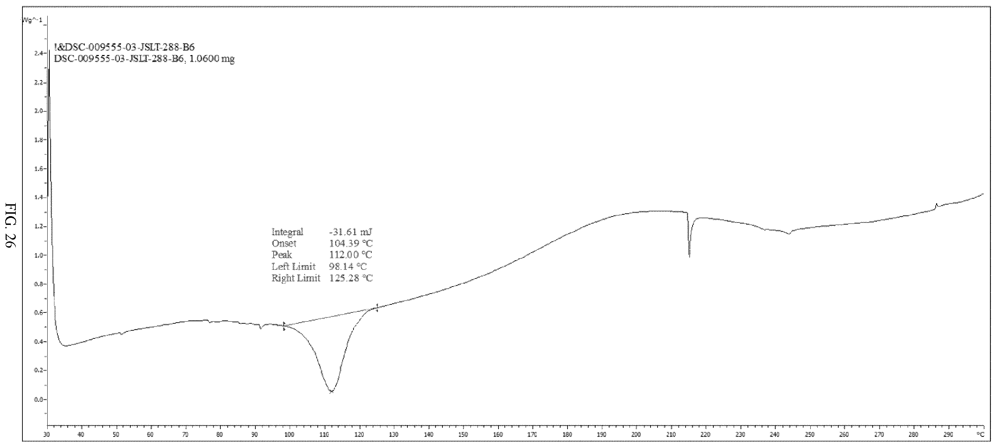

Preferably, crystal form E has a DSC profile substantially shown in FIG. 26.

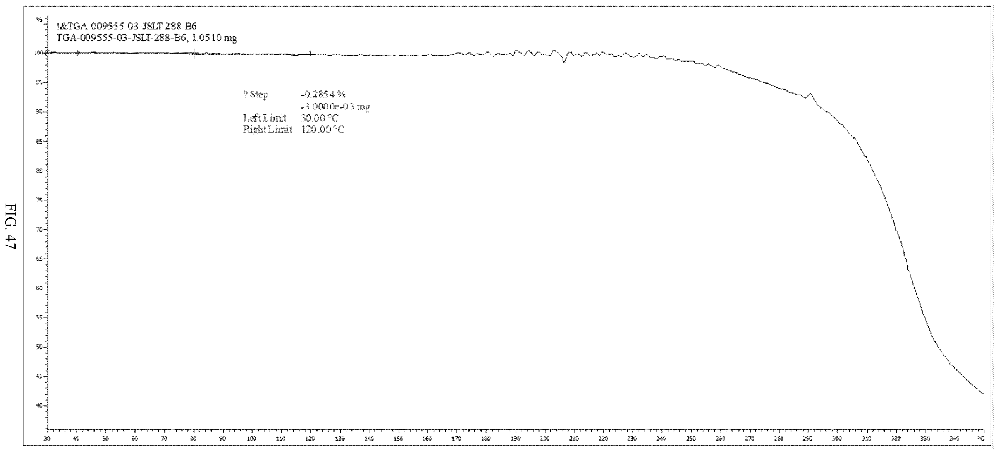

Preferably, crystal form E has a TGA profile substantially shown in FIG. 47.

Crystal form F of compound I provided by the present disclosure has an X-ray powder diffraction pattern comprising peaks at diffraction angles (2θ) of 18.77 ± 0.2°, 25.50 ± 0.2°, 20.98 ± 0.2°, and 23.45 ± 0.2°;
preferably, further comprising peaks at diffraction angles (2θ) of 10.77 ± 0.2°, 12.70 ± 0.2°, 21.33 ± 0.2°, 24.50 ± 0.2°, and 16.89 ± 0.2°;
and more preferably, further comprising peaks at diffraction angles (2θ) of 14.72 ± 0.2°, 19.24 ± 0.2°, 9.68 ± 0.2°, 11.7 ± 0.2°, 20.49 ± 0.2°, and 13.26 ± 0.2°.

Preferably, crystal form F has an X-ray powder diffraction pattern comprising diffraction angles (2θ) shown in Table 6, wherein the 2θ angles have a margin of error of ±0.20°:

**Table 6**

| 2θ (°) | Intensity% | 2θ (°) | Intensity% |
|---|---|---|---|
| 9.68 | 27.1 | 21.33 | 30.0 |
| 10.16 | 14.0 | 22.03 | 4.4 |
| 10.77 | 33.5 | 22.28 | 7.5 |
| 11.45 | 23.0 | 22.94 | 14.5 |
| 11.76 | 26.0 | 23.45 | 50.9 |
| 12.27 | 7.4 | 24.50 | 29.5 |
| 12.70 | 31.0 | 25.50 | 65.5 |
| 13.26 | 23.7 | 26.68 | 23.7 |
| 13.59 | 18.9 | 27.62 | 2.7 |
| 14.72 | 28.2 | 28.15 | 22.2 |
| 15.40 | 1.4 | 28.90 | 5.6 |
| 16.89 | 29.7 | 29.49 | 20.1 |
| 17.91 | 5.9 | 30.14 | 7.5 |
| 18.77 | 100.0 | 31.21 | 3.3 |
| 19.24 | 27.3 | 31.94 | 2.7 |
| 19.54 | 21.3 | 33.50 | 2.3 |
| 20.27 | 12.8 | 34.08 | 2.1 |
| 20.49 | 26.0 | 34.57 | 2.3 |
| 20.98 | 59.3 | | |

Preferably, crystal form F has X-ray powder diffraction intensities shown in Table 6.

Preferably, crystal form F has an X-ray powder diffraction pattern substantially shown in FIG. 6.

Preferably, crystal form F exhibits an endothermic peak in a DSC analysis when heated to a peak temperature of about 169.00 °C.

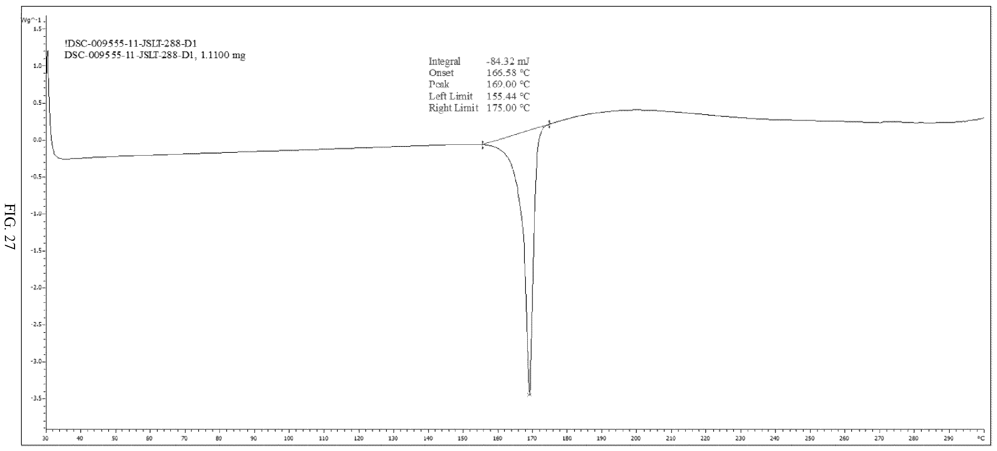

Preferably, crystal form F has a DSC profile substantially shown in FIG. 27.

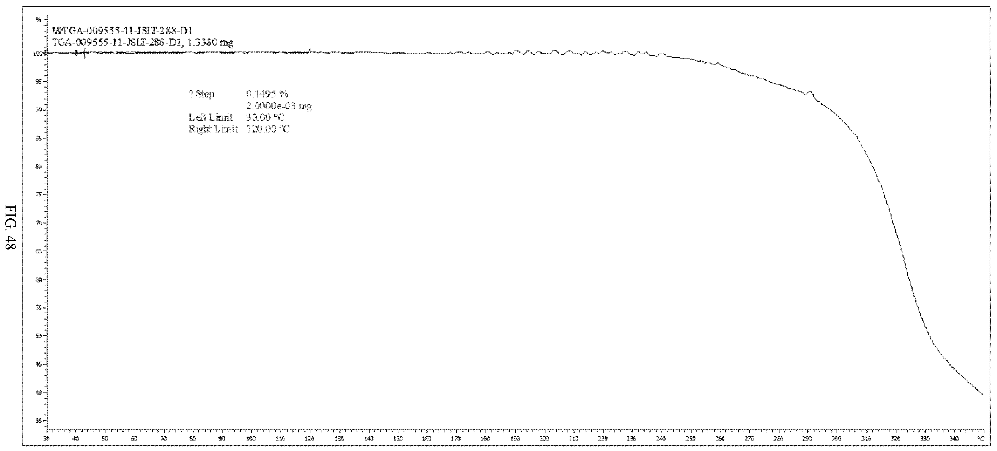

Preferably, crystal form F has a TGA profile substantially shown in FIG. 48.

Crystal form G of a hydrate of compound I provided by the present disclosure has an X-ray powder diffraction pattern comprising peaks at diffraction angles (2θ) of 18.66 ± 0.2°, 18.81 ± 0.2°, 3.62 ± 0.2°, and 22.24 ± 0.2°;
preferably, further comprising peaks at diffraction angles (2θ) of 15.33 ± 0.2°, 17.88 ± 0.2°, 14.76 ± 0.2°, 20.51 ± 0.2°, and 11.08 ± 0.2°;
and more preferably, further comprising peaks at diffraction angles (2θ) of 19.94 ± 0.2°, 26.57 ± 0.2°, 23.22 ± 0.2°, 24.37 ± 0.2°, 7.32 ± 0.2°, and 23.44 ± 0.2°.

Preferably, crystal form G has an X-ray powder diffraction pattern comprising diffraction angles (2θ) shown in Table 7, wherein the 2θ angles have a margin of error of ±0.20°:

**Table 7**

| 2θ (°) | Intensity% | 2θ (°) | Intensity% |
|---|---|---|---|
| 3.62 | 62.0 | 18.81 | 63.5 |
| 7.32 | 21.0 | 19.94 | 33.4 |
| 7.70 | 7.2 | 20.51 | 35.4 |
| 8.72 | 16.8 | 22.24 | 59.6 |
| 10.22 | 12.5 | 23.22 | 25.6 |
| 11.08 | 34.1 | 23.44 | 16.4 |
| 12.48 | 14.6 | 24.37 | 18.8 |
| 14.76 | 40.4 | 26.57 | 27.9 |
| 15.33 | 52.0 | 28.08 | 5.7 |
| 16.45 | 11.6 | 30.21 | 11.1 |
| 17.88 | 50.6 | 31.30 | 7.2 |
| 18.66 | 100.0 | 33.39 | 4.8 |

Preferably, crystal form G has X-ray powder diffraction intensities shown in Table 7.

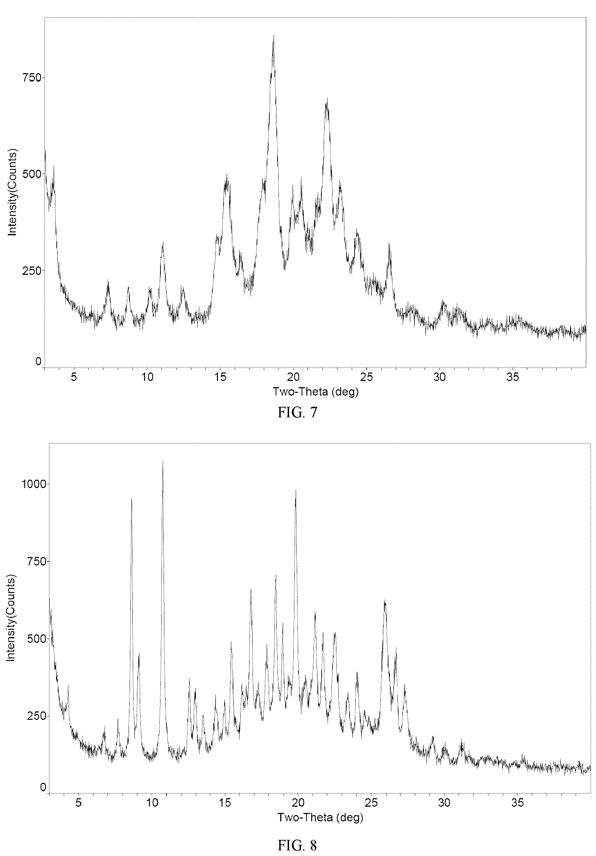

Preferably, crystal form G has an X-ray powder diffraction pattern substantially shown in FIG. 7.

Preferably, crystal form G exhibits endothermic peaks in a DSC analysis when heated to peak temperatures of about 89.95 °C and about 104.07 °C.

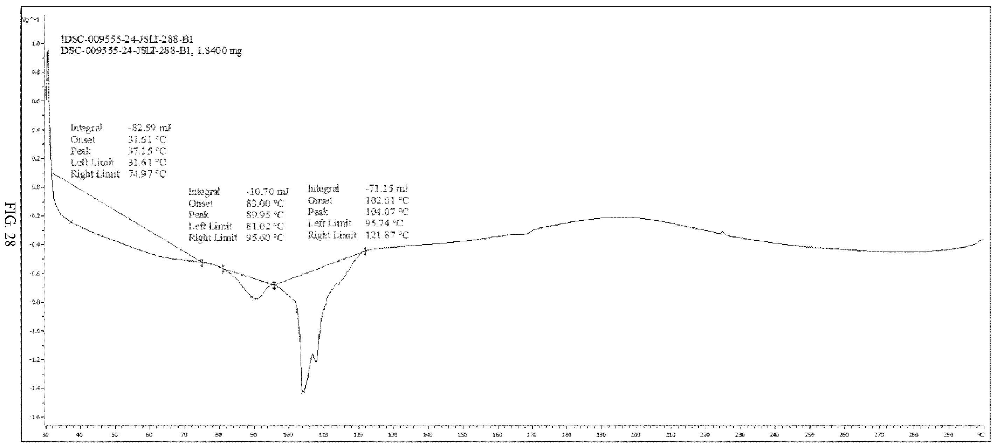

Preferably, crystal form G has a DSC profile substantially shown in FIG. 28.

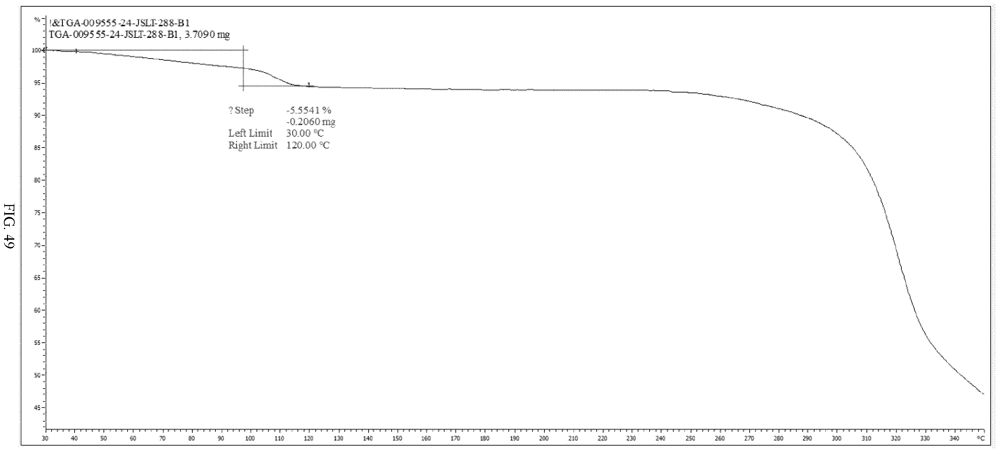

Preferably, crystal form G has a TGA profile substantially shown in FIG. 49.

Preferably, crystal form G is a dihydrate of compound I.

Crystal form H of a methyl isobutyl ketone solvate of compound I provided by the present disclosure has an X-ray powder diffraction pattern comprising peaks at diffraction angles (2θ) of 10.75 ± 0.2°, 8.61 ± 0.2°, 19.84 ± 0.2°, and 18.48 ± 0.2°;
preferably, further comprising peaks at diffraction angles (2θ) of 16.79 ± 0.2°, 25.92 ± 0.2°, 9.11 ± 0.2°, 21.15 ± 0.2°, and 15.43 ± 0.2°;
and more preferably, further comprising peaks at diffraction angles (2θ) of 22.55 ± 0.2°, 3.16 ± 0.2°, 18.95 ± 0.2°, 21.70 ± 0.2°, 12.54 ± 0.2°, and 17.86 ± 0.2°.

Preferably, crystal form H has an X-ray powder diffraction pattern comprising diffraction angles (2θ) shown in Table 8, wherein the 2θ angles have a margin of error of ±0.20°:

**Table 8**

| 2θ (°) | Intensity% | 2θ (°) | Intensity% |
|---|---|---|---|
| 3.16 | 28.1 | 18.95 | 27.4 |
| 4.28 | 12.0 | 19.33 | 9.7 |
| 6.78 | 10.4 | 19.84 | 71.5 |
| 7.69 | 12.5 | 20.51 | 8.3 |
| 8.61 | 86.7 | 21.15 | 32.8 |
| 9.11 | 34.6 | 21.70 | 26.5 |
| 10.75 | 100.0 | 22.55 | 30.4 |
| 12.54 | 24.1 | 23.39 | 12.4 |
| 12.97 | 20.2 | 24.01 | 20.0 |
| 13.53 | 10.7 | 24.52 | 7.0 |
| 14.35 | 16.1 | 25.92 | 43.1 |
| 14.96 | 11.6 | 26.68 | 20.8 |
| 15.43 | 31.2 | 27.29 | 17.2 |
| 16.16 | 9.9 | 29.23 | 6.8 |
| 16.45 | 13.1 | 29.96 | 4.6 |
| 16.79 | 45.1 | 30.06 | 5.6 |
| 17.28 | 10.7 | 31.17 | 7.7 |
| 17.86 | 22.5 | 35.30 | 4.3 |
| 18.48 | 45.3 | | |

Preferably, crystal form H has X-ray powder diffraction intensities shown in Table 8.

Preferably, crystal form H has an X-ray powder diffraction pattern substantially shown in FIG. 8.

Preferably, crystal form H exhibits an endothermic peak in a DSC analysis when heated to a peak temperature of about 87.76 °C.

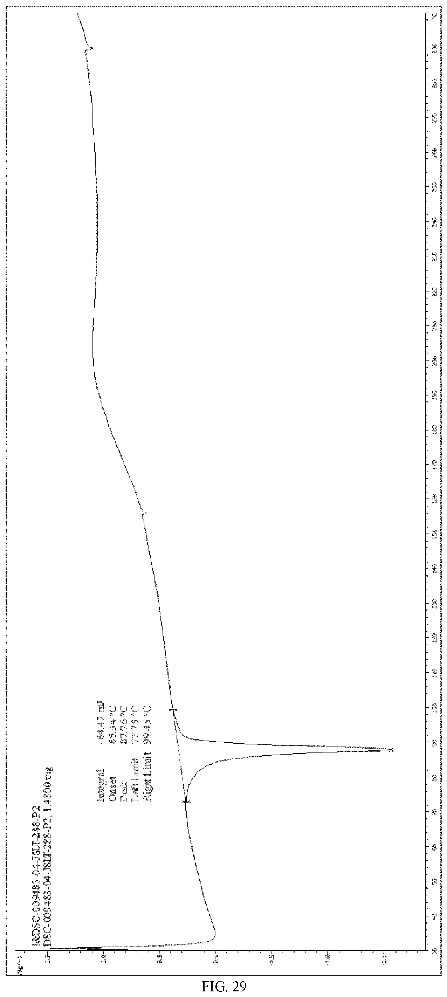

Preferably, crystal form H has a DSC profile substantially shown in FIG. 29.

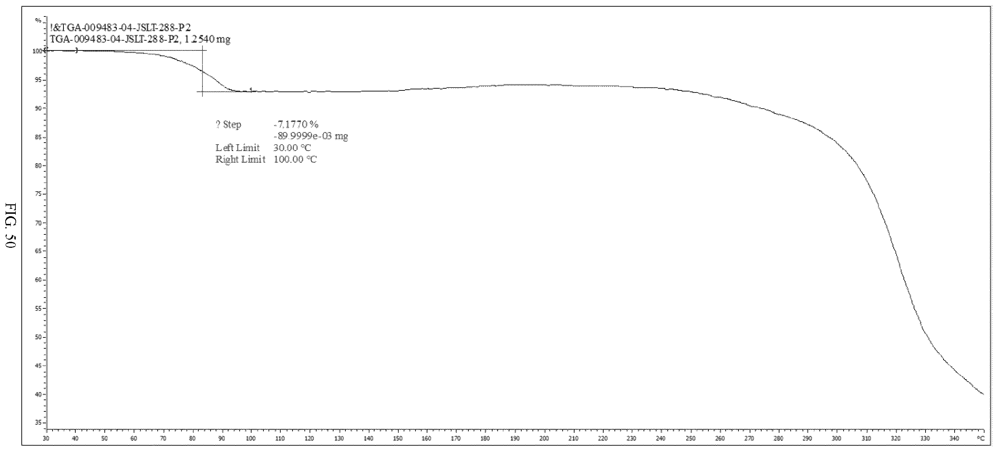

Preferably, crystal form H has a TGA profile substantially shown in FIG. 50.

Preferably, crystal form H is a methyl isobutyl ketone 0.5-solvate of compound I.

Crystal form I-1 of a methyl *tert-butyl* ether solvate of compound I provided by the present disclosure has an X-ray powder diffraction pattern comprising peaks at diffraction angles (2θ) of 8.53 ± 0.2°, 10.75 ± 0.2°, 4.22 ± 0.2°, and 18.39 ± 0.2°;
preferably, further comprising peaks at diffraction angles (2θ) of 17.22 ± 0.2°, 21.48 ± 0.2°, 16.85 ± 0.2°, 17.53 ± 0.2°, and 9.11 ± 0.2°;
and more preferably, further comprising peaks at diffraction angles (2θ) of 12.49 ± 0.2°, 19.61 ± 0.2°, 12.87 ± 0.2°, 15.66 ± 0.2°, 26.02 ± 0.2°, and 22.40 ± 0.2°.

Preferably, crystal form I-1 has an X-ray powder diffraction pattern comprising diffraction angles (2θ) shown in Table 9, wherein the 2θ angles have a margin of error of ±0.20°:

**Table 9**

| 2θ (°) | Intensity% | 2θ (°) | Intensity% |
|---|---|---|---|
| 4.22 | 37.6 | 19.61 | 13.2 |
| 6.84 | 4.8 | 20.57 | 7.0 |
| 8.53 | 100.0 | 21.48 | 23.3 |
| 9.11 | 15.2 | 22.40 | 7.3 |
| 10.75 | 46.0 | 23.41 | 4.4 |
| 12.49 | 13.5 | 23.86 | 3.7 |
| 12.87 | 11.8 | 24.70 | 4.0 |
| 13.69 | 5.1 | 26.02 | 9.1 |
| 14.59 | 3.6 | 26.66 | 2.1 |
| 15.66 | 9.2 | 27.24 | 4.1 |
| 16.40 | 5.1 | 27.69 | 4.0 |
| 16.85 | 21.7 | 28.98 | 4.6 |
| 17.22 | 24.8 | 30.81 | 3.6 |
| 17.53 | 16.1 | 31.13 | 5.1 |
| 18.39 | 30.5 | | 13.2 |

Preferably, crystal form I-1 has X-ray powder diffraction intensities shown in Table 9.

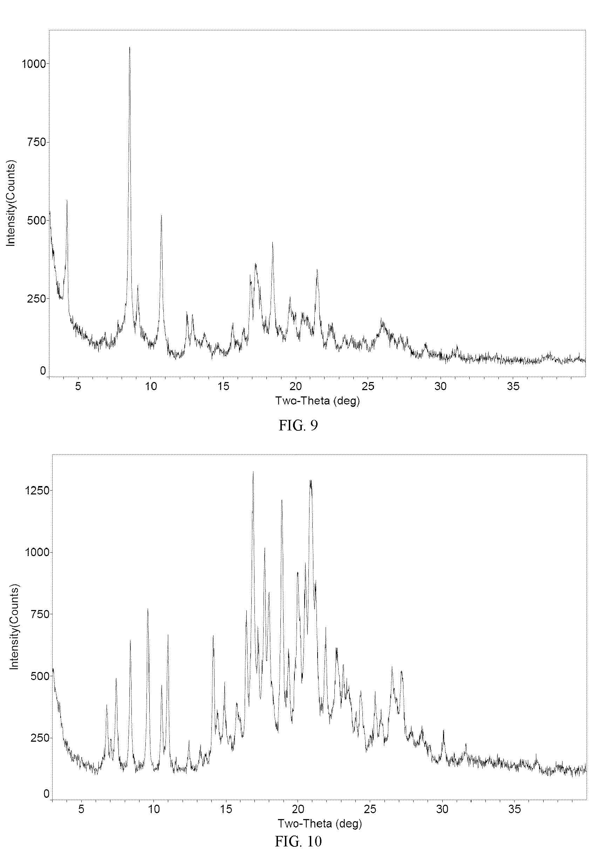

Preferably, crystal form I-1 has an X-ray powder diffraction pattern substantially shown in FIG. 9.

Preferably, crystal form I-1 exhibits an endothermic peak in a DSC analysis when heated to a peak temperature of about 103.27 °C.

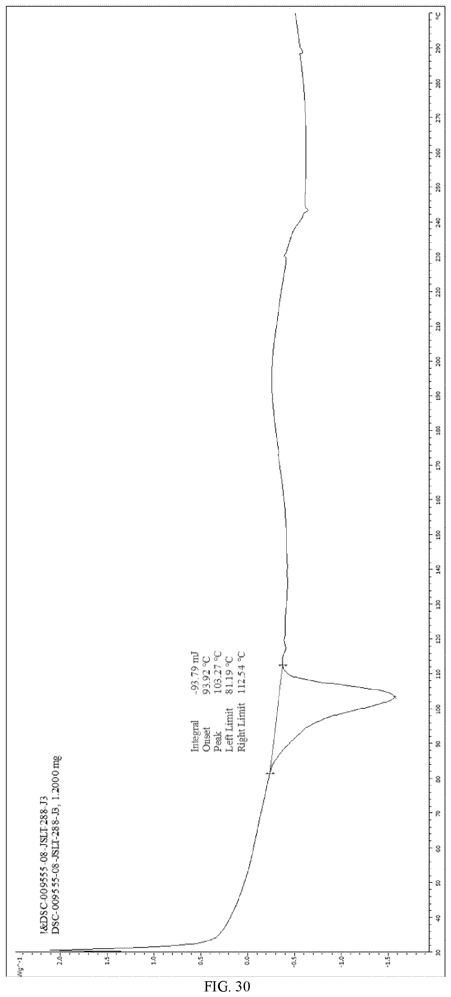

Preferably, crystal form I-1 has a DSC profile substantially shown in FIG. 30.

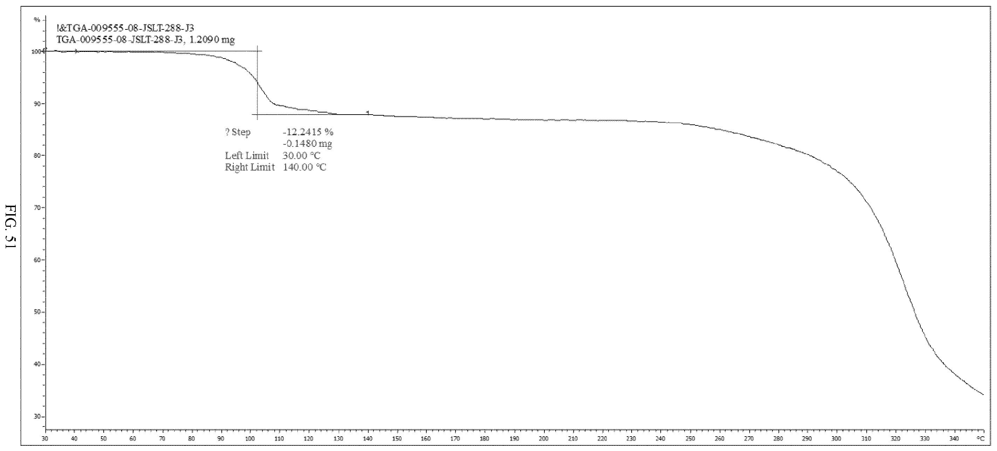

Preferably, crystal form I-1 has a TGA profile substantially shown in FIG. 51.

Preferably, crystal form I-1 is a methyl *tert-butyl* ether monosolvate of compound I.

Crystal form I-2 of a methyl *tert-butyl* ether solvate of compound I provided by the present disclosure has an X-ray powder diffraction pattern comprising peaks at diffraction angles (2θ) of 16.88 ± 0.2°, 18.87 ± 0.2°, 20.90 ± 0.2°, and 9.60 ± 0.2°;
preferably, further comprising peaks at diffraction angles (2θ) of 10.98 ± 0.2°, 8.39 ± 0.2°, 17.68 ± 0.2°, 20.51 ± 0.2°, and 19.97 ± 0.2°;
and more preferably, further comprising peaks at diffraction angles (2θ) of 21.25 ± 0.2°, 17.99 ± 0.2°, 14.10 ± 0.2°, 16.42 ± 0.2°, 7.40 ± 0.2°, and 21.93 ± 0.2°.

Preferably, crystal form I-2 has an X-ray powder diffraction pattern comprising diffraction angles (2θ) shown in Table 10, wherein the 2θ angles have a margin of error of ±0.20°:

**Table 10**

| 2θ (°) | Intensity% | 2θ (°) | Intensity% |
|---|---|---|---|
| 6.74 | 26.1 | 18.87 | 90.9 |
| 7.04 | 12.7 | 19.32 | 28.4 |
| 7.40 | 36.9 | 19.97 | 49.1 |
| 8.39 | 53.3 | 20.51 | 61.9 |
| 9.60 | 67.1 | 20.90 | 86.5 |
| 10.56 | 35.4 | 21.25 | 47.1 |
| 10.98 | 56.4 | 21.93 | 35.7 |
| 11.55 | 4.8 | 22.64 | 24.6 |
| 12.44 | 11.5 | 23.15 | 24.6 |
| 13.24 | 7.7 | 23.56 | 8.7 |
| 13.62 | 3.4 | 24.03 | 9.0 |
| 14.10 | 47.7 | 24.37 | 19.1 |
| 14.39 | 18.1 | 25.36 | 21.4 |
| 14.92 | 27.1 | 25.75 | 12.9 |
| 15.31 | 4.2 | 26.53 | 29.5 |
| 15.74 | 16.2 | 27.17 | 29.6 |
| 16.00 | 8.5 | 27.83 | 6.1 |
| 16.42 | 38.6 | 28.59 | 10.0 |
| 16.88 | 100.0 | 30.09 | 13.5 |
| 17.20 | 31.0 | 31.62 | 8.0 |
| 17.68 | 63.6 | 36.48 | 6.5 |
| 17.99 | 47.6 | 38.31 | 4.3 |

Preferably, crystal form I-2 has X-ray powder diffraction intensities shown in Table 10.

Preferably, crystal form I-2 has an X-ray powder diffraction pattern substantially shown in FIG. 10.

Preferably, crystal form I-2 exhibits an endothermic peak in a DSC analysis when heated to a peak temperature of about 97.11 °C.

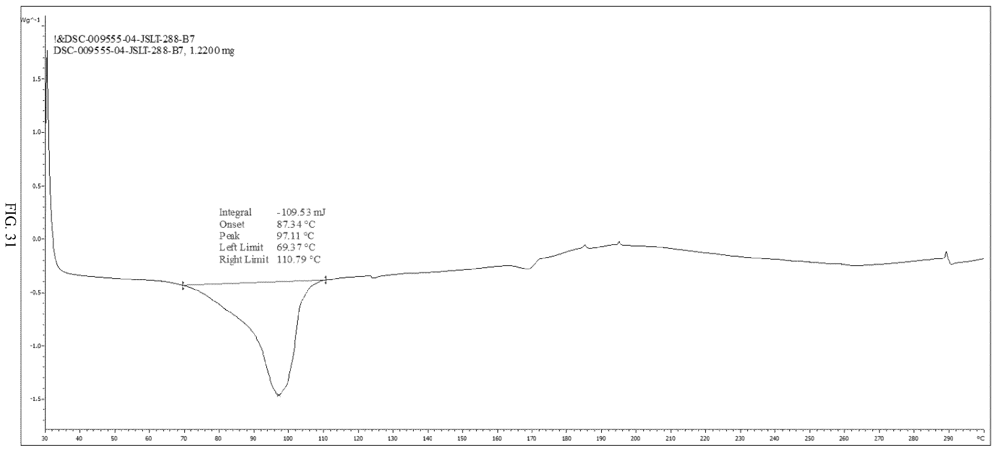

Preferably, crystal form I-2 has a DSC profile substantially shown in FIG. 31.

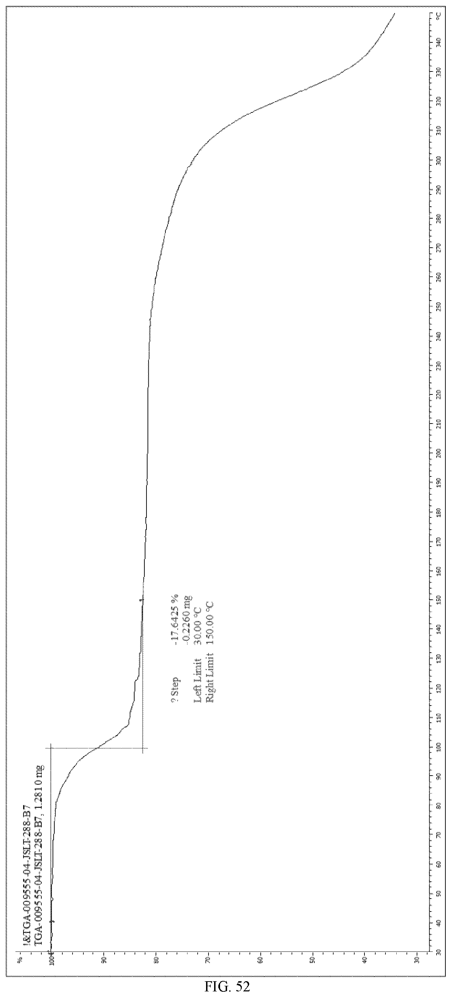

Preferably, crystal form I-2 has a TGA profile substantially shown in FIG. 52.

Preferably, crystal form I-2 is a methyl *tert-butyl* ether 1.5-solvate of compound I.

Crystal form J of an acetone solvate of compound I provided by the present disclosure has an X-ray powder diffraction pattern comprising peaks at diffraction angles (2θ) of 12.02 ± 0.2°, 18.03 ± 0.2°, 8.04 ± 0.2°, and 7.67 ± 0.2°;
preferably, further comprising peaks at diffraction angles (2θ) of 19.54 ± 0.2°, 23.50 ± 0.2°, 16.05 ± 0.2°, 21.21 ± 0.2°, and 13.83 ± 0.2°;
and more preferably, further comprising peaks at diffraction angles (2θ) of 11.10 ± 0.2°, 17.43 ± 0.2°, 16.54 ± 0.2°, 8.92 ± 0.2°, 22.59 ± 0.2°, and 27.39 ± 0.2°.

Preferably, crystal form J has an X-ray powder diffraction pattern comprising diffraction angles (2θ) shown in Table 11, wherein the 2θ angles have a margin of error of ±0.20°:

**Table 11**

| 2θ (°) | Intensity% | 2θ (°) | Intensity% |
|---|---|---|---|
| 6.83 | 20.4 | 16.54 | 28.5 |
| 7.67 | 44.9 | 17.43 | 29.8 |
| 8.04 | 56.1 | 18.03 | 61.4 |
| 8.54 | 21.1 | 19.54 | 43.6 |
| 8.92 | 26.1 | 21.21 | 38.6 |
| 10.69 | 7.6 | 22.59 | 24.3 |
| 11.10 | 31.9 | 23.50 | 41.8 |
| 12.02 | 100.0 | 25.83 | 18.5 |
| 13.83 | 34.5 | 27.39 | 21.4 |
| 14.48 | 16.4 | 29.32 | 9.9 |
| 16.05 | 39.4 | 30.93 | 17.0 |

Preterably, crystal form J has X-ray powder diffraction intensities shown in Table 11.

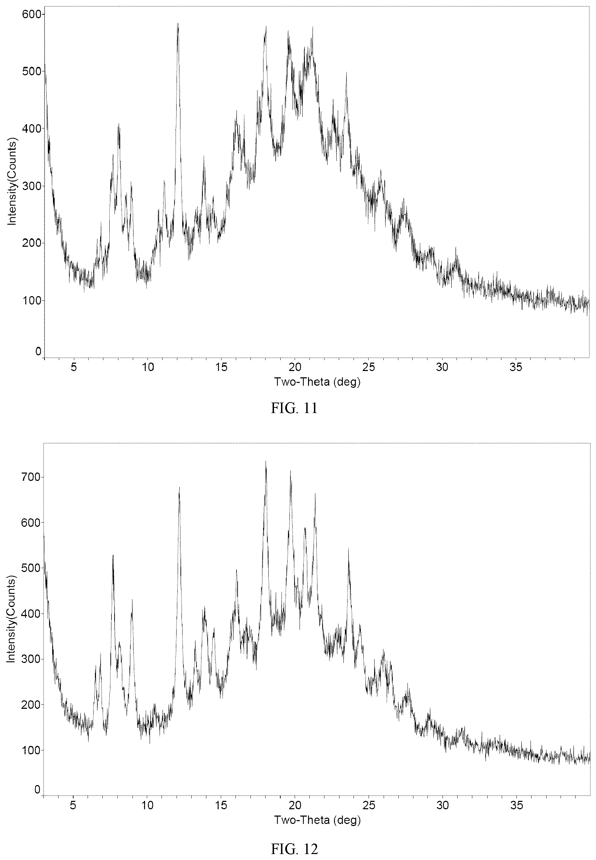

Preferably, crystal form J has an X-ray powder diffraction pattern substantially shown in FIG. 11.

Preferably, crystal form J exhibits an endothermic peak in a DSC analysis when heated to a peak temperature of about 93.18 °C.

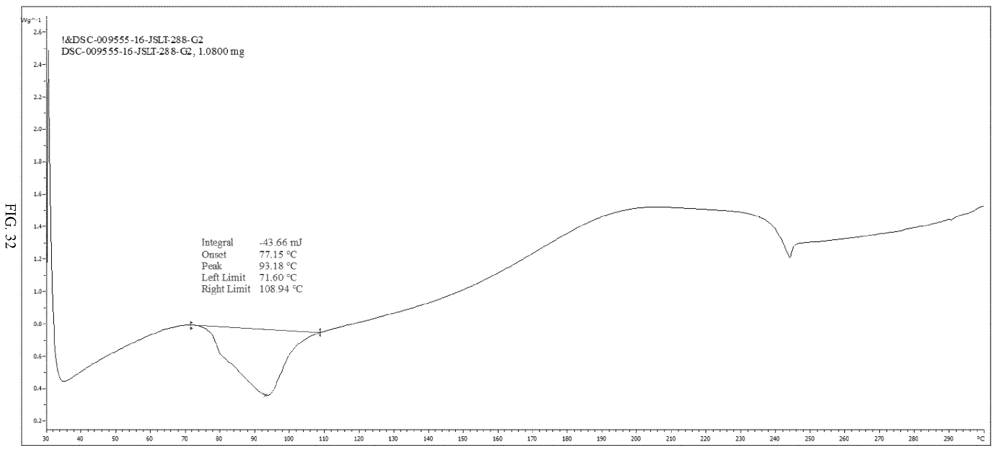

Preferably, crystal form J has a DSC profile substantially shown in FIG. 32.

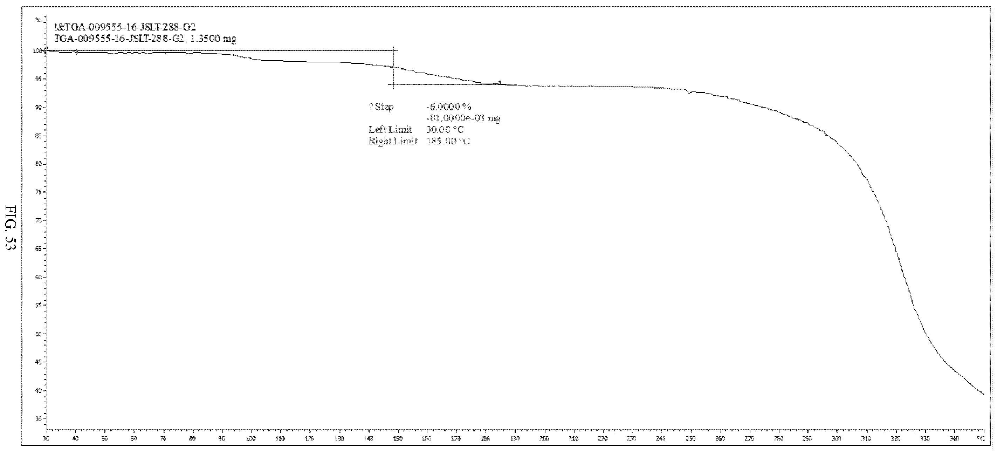

Preferably, crystal form J has a TGA profile substantially shown in FIG. 53.

Preferably, crystal form J is an acetone 0.5-solvate of compound I.

Crystal form K of a n-heptane solvate of compound I provided by the present disclosure has an X-ray powder diffraction pattern comprising peaks at diffraction angles (2θ) of 12.19 ± 0.2°, 18.02 ± 0.2°, 7.69 ± 0.2°, and 19.69 ± 0.2°;
preferably, further comprising peaks at diffraction angles (2θ) of 21.36 ± 0.2°, 8.98 ± 0.2°, 23.63 ± 0.2°, 16.07 ± 0.2°, and 20.72 ± 0.2°;
and more preferably, further comprising peaks at diffraction angles (2θ) of 6.85 ± 0.2°, 8.12 ± 0.2°, 13.75 ± 0.2°, 6.54 ± 0.2°, 14.53 ± 0.2°, and 13.26 ± 0.2°.

Preferably, crystal form K has an X-ray powder diffraction pattern comprising diffraction angles (2θ) shown in Table 12, wherein the 2θ angles have a margin of error of ±0.20°:

**Table 12**

| 2θ (°) | Intensity% | 2θ (°) | Intensity% |
|---|---|---|---|
| 6.54 | 28.2 | 19.69 | 66.0 |
| 6.85 | 33.2 | 20.18 | 14.7 |
| 7.69 | 74.5 | 20.72 | 38.2 |
| 8.12 | 32.8 | 21.36 | 61.4 |
| 8.98 | 53.3 | 23.63 | 47.7 |
| 10.55 | 10.0 | 24.37 | 17.6 |
| 12.19 | 100.0 | 25.37 | 11.2 |
| 13.26 | 21.2 | 26.04 | 20.7 |
| 13.75 | 35.9 | 26.52 | 12.4 |
| 14.53 | 24.7 | 27.44 | 10.6 |
| 15.66 | 21.2 | 27.73 | 14.1 |
| 16.07 | 39.2 | 29.13 | 12.0 |
| 18.02 | 82.0 | 31.20 | 5.4 |

Preferably, crystal form K has X-ray powder diffraction intensities shown in Table 12.

Preferably, crystal form K has an X-ray powder diffraction pattern substantially shown in FIG. 12.

Preferably, crystal form K exhibits an endothermic peak in a DSC analysis when heated to a peak temperature of about 91.51 °C.

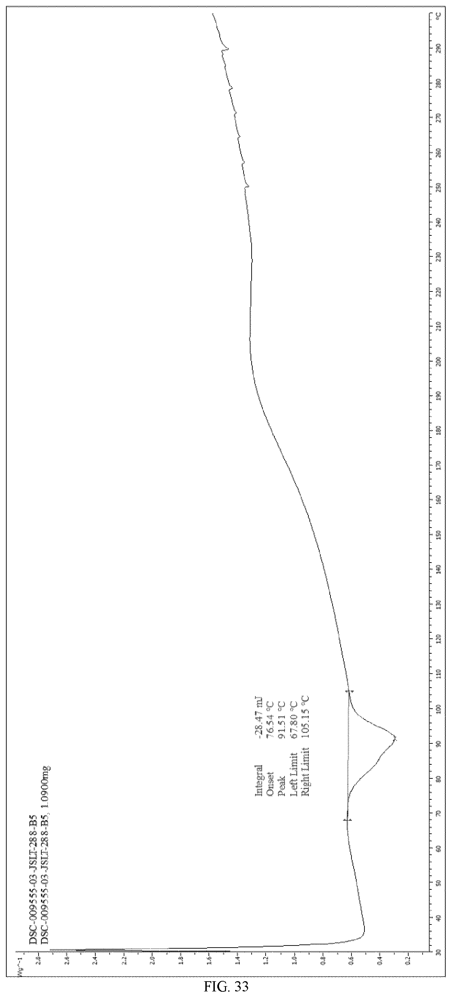

Preferably, crystal form K has a DSC profile substantially shown in FIG. 33.

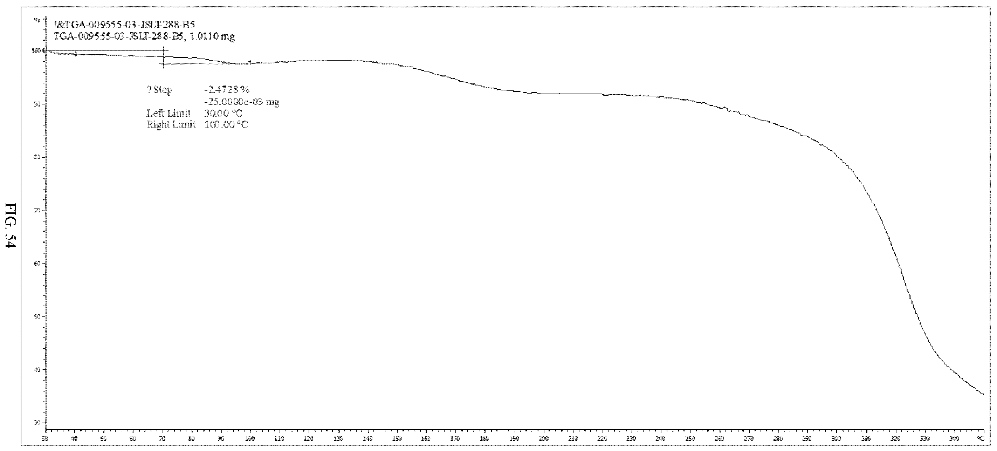

Preferably, crystal form K has a TGA profile substantially shown in FIG. 54.

Preferably, crystal form K is a n-heptane 0.12-solvate of compound I.

Crystal form L of a methylcyclohexane solvate of compound I provided by the present disclosure has an X-ray powder diffraction pattern comprising peaks at diffraction angles (2θ) of 10.98 ± 0.2°, 15.54 ± 0.2°, 9.09 ± 0.2°, and 19.01 ± 0.2°;
preferably, further comprising peaks at diffraction angles (2θ) of 20.39 ± 0.2°, 17.94 ± 0.2°, 8.74 ± 0.2°, 21.03 ± 0.2°, and 13.46 ± 0.2°;
and more preferably, further comprising peaks at diffraction angles (2θ) of 22.86 ± 0.2°, 18.25 ± 0.2°, 25.63 ± 0.2°, 16.90 ± 0.2°, 24.74 ± 0.2°, and 25.95 ± 0.2°.

Preferably, crystal form L has an X-ray powder diffraction pattern comprising diffraction angles (2θ) shown in Table 13, wherein the 2θ angles have a margin of error of ±0.20°:

**Table 13**

| 2θ (°) | Intensity% | 2θ (°) | Intensity% |
|---|---|---|---|
| 5.35 | 23.5 | 16.90 | 35.5 |
| 6.25 | 18.5 | 17.94 | 71.0 |
| 6.73 | 17.5 | 18.25 | 44.0 |
| 7.83 | 24.5 | 19.01 | 78.0 |
| 8.74 | 59.5 | 20.39 | 72.5 |
| 9.09 | 84.0 | 21.03 | 57.0 |
| 10.98 | 100.0 | 22.86 | 48.5 |
| 13.46 | 55.0 | 24.74 | 35.0 |
| 15.54 | 100.0 | 25.63 | 41.5 |
| 16.58 | 26.5 | 25.95 | 33.5 |

Preferably, crystal form L has X-ray powder diffraction intensities shown in Table 13.

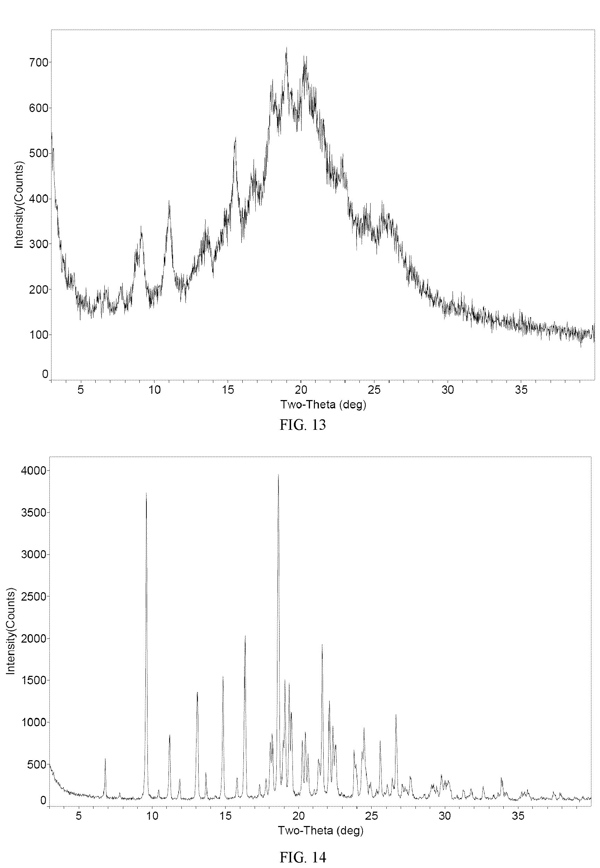

Preferably, crystal form L has an X-ray powder diffraction pattern substantially shown in FIG. 13.

Preferably, crystal form L exhibits an endothermic peak in a DSC analysis when heated to a peak temperature of about 88.68 °C.

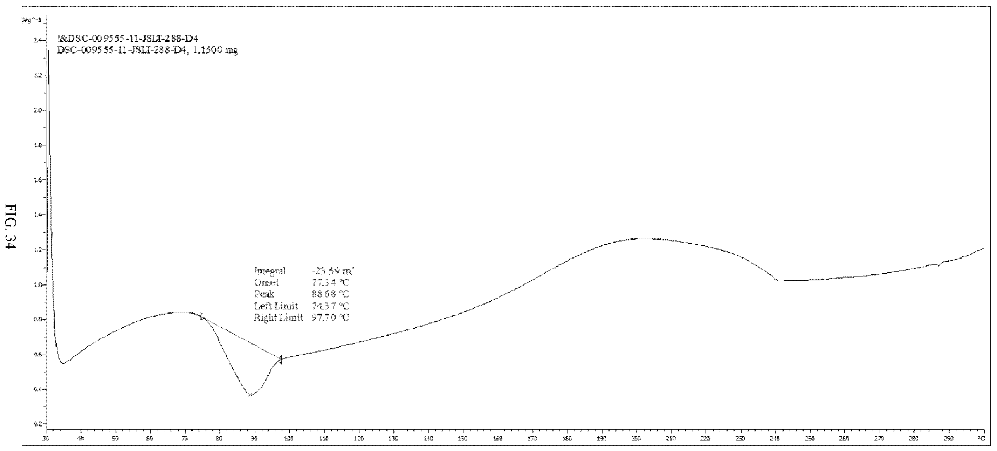

Preferably, crystal form L has a DSC profile substantially shown in FIG. 34.

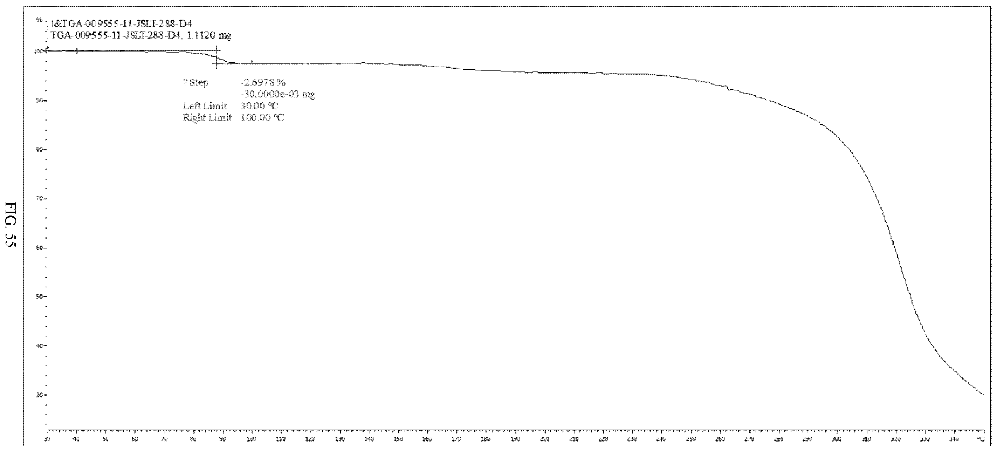

Preferably, crystal form L has a TGA profile substantially shown in FIG. 55.

Preferably, crystal form L is a methylcyclohexane 0.15-solvate of compound I.

Crystal form M of a toluene solvate of compound I provided by the present disclosure has an X-ray powder diffraction pattern comprising peaks at diffraction angles (2θ) of 18.62 ± 0.2°, 9.60 ± 0.2°, 16.34 ± 0.2°, and 21.62 ± 0.2°;
preferably, further comprising peaks at diffraction angles (2θ) of 14.84 ± 0.2°, 19.05 ± 0.2°, 19.36 ± 0.2°, 13.08 ± 0.2°, and 22.11 ± 0.2°;
and more preferably, further comprising peaks at diffraction angles (2θ) of 26.65 ± 0.2°, 24.48 ± 0.2°, 22.36 ± 0.2°, 11.19 ± 0.2°, 20.47 ± 0.2°, and 18.19 ± 0.2°.

Preferably, crystal form M has an X-ray powder diffraction pattern comprising diffraction angles (2θ) shown in Table 14, wherein the 2θ angles have a margin of error of ±0.20°:

**Table 14**

| 2θ (°) | Intensity% | 2θ (°) | Intensity% |
|---|---|---|---|
| 6.79 | 12.5 | 25.34 | 2.4 |
| 7.79 | 1.8 | 25.58 | 17.3 |
| 9.60 | 96.6 | 26.06 | 4.0 |
| 10.46 | 2.6 | 26.41 | 5.7 |
| 11.19 | 20.0 | 26.65 | 25.7 |
| 11.87 | 6.3 | 27.08 | 4.0 |
| 13.08 | 33.9 | 27.28 | 2.6 |
| 13.67 | 8.3 | 27.63 | 6.9 |
| 14.31 | 1.1 | 28.62 | 1.5 |
| 14.84 | 38.6 | 29.21 | 4.0 |
| 15.78 | 6.4 | 29.43 | 3.1 |
| 16.34 | 51.2 | 29.76 | 7.6 |
| 17.33 | 4.0 | 30.00 | 5.1 |
| 18.19 | 18.0 | 30.23 | 5.4 |
| 18.62 | 100.0 | 30.83 | 1.3 |
| 19.05 | 35.4 | 31.24 | 2.9 |
| 19.36 | 34.8 | 31.77 | 3.3 |
| 20.26 | 17.2 | 32.61 | 4.1 |
| 20.47 | 19.8 | 33.34 | 1.3 |
| 20.67 | 12.9 | 33.84 | 6.9 |
| 21.39 | 10.9 | 34.20 | 2.2 |
| 21.62 | 47.0 | 35.25 | 2.5 |
| 22.11 | 29.5 | 35.64 | 2.2 |
| 22.36 | 21.4 | 37.41 | 2.1 |
| 23.82 | 12.6 | 37.85 | 2.2 |
| 24.48 | 21.8 | 38.83 | 1.0 |
| 24.93 | 4.5 | | |

Preferably, crystal form M has X-ray powder diffraction intensities shown in Table 14.

Preferably, crystal form M has an X-ray powder diffraction pattern substantially shown in FIG. 14.

Preferably, crystal form M exhibits an endothermic peak in a DSC analysis when heated to a peak temperature of about 102.43 °C.

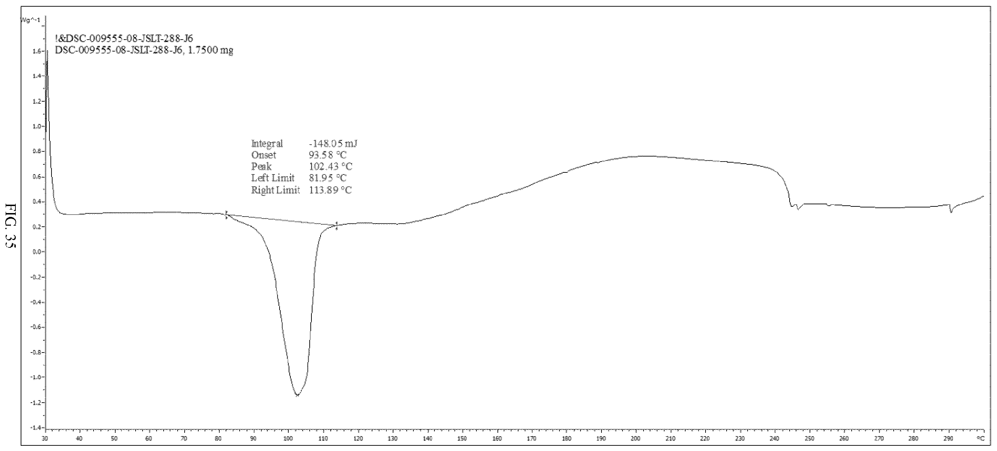

Preferably, crystal form M has a DSC profile substantially shown in FIG. 35.

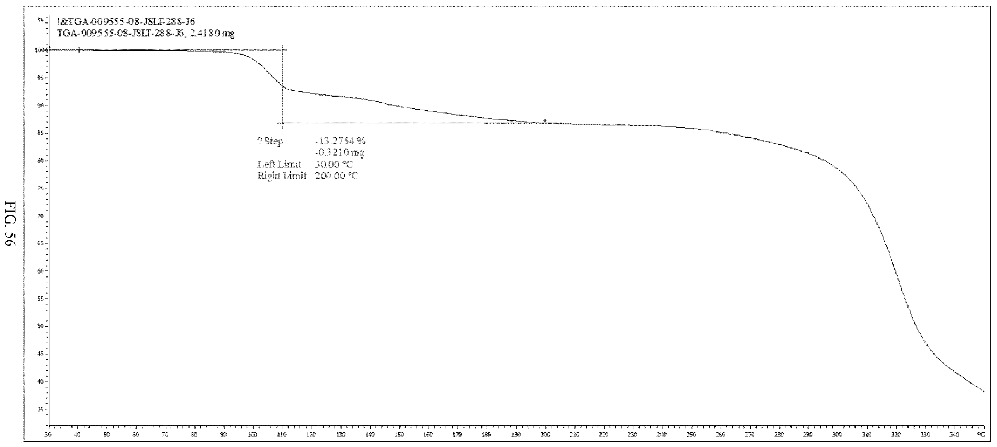

Preferably, crystal form M has a TGA profile substantially shown in FIG. 56.

Preferably, crystal form M is a toluene monosolvate of compound I.

Crystal form N of a dioxane solvate of compound I provided by the present disclosure has an X-ray powder diffraction pattern comprising peaks at diffraction angles (2θ) of 17.10 ± 0.2°, 20.66 ± 0.2°, 22.71 ± 0.2°, and 18.21 ± 0.2°;
preferably, further comprising peaks at diffraction angles (2θ) of 21.70 ± 0.2°, 15.05 ± 0.2°, 20.27 ± 0.2°, 21.97 ± 0.2°, and 8.53 ± 0.2°;
and more preferably, further comprising peaks at diffraction angles (2θ) of 19.59 ± 0.2°, 7.52 ± 0.2°, 11.14 ± 0.2°, 16.83 ± 0.2°, 17.47 ± 0.2°, and 23.57 ± 0.2°.

Preferably, crystal form N has an X-ray powder diffraction pattern comprising diffraction angles (2θ) shown in Table 15, wherein the 2θ angles have a margin of error of ±0.20°:

**Table 15**

| 2θ (°) | Intensity% | 2θ (°) | Intensity% |
|---|---|---|---|
| 6.85 | 7.6 | 21.70 | 40.1 |
| 7.17 | 5.1 | 21.97 | 35.1 |
| 7.52 | 28.2 | 22.71 | 48.0 |
| 8.53 | 36.8 | 23.57 | 26.5 |
| 8.87 | 2.2 | 23.84 | 14.5 |
| 9.77 | 25.7 | 24.44 | 16.9 |
| 10.75 | 19.5 | 25.12 | 8.2 |
| 11.14 | 29.4 | 26.08 | 13.7 |
| 12.63 | 4.2 | 26.98 | 8.4 |
| 13.38 | 7.1 | 27.27 | 14.0 |
| 13.71 | 11.6 | 27.58 | 13.2 |
| 14.34 | 25.9 | 27.94 | 5.5 |
| 15.05 | 38.1 | 28.50 | 4.9 |
| 15.34 | 6.7 | 28.81 | 6.3 |
| 16.12 | 9.4 | 29.16 | 6.3 |
| 16.83 | 28.4 | 29.57 | 4.7 |
| 17.10 | 100.0 | 30.37 | 3.7 |
| 17.47 | 28.2 | 30.86 | 4.5 |
| 17.67 | 15.4 | 31.61 | 3.2 |
| 18.21 | 44.1 | 32.43 | 4.6 |
| 18.46 | 8.5 | 32.78 | 2.4 |
| 18.91 | 6.8 | 34.46 | 3.0 |
| 19.17 | 15.7 | 35.99 | 3.7 |
| 19.59 | 30.8 | 36.62 | 3.9 |
| 20.27 | 35.3 | 36.99 | 4.3 |
| 20.66 | 49.5 | 38.20 | 3.0 |
| 21.19 | 24.9 | 38.95 | 1.1 |

Preferably, crystal form N has X-ray powder diffraction intensities shown in Table 15.

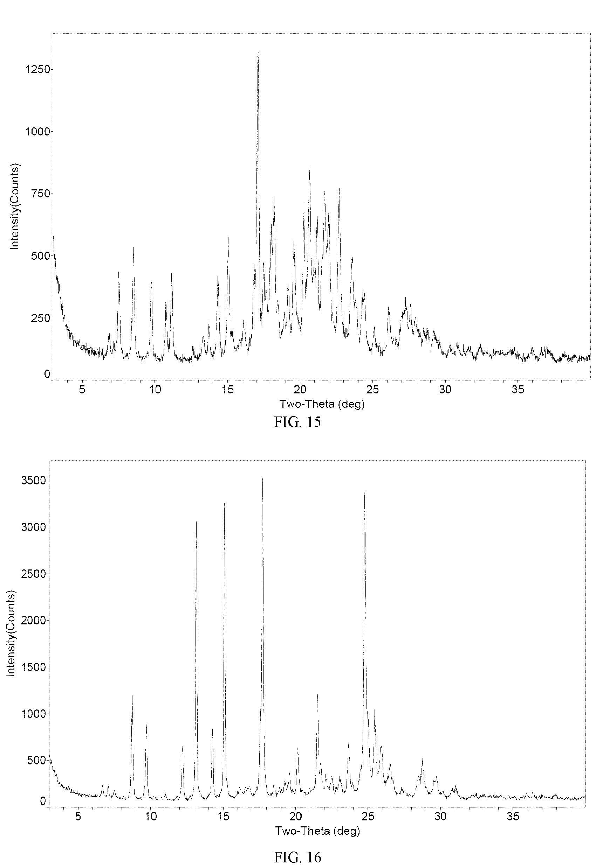

Preferably, crystal form N has an X-ray powder diffraction pattern substantially shown in FIG. 15.

Preferably, crystal form N exhibits an endothermic peak in a DSC analysis when heated to a peak temperature of about 116.48 °C.

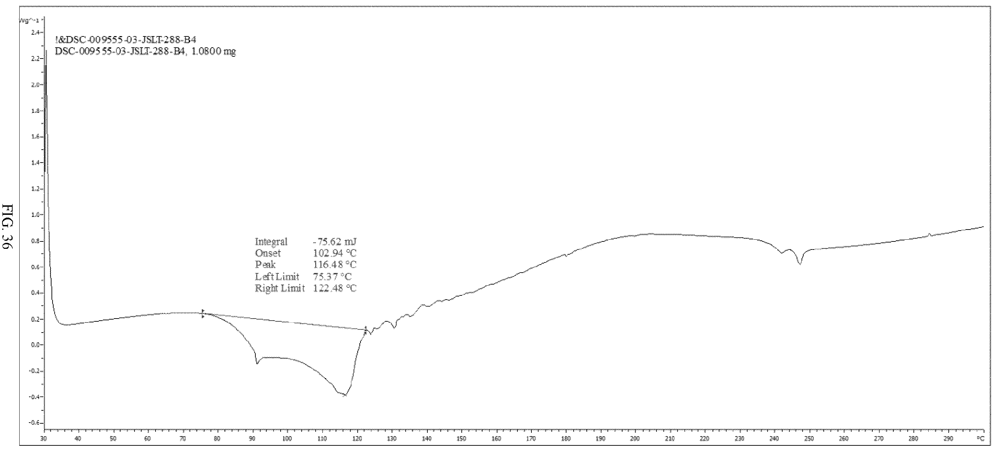

Preferably, crystal form N has a DSC profile substantially shown in FIG. 36.

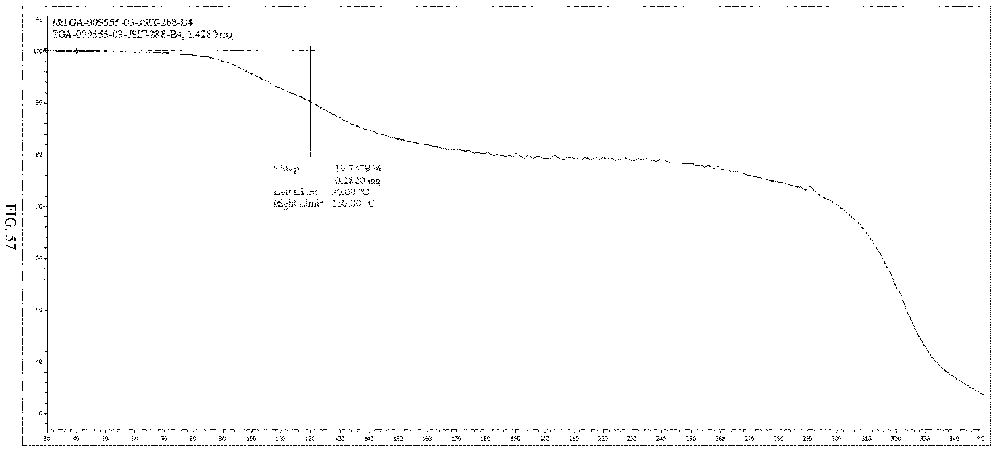

Preferably, crystal form N has a TGA profile substantially shown in FIG. 57.

Preferably, crystal form N is a dioxane 1.5-solvate of compound I.

Crystal form O of a DMF solvate of compound I provided by the present disclosure has an X-ray powder diffraction pattern comprising peaks at diffraction angles (2θ) of 17.72 ± 0.2°, 13.14 ± 0.2°, 15.08 ± 0.2°, and 24.77 ± 0.2°;
preferably, further comprising peaks at diffraction angles (2θ) of 8.72 ± 0.2°, 21.52 ± 0.2°, 9.70 ± 0.2°, 14.26 ± 0.2°, and 25.46 ± 0.2°;
and more preferably, further comprising peaks at diffraction angles (2θ) of 12.19 ± 0.2°, 20.15 ± 0.2°, 25.90 ± 0.2°, 23.66 ± 0.2°, 28.75 ± 0.2°, and 21.73 ± 0.2°.

Preferably, crystal form O has an X-ray powder diffraction pattern comprising diffraction angles (2θ) shown in Table 16, wherein the 2θ angles have a margin of error of ±0.20°:

**Table 16**

| 2θ (°) | Intensity% | 2θ (°) | Intensity% |
|---|---|---|---|
| 6.66 | 3.7 | 22.09 | 5.4 |
| 7.08 | 3.7 | 22.48 | 4.0 |
| 7.52 | 2.3 | 23.06 | 5.4 |
| 8.72 | 32.4 | 23.66 | 15.5 |
| 9.70 | 23.4 | 23.93 | 2.0 |
| 11.01 | 2.2 | 24.77 | 94.4 |
| 12.195 | 16.8 | 25.46 | 21.0 |
| 13.14 | 87.4 | 25.90 | 14.4 |
| 14.26 | 21.4 | 26.53 | 7.5 |
| 15.08 | 92.5 | 27.35 | 1.9 |
| 16.13 | 2.8 | 28.46 | 6.2 |
| 16.59 | 2.8 | 28.75 | 11.3 |
| 16.79 | 3.0 | 29.70 | 5.7 |
| 17.72 | 100.0 | 30.15 | 1.5 |
| 18.51 | 3.1 | 30.81 | 2.7 |
| 18.86 | 1.0 | 31.03 | 3.8 |
| 19.26 | 4.0 | 32.46 | 1.7 |
| 19.56 | 6.5 | 34.16 | 1.2 |
| 20.15 | 14.4 | 34.67 | 1.1 |
| 20.96 | 1.1 | 35.95 | 1.9 |
| 21.52 | 30.2 | 36.35 | 1.8 |
| 21.73 | 9.1 | 37.92 | 1.2 |

Preferably, crystal form O has X-ray powder diffraction intensities shown in Table 16.

Preferably, crystal form O has an X-ray powder diffraction pattern substantially shown in FIG. 16.

Preferably, crystal form O exhibits an endothermic peak in a DSC analysis when heated to a peak temperature of about 123.26 °C.

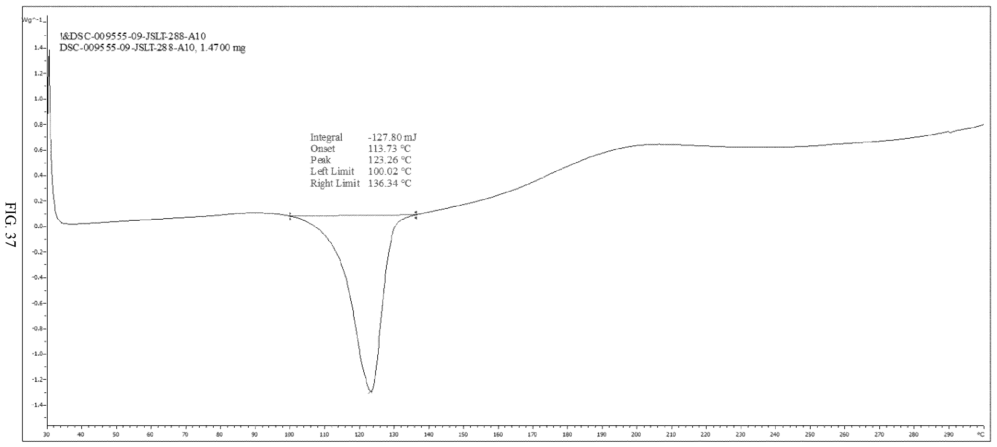

Preferably, crystal form O has a DSC profile substantially shown in FIG. 37.

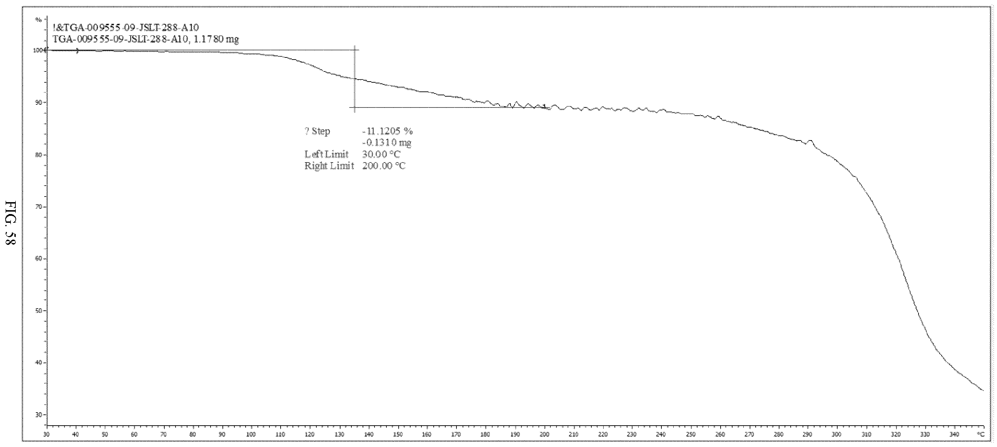

Preferably, crystal form O has a TGA profile substantially shown in FIG. 58.

Preferably, crystal form O is a DMF monosolvate of compound I.

Crystal form P of a N-methylpyrrolidone solvate of compound I provided by the present disclosure has an X-ray powder diffraction pattern comprising peaks at diffraction angles (2θ) of 14.63 ± 0.2°, 13.16 ± 0.2°, 16.98 ± 0.2°, and 14.36 ± 0.2°;
preferably, further comprising peaks at diffraction angles (2θ) of 21.66 ± 0.2°, 23.94 ± 0.2°, 20.22 ± 0.2°, 6.60 ± 0.2°, and 8.41 ± 0.2°;
and more preferably, further comprising peaks at diffraction angles (2θ) of 20.02 ± 0.2°, 11.63 ± 0.2°, 25.14 ± 0.2°, 24.87 ± 0.2°, 21.15 ± 0.2°, and 16.63 ± 0.2°.

Preferably, crystal form P has an X-ray powder diffraction pattern comprising diffraction angles (2θ) shown in Table 17, wherein the 2θ angles have a margin of error of ±0.20°:

**Table 17**

| 2θ (°) | Intensity% | 2θ (°) | Intensity% |
|---|---|---|---|
| 6.60 | 18.6 | 25.14 | 11.6 |
| 8.41 | 14.9 | 25.69 | 9.7 |
| 9.70 | 3.6 | 26.20 | 3.8 |
| 11.63 | 13.6 | 26.53 | 10.0 |
| 13.16 | 80.7 | 27.27 | 3.8 |
| 14.36 | 26.8 | 27.48 | 5.1 |
| 14.63 | 100.0 | 28.76 | 3.8 |
| 15.15 | 2.1 | 29.29 | 5.9 |
| 16.63 | 11.0 | 29.51 | 4.2 |
| 16.98 | 47.9 | 29.86 | 2.2 |
| 17.90 | 10.1 | 30.33 | 3.3 |
| 19.54 | 5.1 | 31.16 | 2.3 |
| 20.02 | 14.6 | 31.77 | 3.1 |
| 20.22 | 25.9 | 33.08 | 2.9 |
| 20.86 | 16.6 | 33.66 | 1.3 |
| 21.15 | 11.3 | 34.22 | 1.0 |
| 21.66 | 24.6 | 34.73 | 2.9 |
| 22.32 | 6.9 | 36.49 | 1.4 |
| 22.77 | 2.7 | 38.25 | 0.8 |
| 23.36 | 5.6 | 38.64 | 1.1 |
| 23.94 | 22.3 | 39.17 | 2.1 |
| 24.87 | 11.4 | | |

Preferably, crystal form P has X-ray powder diffraction intensities shown in Table 17.

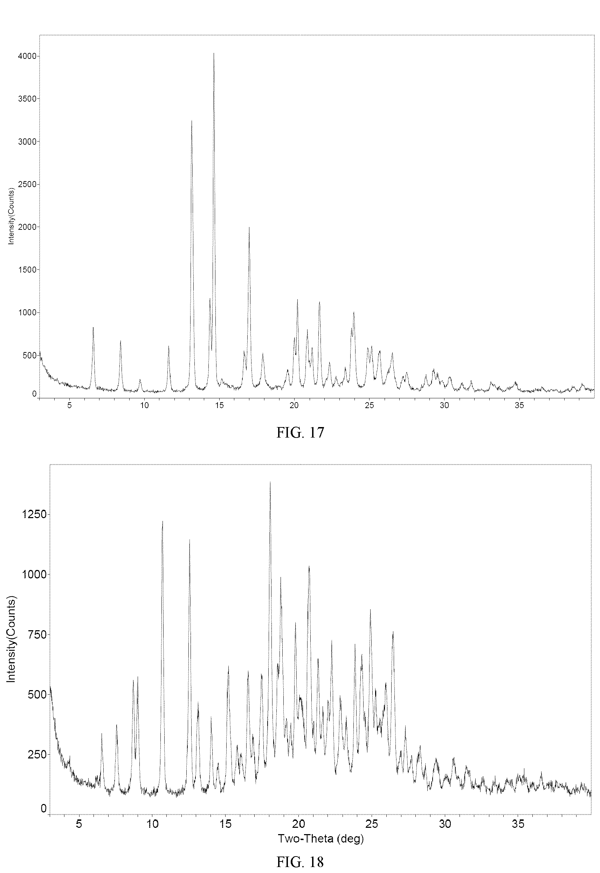

Preferably, crystal form P has an X-ray powder diffraction pattern substantially shown in FIG. 17.

Preferably, crystal form P exhibits an endothermic peak in a DSC analysis when heated to a peak temperature of about 117.63 °C.

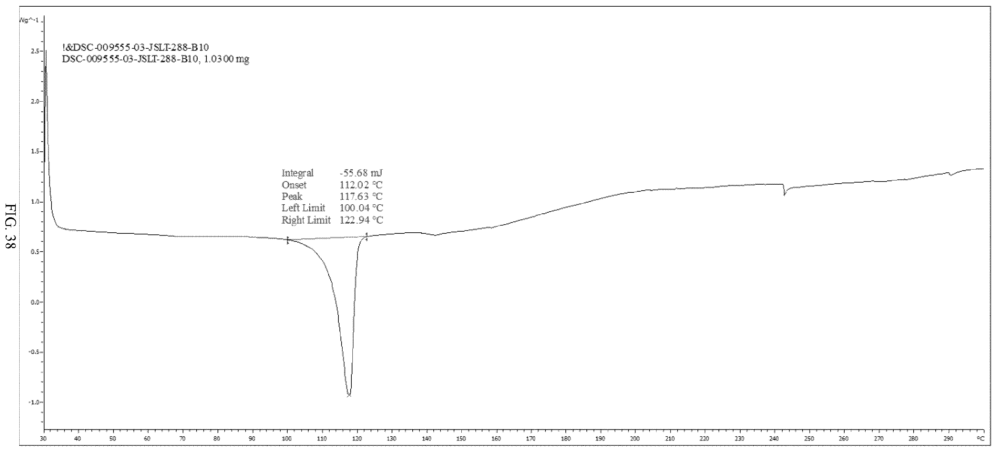

Preferably, crystal form P has a DSC profile substantially shown in FIG. 38.

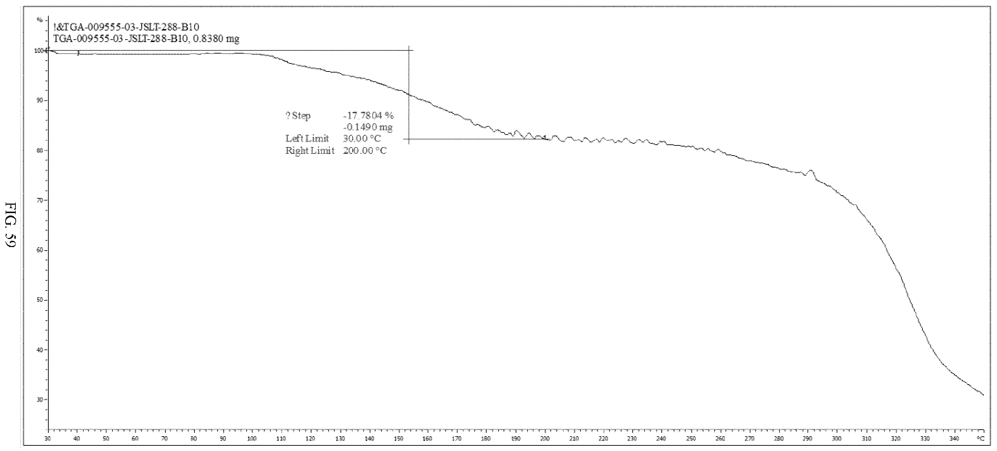

Preferably, crystal form P has a TGA profile substantially shown in FIG. 59.

Preferably, crystal form P is a N-methylpyrrolidone monosolvate of compound I.

Crystal form Q of a n-butanol solvate of compound I provided by the present disclosure has an X-ray powder diffraction pattern comprising peaks at diffraction angles (2θ) of 18.05 ± 0.2°, 10.71 ± 0.2°, 12.54 ± 0.2°, and 18.78 ± 0.2°;
preferably, further comprising peaks at diffraction angles (2θ) of 20.68 ± 0.2°, 24.89 ± 0.2°, 26.45 ± 0.2°, 22.24 ± 0.2°, and 19.81 ± 0.2°;
and more preferably, further comprising peaks at diffraction angles (2θ) of 15.21 ± 0.2°, 9.00 ± 0.2°, 8.70 ± 0.2°, 23.84 ± 0.2°, 16.55 ± 0.2°, and 24.31 ± 0.2°.

Preferably, crystal form Q has an X-ray powder diffraction pattern comprising diffraction angles (2θ) shown in Table 18, wherein the 2θ angles have a margin of error of ±0.20°:

**Table 18**

| 2θ (°) | Intensity% | 2θ (°) | Intensity% |
|---|---|---|---|
| 4.35 | 5.1 | 21.99 | 19.9 |
| 6.25 | 4.2 | 22.24 | 44.3 |
| 6.54 | 20.3 | 22.81 | 27.0 |
| 7.57 | 24.8 | 23.25 | 19.5 |
| 8.70 | 40.6 | 23.84 | 41.0 |
| 9.00 | 42.5 | 24.31 | 35.9 |
| 10.71 | 99.7 | 24.89 | 46.4 |
| 12.54 | 92.2 | 25.25 | 21.1 |
| 13.13 | 32.2 | 25.96 | 20.7 |
| 14.02 | 26.7 | 26.45 | 46.6 |
| 14.50 | 9.3 | 26.99 | 7.5 |
| 15.21 | 42.6 | 27.29 | 17.7 |
| 15.81 | 12.8 | 27.70 | 8.2 |
| 16.07 | 8.7 | 28.30 | 13.3 |
| 16.55 | 38.2 | 28.67 | 7.4 |
| 16.87 | 14.7 | 29.43 | 9.8 |
| 17.45 | 34.1 | 30.13 | 4.7 |
| 18.05 | 100.0 | 30.62 | 10.5 |
| 18.58 | 33.3 | 31.44 | 7.4 |
| 18.78 | 64.5 | 32.08 | 3.2 |
| 19.19 | 11.3 | 32.60 | 4.1 |
| 19.47 | 9.5 | 33.42 | 5.0 |
| 19.81 | 44.9 | 34.24 | 4.2 |
| 20.14 | 18.4 | 35.10 | 5.4 |
| 20.68 | 62.5 | 36.58 | 6.6 |
| 21.01 | 6.9 | 37.58 | 3.8 |
| 21.31 | 32.2 | 39.31 | 4.3 |
| 21.64 | 17.5 | | |

Preferably, crystal form Q has X-ray powder diffraction intensities shown in Table 18.

Preferably, crystal form Q has an X-ray powder diffraction pattern substantially shown in FIG. 18.

Preferably, crystal form Q exhibits an endothermic peak in a DSC analysis when heated to a peak temperature of about 100.61 °C.

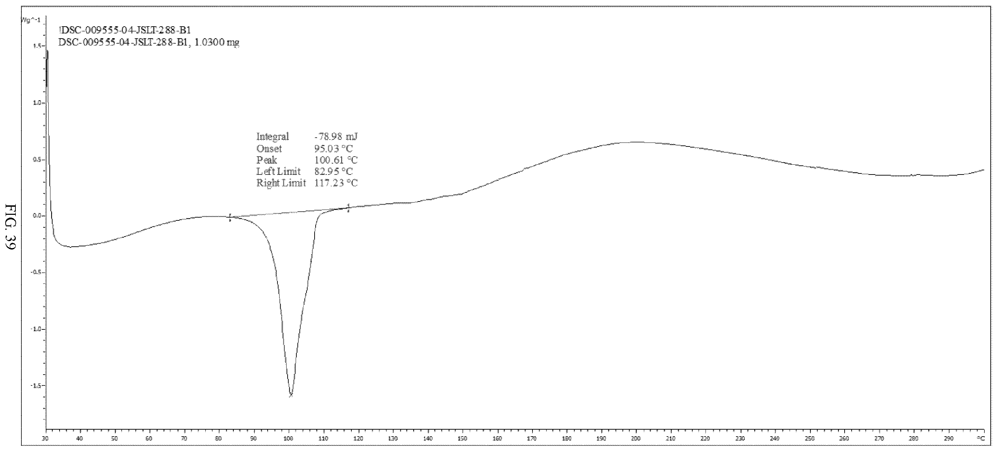

Preferably, crystal form Q has a DSC profile substantially shown in FIG. 39.

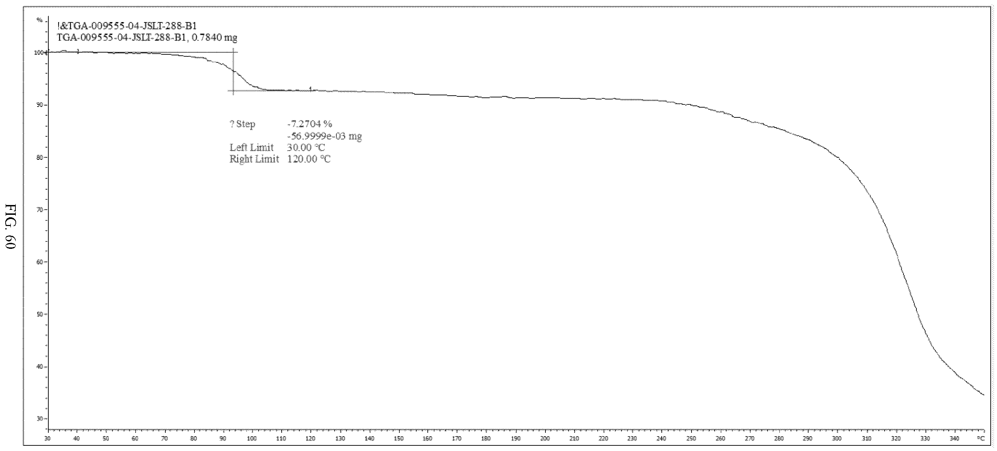

Preferably, crystal form Q has a TGA profile substantially shown in FIG. 60.

Preferably, crystal form Q is a n-butanol 0.5-solvate of compound I.

Crystal form R of a n-propanol solvate of compound I provided by the present disclosure has an X-ray powder diffraction pattern comprising peaks at diffraction angles (2θ) of 10.67 ± 0.2°, 12.52 ± 0.2°, 18.08 ± 0.2°, and 8.98 ± 0.2°;
preferably, further comprising peaks at diffraction angles (2θ) of 6.54 ± 0.2°, 20.68 ± 0.2°, 18.84 ± 0.2°, 7.55 ± 0.2°, and 8.67 ± 0.2°;
and more preferably, further comprising peaks at diffraction angles (2θ) of 19.85 ± 0.2°, 26.59 ± 0.2°, 15.19 ± 0.2°, 13.05 ± 0.2°, 14.06 ± 0.2°, and 16.55 ± 0.2°.

Preferably, crystal form R has an X-ray powder diffraction pattern comprising diffraction angles (2θ) shown in Table 19, wherein the 2θ angles have a margin of error of ±0.20°:

**Table 19**

| 2θ (°) | Intensity% | 2θ (°) | Intensity% |
|---|---|---|---|
| 6.17 | 7.0 | 21.35 | 8.7 |
| 6.54 | 46.7 | 21.70 | 11.4 |
| 7.55 | 34.4 | 22.13 | 7.9 |
| 8.67 | 35.2 | 22.89 | 8.6 |
| 8.98 | 53.5 | 23.19 | 3.8 |
| 10.67 | 100.0 | 23.72 | 5.3 |
| 12.52 | 75.8 | 24.27 | 10.5 |
| 13.05 | 20.8 | 24.91 | 11.0 |
| 14.06 | 20.6 | 25.49 | 9.4 |
| 14.45 | 8.0 | 25.96 | 6.5 |
| 15.19 | 23.9 | 26.59 | 26.2 |
| 16.55 | 20.0 | 27.12 | 3.2 |
| 16.94 | 4.1 | 28.38 | 9.5 |
| 17.45 | 16.9 | 29.51 | 3.4 |
| 18.08 | 57.5 | 30.79 | 2.4 |
| 18.53 | 17.8 | 31.57 | 3.5 |
| 18.84 | 52.1 | 33.45 | 3.1 |
| 19.24 | 14.9 | 35.46 | 2.8 |
| 19.85 | 34.7 | 38.18 | 2.7 |
| 20.68 | 43.4 | | |

Preferably, crystal form R has X-ray powder diffraction intensities shown in Table 19.

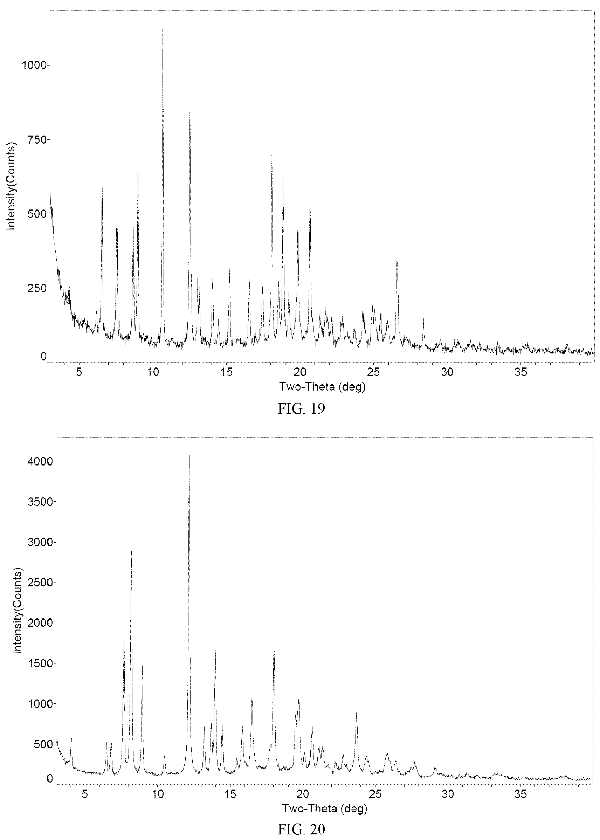

Preferably, crystal form R has an X-ray powder diffraction pattern substantially shown in FIG. 19.

Preferably, crystal form R exhibits an endothermic peak in a DSC analysis when heated to a peak temperature of about 110.90 °C.

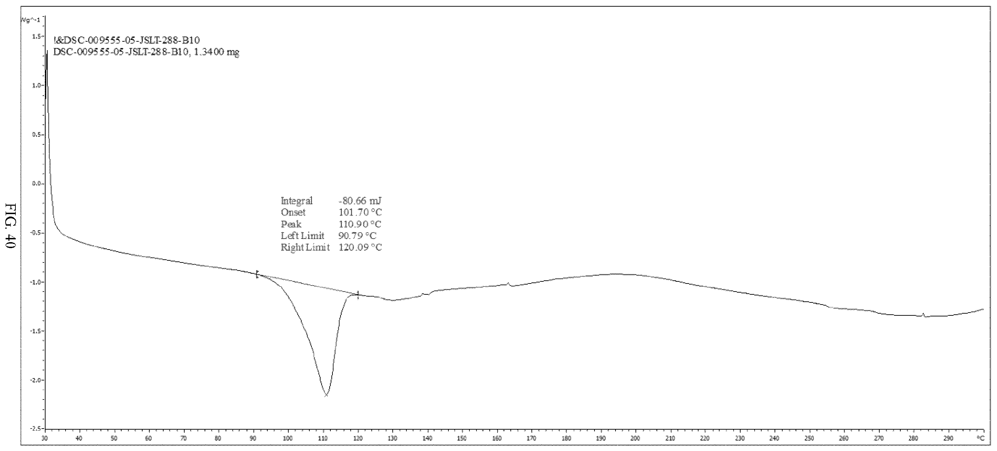

Preferably, crystal form R has a DSC profile substantially shown in FIG. 40.

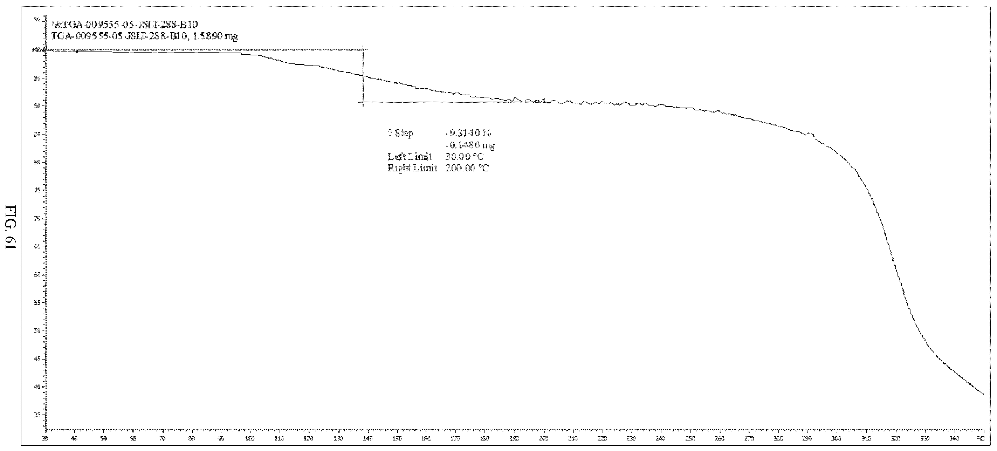

Preferably, crystal form R has a TGA profile substantially shown in FIG. 61.

Preferably, crystal form R is a n-propanol monosolvate of compound I.

Crystal form S of a tetrahydrofuran solvate of compound I provided by the present disclosure has an X-ray powder diffraction pattern comprising peaks at diffraction angles (2θ) of 12.17 ± 0.2°, 8.19 ± 0.2°, 7.67 ± 0.2°, and 13.96 ± 0.2°;
preferably, further comprising peaks at diffraction angles (2θ) of 8.95 ± 0.2°, 18.01 ± 0.2°, 16.50 ± 0.2°, 19.71 ± 0.2°, and 23.70 ± 0.2°;
and more preferably, further comprising peaks at diffraction angles (2θ) of 13.69 ± 0.2°, 13.22 ± 0.2°, 14.45 ± 0.2°, 20.64 ± 0.2°, 15.83 ± 0.2°, and 6.48 ± 0.2°.

Preferably, crystal form S has an X-ray powder diffraction pattern comprising diffraction angles (2θ) shown in Table 20, wherein the 2θ angles have a margin of error of ±0.20°:

**Table 20**

| 2θ (°) | Intensity% | 2θ (°) | Intensity% |
|---|---|---|---|
| 4.07 | 8.4 | 20.12 | 4.4 |
| 6.48 | 10.0 | 20.64 | 12.7 |
| 6.81 | 9.6 | 21.11 | 7.1 |
| 7.67 | 42.3 | 21.35 | 6.9 |
| 8.19 | 69.0 | 21.74 | 1.4 |
| 8.95 | 33.3 | 22.26 | 3.1 |
| 10.46 | 6.0 | 22.79 | 5.6 |
| 12.17 | 100.0 | 23.00 | 2.2 |
| 13.22 | 13.4 | 23.70 | 18.4 |
| 13.69 | 15.3 | 24.37 | 5.3 |
| 13.96 | 38.4 | 25.27 | 1.1 |
| 14.45 | 14.2 | 25.82 | 6.2 |
| 15.43 | 3.3 | 26.37 | 4.4 |
| 15.83 | 14.5 | 27.70 | 4.6 |
| 16.50 | 22.3 | 29.10 | 3.1 |
| 17.76 | 7.6 | 30.81 | 1.1 |
| 18.01 | 37.6 | 31.30 | 1.9 |
| 19.71 | 21.4 | 31.94 | 0.8 |

Preferably, crystal form S has X-ray powder diffraction intensities shown in Table 20.

Preferably, crystal form S has an X-ray powder diffraction pattern substantially shown in FIG. 20.

Preferably, crystal form S exhibits an endothermic peak in a DSC analysis when heated to a peak temperature of about 96.26 °C.

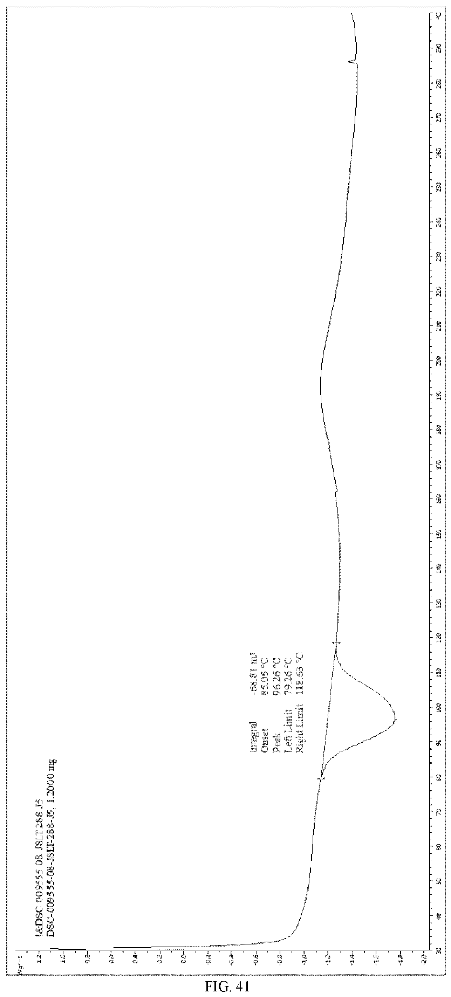

Preferably, crystal form S has a DSC profile substantially shown in FIG. 41.

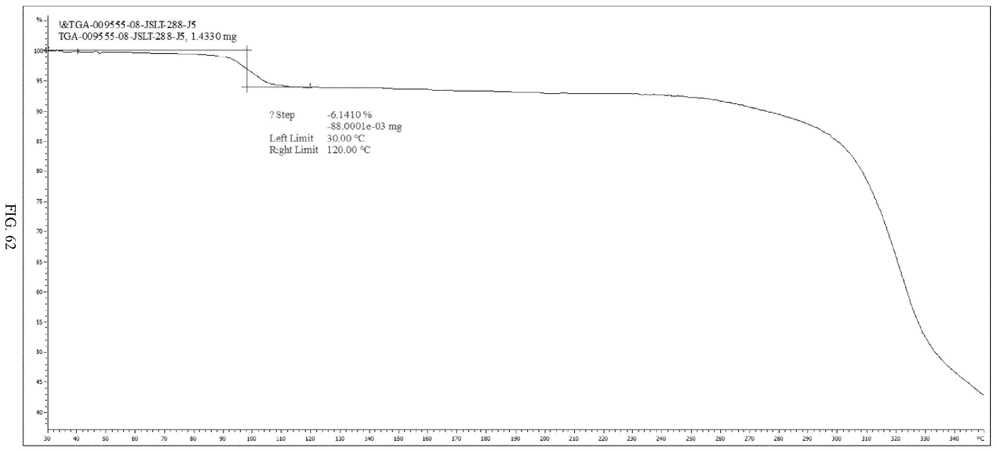

Preferably, crystal form S has a TGA profile substantially shown in FIG. 62.

Preferably, crystal form S is a tetrahydrofuran 0.5-solvate of compound I.

Crystal form T of a 2-methyltetrahydrofuran solvate of compound I provided by the present disclosure has an X-ray powder diffraction pattern comprising peaks at diffraction angles (2θ) of 10.79 ± 0.2°, 8.66 ± 0.2°, 9.12 ± 0.2°, and 16.87 ± 0.2°;
preferably, further comprising peaks at diffraction angles (2θ) of 18.64 ± 0.2°, 15.54 ± 0.2°, 21.27 ± 0.2°, 13.57 ± 0.2°, and 6.72 ± 0.2°;
and more preferably, further comprising peaks at diffraction angles (2θ) of 4.29 ± 0.2°, 14.41 ± 0.2°, 19.99 ± 0.2°, 7.71 ± 0.2°, 16.57 ± 0.2°, and 19.42 ± 0.2°.

Preferably, crystal form T has an X-ray powder diffraction pattern comprising diffraction angles (2θ) shown in Table 21, wherein the 2θ angles have a margin of error of ±0.20°:

**Table 21**

| 2θ (°) | Intensity% | 2θ (°) | Intensity% |
|---|---|---|---|
| 4.29 | 15.2 | 19.42 | 10.6 |
| 6.72 | 16.5 | 19.99 | 14.9 |
| 7.32 | 1.7 | 20.47 | 8.1 |
| 7.71 | 12.7 | 20.71 | 5.8 |
| 8.66 | 71.9 | 21.27 | 18.7 |
| 9.12 | 52.0 | 21.87 | 7.7 |
| 9.62 | 1.9 | 22.30 | 5.0 |
| 10.79 | 100.0 | 22.85 | 3.5 |
| 12.62 | 16.1 | 23.62 | 3.4 |
| 13.05 | 9.5 | 24.14 | 1.4 |
| 13.57 | 19.5 | 25.03 | 2.4 |
| 14.41 | 15.3 | 25.95 | 8.9 |
| 15.54 | 24.9 | 26.22 | 5.7 |
| 16.57 | 10.8 | 26.70 | 2.8 |
| 16.87 | 48.4 | 27.44 | 9.1 |
| 17.45 | 8.1 | 28.17 | 1.9 |
| 17.82 | 4.1 | 29.37 | 1.8 |
| 18.64 | 26.4 | 31.50 | 2.9 |

Preferably, crystal form T has X-ray powder diffraction intensities shown in Table 21.

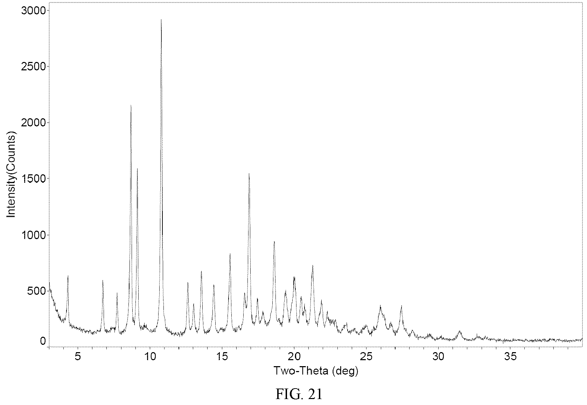

Preferably, crystal form T has an X-ray powder diffraction pattern substantially shown in FIG. 21.

Preferably, crystal form T exhibits an endothermic peak in a DSC analysis when heated to a peak temperature of about 112.95 °C.

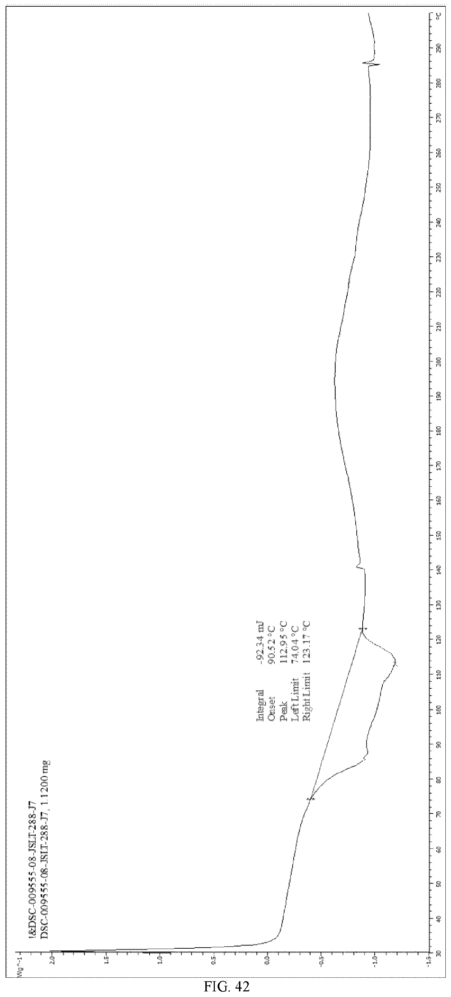

Preferably, crystal form T has a DSC profile substantially shown in FIG. 42.

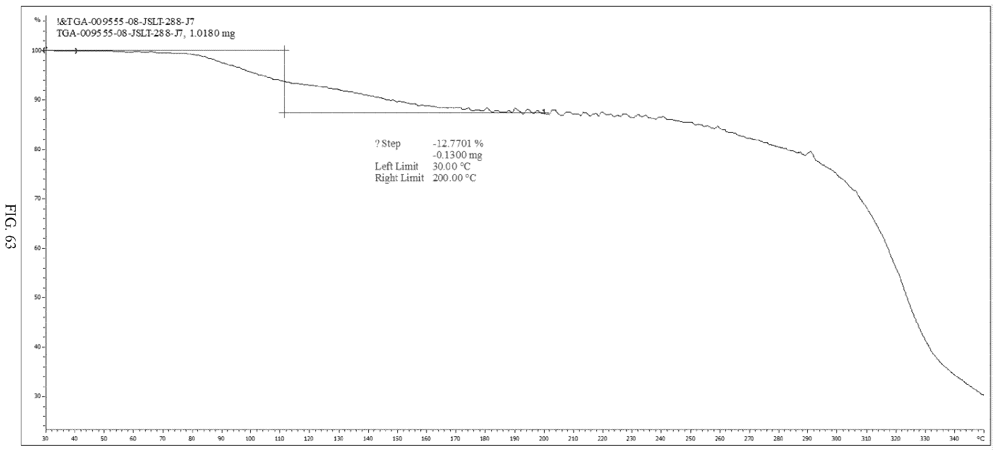

Preferably, crystal form T has a TGA profile substantially shown in FIG. 63.

Preferably, crystal form T is a 2-methyltetrahydrofuran monosolvate of compound I.

The crystal form of a non-solvate of compound II may be the following crystal form 2A, 2B, 2C, 2D, or 2E; the crystal form of a hydrate of compound II may be the following crystal form 2F or 2G; the crystal form of a solvate of compound II may be the following crystal form 2H, 2I, 2J-1, 2J-2, 2K, 2L-1, 2L-2, 2M-1, 2M-2, 2N, 2O, 2P, 2Q-1, 2Q-2, 2R, 2S, 2T, or 2U.

Crystal form 2A of compound II provided by the present disclosure has an X-ray powder diffraction pattern comprising peaks at diffraction angles (2θ) of 13.94 ± 0.2°, 22.07 ± 0.2°, 17.96 ± 0.2°, and 17.57 ± 0.2°;
preferably, further comprising peaks at diffraction angles (2θ) of 13.36 ± 0.2°, 5.92 ± 0.2°, 12.33 ± 0.2°, 23.04 ± 0.2°, and 11.02 ± 0.2°;
and more preferably, further comprising peaks at diffraction angles (2θ) of 22.41 ± 0.2°, 7.42 ± 0.2°, 25.07 ± 0.2°, 27.00 ± 0.2°, 8.71 ± 0.2°, and 16.60 ± 0.2°.

Preferably, crystal form 2A has an X-ray powder diffraction pattern comprising diffraction angles (2θ) shown in Table 2-1, wherein the 2θ angles have a margin of error of ±0.20°:

**Table 2-1**

| 2θ (°) | Intensity% | 2θ (°) | Intensity% |
|---|---|---|---|
| 5.92 | 21.5 | 19.96 | 6.4 |
| 7.42 | 11.0 | 22.07 | 48.6 |
| 8.71 | 7.8 | 22.41 | 11.1 |
| 11.02 | 14.6 | 23.04 | 15.7 |
| 11.92 | 5.2 | 23.78 | 4.8 |
| 12.33 | 17.3 | 25.07 | 9.7 |
| 13.36 | 26.8 | 27.00 | 9.1 |
| 13.94 | 100.0 | 28.22 | 3.8 |
| 16.60 | 6.8 | 29.49 | 2.2 |
| 17.57 | 42.2 | 30.50 | 4.1 |
| 17.96 | 46.1 | 30.99 | 2.2 |

Preferably, crystal form 2A has X-ray powder diffraction intensities shown in Table 2-1.

Preferably, crystal form 2A has an X-ray powder diffraction pattern substantially shown in FIG. 2-1.

Preferably, crystal form 2A exhibits an endothermic peak in a DSC analysis when heated to a peak temperature of about 189.90 °C.

Preferably, crystal form 2A has a DSC profile substantially shown in FIG. 2-26.

Preferably, crystal form 2A has a TGA profile substantially shown in FIG. 2-51.

Crystal form 2B of compound II provided by the present disclosure has an X-ray powder diffraction pattern comprising peaks at diffraction angles (2θ) of 13.16 ± 0.2°, 21.31 ± 0.2°, 25.46 ± 0.2°, and 10.61 ± 0.2°;
preferably, further comprising peaks at diffraction angles (2θ) of 16.79 ± 0.2°, 20.99 ± 0.2°, 18.76 ± 0.2°, 19.59 ± 0.2°, and 9.71 ± 0.2°;
and more preferably, further comprising peaks at diffraction angles (2θ) of 23.72 ± 0.2°, 11.68 ± 0.2°, 23.92 ± 0.2°, 20.57 ± 0.2°, 16.48 ± 0.2°, and 20.23 ± 0.2°.

Preferably, crystal form 2B has an X-ray powder diffraction pattern comprising diffraction angles (2θ) shown in Table 2-2, wherein the 2θ angles have a margin of error of ±0.20°:

**Table 2-2**

| 2θ (°) | Intensity% | 2θ (°) | Intensity% |
|---|---|---|---|
| 9.71 | 59.9 | 19.59 | 62.8 |
| 10.01 | 13.3 | 20.23 | 31.0 |
| 10.61 | 92.7 | 20.57 | 32.2 |
| 11.68 | 35.6 | 20.99 | 81.9 |
| 12.31 | 14.3 | 21.31 | 96.0 |
| 12.71 | 21.4 | 22.16 | 21.4 |
| 13.16 | 100.0 | 23.72 | 47.6 |
| 14.64 | 27.4 | 23.92 | 33.5 |
| 15.87 | 7.1 | 24.56 | 26.4 |
| 16.48 | 31.2 | 25.46 | 95.2 |
| 16.79 | 82.7 | 26.45 | 20.4 |
| 17.15 | 19.1 | 28.30 | 27.7 |
| 17.96 | 25.4 | 29.00 | 10.8 |
| 18.76 | 74.8 | 29.64 | 29.5 |
| 19.02 | 13.9 | 31.51 | 8.7 |

Preferably, crystal form 2B has X-ray powder diffraction intensities shown in Table 2-2.

Preferably, crystal form 2B has an X-ray powder diffraction pattern substantially shown in FIG. 2-2.

Preferably, crystal form 2B exhibits an endothermic peak in a DSC analysis when heated to a peak temperature of about 181.06 °C.

Preferably, crystal form 2B has a DSC profile substantially shown in FIG. 2-27.

Preferably, crystal form 2B has a TGA profile substantially shown in FIG. 2-52.

Crystal form 2C of compound II provided by the present disclosure has an X-ray powder diffraction pattern comprising peaks at diffraction angles (2θ) of 13.63 ± 0.2°, 5.62 ± 0.2°, 17.12 ± 0.2°, and 17.68 ± 0.2°;
preferably, further comprising peaks at diffraction angles (2θ) of 11.94 ± 0.2°, 13.89 ± 0.2°, 22.65 ± 0.2°, 23.63 ± 0.2°, and 17.97 ± 0.2°;
and more preferably, further comprising peaks at diffraction angles (2θ) of 7.46 ± 0.2°, 27.52 ± 0.2°, 8.80 ± 0.2°, 25.15 ± 0.2°, 19.36 ± 0.2°, and 5.88 ± 0.2°.

Preferably, crystal form 2C has an X-ray powder diffraction pattern comprising diffraction angles (2θ) shown in Table 2-3, wherein the 2θ angles have a margin of error of ±0.20°:

**Table 2-3**

| 2θ (°) | Intensity% | 2θ (°) | Intensity% |
|---|---|---|---|
| 5.62 | 60.9 | 17.97 | 12.8 |
| 5.88 | 5.5 | 19.36 | 6.6 |
| 7.46 | 12.4 | 20.47 | 2.7 |
| 8.80 | 7.2 | 21.17 | 3.0 |
| 11.94 | 33.9 | 22.10 | 3.2 |
| 12.32 | 5.3 | 22.65 | 19.9 |
| 13.63 | 100.0 | 23.63 | 15.9 |
| 13.89 | 33.5 | 25.15 | 7.0 |
| 17.12 | 59.5 | 27.52 | 8.6 |
| 17.68 | 42.9 | 28.13 | 4.7 |

Preferably, crystal form 2C has X-ray powder diffraction intensities shown in Table 2-3.

Preferably, crystal form 2C has an X-ray powder diffraction pattern substantially shown in FIG. 2-3.

Preferably, crystal form 2C exhibits an endothermic peak in a DSC analysis when heated to a peak temperature of about 171.63 °C.

Preferably, crystal form 2C has a DSC profile substantially shown in FIG. 2-28.

Preferably, crystal form 2C has a TGA profile substantially shown in FIG. 2-53.

Crystal form 2D of compound II provided by the present disclosure has an X-ray powder diffraction pattern comprising peaks at diffraction angles (2θ) of 5.62 ± 0.2°, 12.01 ± 0.2°, 5.24 ± 0.2°, and 17.72 ± 0.2°;
preferably, further comprising peaks at diffraction angles (2θ) of 13.65 ± 0.2°, 17.16 ± 0.2°, 23.63 ± 0.2°, 19.30 ± 0.2°, and 16.43 ± 0.2°;
and more preferably, further comprising peaks at diffraction angles (2θ) of 15.85 ± 0.2°, 11.04 ± 0.2°, 7.50 ± 0.2°, 5.90 ± 0.2°, 22.18 ± 0.2°, and 22.65 ± 0.2°.

Preferably, crystal form 2D has an X-ray powder diffraction pattern comprising diffraction angles (2θ) shown in Table 2-4, wherein the 2θ angles have a margin of error of ±0.20°:

**Table 2-4**

| 2θ (°) | Intensity% | 2θ (°) | Intensity% |
|---|---|---|---|
| 5.24 | 88.2 | 17.72 | 87.0 |
| 5.62 | 100.0 | 18.98 | 32.3 |
| 5.90 | 42.9 | 19.30 | 61.5 |
| 7.50 | 43.5 | 20.12 | 21.1 |
| 11.04 | 48.4 | 20.49 | 23.0 |
| 12.01 | 90.1 | 21.63 | 12.4 |
| 13.06 | 24.8 | 22.18 | 41.0 |
| 13.65 | 83.9 | 22.43 | 33.5 |
| 14.32 | 32.3 | 22.65 | 34.2 |
| 15.85 | 53.4 | 22.65 | 34.2 |
| 16.43 | 54.7 | 23.63 | 75.2 |
| 17.16 | 77.6 | 27.59 | 21.7 |

Preferably, crystal form 2D has X-ray powder diffraction intensities shown in Table 2-4.

Preferably, crystal form 2D has an X-ray powder diffraction pattern substantially shown in FIG. 2-4.

Preferably, crystal form 2D exhibits an endothermic peak in a DSC analysis when heated to a peak temperature of about 168.79 °C.

Preferably, crystal form 2D has a DSC profile substantially shown in FIG. 2-29.

Preferably, crystal form 2D has a TGA profile substantially shown in FIG. 2-54.

Crystal form 2E of compound II provided by the present disclosure has an X-ray powder diffraction pattern comprising peaks at diffraction angles (2θ) of 17.96 ± 0.2°, 3.34 ± 0.2°, 5.63 ± 0.2°, and 13.63 ± 0.2°;
preferably, further comprising peaks at diffraction angles (2θ) of 17.14 ± 0.2°, 11.96 ± 0.2°, 12.19 ± 0.2°, 13.98 ± 0.2°, and 7.46 ± 0.2°;
and more preferably, further comprising peaks at diffraction angles (2θ) of 19.53 ± 0.2°, 22.14 ± 0.2°, 8.87 ± 0.2°, 19.32 ± 0.2°, 23.90 ± 0.2°, and 19.83 ± 0.2°.

Preferably, crystal form 2E has an X-ray powder diffraction pattern comprising diffraction angles (2θ) shown in Table 2-5, wherein the 2θ angles have a margin of error of ±0.20°:

**Table 2-5**

| 2θ (°) | Intensity% | 2θ (°) | Intensity% |
|---|---|---|---|
| 3.34 | *71.0* | 15.86 | 10.5 |
| 5.63 | 51.8 | 17.14 | 47.4 |
| 5.90 | 11.0 | 17.96 | 100.0 |
| 7.46 | 28.7 | 19.32 | 15.6 |
| 8.24 | 12.6 | 19.53 | 18.2 |
| 8.87 | 16.9 | 19.83 | 13.6 |
| 11.07 | 9.2 | 21.09 | 9.2 |
| 11.96 | 46.2 | 22.14 | 18.2 |
| 12.19 | 42.1 | 23.90 | 15.6 |
| 13.63 | 47.9 | 25.87 | 12.1 |
| 13.98 | 41.5 | 27.48 | 7.9 |

Preferably, crystal form 2E has X-ray powder diffraction intensities shown in Table 2-5.

Preferably, crystal form 2E has an X-ray powder diffraction pattern substantially shown in FIG. 2-5.

Preferably, crystal form 2E exhibits endothermic peaks in a DSC analysis when heated to peak temperatures of about 110.30 °C and about 169.27 °C.

Preferably, crystal form 2E has a DSC profile substantially shown in FIG. 2-30.

Preferably, crystal form 2E has a TGA profile substantially shown in FIG. 2-55.

Crystal form 2F of a hydrate of compound II provided by the present disclosure has an X-ray powder diffraction pattern comprising peaks at diffraction angles (2θ) of 19.51 ± 0.2°, 13.71 ± 0.2°, 14.29 ± 0.2°, and 18.09 ± 0.2°;
preferably, further comprising peaks at diffraction angles (2θ) of 6.42 ± 0.2°, 12.11 ± 0.2°, 24.93 ± 0.2°, 17.86 ± 0.2°, and 20.57 ± 0.2°;
and more preferably, further comprising peaks at diffraction angles (2θ) of 21.37 ± 0.2°, 13.34 ± 0.2°, 23.39 ± 0.2°, 25.15 ± 0.2°, 30.64 ± 0.2°, and 17.25 ± 0.2°.

Preferably, crystal form 2F has an X-ray powder diffraction pattern comprising diffraction angles (2θ) shown in Table 2-6, wherein the 2θ angles have a margin of error of ±0.20°:

**Table 2-6**

| 2θ (°) | Intensity% | 2θ (°) | Intensity% |
|---|---|---|---|
| 6.42 | 29.2 | 20.57 | 18.6 |
| 8.72 | 9.8 | 21.37 | 17.8 |
| 9.50 | 6.1 | 22.23 | 3.3 |
| 11.31 | 10.0 | 22.81 | 6.2 |
| 12.11 | 27.9 | 23.39 | 16.2 |
| 12.95 | 7.9 | 24.03 | 3.1 |
| 13.34 | 16.3 | 24.54 | 6.5 |
| 13.71 | 74.6 | 24.93 | 26.5 |
| 14.29 | 42.3 | 25.15 | 14.0 |
| 14.63 | 5.2 | 25.55 | 3.5 |
| 15.23 | 6.5 | 26.14 | 10.9 |
| 16.46 | 3.8 | 26.41 | 7.6 |
| 17.25 | 12.1 | 27.06 | 9.8 |
| 17.86 | 20.4 | 27.54 | 6.5 |
| 18.09 | 36.8 | 30.64 | 12.6 |
| 19.51 | 100.0 | 31.82 | 4.8 |
| 20.13 | 2.8 | | |

Preferably, crystal form 2F has X-ray powder diffraction intensities shown in Table 2-6.

Preferably, crystal form 2F has an X-ray powder diffraction pattern substantially shown in FIG. 2-6.

Preferably, crystal form 2F exhibits an endothermic peak in a DSC analysis when heated to a peak temperature of about 114.41 °C.

Preferably, crystal form 2F has a DSC profile substantially shown in FIG. 2-31.

Preferably, crystal form 2F has a TGA profile substantially shown in FIG. 2-56.

Preferably, crystal form 2F is a trihydrate of compound II.

Crystal form 2G of a hydrate of compound II provided by the present disclosure has an X-ray powder diffraction pattern comprising peaks at diffraction angles (2θ) of 17.45 ± 0.2°, 13.63 ± 0.2°, 5.61 ± 0.2°, and 5.24 ± 0.2°;
preferably, further comprising peaks at diffraction angles (2θ) of 13.89 ± 0.2°, 10.69 ± 0.2°, 11.78 ± 0.2°, 23.53 ± 0.2°, and 23.86 ± 0.2°;
and more preferably, further comprising peaks at diffraction angles (2θ) of 20.98 ± 0.2°, 27.54 ± 0.2°, 15.46 ± 0.2°, 22.52 ± 0.2°, 6.89 ± 0.2°, and 22.08 ± 0.2°.

Preferably, crystal form 2G has an X-ray powder diffraction pattern comprising diffraction angles (2θ) shown in Table 2-7, wherein the 2θ angles have a margin of error of ±0.20°:

**Table 2-7**

| 2θ (°) | Intensity% | 2θ (°) | Intensity% |
|---|---|---|---|
| 5.24 | 70.0 | 17.45 | 100.0 |
| 5.61 | 74.8 | 20.98 | 25.2 |
| 6.89 | 14.8 | 22.08 | 14.8 |
| 10.69 | 45.7 | 22.52 | 20.5 |
| 11.78 | 33.3 | 23.53 | 31.4 |
| 13.63 | 88.6 | 23.86 | 31.4 |
| 13.89 | 69.5 | 27.54 | 18.6 |
| 15.46 | 21.4 | | |

Preferably, crystal form 2G has X-ray powder diffraction intensities shown in Table 2-7.

Preferably, crystal form 2G has an X-ray powder diffraction pattern substantially shown in FIG. 2-7.

Preferably, crystal form 2G exhibits an endothermic peak in a DSC analysis when heated to a peak temperature of about 169.22 °C.

Preferably, crystal form 2G has a DSC profile substantially shown in FIG. 2-32.

Preferably, crystal form 2G has a TGA profile substantially shown in FIG. 2-57.

Preferably, crystal form 2G is a dihydrate of compound II.

Crystal form 2H of a dimethylsulfoxide solvate of compound II provided by the present disclosure has an X-ray powder diffraction pattern comprising peaks at diffraction angles (2θ) of 14.84 ± 0.2°, 13.42 ± 0.2°, 24.68 ± 0.2°, and 21.70 ± 0.2°;
preferably, further comprising peaks at diffraction angles (2θ) of 8.45 ± 0.2°, 24.46 ± 0.2°, 20.22 ± 0.2°, 17.22 ± 0.2°, and 3.23 ± 0.2°;
and more preferably, further comprising peaks at diffraction angles (2θ) of 14.61 ± 0.2°, 25.32 ± 0.2°, 15.56 ± 0.2°, 22.01 ± 0.2°, 18.52 ± 0.2°, and 21.21 ± 0.2°.

Preferably, crystal form 2H has an X-ray powder diffraction pattern comprising diffraction angles (2θ) shown in Table 2-8, wherein the 2θ angles have a margin of error of ±0.20°:

**Table 2-8**

| 2θ (°) | Intensity% | 2θ (°) | Intensity% |
|---|---|---|---|
| 3.23 | 23.8 | 19.59 | 7.1 |
| 6.64 | 9.9 | 19.84 | 5.6 |
| 7.36 | 4.9 | 20.22 | 26.8 |
| 8.45 | 29.6 | 20.78 | 7.4 |
| 10.03 | 3.7 | 21.21 | 13.2 |
| 11.51 | 6.5 | 21.70 | 29.9 |
| 13.42 | 51.0 | 22.01 | 15.5 |
| 14.61 | 19.5 | 22.41 | 6.8 |
| 14.84 | 100.0 | 22.61 | 3.7 |
| 15.56 | 16.7 | 23.00 | 8.2 |
| 16.63 | 5.9 | 24.46 | 28.3 |
| 17.22 | 25.6 | 24.68 | 30.4 |
| 17.71 | 8.5 | 25.32 | 19.5 |
| 18.52 | 14.5 | 28.24 | 6.2 |
| 18.72 | 8.7 | 28.90 | 6.2 |
| 19.15 | 8.2 | 31.43 | 4.5 |

Preferably, crystal form 2H has X-ray powder diffraction intensities shown in Table 2-8.

Preferably, crystal form 2H has an X-ray powder diffraction pattern substantially shown in FIG. 2-8.

Preferably, crystal form 2H exhibits an endothermic peak in a DSC analysis when heated to a peak temperature of about 118.06 °C.

Preferably, crystal form 2H has a DSC profile substantially shown in FIG. 2-33.

Preferably, crystal form 2H has a TGA profile substantially shown in FIG. 2-58.

Preferably, crystal form 2H is a dimethylsulfoxide monosolvate of compound II.

Crystal form 2I of a methyl *tert-butyl* ether solvate of compound II provided by the present disclosure has an X-ray powder diffraction pattern comprising peaks at diffraction angles (2θ) of 8.74 ± 0.2°, 10.94 ± 0.2°, 4.32 ± 0.2°, and 17.66 ± 0.2°;
preferably, further comprising peaks at diffraction angles (2θ) of 13.18 ± 0.2°, 9.25 ± 0.2°, 18.79 ± 0.2°, 17.12 ± 0.2°, and 12.79°;
and more preferably, further comprising peaks at diffraction angles (2θ) of 22.13 ± 0.2°, 25.90 ± 0.2°, 19.38 ± 0.2°, 20.90 ± 0.2°, 15.91 ± 0.2°, and 6.91 ± 0.2°.

Preferably, crystal form 2I has an X-ray powder diffraction pattern comprising diffraction angles (2θ) shown in Table 2-9, wherein the 2θ angles have a margin of error of ±0.20°:

**Table 2-9**

| 2θ (°) | Intensity% | 2θ (°) | Intensity% |
|---|---|---|---|
| 4.32 | 22.4 | 17.12 | 10.7 |
| 6.91 | 4.0 | 17.66 | 20.1 |
| 8.74 | 100.0 | 18.79 | 11.8 |
| 9.25 | 15.7 | 19.38 | 6.5 |
| 10.94 | 33.9 | 20.90 | 5.8 |
| 12.79 | 8.3 | 22.13 | 7.8 |
| 13.18 | 16.3 | 23.90 | 3.9 |
| 15.91 | 5.0 | 25.90 | 6.7 |

Preferably, crystal form 2I has X-ray powder diffraction intensities shown in Table 2-9.

Preferably, crystal form 2I has an X-ray powder diffraction pattern substantially shown in FIG. 2-9.

Preferably, crystal form 2I exhibits an endothermic peak in a DSC analysis when heated to a peak temperature of about 115.84 °C.

Preferably, crystal form 2I has a DSC profile substantially shown in FIG. 2-34.

Preferably, crystal form 2I has a TGA profile substantially shown in FIG. 2-59.

Preferably, crystal form 2I is a methyl *tert-butyl* ether 0.5-solvate of compound II.

Crystal form 2J-1 of a n-heptane solvate of compound II provided by the present disclosure has an X-ray powder diffraction pattern comprising peaks at diffraction angles (2θ) of 12.15 ± 0.2°, 8.91 ± 0.2°, 8.18 ± 0.2°, and 7.63 ± 0.2°;
preferably, further comprising peaks at diffraction angles (2θ) of 13.93 ± 0.2°, 17.94 ± 0.2°, 19.48 ± 0.2°, 23.72 ± 0.2°, and 15.81 ± 0.2°;
and more preferably, further comprising peaks at diffraction angles (2θ) of 14.37 ± 0.2°, 20.43 ± 0.2°, 6.75 ± 0.2°, 22.68 ± 0.2°, 13.60 ± 0.2°, and 27.41 ± 0.2°.

Preferably, crystal form 2J-1 has an X-ray powder diffraction pattern comprising diffraction angles (2θ) shown in Table 2-10, wherein the 2θ angles have a margin of error of ±0.20°:

**Table 2-10**

| 2θ (°) | Intensity% | 2θ (°) | Intensity% |
|---|---|---|---|
| 6.75 | 18.1 | 17.10 | 10.6 |
| 7.63 | 50.6 | 17.94 | 45.7 |
| 8.18 | 53.7 | 19.48 | 41.6 |
| 8.91 | 81.7 | 20.43 | 20.0 |
| 12.15 | 100.0 | 21.09 | 8.6 |
| 13.07 | 9.9 | 21.39 | 13.8 |
| 13.60 | 16.2 | 22.68 | 17.0 |
| 13.93 | 46.1 | 23.72 | 28.9 |
| 14.37 | 22.8 | 25.68 | 13.6 |
| 15.81 | 24.4 | 27.41 | 14.0 |
| 16.48 | 13.1 | | |

Preferably, crystal form 2J-1 has X-ray powder diffraction intensities shown in Table 2-10.

Preferably, crystal form 2J-1 has an X-ray powder diffraction pattern substantially shown in FIG. 2-10.

Preferably, crystal form 2J-1 exhibits an endothermic peak in a DSC analysis when heated to a peak temperature of about 103.13 °C.

Preferably, crystal form 2J-1 has a DSC profile substantially shown in FIG. 2-35.

Preferably, crystal form 2J-1 has a TGA profile substantially shown in FIG. 2-60.

Preferably, crystal form 2J-1 is a n-heptane 0.2-solvate of compound II.

Crystal form 2J-2 of a n-heptane solvate of compound II provided by the present disclosure has an X-ray powder diffraction pattern comprising peaks at diffraction angles (2θ) of 3.52 ± 0.2°, 17.76 ± 0.2°, 14.19 ± 0.2°, and 10.61 ± 0.2°;
preferably, further comprising peaks at diffraction angles (2θ) of 7.05 ± 0.2°, 21.35 ± 0.2°, 16.24 ± 0.2°, and 19.49 ± 0.2°;

Preferably, crystal form 2J-2 has an X-ray powder diffraction pattern comprising diffraction angles (2θ) shown in Table 2-11, wherein the 2θ angles have a margin of error of ±0.20°:

**Table 2-11**

| 2θ (°) | Intensity% | 2θ (°) | Intensity% |
|---|---|---|---|
| 3.52 | 100 | 16.24 | 6.1 |
| 7.05 | 22.5 | 17.76 | 76.6 |
| 10.61 | 30.2 | 19.49 | 4.8 |
| 14.19 | 41.8 | 21.35 | 12.7 |

Preferably, crystal form 2J-2 has X-ray powder diffraction intensities shown in Table 2-11.

Preferably, crystal form 2J-2 has an X-ray powder diffraction pattern substantially shown in FIG. 2-11.

Preferably, crystal form 2J-2 exhibits an endothermic peak in a DSC analysis when heated to a peak temperature of about 76.82 °C.

Preferably, crystal form 2J-2 has a DSC profile substantially shown in FIG. 2-36.

Preferably, crystal form 2J-2 has a TGA profile substantially shown in FIG. 2-61.

Preferably, crystal form 2J-2 is a n-heptane 0.5-solvate of compound II.

Crystal form 2K of a toluene solvate of compound II provided by the present disclosure has an X-ray powder diffraction pattern comprising peaks at diffraction angles (2θ) of 9.52 ± 0.2°, 18.45 ± 0.2°, 19.09 ± 0.2°, and 21.48 ± 0.2°;
preferably, further comprising peaks at diffraction angles (2θ) of 16.24 ± 0.2°, 14.76 ± 0.2°, 13.04 ± 0.2°, 22.11 ± 0.2°, and 3.50 ± 0.2°;
and more preferably, further comprising peaks at diffraction angles (2θ) of 18.23 ± 0.2°, 17.96 ± 0.2°, 26.57 ± 0.2°, 20.31 ± 0.2°, 14.53 ± 0.2°, and 23.76 ± 0.2°.

Preferably, crystal form 2K has an X-ray powder diffraction pattern comprising diffraction angles (2θ) shown in Table 2-12, wherein the 2θ angles have a margin of error of ±0.20°:

**Table 2-12**

| 2θ (°) | Intensity% | 2θ (°) | Intensity% |
|---|---|---|---|
| 3.50 | 23.4 | 19.36 | 17.2 |
| 5.62 | 11.7 | 20.31 | 20.5 |
| 7.73 | 8.2 | 20.51 | 16.6 |
| 9.52 | 100.0 | 21.05 | 4.3 |
| 10.46 | 4.9 | 21.48 | 47.9 |
| 11.15 | 16.8 | 22.11 | 31.7 |
| 11.75 | 6.0 | 22.48 | 11.3 |
| 11.94 | 7.3 | 23.76 | 17.3 |
| 13.04 | 31.7 | 24.15 | 15.1 |
| 13.63 | 19.5 | 24.44 | 17.1 |
| 13.85 | 5.5 | 25.36 | 15.6 |
| 14.76 | 35.1 | 25.78 | 3.4 |
| 15.71 | 3.5 | 26.31 | 6.0 |
| 16.24 | 45.5 | 26.57 | 20.8 |
| 17.12 | 11.5 | 27.19 | 7.8 |
| 17.69 | 13.7 | 27.77 | 4.1 |
| 17.96 | 22.0 | 28.96 | 4.1 |
| 18.23 | 23.2 | 29.51 | 6.4 |
| 18.45 | 70.8 | 29.88 | 4.8 |
| 18.80 | 17.0 | 31.76 | 4.7 |
| 19.09 | 49.3 | 33.54 | 5.0 |

Preferably, crystal form 2K has X-ray powder diffraction intensities shown in Table 2-12.

Preferably, crystal form 2K has an X-ray powder diffraction pattern substantially shown in FIG. 2-12.

Preferably, crystal form 2K exhibits endothermic peaks in a DSC analysis when heated to peak temperatures of about 107.92 °C, about 172.19 °C, and about 184.68 °C.

Preferably, crystal form 2K has a DSC profile substantially shown in FIG. 2-37.

Preferably, crystal form 2K has a TGA profile substantially shown in FIG. 2-62.

Preferably, crystal form 2K is a toluene 0.75-solvate of compound II.

Crystal form 2L-1 of a methyl isobutyl ketone solvate of compound II provided by the present disclosure has an X-ray powder diffraction pattern comprising peaks at diffraction angles (2θ) of 8.66 ± 0.2°, 10.79 ± 0.2°, 26.00 ± 0.2°, and 19.62 ± 0.2°;
preferably, further comprising peaks at diffraction angles (2θ) of 9.13 ± 0.2°, 18.60 ± 0.2°, 16.90 ± 0.2°, 15.58 ± 0.2°, and 19.43 ± 0.2°;
and more preferably, further comprising peaks at diffraction angles (2θ) of 13.03 ± 0.2°, 17.43 ± 0.2°, 6.74 ± 0.2°, 26.72 ± 0.2°, 26.27 ± 0.2°, and 22.40 ± 0.2°.

Preferably, crystal form 2L-1 has an X-ray powder diffraction pattern comprising diffraction angles (2θ) shown in Table 2-13, wherein the 2θ angles have a margin of error of ±0.20°:

**Table 2-13**

| 2θ (°) | Intensity% | 2θ (°) | Intensity% |
|---|---|---|---|
| 4.28 | 9.0 | 18.60 | 32.9 |
| 6.40 | 13.3 | 19.43 | 24.2 |
| 6.74 | 21.4 | 19.62 | 40.4 |
| 7.71 | 7.4 | 20.04 | 9.6 |
| 8.66 | 100.0 | 20.71 | 10.6 |
| 9.13 | 39.7 | 20.94 | 11.7 |
| 10.79 | 77.4 | 21.32 | 12.5 |
| 12.64 | 16.5 | 21.62 | 10.6 |
| 13.03 | 23.1 | 21.91 | 11.7 |
| 13.60 | 12.5 | 22.40 | 19.1 |
| 14.04 | 9.2 | 22.88 | 8.1 |
| 14.51 | 12.1 | 23.59 | 14.1 |
| 14.91 | 8.2 | 24.03 | 9.2 |
| 15.58 | 24.7 | 26.00 | 41.4 |
| 16.54 | 8.9 | 26.27 | 19.5 |
| 16.90 | 31.7 | 26.72 | 20.3 |
| 17.43 | 21.7 | 27.44 | 16.6 |
| 17.84 | 6.2 | | |

Preferably, crystal form 2L-1 has X-ray powder diffraction intensities shown in Table 2-13.

Preferably, crystal form 2L-1 has an X-ray powder diffraction pattern substantially shown in FIG. 2-13.

Preferably, crystal form 2L-1 exhibits an endothermic peak in a DSC analysis when heated to a peak temperature of about 106.45 °C.

Preferably, crystal form 2L-1 has a DSC profile substantially shown in FIG. 2-38.

Preferably, crystal form 2L-1 has a TGA profile substantially shown in FIG. 2-63.

Preferably, crystal form 2L-1 is a methyl isobutyl ketone 0.75-solvate of compound II.

Crystal form 2L-2 of a methyl isobutyl ketone solvate of compound II provided by the present disclosure has an X-ray powder diffraction pattern comprising peaks at diffraction angles (2θ) of 8.68 ± 0.2°, 10.83 ± 0.2°, 13.10 ± 0.2°, and 11.06 ± 0.2°;
preferably, further comprising peaks at diffraction angles (2θ) of 17.49 ± 0.2°, 9.15 ± 0.2°, 16.96 ± 0.2°, 18.69 ± 0.2°, and 6.74 ± 0.2°;
and more preferably, further comprising peaks at diffraction angles (2θ) of 12.62 ± 0.2°, 19.63 ± 0.2°, 19.37 ± 0.2°, 22.01 ± 0.2°, 15.65 ± 0.2°, and 21.33 ± 0.2°.

Preferably, crystal form 2L-2 has an X-ray powder diffraction pattern comprising diffraction angles (2θ) shown in Table 2-14, wherein the 2θ angles have a margin of error of ±0.20°:

**Table 2-14**

| 2θ (°) | Intensity% | 2θ (°) | Intensity% |
|---|---|---|---|
| 6.74 | 12.1 | 17.49 | 23.3 |
| 8.68 | 100.0 | 18.69 | 13.8 |
| 9.15 | 17.9 | 19.37 | 8.9 |
| 10.83 | 41.7 | 19.63 | 9.4 |
| 11.06 | 28.2 | 20.90 | 6.2 |
| 12.62 | 11.6 | 21.33 | 8.0 |
| 13.10 | 29.5 | 22.01 | 8.7 |
| 15.65 | 8.5 | 22.41 | 7.2 |
| 16.96 | 14.8 | 27.52 | 5.4 |

Preferably, crystal form 2L-2 has X-ray powder diffraction intensities shown in Table 2-14.

Preferably, crystal form 2L-2 has an X-ray powder diffraction pattern substantially shown in FIG. 2-14.

Preferably, crystal form 2L-2 exhibits an endothermic peak in a DSC analysis when heated to a peak temperature of about 102.87 °C.

Preferably, crystal form 2L-2 has a DSC profile substantially shown in FIG. 2-39.

Preferably, crystal form 2L-2 has a TGA profile substantially shown in FIG. 2-64.

Preferably, crystal form 2L-2 is a methyl isobutyl ketone 0.3-solvate of compound II.

Crystal form 2M-1 of a cyclopentyl methyl ether solvate of compound II provided by the present disclosure has an X-ray powder diffraction pattern comprising peaks at diffraction angles (2θ) of 8.78 ± 0.2°, 10.98 ± 0.2°, 17.67 ± 0.2°, and 17.20 ± 0.2°;
preferably, further comprising peaks at diffraction angles (2θ) of 19.32 ± 0.2°, 25.69 ± 0.2°, 13.23 ± 0.2°, 22.24 ± 0.2°, and 9.29 ± 0.2°;
and more preferably, further comprising peaks at diffraction angles (2θ) of 18.93 ± 0.2°, 26.53 ± 0.2°, 26.04 ± 0.2°, 15.87 ± 0.2°, 21.25 ± 0.2°, and 12.85 ± 0.2°.

Preferably, crystal form 2M-1 has an X-ray powder diffraction pattern comprising diffraction angles (2θ) shown in Table 2-15, wherein the 2θ angles have a margin of error of ±0.20°:

**Table 2-15**

| 2θ (°) | Intensity% | 2θ (°) | Intensity% |
|---|---|---|---|
| 5.18 | 6.5 | 17.67 | 28.0 |
| 6.48 | 4.0 | 18.93 | 20.2 |
| 6.85 | 8.9 | 19.32 | 27.7 |
| 8.78 | 100.0 | 20.36 | 4.3 |
| 9.29 | 22.4 | 20.65 | 8.9 |
| 10.98 | 43.6 | 21.25 | 10.9 |
| 12.85 | 9.8 | 22.24 | 22.8 |
| 13.23 | 23.4 | 23.20 | 5.3 |
| 13.63 | 7.1 | 23.84 | 9.1 |
| 13.88 | 7.2 | 25.69 | 27.4 |
| 14.70 | 3.8 | 26.04 | 15.9 |
| 15.42 | 6.6 | 26.53 | 17.2 |
| 15.87 | 12.0 | 27.21 | 9.0 |
| 16.48 | 3.6 | 28.00 | 3.6 |
| 17.20 | 27.7 | | |

Preferably, crystal form 2M-1 has X-ray powder diffraction intensities shown in Table 2-15.

Preferably, crystal form 2M-1 has an X-ray powder diffraction pattern substantially shown in FIG. 2-15.

Preferably, crystal form 2M-1 exhibits an endothermic peak in a DSC analysis when heated to a peak temperature of about 113.56 °C.

Preferably, crystal form 2M-1 has a DSC profile substantially shown in FIG. 2-40.

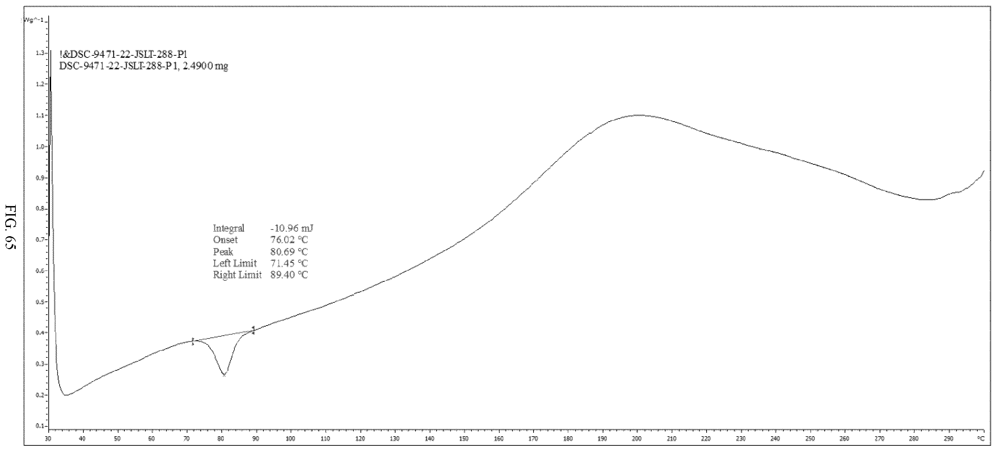

Preferably, crystal form 2M-1 has a TGA profile substantially shown in FIG. 2-65.

Preferably, crystal form 2M-1 is a cyclopentyl methyl ether monosolvate of compound II.

Crystal form 2M-2 of a cyclopentyl methyl ether solvate of compound II provided by the present disclosure has an X-ray powder diffraction pattern comprising peaks at diffraction angles (2θ) of 5.16 ± 0.2°, 10.83 ± 0.2°, 15.66 ± 0.2°, and 21.29 ± 0.2°;
preferably, further comprising peaks at diffraction angles (2θ) of 17.63 ± 0.2°, 17.90 ± 0.2°, 8.80 ± 0.2°, 13.03 ± 0.2°, and 23.88 ± 0.2°;
and more preferably, further comprising peaks at diffraction angles (2θ) of 25.73 ± 0.2°, 11.99 ± 0.2°, 19.28 ± 0.2°, 20.20 ± 0.2°, 23.57 ± 0.2°, and 22.20 ± 0.2°.

Preferably, crystal form 2M-2 has an X-ray powder diffraction pattern comprising diffraction angles (2θ) shown in Table 2-16, wherein the 2θ angles have a margin of error of ±0.20°:

**Table 2-16**

| 2θ (°) | Intensity% | 2θ (°) | Intensity% |
|---|---|---|---|
| 5.16 | 100.0 | 19.28 | 39.3 |
| 7.01 | 23.1 | 19.77 | 13.6 |
| 8.80 | 65.2 | 20.20 | 35.4 |
| 10.83 | 95.3 | 21.29 | 74.1 |
| 11.99 | 42.3 | 21.68 | 16.4 |
| 13.03 | 62.4 | 22.207 | 33.4 |
| 13.26 | 21.4 | 22.50 | 24.0 |
| 14.10 | 27.3 | 23.57 | 34.5 |
| 15.66 | 83.0 | 23.88 | 49.9 |
| 17.23 | 26.2 | 25.73 | 48.5 |
| 17.63 | 73.8 | 26.53 | 15.3 |
| 17.90 | 68.2 | 27.39 | 17.5 |
| 18.95 | 32.6 | 29.93 | 21.7 |

Preferably, crystal form 2M-2 has X-ray powder diffraction intensities shown in Table 2-16.

Preferably, crystal form 2M-2 has an X-ray powder diffraction pattern substantially shown in FIG. 2-16.

Preferably, crystal form 2M-2 exhibits endothermic peaks in a DSC analysis when heated to peak temperatures of about 110.50 °C and about 165.01 °C.

Preferably, crystal form 2M-2 has a DSC profile substantially shown in FIG. 2-41.

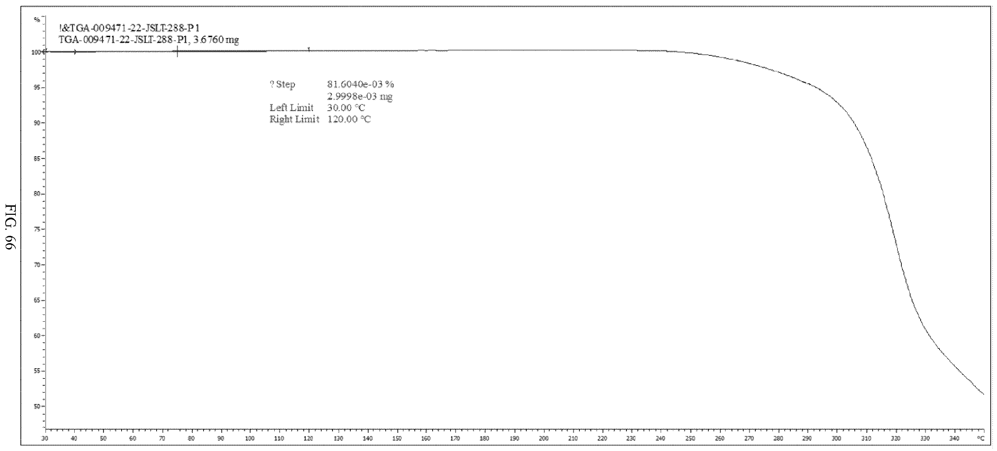

Preferably, crystal form 2M-2 has a TGA profile substantially shown in FIG. 2-66.

Preferably, crystal form 2M-2 is a cyclopentyl methyl ether monosolvate of compound II.

Crystal form 2N of a methyl ethyl ketone solvate of compound II provided by the present disclosure has an X-ray powder diffraction pattern comprising peaks at diffraction angles (2θ) of 3.34 ± 0.2°, 10.88 ± 0.2°, 9.13 ± 0.2°, and 6.64 ± 0.2°;
preferably, further comprising peaks at diffraction angles (2θ) of 7.73 ± 0.2°, 19.87 ± 0.2°, 8.78 ± 0.2°, 12.78 ± 0.2°, and 6.25 ± 0.2°;
and more preferably, further comprising peaks at diffraction angles (2θ) of 18.29 ± 0.2°, 19.08 ± 0.2°, 21.19 ± 0.2°, 11.69 ± 0.2°, 19.49 ± 0.2°, and 17.01 ± 0.2°.

Preferably, crystal form 2N has an X-ray powder diffraction pattern comprising diffraction angles (2θ) shown in Table 2-17, wherein the 2θ angles have a margin of error of ±0.20°:

**Table 2-17**

| 2θ (°) | Intensity% | 2θ (°) | Intensity% |
|---|---|---|---|
| 3.34 | 100.0 | 12.78 | 27.7 |
| 6.25 | 25.7 | 17.01 | 19.0 |
| 6.64 | 57.7 | 18.29 | 25.3 |
| 7.73 | 45.1 | 19.08 | 24.1 |
| 8.78 | 33.2 | 19.49 | 19.8 |
| 9.13 | 87.7 | 19.87 | 35.2 |
| 10.88 | 97.2 | 21.19 | 21.7 |
| 11.69 | 20.2 | | |

Preferably, crystal form 2N has X-ray powder diffraction intensities shown in Table 2-17.

Preferably, crystal form 2N has an X-ray powder diffraction pattern substantially shown in FIG. 2-17.

Preferably, crystal form 2N exhibits endothermic peaks in a DSC analysis when heated to peak temperatures of about 102.66 °C and about 113.16 °C.

Preferably, crystal form 2N has a DSC profile substantially shown in FIG. 2-42.

Preferably, crystal form 2N has a TGA profile substantially shown in FIG. 2-67.

Preferably, crystal form 2N is a methyl ethyl ketone 0.3-solvate of compound II.

Crystal form 2O of a methylcyclohexane solvate of compound II provided by the present disclosure has an X-ray powder diffraction pattern comprising peaks at diffraction angles (2θ) of 8.72 ± 0.2°, 10.92 ± 0.2°, 13.14 ± 0.2°, and 9.23 ± 0.2°;
preferably, further comprising peaks at diffraction angles (2θ) of 17.10 ± 0.2°, 18.78 ± 0.2°, 17.55 ± 0.2°, 15.79 ± 0.2°, and 6.89 ± 0.2°;
and more preferably, further comprising peaks at diffraction angles (2θ) of 22.05 ± 0.2°, 12.77 ± 0.2°, 19.30 ± 0.2°, 20.84 ± 0.2°, 23.74 ± 0.2°, and 26.45 ± 0.2°.

Preferably, crystal form 2O has an X-ray powder diffraction pattern comprising diffraction angles (2θ) shown in Table 2-18, wherein the 2θ angles have a margin of error of ±0.20°:

**Table 2-18**

| 2θ (°) | Intensity% | 2θ (°) | Intensity% |
|---|---|---|---|
| 6.49 | 5.1 | 16.70 | 3.5 |
| 6.89 | 10.9 | 17.10 | 14.8 |
| 7.92 | 4.3 | 17.55 | 12.4 |
| 8.72 | 100.0 | 18.78 | 14.2 |
| 9.23 | 18.1 | 19.30 | 7.8 |
| 10.92 | 36.2 | 20.25 | 3.3 |
| 12.77 | 9.6 | 20.84 | 6.9 |
| 13.14 | 24.8 | 21.65 | 3.1 |
| 13.80 | 4.7 | 22.05 | 10.6 |
| 14.70 | 3.8 | 23.74 | 6.8 |
| 15.79 | 11.4 | 26.45 | 5.5 |

Preferably, crystal form 2O has X-ray powder diffraction intensities shown in Table 2-18.

Preferably, crystal form 2O has an X-ray powder diffraction pattern substantially shown in FIG. 2-18.

Preferably, crystal form 2O exhibits an endothermic peak in a DSC analysis when heated to a peak temperature of about 111.80 °C.

Preferably, crystal form 2O has a DSC profile substantially shown in FIG. 2-43.

Preferably, crystal form 2O has a TGA profile substantially shown in FIG. 2-68.

Preferably, crystal form 2O is a methylcyclohexane monosolvate of compound II.

Crystal form 2P of a DMF solvate of compound II provided by the present disclosure has an X-ray powder diffraction pattern comprising peaks at diffraction angles (2θ) of 10.98 ± 0.2°, 9.31 ± 0.2°, 17.68 ± 0.2°, and 25.67 ± 0.2°;
preferably, further comprising peaks at diffraction angles (2θ) of 19.65 ± 0.2°, 5.78 ± 0.2°, 6.24 ± 0.2°, 16.54 ± 0.2°, and 21.19 ± 0.2°;
and more preferably, further comprising peaks at diffraction angles (2θ) of 24.09 ± 0.2°, 18.25 ± 0.2°, 25.26 ± 0.2°, 20.67 ± 0.2°, 18.84 ± 0.2°, and 22.19 ± 0.2°.

Preferably, crystal form 2P has an X-ray powder diffraction pattern comprising diffraction angles (2θ) shown in Table 2-19, wherein the 2θ angles have a margin of error of ±0.20°:

**Table 2-19**

| 2θ (°) | Intensity% | 2θ (°) | Intensity% |
|---|---|---|---|
| 5.78 | 21 | 18.56 | 9.1 |
| 6.24 | 20.3 | 18.84 | 13.1 |
| 9.31 | 69.8 | 19.65 | 29.4 |
| 10.98 | 100.0 | 20.67 | 13.5 |
| 11.63 | 11.0 | 21.19 | 16.9 |
| 11.88 | 9.4 | 22.19 | 11.1 |
| 12.56 | 8.8 | 24.09 | 16.9 |
| 14.93 | 8.9 | 24.48 | 8.6 |
| 16.54 | 20.2 | 25.26 | 14.6 |
| 17.68 | 49.9 | 25.67 | 31.3 |
| 18.25 | 15.8 | 27.95 | 6.7 |

Preferably, crystal form 2P has X-ray powder diffraction intensities shown in Table 2-19.

Preferably, crystal form 2P has an X-ray powder diffraction pattern substantially shown in FIG. 2-19.

Preferably, crystal form 2P exhibits an endothermic peak in a DSC analysis when heated to a peak temperature of about 105.14 °C.

Preferably, crystal form 2P has a DSC profile substantially shown in FIG. 2-44.

Preferably, crystal form 2P has a TGA profile substantially shown in FIG. 2-69.

Preferably, crystal form 2P is a DMF monosolvate of compound II.

Crystal form 2Q-1 of a 2-methyltetrahydrofuran solvate of compound II provided by the present disclosure has an X-ray powder diffraction pattern comprising peaks at diffraction angles (2θ) of 8.74 ± 0.2°, 10.89 ± 0.2°, 3.53 ± 0.2°, and 17.90 ± 0.2°;
preferably, further comprising peaks at diffraction angles (2θ) of 18.77 ± 0.2°, 17.65 ± 0.2°, 16.93 ± 0.2°, 7.54 ± 0.2°, and 9.19 ± 0.2°;
and more preferably, further comprising peaks at diffraction angles (2θ) of 13.18 ± 0.2°, 8.45 ± 0.2°, 9.63 ± 0.2°, 22.07 ± 0.2°, 6.76 ± 0.2°, and 12.81 ± 0.2°.

Preferably, crystal form 2Q-1 has an X-ray powder diffraction pattern comprising diffraction angles (2θ) shown in Table 2-20, wherein the 2θ angles have a margin of error of ±0.20°:

**Table 2-20**

| 2θ (°) | Intensity% | 2θ (°) | Intensity% |
|---|---|---|---|
| 3.53 | 40.6 | 13.18 | 16.7 |
| 6.76 | 10.9 | 14.28 | 8.6 |
| 7.11 | 7.9 | 15.69 | 7.9 |
| 7.54 | 17.6 | 16.24 | 9.2 |
| 8.45 | 15.7 | 16.93 | 20.9 |
| 8.74 | 100.0 | 17.65 | 21.3 |
| 9.19 | 17.6 | 17.90 | 28.5 |
| 9.63 | 12.6 | 18.77 | 21.8 |
| 10.89 | 61.5 | 20.83 | 9.2 |
| 12.81 | 10.9 | 22.07 | 12.3 |

Preferably, crystal form 2Q-1 has X-ray powder diffraction intensities shown in Table 2-20.

Preferably, crystal form 2Q-1 has an X-ray powder diffraction pattern substantially shown in FIG. 2-20. Preferably, crystal form 2Q-1 exhibits an endothermic peak in a DSC analysis when heated to a peak temperature of about 111.13 °C.

Preferably, crystal form 2Q-1 has a DSC profile substantially shown in FIG. 2-45.

Preferably, crystal form 2Q-1 has a TGA profile substantially shown in FIG. 2-70.

Preferably, crystal form 2Q-1 is a 2-methyltetrahydrofuran monosolvate of compound II.

Crystal form 2Q-2 of a 2-methyltetrahydrofuran solvate of compound II provided by the present disclosure has an X-ray powder diffraction pattern comprising peaks at diffraction angles (2θ) of 8.76 ± 0.2°, 10.89 ± 0.2°, 9.21 ± 0.2°, and 17.02 ± 0.2°;
preferably, further comprising peaks at diffraction angles (2θ) of 18.80 ± 0.2°, 6.79 ± 0.2°, 19.65 ± 0.2°, 17.63 ± 0.2°, and 26.08 ± 0.2°;
and more preferably, further comprising peaks at diffraction angles (2θ) of 13.18 ± 0.2°, 15.68 ± 0.2°, 4.34 ± 0.2°, 13.70 ± 0.2°, 12.74 ± 0.2°, and 21.46 ± 0.2°.

Preferably, crystal form 2Q-2 has an X-ray powder diffraction pattern comprising diffraction angles (2θ) shown in Table 2-21, wherein the 2θ angles have a margin of error of ±0.20°:

**Table 2-21**

| 2θ (°) | Intensity% | 2θ (°) | Intensity% |
|---|---|---|---|
| 4.34 | 18.6 | 18.58 | 9.0 |
| 6.44 | 11.4 | 18.80 | 30.6 |
| 6.79 | 26.3 | 19.65 | 23.4 |
| 7.79 | 5.7 | 20.21 | 6.6 |
| 8.76 | 100.0 | 20.64 | 5.9 |
| 9.21 | 42.7 | 20.97 | 7.0 |
| 10.89 | 85.3 | 21.46 | 11.7 |
| 12.74 | 12.7 | 22.08 | 11.7 |
| 13.18 | 19.8 | 22.48 | 10.0 |
| 13.70 | 13.6 | 23.066 | 5.4 |
| 14.53 | 8.7 | 24.17 | 4.0 |
| 15.68 | 19.3 | 26.08 | 20.2 |
| 16.75 | 11.3 | 26.81 | 8.0 |
| 17.02 | 36.1 | 27.62 | 9.2 |
| 17.63 | 22.9 | 18.58 | 9.0 |

Preferably, crystal form 2Q-2 has X-ray powder diffraction intensities shown in Table 2-21.

Preferably, crystal form 2Q-2 has an X-ray powder diffraction pattern substantially shown in FIG. 2-21.

Preferably, crystal form 2Q-2 exhibits an endothermic peak in a DSC analysis when heated to a peak temperature of about 114.18 °C.

Preferably, crystal form 2Q-2 has a DSC profile substantially shown in FIG. 2-46.

Preferably, crystal form 2Q-2 has a TGA profile substantially shown in FIG. 2-71.

Preferably, crystal form 2Q-2 is a 2-methyltetrahydrofuran monosolvate of compound II.

Crystal form 2R of a N-methylpyrrolidone solvate of compound II provided by the present disclosure has an X-ray powder diffraction pattern comprising peaks at diffraction angles (2θ) of 10.79 ± 0.2°, 9.15 ± 0.2°, 3.44 ± 0.2°, and 17.63 ± 0.2°;
preferably, further comprising peaks at diffraction angles (2θ) of 25.83 ± 0.2°, 19.26 ± 0.2°, 20.86 ± 0.2°, 16.76 ± 0.2°, and 25.48 ± 0.2°;
and more preferably, further comprising peaks at diffraction angles (2θ) of 16.51 ± 0.2°, 9.55 ± 0.2°, 17.18 ± 0.2°, 19.54 ± 0.2°, 14.08 ± 0.2°, and 7.34 ± 0.2°.

Preferably, crystal form 2R has an X-ray powder diffraction pattern comprising diffraction angles (2θ) shown in Table 2-22, wherein the 2θ angles have a margin of error of ±0.20°:

**Table 2-22**

| 2θ (°) | Intensity% | 2θ (°) | Intensity% |
|---|---|---|---|
| 3.44 | 62.9 | 16.76 | 33.3 |
| 6.11 | 13.9 | 17.18 | 22.9 |
| 6.68 | 12.9 | 17.63 | 59.4 |
| 7.01 | 13.9 | 17.97 | 14.3 |
| 7.34 | 21.4 | 18.41 | 20.4 |
| 8.33 | 13.7 | 19.26 | 41.6 |
| 9.15 | 77.6 | 19.54 | 22.0 |
| 9.55 | 24.3 | 20.47 | 10.2 |
| 10.79 | 100.0 | 20.86 | 37.1 |
| 11.43 | 14.7 | 21.49 | 13.1 |
| 11.66 | 14.7 | 22.21 | 16.1 |
| 12.31 | 11.8 | 23.23 | 18.4 |
| 13.78 | 16.7 | 23.80 | 18.8 |
| 14.08 | 21.6 | 24.09 | 14.5 |
| 14.67 | 17.6 | 25.48 | 28.4 |
| 15.36 | 8.4 | 25.83 | 49.0 |
| 16.51 | 26.1 | 27.19 | 16.9 |

Preferably, crystal form 2R has X-ray powder diffraction intensities shown in Table 2-22.

Preferably, crystal form 2R has an X-ray powder diffraction pattern substantially shown in FIG. 2-22.

Preferably, crystal form 2R exhibits an endothermic peak in a DSC analysis when heated to a peak temperature of about 128.45 °C.

Preferably, crystal form 2R has a DSC profile substantially shown in FIG. 2-47.

Preferably, crystal form 2R has a TGA profile substantially shown in FIG. 2-72.

Preferably, crystal form 2R is a N-methylpyrrolidone monosolvate of compound II.

Crystal form 2S of a trifluoroethanol solvate of compound II provided by the present disclosure has an X-ray powder diffraction pattern comprising peaks at diffraction angles (2θ) of 17.64 ± 0.2°, 4.01 ± 0.2°, 8.72 ± 0.2°, and 8.28 ± 0.2°;
preferably, further comprising peaks at diffraction angles (2θ) of 13.63 ± 0.2°, 10.89 ± 0.2°, 12.17 ± 0.2°, 19.65 ± 0.2°, and 23.65 ± 0.2°;
and more preferably, further comprising peaks at diffraction angles (2θ) of 26.08 ± 0.2°, 15.8 ± 0.2°, 21.35 ± 0.2°, 7.61 ± 0.2°, 22.49 ± 0.2°, and 5.61 ± 0.2°.

Preferably, crystal form 2S has an X-ray powder diffraction pattern comprising diffraction angles (2θ) shown in Table 2-23, wherein the 2θ angles have a margin of error of ±0.20°:

**Table 2-23**

| 2θ (°) | Intensity% | 2θ (°) | Intensity% |
|---|---|---|---|
| 4.01 | 60.0 | 15.81 | 26.3 |
| 5.61 | 16.9 | 17.64 | 100.0 |
| 7.61 | 21.9 | 19.65 | 34.4 |
| 8.28 | 54.7 | 21.35 | 24.4 |
| 8.72 | 57.2 | 22.49 | 17.5 |
| 10.89 | 41.6 | 23.65 | 34.1 |
| 12.17 | 39.4 | 26.08 | 29.4 |
| 13.63 | 42.5 | | |

Preferably, crystal form 2S has X-ray powder diffraction intensities shown in Table 2-23.

Preferably, crystal form 2S has an X-ray powder diffraction pattern substantially shown in FIG. 2-23.

Preferably, crystal form 2S exhibits endothermic peaks in a DSC analysis when heated to peak temperatures of about 123.79 °C and about 169.39 °C.

Preferably, crystal form 2S has a DSC profile substantially shown in FIG. 2-48.

Preferably, crystal form 2S has a TGA profile substantially shown in FIG. 2-73.

Preferably, crystal form 2S is a trifluoroethanol monosolvate of compound II.

Crystal form 2T of a tetrahydrofuran solvate of compound II provided by the present disclosure has an X-ray powder diffraction pattern comprising peaks at diffraction angles (2θ) of 12.17 ± 0.2°, 8.92 ± 0.2°, 7.63 ± 0.2°, and 8.22 ± 0.2°;
preferably, further comprising peaks at diffraction angles (2θ) of 13.99 ± 0.2°, 17.96 ± 0.2°, 9.27 ± 0.2°, 19.51 ± 0.2°, and 13.57 ± 0.2°;
and more preferably, further comprising peaks at diffraction angles (2θ) of 11.10 ± 0.2°, 15.89 ± 0.2°, 14.33 ± 0.2°, 19.77 ± 0.2°, 20.39 ± 0.2°, and 16.61 ± 0.2°.

Preferably, crystal form 2T has an X-ray powder diffraction pattern comprising diffraction angles (2θ) shown in Table 2-24, wherein the 2θ angles have a margin of error of ±0.20°:

**Table 2-24**

| 2θ (°) | Intensity% | 2θ (°) | Intensity% |
|---|---|---|---|
| 4.51 | 14.5 | 15.38 | 13.2 |
| 6.37 | 10.5 | 15.89 | 25.4 |
| 6.70 | 16.3 | 16.61 | 19.9 |
| 7.63 | 57.2 | 17.96 | 35.8 |
| 8.22 | 39.2 | 19.51 | 28.9 |
| 8.92 | 75.5 | 19.77 | 22.2 |
| 9.27 | 28.9 | 20.39 | 21.8 |
| 10.51 | 8.6 | 21.15 | 12.2 |
| 11.10 | 26.2 | 21.55 | 10.9 |
| 12.17 | 100.0 | 22.80 | 12.8 |
| 13.05 | 16.3 | 23.78 | 18.5 |
| 13.57 | 28.5 | 25.98 | 10.3 |
| 13.99 | 38.8 | 27.38 | 7.3 |
| 14.33 | 22.4 | | |

Preferably, crystal form 2T has X-ray powder diffraction intensities shown in Table 2-24.

Preferably, crystal form 2T has an X-ray powder diffraction pattern substantially shown in FIG. 2-24.

Preferably, crystal form 2T exhibits an endothermic peak in a DSC analysis when heated to a peak temperature of about 110.50 °C.

Preferably, crystal form 2T has a DSC profile substantially shown in FIG. 2-49.

Preferably, crystal form 2T has a TGA profile substantially shown in FIG. 2-74.

Preferably, crystal form 2T is a tetrahydrofuran 0.3-solvate of compound II.

Crystal form 2U of a dioxane solvate of compound II provided by the present disclosure has an X-ray powder diffraction pattern comprising peaks at diffraction angles (2θ) of 8.86 ± 0.2°, 10.98 ± 0.2°, 9.27 ± 0.2°, and 17.12 ± 0.2°;
preferably, further comprising peaks at diffraction angles (2θ) of 19.05 ± 0.2°, 19.55 ± 0.2°, 15.70 ± 0.2°, 13.36 ± 0.2°, and 6.79 ± 0.2°;
and more preferably, further comprising peaks at diffraction angles (2θ) of 25.85 ± 0.2°, 12.87 ± 0.2°, 22.48 ± 0.2°, 13.69 ± 0.2°, 4.39 ± 0.2°, and 17.86 ± 0.2°.

Preferably, crystal form 2U has an X-ray powder diffraction pattern comprising diffraction angles (2θ) shown in Table 2-25, wherein the 2θ angles have a margin of error of ±0.20°:

**Table 2-25**

| 2θ (°) | Intensity% | 2θ (°) | Intensity% |
|---|---|---|---|
| 4.39 | 9.6 | 15.70 | 19.5 |
| 6.35 | 8.3 | 17.12 | 39.0 |
| 6.79 | 16 | 17.86 | 9.1 |
| 7.77 | 6.3 | 19.05 | 21.8 |
| 8.86 | 100.0 | 19.55 | 21.1 |
| 9.27 | 44.9 | 20.36 | 6.8 |
| 10.98 | 71.6 | 20.70 | 8.5 |
| 12.87 | 13.2 | 21.07 | 7.3 |
| 13.36 | 17.0 | 21.48 | 8.0 |
| 13.69 | 12.3 | 22.48 | 12.6 |
| 14.59 | 8.6 | 25.85 | 15.5 |

Preferably, crystal form 2U has X-ray powder diffraction intensities shown in Table 2-25.

Preferably, crystal form 2U has an X-ray powder diffraction pattern substantially shown in FIG. 2-25.

Preferably, crystal form 2U exhibits an endothermic peak in a DSC analysis when heated to a peak temperature of about 109.16 °C.

Preferably, crystal form 2U has a DSC profile substantially shown in FIG. 2-50.

Preferably, crystal form 2U has a TGA profile substantially shown in FIG. 2-75.

Preferably, crystal form 2U is a dioxane 0.5-solvate of compound II.

In a second aspect of the present disclosure, provided is a method for preparing the polymorph of compound III described above, selected from:
method I, comprising: step 1, dissolving or dispersing compound III in a solvent; and step 2, stirring at 0-50 °C for crystallization, or adding an antisolvent into the clarified solution of the compound for crystallization, or slowly evaporating the clarified solution of the compound;
method II, comprising: dispersing compound III in a solvent or an atmosphere of the media to give a crystal; and
method III, comprising: combining method I and method II to give the polymorph of compound III.

As a further preferred embodiment, the solvent is water, an organic solvent, or a mixed solvent thereof, and the organic solvent is selected from alcohols, chloroalkanes, ketones, ethers, cyclic ethers, esters, alkanes, cycloalkanes, benzenoids, amides, sulfoxides, and a mixture thereof; preferably, the organic solvent is selected from methanol, ethanol, *n*-propanol, isopropanol, *n*-butanol, trifluoroethanol, acetonitrile, acetone, methyl ethyl ketone, methyl isobutyl ketone, 1,4-dioxane, tetrahydrofuran, 2-methyltetrahydrofuran, *N,N-dimethylformamide, N,N-dimethylacetamide, N*-methylpyrrolidone, dimethylsulfoxide, ethyl acetate, isopropyl acetate, dichloromethane, trichloroethane, tetrachloromethane, methyl *tert*-butyl ether, 2-methoxyethyl ether, isopropyl ether, diethyl ether, *n*-heptane, *n*-hexane, isooctane, pentane, cyclohexane, cyclopentane, methylcyclohexane, benzene, toluene, xylene, and a mixture thereof.

In a third aspect of the present disclosure, provided is a pharmaceutical composition, comprising at least one of the polymorphs of compound III described above and a pharmaceutically acceptable carrier.

In a fourth aspect of the present disclosure, provided is use of the polymorph of compound III described above for manufacturing a medicament for the treatment of a metabolic disease, tumor, autoimmune disease, or metastatic disease.

In a fifth aspect of the present disclosure, provided is the polymorph of compound III described above for use as a medicament for the treatment of a metabolic disease, tumor, autoimmune disease, or metastatic disease.

In a sixth aspect of the present disclosure, provided is the polymorph of compound III described above for use as a medicament for the prevention or treatment of T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, or polycystic ovary syndrome, and the treatment of addiction.

As a preferred embodiment, the polymorph of compound III described above is for use as a medicament for the treatment of T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, obesity, eating disorders, weight gain caused by use of other medicaments, excessive sugar craving, dyslipidemia, or hyperinsulinemia.

The present disclosure further provides a method for the treatment of a disease, comprising: administering to an individual in need a therapeutically effective amount of at least one of the polymorph of compound III or the pharmaceutical composition described above.

According to the embodiments of the present disclosure, the disease is selected from a metabolic disease, a tumor, an autoimmune disease, and a metastatic disease.

According to the embodiments of the present disclosure, the disease is selected from T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, and polycystic ovary syndrome.

### Beneficial Effects

The present disclosure provides a polymorph of compound III and a method for preparing same. Compared with amorphous forms, the obtained polymorph has the advantages of good stability, good flowability, and easy pulverization, making it more suitable for the development of clinical formulations. The method provided by the present disclosure features simplified processes, ease of implementation, mild conditions for reaction, and high product yields. Moreover, the present disclosure does not require excessive purification procedures and features safe and environment-friendly operations, thus favoring the industrial production of polymorphs. The present disclosure also meets the requirements of the development of clinical pharmaceutical formulations, has very important clinical application value, and is expected to accelerate the development of a new-generation GLP-1R small-molecular agonist.

### BRIEF DESCRIPTION OF THE DRAWINGS

FIG. 1 illustrates the X-ray powder diffraction pattern of crystal form A of compound I of the present disclosure.
The abscissa denotes 2θ values (degrees), and the ordinate denotes peak intensity.
FIG. 2 illustrates the X-ray powder diffraction pattern of crystal form B of compound I of the present disclosure.
The abscissa denotes 2θ values (degrees), and the ordinate denotes peak intensity.
FIG. 3 illustrates the X-ray powder diffraction pattern of crystal form C of compound I of the present disclosure. The abscissa denotes 2θ values (degrees), and the ordinate denotes peak intensity.
FIG. 4 illustrates the X-ray powder diffraction pattern of crystal form D of compound I of the present disclosure. The abscissa denotes 2θ values (degrees), and the ordinate denotes peak intensity.
FIG. 5 illustrates the X-ray powder diffraction pattern of crystal form E of compound I of the present disclosure.
The abscissa denotes 2θ values (degrees), and the ordinate denotes peak intensity.
FIG. 6 illustrates the X-ray powder diffraction pattern of crystal form F of compound I of the present disclosure. The abscissa denotes 2θ values (degrees), and the ordinate denotes peak intensity.
FIG. 7 illustrates the X-ray powder diffraction pattern of crystal form G of a hydrate of compound I of the present disclosure. The abscissa denotes 2θ values (degrees), and the ordinate denotes peak intensity.
FIG. 8 illustrates the X-ray powder diffraction pattern of crystal form H of a methyl isobutyl ketone solvate of compound I of the present disclosure. The abscissa denotes 2θ values (degrees), and the ordinate denotes peak intensity.
FIG. 9 illustrates the X-ray powder diffraction pattern of crystal form I-1 of a methyl *tert-*butyl ether solvate of compound I of the present disclosure. The abscissa denotes 2θ values (degrees), and the ordinate denotes peak intensity.
FIG. 10 illustrates the X-ray powder diffraction pattern of crystal form I-2 of a methyl *tert-*butyl ether solvate of compound I of the present disclosure. The abscissa denotes 2θ values (degrees), and the ordinate denotes peak intensity.
FIG. 11 illustrates the X-ray powder diffraction pattern of crystal form J of an acetone solvate of compound I of the present disclosure. The abscissa denotes 2θ values (degrees), and the ordinate denotes peak intensity.
FIG. 12 illustrates the X-ray powder diffraction pattern of crystal form K of a *n*-heptane solvate of compound I of the present disclosure. The abscissa denotes 2θ values (degrees), and the ordinate denotes peak intensity.
FIG. 13 illustrates the X-ray powder diffraction pattern of crystal form L of a methylcyclohexane solvate of compound I of the present disclosure. The abscissa denotes 2θ values (degrees), and the ordinate denotes peak intensity.
FIG. 14 illustrates the X-ray powder diffraction pattern of crystal form M of a toluene solvate of compound I of the present disclosure. The abscissa denotes 2θ values (degrees), and the ordinate denotes peak intensity.
FIG. 15 illustrates the X-ray powder diffraction pattern of crystal form N of a dioxane solvate of compound I of the present disclosure. The abscissa denotes 2θ values (degrees), and the ordinate denotes peak intensity.
FIG. 16 illustrates the X-ray powder diffraction pattern of crystal form O of a DMF solvate of compound I of the present disclosure. The abscissa denotes 2θ values (degrees), and the ordinate denotes peak intensity.
FIG. 17 illustrates the X-ray powder diffraction pattern of crystal form P of a *N*-methylpyrrolidone solvate of compound I of the present disclosure. The abscissa denotes 2θ values (degrees), and the ordinate denotes peak intensity.
FIG. 18 illustrates the X-ray powder diffraction pattern of crystal form Q of a *n*-butanol solvate of compound I of the present disclosure. The abscissa denotes 2θ values (degrees), and the ordinate denotes peak intensity.
FIG. 19 illustrates the X-ray powder diffraction pattern of crystal form R of a *n*-propanol solvate of compound I of the present disclosure. The abscissa denotes 2θ values (degrees), and the ordinate denotes peak intensity.
FIG. 20 illustrates the X-ray powder diffraction pattern of crystal form S of a tetrahydrofuran solvate of compound I of the present disclosure. The abscissa denotes 2θ values (degrees), and the ordinate denotes peak intensity.
FIG. 21 illustrates the X-ray powder diffraction pattern of crystal form T of a 2-methyltetrahydrofuran solvate of compound I of the present disclosure. The abscissa denotes 2θ values (degrees), and the ordinate denotes peak intensity.
FIG. 22 illustrates the DSC profile of crystal form A of compound I of the present disclosure. The abscissa denotes temperature (°C), and the ordinate denotes heat flow (mW).
FIG. 23 illustrates the DSC profile of crystal form B of compound I of the present disclosure. The abscissa denotes temperature (°C), and the ordinate denotes heat flow (mW).
FIG. 24 illustrates the DSC profile of crystal form C of compound I of the present disclosure. The abscissa denotes temperature (°C), and the ordinate denotes heat flow (mW).
FIG. 25 illustrates the DSC profile of crystal form D of compound I of the present disclosure. The abscissa denotes temperature (°C), and the ordinate denotes heat flow (mW).
FIG. 26 illustrates the DSC profile of crystal form E of compound I of the present disclosure. The abscissa denotes temperature (°C), and the ordinate denotes heat flow (mW).
FIG. 27 illustrates the DSC profile of crystal form F of compound I of the present disclosure. The abscissa denotes temperature (°C), and the ordinate denotes heat flow (mW).
FIG. 28 illustrates the DSC profile of crystal form G of a hydrate of compound I of the present disclosure. The abscissa denotes temperature (°C), and the ordinate denotes heat flow (mW).
FIG. 29 illustrates the DSC profile of crystal form H of a methyl isobutyl ketone solvate of compound I of the present disclosure. The abscissa denotes temperature (°C), and the ordinate denotes heat flow (mW).
FIG. 30 illustrates the DSC profile of crystal form I-1 of a methyl *tert-*butyl ether solvate of compound I of the present disclosure. The abscissa denotes temperature (°C), and the ordinate denotes heat flow (mW).
FIG. 31 illustrates the DSC profile of crystal form I-2 of a methyl *tert-*butyl ether solvate of compound I of the present disclosure. The abscissa denotes temperature (°C), and the ordinate denotes heat flow (mW).
FIG. 32 illustrates the DSC profile of crystal form J of an acetone solvate of compound I of the present disclosure. The abscissa denotes temperature (°C), and the ordinate denotes heat flow (mW).
FIG. 33 illustrates the DSC profile of crystal form K of a *n*-heptane solvate of compound I of the present disclosure. The abscissa denotes temperature (°C), and the ordinate denotes heat flow (mW).
FIG. 34 illustrates the DSC profile of crystal form L of a methylcyclohexane solvate of compound I of the present disclosure. The abscissa denotes temperature (°C), and the ordinate denotes heat flow (mW).
FIG. 35 illustrates the DSC profile of crystal form M of a toluene solvate of compound I of the present disclosure. The abscissa denotes temperature (°C), and the ordinate denotes heat flow (mW). FIG. 36 illustrates the DSC profile of crystal form N of a dioxane solvate of compound I of the present disclosure.
The abscissa denotes temperature (°C), and the ordinate denotes heat flow (mW). FIG. 37 illustrates the DSC profile of crystal form O of a DMF solvate of compound I of the present disclosure. The abscissa denotes temperature (°C), and the ordinate denotes heat flow (mW).
FIG. 38 illustrates the DSC profile of crystal form P of a N-methylpyrrolidone solvate of compound I of the present disclosure. The abscissa denotes temperature (°C), and the ordinate denotes heat flow (mW).
FIG. 39 illustrates the DSC profile of crystal form Q of a n-butanol solvate of compound I of the present disclosure. The abscissa denotes temperature (°C), and the ordinate denotes heat flow (mW).
FIG. 40 illustrates the DSC profile of crystal form R of a n-propanol solvate of compound I of the present disclosure. The abscissa denotes temperature (°C), and the ordinate denotes heat flow (mW).
FIG. 41 illustrates the DSC profile of crystal form S of a tetrahydrofuran solvate of compound I of the present disclosure. The abscissa denotes temperature (°C), and the ordinate denotes heat flow (mW).
FIG. 42 illustrates the DSC profile of crystal form T of a 2-methyltetrahydrofuran solvate of compound I of the present disclosure. The abscissa denotes temperature (°C), and the ordinate denotes heat flow (mW).
FIG. 43 illustrates the TGA profile of crystal form A of compound I of the present disclosure. The abscissa denotes temperature (°C), and the ordinate denotes weight (%).
FIG. 44 illustrates the TGA profile of crystal form B of compound I of the present disclosure. The abscissa denotes temperature (°C), and the ordinate denotes weight (%).
FIG. 45 illustrates the TGA profile of crystal form C of compound I of the present disclosure. The abscissa denotes temperature (°C), and the ordinate denotes weight (%).
FIG. 46 illustrates the TGA profile of crystal form D of compound I of the present disclosure. The abscissa denotes temperature (°C), and the ordinate denotes weight (%).
FIG. 47 illustrates the TGA profile of crystal form E of compound I of the present disclosure. The abscissa denotes temperature (°C), and the ordinate denotes weight (%).
FIG. 48 illustrates the TGA profile of crystal form F of compound I of the present disclosure. The abscissa denotes temperature (°C), and the ordinate denotes weight (%).
FIG. 49 illustrates the TGA profile of crystal form G of a hydrate of compound I of the present disclosure. The abscissa denotes temperature (°C), and the ordinate denotes weight (%).
FIG. 50 illustrates the TGA profile of crystal form H of a methyl isobutyl ketone solvate of compound I of the present disclosure. The abscissa denotes temperature (°C), and the ordinate denotes weight (%).
FIG. 51 illustrates the TGA profile of crystal form I-1 of a methyl *tert-*butyl ether solvate of compound I of the present disclosure. The abscissa denotes temperature (°C), and the ordinate denotes weight (%).
FIG. 52 illustrates the TGA profile of crystal form I-2 of a methyl *tert-*butyl ether solvate of compound I of the present disclosure. The abscissa denotes temperature (°C), and the ordinate denotes weight (%).
FIG. 53 illustrates the TGA profile of crystal form J of an acetone solvate of compound I of the present disclosure. The abscissa denotes temperature (°C), and the ordinate denotes weight (%).
FIG. 54 illustrates the TGA profile of crystal form K of a n-heptane solvate of compound I of the present disclosure. The abscissa denotes temperature (°C), and the ordinate denotes weight (%).
FIG. 55 illustrates the TGA profile of crystal form L of a methylcyclohexane solvate of compound I of the present disclosure. The abscissa denotes temperature (°C), and the ordinate denotes weight (%).
FIG. 56 illustrates the TGA profile of crystal form M of a toluene solvate of compound I of the present disclosure. The abscissa denotes temperature (°C), and the ordinate denotes weight (%).
FIG. 57 illustrates the TGA profile of crystal form N of a dioxane solvate of compound I of the present disclosure. The abscissa denotes temperature (°C), and the ordinate denotes weight (%).
FIG. 58 illustrates the TGA profile of crystal form O of a DMF solvate of compound I of the present disclosure. The abscissa denotes temperature (°C), and the ordinate denotes weight (%).
FIG. 59 illustrates the TGA profile of crystal form P of a N-methylpyrrolidone solvate of compound I of the present disclosure. The abscissa denotes temperature (°C), and the ordinate denotes weight (%).
FIG. 60 illustrates the TGA profile of crystal form Q of a n-butanol solvate of compound I of the present disclosure. The abscissa denotes temperature (°C), and the ordinate denotes weight (%).
FIG. 61 illustrates the TGA profile of crystal form R of a *n*-propanol solvate of compound I of the present disclosure. The abscissa denotes temperature (°C), and the ordinate denotes weight (%).
FIG. 62 illustrates the TGA profile of crystal form S of a tetrahydrofuran solvate of compound I of the present disclosure. The abscissa denotes temperature (°C), and the ordinate denotes weight (%).
FIG. 63 illustrates the TGA profile of crystal form T of a 2-methyltetrahydrofuran solvate of compound I of the present disclosure. The abscissa denotes temperature (°C), and the ordinate denotes weight (%).
FIG. 64 illustrates the X-ray powder diffraction pattern of an amorphous solid of compound I of the present disclosure. The abscissa denotes 2θ values (degrees), and the ordinate denotes peak intensity.
FIG. 65 illustrates the DSC profile of an amorphous solid of compound I of the present disclosure. The abscissa denotes temperature (°C), and the ordinate denotes heat flow (mW).
FIG. 66 illustrates the TGA profile of an amorphous solid of compound I of the present disclosure. The abscissa denotes temperature (°C), and the ordinate denotes weight (%).
FIG. 67 illustrates the DVS plot of an amorphous solid of compound I of the present disclosure. The abscissa denotes relative humidity (%), and the ordinate denotes weight change (%).
FIG. 68 illustrates the DVS plot of crystal form D of compound I of the present disclosure. The abscissa denotes relative humidity (%), and the ordinate denotes weight change (%).
FIG. 2-1 illustrates the X-ray powder diffraction pattern of crystal form 2A of compound II of the present disclosure. The abscissa denotes 2θ values (degrees), and the ordinate denotes peak intensity.
FIG. 2-2 illustrates the X-ray powder diffraction pattern of crystal form 2B of compound II of the present disclosure. The abscissa denotes 2θ values (degrees), and the ordinate denotes peak intensity.
FIG. 2-3 illustrates the X-ray powder diffraction pattern of crystal form 2C of compound II of the present disclosure. The abscissa denotes 2θ values (degrees), and the ordinate denotes peak intensity.
FIG. 2-4 illustrates the X-ray powder diffraction pattern of crystal form 2D of compound II of the present disclosure. The abscissa denotes 2θ values (degrees), and the ordinate denotes peak intensity.
FIG. 2-5 illustrates the X-ray powder diffraction pattern of crystal form 2E of compound II of the present disclosure. The abscissa denotes 2θ values (degrees), and the ordinate denotes peak intensity.
FIG. 2-6 illustrates the X-ray powder diffraction pattern of crystal form 2F of a hydrate of compound II of the present disclosure. The abscissa denotes 2θ values (degrees), and the ordinate denotes peak intensity.
FIG. 2-7 illustrates the X-ray powder diffraction pattern of crystal form 2G of a hydrate of compound II of the present disclosure. The abscissa denotes 2θ values (degrees), and the ordinate denotes peak intensity.
FIG. 2-8 illustrates the X-ray powder diffraction pattern of crystal form 2H of a dimethylsulfoxide solvate of compound II of the present disclosure. The abscissa denotes 2θ values (degrees), and the ordinate denotes peak intensity.
FIG. 2-9 illustrates the X-ray powder diffraction pattern of crystal form 2I of a methyl *tert-*butyl ether solvate of compound II of the present disclosure. The abscissa denotes 2θ values (degrees), and the ordinate denotes peak intensity.
FIG. 2-10 illustrates the X-ray powder diffraction pattern of crystal form 2J-1 of a *n*-heptane solvate of compound II of the present disclosure. The abscissa denotes 2θ values (degrees), and the ordinate denotes peak intensity.
FIG. 2-11 illustrates the X-ray powder diffraction pattern of crystal form 2J-2 of a *n*-heptane solvate of compound II of the present disclosure. The abscissa denotes 2θ values (degrees), and the ordinate denotes peak intensity.
FIG. 2-12 illustrates the X-ray powder diffraction pattern of crystal form 2K of a toluene solvate of compound II of the present disclosure. The abscissa denotes 2θ values (degrees), and the ordinate denotes peak intensity.
FIG. 2-13 illustrates the X-ray powder diffraction pattern of crystal form 2L-1 of a methyl isobutyl ketone solvate of compound II of the present disclosure. The abscissa denotes 2θ values (degrees), and the ordinate denotes peak intensity.
FIG. 2-14 illustrates the X-ray powder diffraction pattern of crystal form 2L-2 of a methyl isobutyl ketone solvate of compound II of the present disclosure. The abscissa denotes 2θ values (degrees), and the ordinate denotes peak intensity.
FIG. 2-15 illustrates the X-ray powder diffraction pattern of crystal form 2M-1 of a cyclopentyl methyl ether solvate of compound II of the present disclosure. The abscissa denotes 2θ values (degrees), and the ordinate denotes peak intensity.
FIG. 2-16 illustrates the X-ray powder diffraction pattern of crystal form 2M-2 of a cyclopentyl methyl ether solvate of compound II of the present disclosure. The abscissa denotes 2θ values (degrees), and the ordinate denotes peak intensity.
FIG. 2-17 illustrates the X-ray powder diffraction pattern of crystal form 2N of a methyl ethyl ketone solvate of compound II of the present disclosure. The abscissa denotes 2θ values (degrees), and the ordinate denotes peak intensity.
FIG. 2-18 illustrates the X-ray powder diffraction pattern of crystal form 2θ of a methylcyclohexane solvate of compound II of the present disclosure. The abscissa denotes 2θ values (degrees), and the ordinate denotes peak intensity.
FIG. 2-19 illustrates the X-ray powder diffraction pattern of crystal form 2P of a DMF solvate of compound II of the present disclosure. The abscissa denotes 2θ values (degrees), and the ordinate denotes peak intensity.
FIG. 2-20 illustrates the X-ray powder diffraction pattern of crystal form 2Q-1 of a 2-methyltetrahydrofuran solvate of compound II of the present disclosure. The abscissa denotes 2θ values (degrees), and the ordinate denotes peak intensity.
FIG. 2-21 illustrates the X-ray powder diffraction pattern of crystal form 2Q-2 of a 2-methyltetrahydrofuran solvate of compound II of the present disclosure. The abscissa denotes 2θ values (degrees), and the ordinate denotes peak intensity.
FIG. 2-22 illustrates the X-ray powder diffraction pattern of crystal form 2R of a N-methylpyrrolidone solvate of compound II of the present disclosure. The abscissa denotes 2θ values (degrees), and the ordinate denotes peak intensity.
FIG. 2-23 illustrates the X-ray powder diffraction pattern of crystal form 2S of a trifluoroethanol solvate of compound II of the present disclosure. The abscissa denotes 2θ values (degrees), and the ordinate denotes peak intensity.
FIG. 2-24 illustrates the X-ray powder diffraction pattern of crystal form 2T of a tetrahydrofuran solvate of compound II of the present disclosure. The abscissa denotes 2θ values (degrees), and the ordinate denotes peak intensity.
FIG. 2-25 illustrates the X-ray powder diffraction pattern of crystal form 2U of a dioxane solvate of compound II of the present disclosure. The abscissa denotes 2θ values (degrees), and the ordinate denotes peak intensity.
FIG. 2-26 illustrates the DSC profile of crystal form 2A of compound II of the present disclosure. The abscissa denotes temperature (°C), and the ordinate denotes heat flow (mW).
FIG. 2-27 illustrates the DSC profile of crystal form 2B of compound II of the present disclosure. The abscissa denotes temperature (°C), and the ordinate denotes heat flow (mW).
FIG. 2-28 illustrates the DSC profile of crystal form 2C of compound II of the present disclosure. The abscissa denotes temperature (°C), and the ordinate denotes heat flow (mW).
FIG. 2-29 illustrates the DSC profile of crystal form 2D of compound II of the present disclosure. The abscissa denotes temperature (°C), and the ordinate denotes heat flow (mW).
FIG. 2-30 illustrates the DSC profile of crystal form 2E of compound II of the present disclosure. The abscissa denotes temperature (°C), and the ordinate denotes heat flow (mW).
FIG. 2-31 illustrates the DSC profile of crystal form 2F of a hydrate of compound II of the present disclosure. The abscissa denotes temperature (°C), and the ordinate denotes heat flow (mW).
FIG. 2-32 illustrates the DSC profile of crystal form 2G of a hydrate of compound II of the present disclosure. The abscissa denotes temperature (°C), and the ordinate denotes heat flow (mW).
FIG. 2-33 illustrates the DSC profile of crystal form 2H of a dimethylsulfoxide solvate of compound II of the present disclosure. The abscissa denotes temperature (°C), and the ordinate denotes heat flow (mW).
FIG. 2-34 illustrates the DSC profile of crystal form 2I of a methyl *tert-*butyl ether solvate of compound II of the present disclosure. The abscissa denotes temperature (°C), and the ordinate denotes heat flow (mW).
FIG. 2-35 illustrates the DSC profile of crystal form 2J-1 of a *n*-heptane solvate of compound II of the present disclosure. The abscissa denotes temperature (°C), and the ordinate denotes heat flow (mW).
FIG. 2-36 illustrates the DSC profile of crystal form 2J-2 of a *n*-heptane solvate of compound II of the present disclosure. The abscissa denotes temperature (°C), and the ordinate denotes heat flow (mW).
FIG. 2-37 illustrates the DSC profile of crystal form 2K of a toluene solvate of compound II of the present disclosure. The abscissa denotes temperature (°C), and the ordinate denotes heat flow (mW).
FIG. 2-38 illustrates the DSC profile of crystal form 2L-1 of a methyl isobutyl ketone solvate of compound II of the present disclosure. The abscissa denotes temperature (°C), and the ordinate denotes heat flow (mW).
FIG. 2-39 illustrates the DSC profile of crystal form 2L-2 of a methyl isobutyl ketone solvate of compound II of the present disclosure. The abscissa denotes temperature (°C), and the ordinate denotes heat flow (mW).
FIG. 2-40 illustrates the DSC profile of crystal form 2M-1 of a cyclopentyl methyl ether solvate of compound II of the present disclosure. The abscissa denotes temperature (°C), and the ordinate denotes heat flow (mW).
FIG. 2-41 illustrates the DSC profile of crystal form 2M-2 of a cyclopentyl methyl ether solvate of compound II of the present disclosure. The abscissa denotes temperature (°C), and the ordinate denotes heat flow (mW).
FIG. 2-42 illustrates the DSC profile of crystal form 2N of a methyl ethyl ketone solvate of compound II of the present disclosure. The abscissa denotes temperature (°C), and the ordinate denotes heat flow (mW).
FIG. 2-43 illustrates the DSC profile of crystal form 2O of a methylcyclohexane solvate of compound II of the present disclosure. The abscissa denotes temperature (°C), and the ordinate denotes heat flow (mW).
FIG. 2-44 illustrates the DSC profile of crystal form 2P of a DMF solvate of compound II of the present disclosure. The abscissa denotes temperature (°C), and the ordinate denotes heat flow (mW).
FIG. 2-45 illustrates the DSC profile of crystal form 2Q-1 of a 2-methyltetrahydrofuran solvate of compound II of the present disclosure. The abscissa denotes temperature (°C), and the ordinate denotes heat flow (mW).
FIG. 2-46 illustrates the DSC profile of crystal form 2Q-2 of a 2-methyltetrahydrofuran solvate of compound II of the present disclosure. The abscissa denotes temperature (°C), and the ordinate denotes heat flow (mW).
FIG. 2-47 illustrates the DSC profile of crystal form 2R of a *N*-methylpyrrolidone solvate of compound II of the present disclosure. The abscissa denotes temperature (°C), and the ordinate denotes heat flow (mW).
FIG. 2-48 illustrates the DSC profile of crystal form 2S of a trifluoroethanol solvate of compound II of the present disclosure. The abscissa denotes temperature (°C), and the ordinate denotes heat flow (mW).
FIG. 2-49 illustrates the DSC profile of crystal form 2T of a tetrahydrofuran solvate of compound II of the present disclosure. The abscissa denotes temperature (°C), and the ordinate denotes heat flow (mW).
FIG. 2-50 illustrates the DSC profile of crystal form 2U of a dioxane solvate of compound II of the present disclosure. The abscissa denotes temperature (°C), and the ordinate denotes heat flow (mW).
FIG. 2-51 illustrates the TGA profile of crystal form 2A of compound II of the present disclosure. The abscissa denotes temperature (°C), and the ordinate denotes weight (%).
FIG. 2-52 illustrates the TGA profile of crystal form 2B of compound II of the present disclosure. The abscissa denotes temperature (°C), and the ordinate denotes weight (%).
FIG. 2-53 illustrates the TGA profile of crystal form 2C of compound II of the present disclosure. The abscissa denotes temperature (°C), and the ordinate denotes weight (%).
FIG. 2-54 illustrates the TGA profile of crystal form 2D of compound II of the present disclosure. The abscissa denotes temperature (°C), and the ordinate denotes weight (%).
FIG. 2-55 illustrates the TGA profile of crystal form 2E of compound II of the present disclosure. The abscissa denotes temperature (°C), and the ordinate denotes weight (%).
FIG. 2-56 illustrates the TGA profile of crystal form 2F of a hydrate of compound II of the present disclosure. The abscissa denotes temperature (°C), and the ordinate denotes weight (%).
FIG. 2-57 illustrates the TGA profile of crystal form 2G of a hydrate of compound II of the present disclosure. The abscissa denotes temperature (°C), and the ordinate denotes weight (%).
FIG. 2-58 illustrates the TGA profile of crystal form 2H of a dimethylsulfoxide solvate of compound II of the present disclosure. The abscissa denotes temperature (°C), and the ordinate denotes weight (%).
FIG. 2-59 illustrates the TGA profile of crystal form 2I of a methyl *tert-*butyl ether solvate of compound II of the present disclosure. The abscissa denotes temperature (°C), and the ordinate denotes weight (%).
FIG. 2-60 illustrates the TGA profile of crystal form 2J-1 of a *n*-heptane solvate of compound II of the present disclosure. The abscissa denotes temperature (°C), and the ordinate denotes weight (%).
FIG. 2-61 illustrates the TGA profile of crystal form 2J-2 of a *n*-heptane solvate of compound II of the present disclosure. The abscissa denotes temperature (°C), and the ordinate denotes weight (%).
FIG. 2-62 illustrates the TGA profile of crystal form 2K of a toluene solvate of compound II of the present disclosure. The abscissa denotes temperature (°C), and the ordinate denotes weight (%).
FIG. 2-63 illustrates the TGA profile of crystal form 2L-1 of a methyl isobutyl ketone solvate of compound II of the present disclosure. The abscissa denotes temperature (°C), and the ordinate denotes weight (%).
FIG. 2-64 illustrates the TGA profile of crystal form 2L-2 of a methyl isobutyl ketone solvate of compound II of the present disclosure. The abscissa denotes temperature (°C), and the ordinate denotes weight (%).
FIG. 2-65 illustrates the TGA profile of crystal form 2M-1 of a cyclopentyl methyl ether solvate of compound II of the present disclosure. The abscissa denotes temperature (°C), and the ordinate denotes weight (%).
FIG. 2-66 illustrates the TGA profile of crystal form 2M-2 of a cyclopentyl methyl ether solvate of compound II of the present disclosure. The abscissa denotes temperature (°C), and the ordinate denotes weight (%).
FIG. 2-67 illustrates the TGA profile of crystal form 2N of a methyl ethyl ketone solvate of compound II of the present disclosure. The abscissa denotes temperature (°C), and the ordinate denotes weight (%).
FIG. 2-68 illustrates the TGA profile of crystal form 2O of a methylcyclohexane solvate of compound II of the present disclosure. The abscissa denotes temperature (°C), and the ordinate denotes weight (%).
FIG. 2-69 illustrates the TGA profile of crystal form 2P of a DMF solvate of compound II of the present disclosure. The abscissa denotes temperature (°C), and the ordinate denotes weight (%).
FIG. 2-70 illustrates the TGA profile of crystal form 2Q-1 of a 2-methyltetrahydrofuran solvate of compound II of the present disclosure. The abscissa denotes temperature (°C), and the ordinate denotes weight (%).
FIG. 2-71 illustrates the TGA profile of crystal form 2Q-2 of a 2-methyltetrahydrofuran solvate of compound II of the present disclosure. The abscissa denotes temperature (°C), and the ordinate denotes weight (%).
FIG. 2-72 illustrates the TGA profile of crystal form 2R of a N-methylpyrrolidone solvate of compound II of the present disclosure. The abscissa denotes temperature (°C), and the ordinate denotes weight (%).
FIG. 2-73 illustrates the TGA profile of crystal form 2S of a trifluoroethanol solvate of compound II of the present disclosure. The abscissa denotes temperature (°C), and the ordinate denotes weight (%).
FIG. 2-74 illustrates the TGA profile of crystal form 2T of a tetrahydrofuran solvate of compound II of the present disclosure. The abscissa denotes temperature (°C), and the ordinate denotes weight (%).
FIG. 2-75 illustrates the TGA profile of crystal form 2U of a dioxane solvate of compound II of the present disclosure. The abscissa denotes temperature (°C), and the ordinate denotes weight (%).
FIG. 2-76 illustrates the X-ray powder diffraction pattern of compound II of the present disclosure. The abscissa denotes 2θ values (degrees), and the ordinate denotes peak intensity.
FIG. 2-77 illustrates the DSC profile of compound II of the present disclosure. The abscissa denotes temperature (°C), and the ordinate denotes heat flow (mW).
FIG. 2-78 illustrates the TGA profile of compound II of the present disclosure. The abscissa denotes temperature (°C), and the ordinate denotes weight (%).
FIG. 2-79 illustrates the DVS plot of crystal form 2A of compound II of the present disclosure. The abscissa denotes relative humidity (%), and the ordinate denotes weight change (%).

### Definitions and Description

Unless otherwise stated, the following terms used in the specification and the claims have the following meanings. A particular phrase or term, unless otherwise specifically defined, should not be considered uncertain or unclear, but construed according to its common meaning. When referring to a trade name, it is intended to refer to its corresponding commercial product or its active ingredient.

The "pharmaceutical composition" refers to a mixture containing one or more of the compounds described herein or a physiologically/pharmaceutically acceptable salt or prodrug thereof and other chemical components, as well as other components such as physiological/pharmaceutically acceptable carriers and excipients. The purpose of the pharmaceutical composition is to facilitate the administration to an organism, promote the absorption of the active ingredient, and thereby exert biological activity.

As used herein, the "polymorph" refers to crystal forms having the same chemical composition but different spatial arrangements of molecules, atoms, and/or ions composing the crystal. Although polymorphs have the same chemical composition, they differ in packing and geometric arrangement and may exhibit different physical properties such as melting point, shape, color, density, hardness, deformability, stability, solubility, dissolution rate, and the like. Two polymorphs may be monotropic or enantiotropic, depending on their temperature-stability relationship. For a monotropic system, the relative stability between the two solid phases remains unchanged upon temperature change. In contrast, in an enantiotropic system, there is a transition temperature at which the two phases are switched in stability ((Theory and Origin of Polymorphism in "Polymorphism in Pharmaceutical Solids" (1999) ISBN: )-8247-0237 ). The phenomenon that such compounds exist in different crystal structures is called polymorphism.

The various crystal structures of the present disclosure can be distinguished from one another using various analytical techniques known to those of ordinary skill in the art. Such techniques include, but are not limited to, X-ray powder diffraction (XRPD), differential scanning calorimetry (DSC), and/or thermogravimetric analysis (TGA).

The term "room temperature" or "RT" as used herein refers to an ambient temperature of 20 to 25 °C (68-77 °F).

The term "substantially identical" as used herein with respect to X-ray diffraction peak positions is meant to account for typical peak positions and intensity variability. For example, those skilled in the art will appreciate that the peak position (2θ) may vary due to different XRPD systems, sometimes by up to 0.2°. Furthermore, those skilled in the art will appreciate that factors such as XRPD sample preparation, XRPD system, sample crystallinity, sample amount, and preferred crystal orientation will result in variations in the relative peak intensities in the XRPD diffraction patterns of the samples.

The intermediate compounds of the present disclosure can be prepared by a variety of synthetic methods well known to those skilled in the art, including the specific embodiments listed below, embodiments formed by combinations thereof with other chemical synthetic methods, and equivalent substitutions thereof known to those skilled in the art. Preferred embodiments include but are not limited to the examples of the present disclosure.

The chemical reactions of the specific embodiments of the present disclosure are carried out in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction scheme based on the existing embodiments.

The present disclosure is described in detail below through examples; however, these examples are not intended to limit the present disclosure in any way.

All solvents used in the present disclosure are commercially available and can be used without further purification. Unless otherwise stated, all reactions in the present disclosure were performed under continuous magnetic stirring in dry solvents, and the temperatures are expressed in degrees Celsius (°C).

### Methods and Materials

The structures of the compounds were determined by nuclear magnetic resonance (NMR). The NMR shifts (δ) are given in parts per million (ppm). The NMR analyses were performed using the Bruker avance-400MHz NMR spectrometer with deuterated dimethylsulfoxide (DMSO-*d₆*) or deuterated methanol (MeOD-*d₄*) as the solvent and tetramethylsilane (TMS) as the internal standard, and the chemical shifts are expressed in 10⁻⁶ ppm.

The HPLC procedures were performed using the Agilent 1260 high performance liquid chromatograph or an equivalent high performance liquid chromatograph (with a Sunfire C18 150 × 4.6 m chromatography column or an equivalent chromatography column).

The polymorphs of compound III were characterized by X-ray powder diffraction patterns. The X-ray powder diffraction patterns of the salts were collected on the Bruker D8 Advance powder diffractometer operating in reflection mode using Cu Kα radiation. The instrument used Cu Kα radiation (40 kV, 40 mA) and operated at room temperature using the SSD160-2 detector. The 2θ scan range was from 3° to 40°, and the scan speed was 0.1 s/step. The diffraction patterns were analyzed using the DIFFRAC.MEA.CENTER software.

XRPD samples were prepared by placing a sample on a monocrystal silicon wafer and pressing the sample powder with a glass slide or an equivalent to ensure that the sample had a flat surface and an appropriate height. The sample holder was then placed into the Bruker D8 Advance system, and the X-ray powder diffraction pattern was collected using the instrumental parameters described above. Measurement differences associated with such X-ray powder diffraction analysis results may result from a variety of factors including: (a) errors in sample preparation (e.g., sample height), (b) instrument errors, (c) calibration errors, (d) operator errors (including those errors present when determining the peak positions), and (e) the nature of the material (e.g., preferred orientation errors). Calibration errors and sample height errors often result in a shift of all the peaks in the same direction. In general, this correction factor will bring the measured peak positions into agreement with the expected peak positions and may be in the range of the expected 2θ value ± 0.2°.

The experimental method for characterizing a crystal form of an acid salt or a base salt of compound III by differential scanning calorimetry (DSC) was as follows: A small amount of a polymorph of compound III in the crystal form was taken and placed into an aluminum crucible that matches the instrument and can be sealed with a lid. After the sample was loaded, the crucible was sealed with an aluminum plate and then sent into the instrument for analysis. In the present disclosure, the instrument model used in the differential scanning calorimetry analysis was METTLER TOLEDO DSC 3, and scan parameters were set as follows: a nitrogen atmosphere was used, and the temperature ramp rate was 10.0 k/min.

The experimental method for characterizing a polymorph of compound III by thermogravimetric analysis (TGA) was as follows: A small amount of a powder of a polymorph of compound III was taken and placed into an aluminum oxide crucible that matches the instrument. After the sample was loaded, the crucible was sent into the instrument for analysis. In the present disclosure, the instrument model used in the differential scanning calorimetry analysis was METTLER TOLEDO TGA 2, and scan parameters were set as follows: a nitrogen atmosphere was used, and the temperature ramp rate was 10.0 k/min.

The experimental method for characterizing an acid salt or a base salt of compound III by dynamic vapor sorption (DVS) was as follows: A small amount of a powder of a polymorph of compound III was taken and placed into a precise sample tray that matches the instrument. After the sample was loaded, the sample tray was sent into the instrument for analysis. In the present disclosure, the instrument model used in the dynamic vapor sorption analysis was Intrinsic PLUS; the experimental parameter was set as follows: a constant temperature of 25 °C was set; the mass percent change rate per unit time (dm/dt) = 0.02%/min was used as a criterion for determining that a balance is achieved; the program humidity change cycle was set as follows: the initial relative humidity was 0%, and the endpoint relative humidity was 90%.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure.

Unless otherwise stated, the starting materials and reagents used in the following examples are all commercially **available products or can be prepared by using known methods.**

### Example 1. Preparation of (S)-2-((4-((6-((4-chloro-2-fluorophenoxy)methyl)pyridin-2-yl)oxy)piperidin-1-yl)methyl)-1-(oxetan-2-ylmethy l)-1H-benzo[d]imidazole-6-carboxylic acid (amorphous form of compound I)

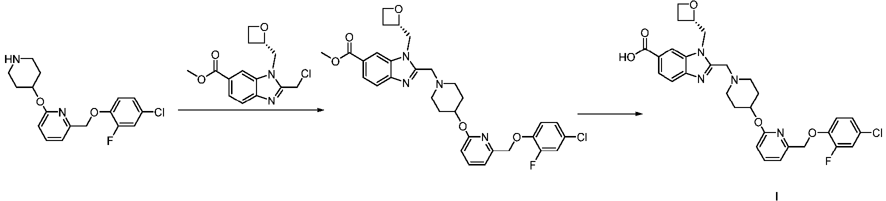

### Step 1: Synthesis of methyl (S)-2-((4-((6-((4-chloro-2-fluorophenoxy)methyl)pyridin-2-yl)oxy)piperidin-1-yl)methyl)-1-(oxetan-2-ylmethyl)-1 H-benzo[d]imidazole-6-carboxylate

A mixed solution of methyl (*S*)-2-(chloromethyl)-1-(oxetan-2-ylmethyl)-1*H*-benzo[*d*]imidazole-6-carboxylate (1.5 g, 5.1 mmol), 2-(4-chloro-2-fluorophenoxy)methyl)-6-(piperidin-4-yloxy)pyridine (1.8 g, 5.5 mmol), and potassium carbonate (1.8 g, 13.0 mmol) in *N,N*-dimethylformamide (80 mL) was stirred at 60 °C for 3 h, then quenched with water (100 mL), and extracted with ethyl acetate (50 mL × 3). The combined organic layers were washed with brine (50 mL × 3), dried over anhydrous sodium sulfate, filtered, and concentrated. The residue was purified by silica gel column chromatography (dichloromethane/methanol = 20/1) to give methyl (*S*)-2-((4-((6-((4-chloro-2-fluorophenoxy)methyl)pyridin-2-yl)oxy)piperidin-1-yl)methyl)-1-(oxetan-2-ylmethyl)-1 *H*-benzo[*d*]imidazole-6-carboxylate (1.0 g, 33.5% yield).

### Step 2: Synthesis of (S)-2-((4-((6-((4-chloro-2-fluorophenoxy)methyl)pyridin-2-yl)oxy)piperidin-1-yl)methyl)-1-(oxetan-2-ylmethyl)-1 H-benzo[d]imidazole-6-carboxylate

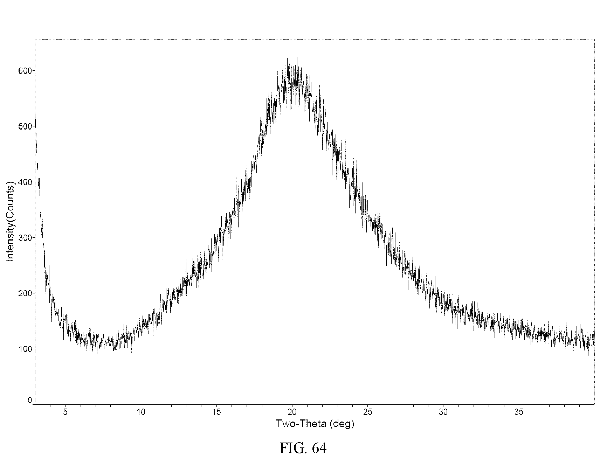

Lithium hydroxide (0.13 g, 5.4 mmol) was added to a mixed solution of methyl (*S*)-2-((4-((6-((4-chloro-2-fluorophenoxy)methyl)pyridin-2-yl)oxy)piperidin-1-yl)methyl)-1-(oxetan-2-ylmethyl)-1 *H*-benzo[*d*]imidazole-6-carboxylate (1.0 g, 1.7 mmol) in tetrahydrofuran/water (20 mL/20 mL), and the mixture was stirred at room temperature for 16 h. The pH of the resulting mixture was adjusted to 5-6 with formic acid, and the solvent was removed *in vacuo.* The residue was purified by reverse-phase flash column chromatography to give (*S*)-2-((4-((6-((4-chloro-2-fluorophenoxy)methyl)pyridin-2-yl)oxy)piperidin-1-yl)methyl)-1-(oxetan-2-ylmethyl)-1 *H*-benzo[*d*]imidazole-6-carboxylic acid (0.69 g, 70.5% yield). The product was characterized by XRPD, DSC, and TGA. The resultant compound I was an amorphous form, with its X-ray powder diffraction pattern, DSC profile, and TGA profile shown in FIGs. 64, 65, and 66, respectively. ¹H NMR (400 MHz, DMSO-*d₆*): δ 8.27 (s, 1 H), 7.80 (dd, *J*=8.4 Hz, 1.2 Hz, 1 H), 7.72 (t, *J*=7.6 Hz, 1 H), 7.64 (d, *J*=8.4 Hz, 1 H), 7.44 (dd, *J=*11.2 Hz, 2.0 Hz, 1 H), 7.28 (t, *J*=8.8 Hz, 1 H), 7.18 (d, *J*=8.4 Hz, 1 H), 7.04 (d, *J*=7.2 Hz, 1 H), 6.72 (d, *J*=8.0 Hz, 1 H), 5.18 (s, 2 H), 5.12-5.06 (m, 1 H), 4.95-4.93 (m, 1 H), 4.81-4.76 (m, 1 H), 4.66-4.62 (m, 1 H), 4.51-4.49 (m, 1 H), 4.38-4.36 (m, 1 H), 3.94 (d, *J*=13.6 Hz, 1 H), 3.78 (d, *J*=13.6 Hz, 1 H), 2.79-2.67 (m, 2 H), 2.46-2.41 (m, 1 H), 2.32 (s, 2 H), 1.92-1.91 (m, 2 H), 1.63-1.59 (m, 2 H).

### Example 2. Preparation of crystal form A of compound I

200 mg of compound I was added to 7.5 mL of isopropanol. The mixture was ultrasonicated for dissolution at 50 °C and filtered. The filtrate was stirred at 5 °C for about 16 h and filtered. The filter cake was dried in an oven at 50 °C to give crystal form A of the compound. The product was analyzed and characterized by XRPD (FIG. 1), DSC (FIG. 22), and TGA (FIG. 43).

### Example 3. Preparation of crystal form B of compound I

200 mg of compound I was added to 3 mL of ethyl acetate. The mixture was ultrasonicated for dissolution at 50 °C and filtered. The filtrate was stirred at 5 °C for about 16 h and filtered. The filter cake was dried in an oven at 50 °C to give crystal form B of the compound. The product was analyzed and characterized by XRPD (FIG. 2), DSC (FIG. 23), and TGA (FIG. 44).

### Example 4. Preparation of crystal form C of compound I

200 mg of compound I was added to 9 mL of acetonitrile. The mixture was ultrasonicated for dissolution at 50 °C and filtered. The filtrate was stirred at 5 °C for about 16 h and filtered. The filter cake was dried in an oven at 50 °C to give crystal form C of the compound. The product was analyzed and characterized by XRPD (FIG. 3), DSC (FIG. 24), and TGA (FIG. 45).

### Example 5. Preparation of crystal form D of compound I

200 mg of compound I was added to 2.5 mL of isopropyl ether/ethyl acetate (6:1, v:v). The mixture was stirred at 50 °C for 2 h, cooled to 5 °C, stirred for 2 h, and filtered. The filter cake was dried in an oven at 50 °C to give crystal form D of the compound. The product was analyzed and characterized by XRPD (FIG. 4), DSC (FIG. 25), and TGA (FIG. 46).

### Example 6. Preparation of crystal form E of compound I

20 mg of compound I was suspended in 0.5 mL of *n*-hexane/dichloromethane (3:1, v:v). The mixture was stirred at 5 °C for 3 d and filtered. The filter cake was dried in an oven at 50 °C to give crystal form E of the compound. The product was analyzed and characterized by XRPD (FIG. 5), DSC (FIG. 26), and TGA (FIG. 47).

### Example 7. Preparation of crystal form F of compound I

20 mg of compound I was suspended in 0.5 mL of water/acetone (6:1, v:v). The mixture was stirred at 50 °C for 2 h, cooled to 5 °C, stirred for 2 h, and filtered. The filter cake was dried in an oven at 50 °C to give crystal form F of the compound. The product was analyzed and characterized by XRPD (FIG. 6), DSC (FIG. 27), and TGA (FIG. 48).

### Example 8. Preparation of crystal form G of hydrate of compound I

100 mg of compound I was suspended in 2.5 mL of water. The mixture was stirred at 5 °C for 3 d and filtered. The filter cake was dried in an oven at 50 °C to give crystal form G of a hydrate of the compound. The product was analyzed and characterized by XRPD (FIG. 7), DSC (FIG. 28), and TGA (FIG. 49).

### Example 9. Preparation of crystal form H of methyl isobutyl ketone solvate of compound I

20 mg of compound I was suspended in 0.5 mL of methyl isobutyl ketone. The mixture was stirred at room temperature for 3 d and filtered. The filter cake was dried in an oven at 40 °C to give crystal form H of a methyl isobutyl ketone solvate of the compound. The product was analyzed and characterized by XRPD (FIG. 8), DSC (FIG. 29), and TGA (FIG. 50). ¹H NMR (400 MHz, DMSO-*d₆*): δ 12.73 (br, 1H), 8.27 (s, 1 H), 7.81 (dd, *J*=8.4 Hz, 1.2 Hz, 1 H), 7.74 (t, *J*=7.6 Hz, 1 H), 7.66 (d, *J*=8.4 Hz, 1 H), 7.43 (dd, *J*=11.2 Hz, 2.0 Hz, 1 H), 7.30 (t, *J*=8.8 Hz, 1 H), 7.17 (d, *J*=8.4 Hz, 1 H), 7.04 (d, *J*=7.2 Hz, 1 H), 6.71 (d, *J=8.0* Hz, 1 H), 5.18 (s, 2 H), 5.10-5.08 (m, 1 H), 4.95-4.93 (m, 1 H), 4.78-4.76 (m, 1 H), 4.66-4.63 (m, 1 H), 4.51-4.49 (m, 1 H), 4.38-4.36 (m, 1 H), 3.95 (d, *J*=13.6 Hz, 1 H), 3.78 (d, *J*=13.6 Hz, 1 H), 2.73-2.69 (m, 3 H), 2.32-2.28 (m, 4 H), 2.06-1.91 (m, 4 H), 1.63-1.59 (m, 2 H), 0.86-0.84 (d, *J*=6.8 Hz, 4H).

### Example 10. Preparation of crystal form I-1 of methyl tert-butyl ether solvate of compound I

20 mg of compound I was suspended in 1.0 mL of ethyl acetate. The mixture was filtered to give a clear solution. This solution was then transferred into a 20-mL vial containing 3 mL of methyl *tert-*butyl ether. The 20-mL vial was sealed with a cap, let stand at room temperature for 3 d, and filtered. The filter cake was dried in an oven at 50 °C to give crystal form I-1 of a methyl *tert-butyl* ether solvate of the compound. The product was analyzed and characterized by XRPD (FIG. 9), DSC (FIG. 30), and TGA (FIG. 51). ¹H NMR (400 MHz, DMSO-*d₆*): δ 12.72 (br, 1H), 8.27 (s, 1 H), 7.81 (d, *J=8.4* Hz, 1 H), 7.75 (t, J=7.6 Hz, 1 H), 7.67 (d, *J=8.4* Hz, 1 H), 7.44 (dd, *J=11.2* Hz, 2.0 Hz, 1 H), 7.30 (t, *J=8.8* Hz, 1 H), 7.18 (d, *J=8.4* Hz, 1 H), 7.03 (d, *J=7.2* Hz, 1 H), 6.72 (d, *J=8.0* Hz, 1 H), 5.18 (s, 2 H), 5.10-5.08 (m, 1 H), 4.95-4.93 (m, 1 H), 4.78-4.76 (m, 1 H), 4.66-4.63 (m, 1 H), 4.50-4.49 (m, 1 H), 4.38-4.36 (m, 1 H), 3.96 (d, J=13.6 Hz, 1 H), 3.79 (d, *J* =13.6 Hz, 1 H), 3.08 (s, 3 H), 2.72-2.70 (m, 3 H), 2.32-2.28 (m, 4 H), 1.99-1.91 (m, 4 H), 1.63-1.59 (m, 2 H), 1.11 (s, 8H).

### Example 11. Preparation of crystal form I-2 of methyl tert-butyl ether solvate of compound I

20 mg of compound I was suspended in 0.5 mL of methyl *tert-butyl* ether/isopropyl acetate (4:1, v:v). The mixture was stirred at room temperature for 3 d and filtered. The filter cake was dried in an oven at 50 °C to give crystal form I-2 of a methyl *tert-*butyl ether solvate of the compound. The product was analyzed and characterized by XRPD (FIG. 10), DSC (FIG. 31), and TGA (FIG. 52). ¹H NMR (400 MHz, DMSO-*d₆*): δ 12.72 (br, 1H), 8.27 (s, 1 H), 7.81 (d, *J*=8.4 Hz, 1 H), 7.76 (t, *J*=7.6 Hz, 1 H), 7.67 (d, *J*=8.4 Hz, 1 H), 7.45 (dd, *J=*11.2 Hz, 2.0 Hz, 1 H), 7.31 (t, *J*=8.8 Hz, 1 H), 7.18 (d, *J*=8.4 Hz, 1 H), 7.04 (d, *J*=7.2 Hz, 1 H), 6.72 (d, *J*=8.0 Hz, 1 H), 5.18 (s, 2 H), 5.11-5.09 (m, 1 H), 4.96-4.94 (m, 1 H), 4.78-4.76 (m, 1 H), 4.66-4.63 (m, 1 H), 4.50-4.49 (m, 1 H), 4.38-4.36 (m, 1 H), 3.96 (d, *J*=13.6 Hz, 1 H), 3.79 (d, *J*=13.6 Hz, 1 H), 3.08 (s, 4 H), 2.73-2.70 (m, 3 H), 2.32-2.28 (m, 3 H), 1.99-1.91 (m, 2 H), 1.63-1.59 (m, 2 H), 1.11 (s, 12H).

### Example 12. Preparation of crystal form J of acetone solvate of compound I

To about 20 mg of compound I was added 0.5 mL of acetone in three portions. The mixture was ground for 5 min after each addition. The resultant solid was dried in an oven at 50 °C to give crystal form J of an acetone solvate of the compound. The product was analyzed and characterized by XRPD (FIG. 11), DSC (FIG. 32), and TGA (FIG. 53). ¹H NMR (400 MHz, DMSO-*d₆*): δ 12.70 (br, 1H), 8.27 (s, 1 H), 7.81 (dd, *J*=8.4 Hz, 1.2 Hz, 1 H), 7.72 (t, *J*=7.6 Hz, 1 H), 7.64 (d, *J*=8.4 Hz, 1 H), 7.44 (dd, *J*=11.2 Hz, 2.0 Hz, 1 H), 7.28 (t, *J*=8.8 Hz, 1 H), 7.18 (d, *J*=8.4 Hz, 1 H), 7.04 (d, *J*=7.2 Hz, 1 H), 6.72 (d, *J*=8.*0* Hz, 1 H), 5.18 (s, 2 H), 5.12-5.07 (m, 1 H), 4.95-4.92 (m, 1 H), 4.81-4.77 (m, 1 H), 4.66-4.63 (m, 1 H), 4.52-4.49 (m, 1 H), 4.38-4.36 (m, 1 H), 3.94 (d, *J* =13.6 Hz, 1 H), 3.78 (d, *J*=13.6 Hz, 1 H), 2.73-2.69 (m, 3 H), 2.46-2.32 (m, 3H), 2.09 (s, 3 H), 1.92-1.91 (m, 2 H), 1.63-1.59 (m, 2 H).

### Example 13. Preparation of crystal form K of n-heptane solvate of compound I

About 20 mg of compound I was suspended in 0.5 mL of *n*-heptane/tetrahydrofuran (3:1, v:v). The mixture was stirred at 5 °C for 3 d and filtered. The filter cake was dried in an oven at 50 °C to give crystal form K of a *n*-heptane solvate of the compound. The product was analyzed and characterized by XRPD (FIG. 12), DSC (FIG. 33), and TGA (FIG. 54). ¹H NMR (400 MHz, DMSO-*d₆*): δ 12.80 (br, 1H), 8.26 (s, 1 H), 7.81 (dd, *J*=8.4 Hz, 1.2 Hz, 1 H), 7.79 (t, *J*=7.6 Hz, 1 H), 7.72 (d, *J*=8.4 Hz, 1 H), 7.45 (dd, *J*=11.2 Hz, 2.0 Hz, 1 H), 7.29 (t, *J*=8.8 Hz, 1 H), 7.18 (d, *J*=8.4 Hz, 1 H), 7.04 (d, *J*=7.2 Hz, 1 H), 6.72 (d, *J*=8.0 Hz, 1 H), 5.18 (s, 2 H), 5.10-5.08 (m, 1 H), 4.94-4.92 (m, 1 H), 4.81-4.78 (m, 1 H), 4.66-4.63 (m, 1 H), 4.50-4.49 (m, 1 H), 4.38-4.36 (m, 1 H), 3.94 (d, *J* =13.6 Hz, 1 H), 3.78 (d, *J* =13.6 Hz, 1 H), 2.73-2.69 (m, 3 H), 2.43-2.32 (m, 3H), 1.92-1.91 (m, 2 H), 1.63-1.61 (m, 2 H), 1.24 (s, 1.2 H), 0.87-0.85 (m, 0.7 H).

### Example 14. Preparation of crystal form L of methylcyclohexane solvate of compound I

About 20 mg of compound I was suspended in 0.5 mL of methylcyclohexane/tetrahydrofuran (6:1, v:v). The mixture was stirred at 50 °C for 2 h, cooled to 5 °C, stirred for another 2 h, and filtered. The filter cake was dried in an oven at 50 °C to give crystal form L of a methylcyclohexane solvate of the compound. The product was analyzed and characterized by XRPD (FIG. 13), DSC (FIG. 34), and TGA (FIG. 55). ¹H NMR (400 MHz, DMSO-*d₆*): δ 12.81 (br, 1H), 8.26 (s, 1 H), 7.81 (dd, *J*=8.4 Hz, 1.2 Hz, 1 H), 7.79 (t, J=7.6 Hz, 1 H), 7.72 (d, *J*=8.4 Hz, 1 H), 7.45 (dd, *J*=11.2 Hz, 2.0 Hz, 1 H), 7.29 (t, *J*=8.8 Hz, 1 H), 7.19 (d, *J*=8.4 Hz, 1 H), 7.04 (d, *J*=7.2 Hz, 1 H), 6.72 (d, *J*=8.0 Hz, 1 H), 5.18 (s, 2 H), 5.10-5.08 (m, 1 H), 4.94-4.92 (m, 1 H), 4.81-4.78 (m, 1 H), 4.66-4.63 (m, 1 H), 4.50-4.49 (m, 1 H), 4.38-4.36 (m, 1 H), 3.95 (d, *J*=13.6 Hz, 1 H), 3.79 (d, *J*=13.6 Hz, 1 H), 2.73-2.70 (m, 3 H), 2.43-2.32 (m, 3H), 1.92-1.91 (m, 2 H), 1.63-1.59 (m, 2 H), 1.25-0.83 (m, 2 H).

### Example 15. Preparation of crystal form M of toluene solvate of compound I

About 20 mg of compound I was dissolved in 0.5 mL of toluene. The mixture was filtered to give a clear solution. The solution was then transferred into a 20-mL vial containing 3 mL of isopropyl ether. The 20-mL vial was sealed with a cap, let stand at room temperature for 2 d, and filtered. The filter cake was dried in an oven at 50 °C to give crystal form M of a toluene solvate of the compound. The product was analyzed and characterized by XRPD (FIG. 14), DSC (FIG. 35), and TGA (FIG. 56). ¹H NMR (400 MHz, DMSO-*d₆*): δ 12.73 (br, 1H), 8.26 (s, 1 H), 7.81 (dd, *J*=8.4 Hz, 1.2 Hz, 1 H), 7.79 (t, *J*=7.6 Hz, 1 H), 7.72 (d, *J*=8.4 Hz, 1 H), 7.45 (dd, *J*=11.2 Hz, 2.0 Hz, 1 H), 7.30-7.14 (m, 8 H), 7.04 (d, *J*=7.2 Hz, 1 H), 6.72 (d, *J*=8.0 Hz, 1 H), 5.18 (s, 2 H), 5.10-5.08 (m, 1 H), 4.94-4.92 (m, 1 H), 4.81-4.78 (m, 1 H), 4.66-4.63 (m, 1 H), 4.50-4.49 (m, 1 H), 4.38-4.36 (m, 1 H), 3.95 (d, *J=* 13.6 Hz, 1 H), 3.79 (d, *J* = 13.6 Hz, 1 H), 2.73-2.70 (m, 3 H), 2.43-2.30 (m, 7 H), 1.92-1.91 (m, 2 H), 1.63-1.59 (m, 2 H).

### Example 16. Preparation of crystal form N of dioxane solvate of compound I

About 20 mg of compound I was suspended in 0.5 mL of n-heptane/dioxane (3:1, v:v). The mixture was stirred at 5 °C for 3 d and filtered. The filter cake was dried in an oven at 50 °C to give crystal form N of a dioxane solvate of the compound. The product was analyzed and characterized by XRPD (FIG. 15), DSC (FIG. 36), and TGA (FIG. 57). ¹H NMR (400 MHz, DMSO-*d₆*): δ 12.81 (br, 1H), 8.26 (s, 1 H), 7.81 (dd, *J*=8.4 Hz, 1.2 Hz, 1 H), 7.79 (t, J=7.6 Hz, 1 H), 7.72 (d, *J=8.4* Hz, 1 H), 7.45 (dd, *J=11.2* Hz, 2.0 Hz, 1 H), 7.29 (t, *J=8.8* Hz, 1 H), 7.19 (d, *J=8.4* Hz, 1 H), 7.04 (d, J=7.2 Hz, 1 H), 6.72 (d, *J=8.0* Hz, 1 H), 5.18 (s, 2 H), 5.10-5.08 (m, 1 H), 4.94-4.92 (m, 1 H), 4.81-4.78 (m, 1 H), 4.66-4.63 (m, 1 H), 4.50-4.49 (m, 1 H), 4.38-4.36 (m, 1 H), 3.94 (d, *J* =13.6 Hz, 1 H), 3.79 (d, J=13.6 Hz, 1 H), 3.57 (s, 14 H), 2.73-2.69 (m, 3 H), 2.51-2.32 (m, 3H), 1.92-1.91 (m, 2 H), 1.63-1.61 (m, 2 H).

### Example 17. Preparation of crystal form O of DMF solvate of compound I

About 20 mg of compound I was dissolved in 0.5 mL of DMF/isopropyl ether (1:4, v:v). The solution was exposed to the ambience, let stand at room temperature for evaporation for 1 d, and filtered. The filter cake was dried in an oven at 50 °C to give crystal form O of a DMF solvate of the compound. The product was analyzed and characterized by XRPD (FIG. 16), DSC (FIG. 37), and TGA (FIG. 58). ¹H NMR (400 MHz, DMSO-*d₆*): δ 12.81 (br, 1H), 8.26 (s, 1 H), 7.81 (dd, J=8.4 Hz, 1.2 Hz, 1 H), 7.79 (t, J=7.6 Hz, 1 H), 7.72 (d, *J=8.4* Hz, 1 H), 7.45 (dd, *J*=11.2 Hz, 2.0 Hz, 1 H), 7.29 (t, *J*=8.8 Hz, 1 H), 7.20 (d, *J*=8.4 Hz, 1 H), 7.04 (d, *J*=7.2 Hz, 1 H), 6.72 (d, *J*=8.0 Hz, 1 H), 5.18 (s, 2 H), 5.10-5.08 (m, 1 H), 4.94-4.92 (m, 1 H), 4.78-4.76 (m, 1 H), 4.66-4.63 (m, 1 H), 4.51-4.49 (m, 1 H), 4.38-4.36 (m, 1 H), 3.94 (d, *J*=13.6 Hz, 1 H), 3.79 (d, *J*=13.6 Hz, 1 H), 2.89 (s, 3H), 2.73-2.70 (m, 3 H), 2.43-2.32 (m, 3H), 1.92-1.91 (m, 2 H), 1.63-1.59 (m, 2 H).

### Example 18. Preparation of crystal form P of N-methylpyrrolidone solvate of compound I

About 20 mg of compound I was suspended in 0.5 mL of *N*-methylpyrrolidone/isopropyl ether (1:9, v:v). The mixture was stirred at 5 °C for 3 d and filtered. The filter cake was dried in an oven at 50 °C to give crystal form P of a N-methylpyrrolidone solvate of the compound. The product was analyzed and characterized by XRPD (FIG. 17), DSC (FIG. 38), and TGA (FIG. 59). ¹H NMR (400 MHz, DMSO-*d₆*): δ 12.76 (br, 1H), 8.27 (s, 1 H), 7.81 (dd, *J*=8.4 Hz, 1.2 Hz, 1 H), 7.79 (t, *J*=7.6 Hz, 1 H), 7.72 (d, *J*=8.4 Hz, 1 H), 7.45 (dd, *J*=11.2 Hz, 2.0 Hz, 1 H), 7.29 (t, *J*=8.8 Hz, 1 H), 7.19 (d, *J*=8.4 Hz, 1 H), 7.04 (d, *J*=7.2 Hz, 1 H), 6.72 (d, *J*=8.0 Hz, 1 H), 5.19 (s, 2 H), 5.10-5.09 (m, 1 H), 4.94-4.92 (m, 1 H), 4.81-4.78 (m, 1 H), 4.66-4.63 (m, 1 H), 4.50-4.49 (m, 1 H), 4.38-4.36 (m, 1 H), 3.96 (d, *J*=13.5 Hz, 1 H), 3.78 (d, *J*=13.5 Hz, 1 H), 3.32-3.29 (m, 3 H), 2.73-2.70 (m, 6 H), 2.43-2.32 (m, 3H), 2.20-2.16 (m, 2 H), 1.94-1.88 (m, 4 H), 1.63-1.61 (m, 2 H).

### Example 19. Preparation of crystal form Q of n-butanol solvate of compound I

About 20 mg of compound I was suspended in 0.5 mL of n-butanol. The mixture was stirred at 50 °C for 3 d and filtered. The filter cake was dried in an oven at 50 °C to give crystal form Q of a n-butanol solvate of the compound. The product was analyzed and characterized by XRPD (FIG. 18), DSC (FIG. 39), and TGA (FIG. 60). ¹H NMR (400 MHz, DMSO-*d₆*): δ 12.73 (br, 1H), 8.27 (s, 1 H), 7.81 (dd, *J*=8.4 Hz, 1.2 Hz, 1 H), 7.79 (t, *J*=7.6 Hz, 1 H), 7.73 (d, *J*=8.4 Hz, 1 H), 7.45 (dd, *J*=11.2 Hz, 2.0 Hz, 1 H), 7.29 (t, *J*=8.8 Hz, 1 H), 7.20 (d, *J*=8.4 Hz, 1 H), 7.04 (d, *J*=7.2 Hz, 1 H), 6.72 (d, *J*=8.0 Hz, 1 H), 5.18 (s, 2 H), 5.10-5.08 (m, 1 H), 4.94-4.92 (m, 1 H), 4.81-4.78 (m, 1 H), 4.66-4.63 (m, 1 H), 4.50-4.49 (m, 1 H), 4.39-4.31 (m, 1.8 H), 3.94 (d, *J*=13.6 Hz, 1 H), 3.79 (d, *J*=13.6 Hz, 1 H), 3.39-3.38 (m, 1.6 H), 2.70-2.69 (m, 3 H), 2.43-2.32 (m, 3H), 1.92-1.91 (m, 2 H), 1.63-1.59 (m, 2 H), 1.39-1.28 (m, 3.2 H), 0.88-0.85 (m, 2.3H).

### Example 20. Preparation of crystal form R of n-propanol solvate of compound I

About 20 mg of compound I was suspended in 0.5 mL of methyl *tert-butyl* ether/n-propanol (1:1, v:v). The mixture was stirred at 50 °C for 3 d and filtered. The filter cake was dried in an oven at 50 °C to give crystal form R of a n-propanol solvate of the compound. The product was analyzed and characterized by XRPD (FIG. 19), DSC (FIG. 40), and TGA (FIG. 61). ¹H NMR (400 MHz, DMSO-*d₆*): δ 12.80 (br, 1H), 8.27 (s, 1 H), 7.81 (dd, *J*=8.4 Hz, 1.2 Hz, 1 H), 7.79 (t, *J*=7.6 Hz, 1 H), 7.72 (d, *J*=8.4 Hz, 1 H), 7.45 (dd, *J*=11.2 Hz, 2.0 Hz, 1 H), 7.29 (t, *J*=8.8 Hz, 1 H), 7.19 (d, *J*=8.4 Hz, 1 H), 7.04 (d, *J*=7.2 Hz, 1 H), 6.72 (d, *J*=8.0 Hz, 1 H), 5.18 (s, 2 H), 5.10-5.08 (m, 1 H), 4.94-4.92 (m, 1 H), 4.81-4.78 (m, 1 H), 4.66-4.63 (m, 1 H), 4.50-4.49 (m, 1 H), 4.38-4.36 (m, 1.6 H), 3.95 (d, J=13.6 Hz, 1 H), 3.79 (d, J=13.6 Hz, 1 H), 3.36-3.32 (m, 1H), 2.71-2.69 (m, 3 H), 2.49-2.32 (m, 3H), 1.92-1.91 (m, 2 H), 1.63-1.59 (m, 2 H), 1.44-1.39 (m, 1.6 H), 0.85-0.82 (m, 2.5 H).

### Example 21. Preparation of crystal form S of tetrahydrofuran solvate of compound I

About 20 mg of compound I was dissolved in 0.5 mL of tetrahydrofuran. The solution was then transferred into a 20-mL vial containing 3 mL of isopropyl ether. The 20-mL vial was sealed with a cap, let stand at room temperature for 2 d, and filtered. The filter cake was dried in an oven at 50 °C to give crystal form S of a tetrahydrofuran solvate of the compound. The product was analyzed and characterized by XRPD (FIG. 20), DSC (FIG. 41), and TGA (FIG. 62). ¹H NMR (400 MHz, DMSO-*d₆*): δ 12.80 (br, 1H), 8.26 (s, 1 H), 7.81 (dd, J=8.4 Hz, 1.2 Hz, 1 H), 7.79 (t, J=7.6 Hz, 1 H), 7.72 (d, *J*=8.4 Hz, 1 H), 7.45 (dd, *J=*11.2 Hz, 2.0 Hz, 1 H), 7.29 (t, J=8.8 Hz, 1 H), 7.19 (d, *J=8.4* Hz, 1 H), 7.04 (d, J=7.2 Hz, 1 H), 6.72 (d, *J=8.0* Hz, 1 H), 5.18 (s, 2 H), 5.10-5.08 (m, 1 H), 4.94-4.92 (m, 1 H), 4.78-4.76 (m, 1 H), 4.66-4.63 (m, 1 H), 4.50-4.49 (m, 1 H), 4.38-4.36 (m, 1 H), 3.95 (d, *J* =13.6 Hz, 1 H), 3.79 (d, *J* = 13.6 Hz, 1 H), 3.62-3.59 (m, 2.8 H), 2.73-2.69 (m, 3 H), 2.43-2.32 (m, 3H), 1.92-1.90 (m, 2 H), 1.78-1.74 (m, 2.7 H), 1.63-1.61 (m, 2 H).

### Example 22. Preparation of crystal form T of 2-methyltetrahydrofuran solvate of compound I

About 20 mg of compound I was dissolved in 0.5 mL of 2-methyltetrahydrofuran. The solution was then transferred into a 20-mL vial containing 3 mL of diethyl ether. The 20-mL vial was sealed with a cap, let stand at room temperature for 2 d, and filtered. The filter cake was dried in an oven at 50 °C to give crystal form T of a 2-methyltetrahydrofuran solvate of the compound. The product was analyzed and characterized by XRPD (FIG. 21), DSC (FIG. 42), and TGA (FIG. 63). ¹H NMR (400 MHz, DMSO-*d₆*): δ 12.80 (br, 1H), 8.27 (s, 1 H), 7.81 (dd, *J*=8.4 Hz, 1.2 Hz, 1 H), 7.79 (t, *J*=7.6 Hz, 1 H), 7.72 (d, *J*=8.4 Hz, 1 H), 7.45 (dd, *J*=11.2 Hz, 2.0 Hz, 1 H), 7.29 (t, *J*=8.8 Hz, 1 H), 7.19 (d, *J*=8.4 Hz, 1 H), 7.04 (d, *J*=7.2 Hz, 1 H), 6.72 (d, *J*=8.0 Hz, 1 H), 5.18 (s, 2 H), 5.10-5.08 (m, 1 H), 4.94-4.92 (m, 1 H), 4.78-4.76 (m, 1 H), 4.66-4.63 (m, 1 H), 4.50-4.49 (m, 1 H), 4.38-4.36 (m, 1 H), 3.95 (d, *J*=13.6 Hz, 1 H), 3.83-3.74 (m, 2.5 H), 3.56-3.54 (m, 1 H), 2.73-2.69 (m, 3 H), 2.43-2.32 (m, 3H), 1.94-1.89 (m, 3 H), 1.89-1.79 (m, 1.6 H), 1.63-1.61 (m, 2 H), 1.36-1.26 (m, 2H), 1.12 (d, *J*=6.8 Hz, 2.3H).

### Example 2-1. Preparation of (S)-2-((4-((6-((4-cyano-2-fluorophenoxy)methyl)pyridin-2-yl)oxy)piperidin-1-yl)methyl)-1-(oxetan-2-ylmethyl )-1H-benzo[d]imidazole-6-carboxylic acid

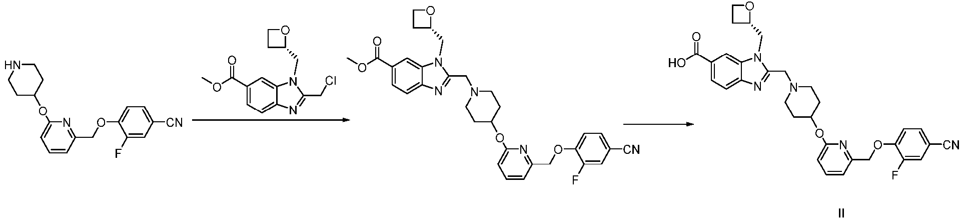

### Step 1: Synthesis of methyl (S)-2-((4-((6-((4-cyano-2-fluorophenoxy)methyl)pyridin-2-yl)oxy)piperidin-1-yl)methyl)-1-(oxetan-2-ylmethyl)-1 H-benzo[d]imidazole-6-carboxylate

A mixed solution of methyl (*S*)-2-(chloromethyl)-1-(oxetan-2-ylmethyl)-1H-benzo[*d*]imidazole-6-carboxylate (1.5 g, 5.1 mmol), 2-(4-cyano-2-fluorophenoxy)methyl)-6-(piperidin-4-yloxy)pyridine (1.8 g, 5.5 mmol), and potassium carbonate (1.8 g, 13.0 mmol) in *N,N*-dimethylformamide (80 mL) was stirred at 60 °C for 3 h, then quenched with water (100 mL), and extracted with ethyl acetate (50 mL × 3). The combined organic layers were washed with brine (50 mL × 3), dried over anhydrous sodium sulfate, filtered, and concentrated. The residue was purified by silica gel column chromatography (dichloromethane/methanol = 20/1) to give methyl (*S*)-2-((4-((6-((4-cyano-2-fluorophenoxy)methyl)pyridin-2-yl)oxy)piperidin-1-yl)methyl)-1-(oxetan-2-ylmethyl)-1 *H*-benzo[*d*]imidazole-6-carboxylate (1.1 g, 37.2% yield).

### Step 2: Synthesis of (S)-2-((4-((6-((4-cyano-2-fluorophenoxy)methyl)pyridin-2-yl)oxy)piperidin-1-yl)methyl)-1-(oxetan-2-ylmethyl)-1 H-benzo[d]imidazole-6-carboxylate

Lithium hydroxide (0.13 g, 5.4 mmol) was added to a mixed solution of methyl (*S*)-2-((4-((6-((4-cyano-2-fluorophenoxy)methyl)pyridin-2-yl)oxy)piperidin-1-yl)methyl)-1-(oxetan-2-ylmethyl)-1 *H*-benzo[*d*]imidazole-6-carboxylate (1.1 g, 1.9 mmol) in tetrahydrofuran/water (20 mL/20 mL), and the mixture was stirred at room temperature for 16 h. The pH of the resulting mixture was adjusted to 5-6 with formic acid, and the solvent was removed *in vacuo.* The residue was purified by reverse-phase flash column chromatography to give (*S*)-2-((4-((6-((4-cyano-2-fluorophenoxy)methyl)pyridin-2-yl)oxy)piperidin-1-yl)methyl)-1-(oxetan-2-ylmethyl)-1 *H*-benzo[*d*]imidazole-6-carboxylic acid (0.70 g, 65.5% yield). The resultant compound II was confirmed by XRPD, DSC, and TGA characterizations as a partially crystalline compound with its X-ray powder diffraction pattern, DSC profile, and TGA profile shown in FIGs. 2-76, 2-77, and 2-78, respectively. ¹HNMR (400 MHz, DMSO-*d₆*): δ 8.23 (s, 1 H), 7.88 (dd, *J*=2.0 Hz, 11.6 Hz, 1 H), 7.80 (dd, *J =* 1.6, 8.8 Hz, 1 H), 7.73 (t, *J =* 8.0 Hz, 1 H), 7.67 (d, *J* =8.4 Hz, 1 H), 7.60 (d, *J* =8.8 Hz, 1 H), 7.45 (t, *J =* 8.4 Hz, 1 H), 7.06 (d, *J* =7.6 Hz, 1 H), 6.74 (d*, J =*8.4 Hz*,* 1 H), 5.31 (s, 2 H), 5.10-5.08 (m, 1 H), 4.92-4.90 (m, 1H), 4.80-4.74 (m, 1 H), 4.65-4.61 (m, 1 H), 4.50-4.47 (m, 1 H), 4.40-4.35 (m, 1 H), 3.93 (d, *J =* 13.6 Hz, 1 H), 3.78 (d, *J =* 13.6 Hz, 1 H), 2.79-2.67 (m, 3 H), 2.51-2.41 (m, 1 H), 2.32-2.27 (m, 2 H), 1.92-1.89 (m, 2 H) 1.63-1.60 (m, 2 H).

### Example 2-2. Preparation of crystal form 2A of compound II

200 mg of compound II was added to 5.0 mL of isopropanol. The mixture was stirred at room temperature for 1 d and filtered. The filter cake was dried in an oven at 50 °C to give crystal form 2A of compound II. The product was analyzed and characterized by XRPD (FIG. 2-1), DSC (FIG. 2-26), and TGA (FIG. 2-51). ¹HNMR (400 MHz, DMSO-*d₆*): δ 12.76 (s, 1 H), 8.27 (s, 1 H), 7.88 (dd, *J*=2.0 Hz, 11.6 Hz, 1 H), 7.80 (dd, *J* = 1.6, 8.8 Hz, 1 H), 7.72 (t, *J =* 8.0 Hz, 1 H), 7.71-7.64 (m, 2 H), 7.44 (t, *J =* 8.4 Hz, 1 H), 7.06 (d, *J* =7.6 Hz, 1 H), 6.74 (d, *J* =8.4 Hz, 1 H), 5.31 (s, 2 H), 5.10-5.08 (m, 1 H), 4.92-4.90 (m, 1H), 4.80-4.74 (m, 1 H), 4.66-4.61 (m, 1 H), 4.51-4.49 (m, 1 H), 4.40-4.36 (m, 1 H), 3.94 (d, *J =* 13.5 Hz, 1 H), 3.78 (d, *J =* 13.5 Hz, 1 H), 2.73-2.69 (m, 3 H), 2.50-2.30 (m, 3 H), 1.91-1.89 (m, 2 H) 1.63-1.61 (m, 2 H).

### Example 2-3. Preparation of crystal form 2B of compound II

20 mg of compound II was added to 0.5 mL of acetone/water (1:6, v:v). The mixture was stirred at 50 °C for 3 d and filtered. The filter cake was dried in an oven at 50 °C to give crystal form 2B of compound II. The product was analyzed and characterized by XRPD (FIG. 2-2), DSC (FIG. 2-27), and TGA (FIG. 2-52). ¹HNMR (400 MHz, DMSO-*d₆*): δ 12.76 (br, 1 H), 8.26 (s, 1 H), 7.88 (dd, *J*=2.0 Hz, 11.6 Hz, 1 H), 7.80 (dd, *J*= 1.6, 8.8 Hz, 1 H), 7.72 (t, *J =* 8.0 Hz, 1 H), 7.71-7.65 (m, 2 H), 7.45 (t, *J* = 8.4 Hz, 1 H), 7.06 (d, *J* =7.6 Hz, 1 H), 6.74 (d, *J* =8.4 Hz, 1 H), 5.31 (s, 2 H), 5.10-5.08 (m, 1 H), 4.92-4.90 (m, 1H), 4.80-4.74 (m, 1 H), 4.66-4.63 (m, 1 H), 4.51-4.49 (m, 1 H), 4.40-4.36 (m, 1 H), 3.94 (d, *J =* 13.5 Hz, 1 H), 3.78 (d, *J =* 13.5 Hz, 1 H), 2.70-2.69 (m, 3 H), 2.51-2.30 (m, 3 H), 1.91-1.89 (m, 2 H) 1.61-1.59 (m, 2 H).

### Example 2-4. Preparation of crystal form 2C of compound II

20 mg of compound II was dissolved in 0.2 mL ethanol/toluene (2:1, v:v). The mixture was let stand at room temperature to slowly evaporate the solvent. The resultant solid was dried at 50 °C to give crystal form 2C of compound II. The product was analyzed and characterized by XRPD (FIG. 2-3), DSC (FIG. 2-28), and TGA (FIG. 2-53). ¹HNMR (400 MHz, DMSO-*d₆*): δ 12.76 (br, 1 H), 8.26 (s, 1 H), 7.88 (dd, *J*=2.0 Hz, 11.6 Hz, 1 H), 7.80 (dd, *J=* 1.6, 8.8 Hz, 1 H), 7.72 (t, *J =* 8.0 Hz, 1 H), 7.71-7.65 (m, 2 H), 7.45 (t, *J =* 8.5 Hz, 1 H), 7.05 (d, *J* =7.5 Hz, 1 H), 6.74 (d, *J* =8.5 Hz, 1 H), 5.31 (s, 2 H), 5.10-5.09 (m, 1 H), 4.92-4.91 (m, 1H), 4.80-4.75 (m, 1 H), 4.66-4.64 (m, 1 H), 4.51-4.49 (m, 1 H), 4.39-4.36 (m, 1 H), 3.94 (d, *J=* 13.5 Hz, 1 H), 3.78 (d, *J=* 13.5 Hz, 1 H), 2.70-2.69 (m, 3 H), 2.50-2.30 (m, 3 H), 1.91-1.89 (m, 2 H) 1.61-1.59 (m, 2 H).

### Example 2-5. Preparation of crystal form 2D of compound II

20 mg of compound II was added to 0.5 mL of isopropyl acetate/dichloromethane (6:1, v:v). The mixture was stirred at room temperature for 3 d and filtered. The filter cake was dried in an oven at 50 °C to give crystal form 2D of compound II. The product was analyzed and characterized by XRPD (FIG. 2-4), DSC (FIG. 2-29), and TGA (FIG. 2-54). ¹HNMR (400 MHz, DMSO-*d₆*): δ 12.77 (br, 1 H), 8.26 (s, 1 H), 7.88 (dd, *J=*2.0 Hz, 11.5 Hz, 1 H), 7.80 (dd, *J =* 1.7, 8.8 Hz, 1 H), 7.72 (t, *J =* 8.0 Hz, 1 H), 7.71-7.63 (m, 2 H), 7.45 (t, *J =* 8.5 Hz, 1 H), 7.06 (d, *J* =7.5 Hz, 1 H), 6.74 (d, *J* =8.6 Hz, 1 H), 5.31 (s, 2 H), 5.10-5.08 (m, 1 H), 4.92-4.91 (m, 1H), 4.80-4.75 (m, 1 H), 4.66-4.64 (m, 1 H), 4.51-4.49 (m, 1 H), 4.39-4.36 (m, 1 H), 3.92 (d, *J=* 13.5 Hz, 1 H), 3.76 (d, *J =* 13.5 Hz, 1 H), 2.76-2.71 (m, 3 H), 2.50-2.30 (m, 3 H), 1.96-1.90 (m, 2 H) 1.61-1.59 (m, 2 H).

### Example 2-6. Preparation of crystal form 2E of compound II

20 mg of compound II was suspended in 0.5 mL of chloroform/*n*-heptane (1:9, v:v). The mixture was stirred at 50 °C for 3 d and filtered. The filter cake was dried in an oven at 50 °C to give crystal form 2E of compound II. The product was analyzed and characterized by XRPD (FIG. 2-5), DSC (FIG. 2-30), and TGA (FIG. 2-55). ¹HNMR (400 MHz, DMSO-*d₆*): δ 12.75 (br, 1 H), 8.26 (s, 1 H), 7.88 (dd, J=2.0 Hz, 11.5 Hz, 1 H), 7i.80 (dd, J = 1.7, 8.5 Hz, 1 H), 7.72 (t, *J* = 8.1 Hz, 1 H), 7.71-7.63 (m, 2 H), 7.45 (t, *J* = 8.6 Hz, 1 H), 7.05 (d, *J* =7.5 Hz, 1 H), 6.73 (d,*J* =8.5 Hz, 1 H), 5.31 (s, 2 H), 5.11-5.08 (m, 1 H), 4.92-4.90 (m, 1H), 4.80-4.77 (m, 1 H), 4.66-4.64 (m, 1 H), 4.50-4.48 (m, 1 H), 4.38-4.36 (m, 1 H), 3.92 (d, *J =* 13.5 Hz, 1 H), 3.78 (d, *J =* 13.5 Hz, 1 H), 2.67-2.60 (m, 3 H), 2.50-2.30 (m, 3 H), 1.90-1.88 (m, 2 H) 1.62-1.60 (m, 2 H).

### Example 2-7. Preparation of crystal form 2F of hydrate of compound II

20 mg of compound II was suspended in 0.5 mL of ethanol/water (1:4, v:v). The mixture was stirred at room temperature for 3 d and filtered. The filter cake was dried in an oven at 50 °C to give crystal form 2F of compound II. The product was analyzed and characterized by XRPD (FIG. 2-6), DSC (FIG. 2-31), and TGA (FIG. 2-56). ¹HNMR (400 MHz, DMSO-*d₆*): δ 12.69 (br, 1 H), 8.26 (s, 1 H), 7.87 (dd, *J*=2.0 Hz, 11.5 Hz, 1 H), 7.80 (dd, *J =* 1.7, 8.5 Hz, 1 H), 7.73 (t, *J =* 8.1 Hz, 1 H), 7.71-7.63 (m, 2 H), 7.44 (t, *J =* 8.6 Hz, 1 H), 7.06 (d, *J* =7.5 Hz, 1 H), 6.74 (d, *J* =8.5 Hz, 1 H), 5.31 (s, 2 H), 5.11-5.08 (m, 1 H), 4.92-4.90 (m, 1H), 4.80-4.77 (m, 1 H), 4.66-4.64 (m, 1 H), 4.50-4.48 (m, 1 H), 4.38-4.36 (m, 1 H), 3.92 (d, *J =* 13.5 Hz, 1 H), 3.78 (d, *J =* 13.5 Hz, 1 H), 2.67-2.60 (m, 3 H), 2.50-2.30 (m, 3 H), 1.90-1.88 (m, 2 H), 1.62-1.60 (m, 2 H).

### Example 2-8. Preparation of crystal form 2G of hydrate of compound II

20 mg of compound II was suspended in 0.5 mL of ethyl acetate/toluene (1:3, v:v). The mixture was stirred at 50 °C for 3 d and filtered. The filter cake was dried in an oven at 50 °C to give crystal form 2G of a hydrate of compound II. The product was analyzed and characterized by XRPD (FIG. 2-7), DSC (FIG. 2-32), and TGA (FIG. 2-57). ¹HNMR (400 MHz, DMSO-*d₆*): δ 12.71 (br, 1 H), 8.26 (s, 1 H), 7.87 (dd, *J=*2.0 Hz, 11.5 Hz, 1 H), 7.80 (dd, *J =* 1.7, 8.5 Hz, 1 H), 7.73 (t, *J =* 8.1 Hz, 1 H), 7.71-7.63 (m, 2 H), 7.445 (t, *J =* 8.5 Hz, 1 H), 7.06 (d, *J* = 7.5 Hz, 1 H), 6.73 (d, *J* =8.5 Hz, 1 H), 5.31 (s, 2 H), 5.10-5.08 (m, 1 H), 4.92-4.90 (m, 1H), 4.80-4.78 (m, 1 H), 4.66-4.64 (m, 1 H), 4.50-4.48 (m, 1 H), 4.38-4.36 (m, 1 H), 3.94 (d, *J =* 13.5 Hz, 1 H), 3.78 (d, *J =* 13.5 Hz, 1 H), 2.70-2.69 (m, 3 H), 2.50-2.30 (m, 3 H), 1.90-1.88 (m, 2 H) 1.61-1.59 (m, 2 H).

### Example 2-9. Preparation of crystal form 2H of dimethylsulfoxide solvate of compound II

20 mg of compound II was transferred into a 3-mL vial before the vial was placed into a 20-mL vial containing 3 mL of DMSO. The 20-mL vial was sealed with a cap and let stand at room temperature for 10 d. The resultant solid was dried in an oven at 50 °C to give crystal form 2H of a hydrate of compound II. The product was analyzed and characterized by XRPD (FIG. 2-8), DSC (FIG. 2-33), and TGA (FIG. 2-58). ¹H NMR (400 MHz,CD₃OD): δ 8.23 (s, 1 H), 7.97 (dd, *J*=8.5 Hz, 1.2 Hz, 1 H), 7.69-7.65 (m, 2 H), 7.56 (dd, *J* = 11.2 Hz, 2.0 Hz, 1 H), 7.50 (d, *J =*11.2 Hz, 2.0 Hz, 1 H), 7.32 (t, *J*=8.7 Hz, 1 H), 7.05 (d, *J*=8.5 Hz, 1 H), 7.51-7.49 (m, 1 H), 6.71 (d, *J*=8.0 Hz, 1 H), 5.25 (s, 3 H), 5.15-5.03 (m, 1 H), 4.74-4.73 (m, 2 H), 4.51-4.49 (m, 1 H), 4.04 (d, *J* = 13.5 Hz, 1 H), 3.94 (d, *J* =13.5 Hz, 1 H), 2.83-2.79 (m, 3 H), 2.65 (s, 6 H), 2.48-2.46 (m, 3 H), 2.01-1.99 (m, 2 H), 1.30-1.29 (m, 2 H).

### Example 2-10. Preparation of crystal form 2I of methyl tert-butyl ether solvate of compound II

20 mg of compound II was suspended in 0.5 mL of dioxane/methyl *tert-*butyl ether (1:9, v:v). The mixture was stirred at 50 °C for 2 h, cooled to 5 °C, stirred for another 2 h, and filtered. The filter cake was dried in an oven at 50 °C to give crystal form 2I of a methyl *tert-*butyl ether solvate of compound II. The product was analyzed and characterized by XRPD (FIG. 2-9), DSC (FIG. 2-34), and TGA (FIG. 2-59). ¹HNMR (400 MHz, DMSO-*d₆*): δ 12.76 (br, 1 H), 8.26 (s, 1 H), 7.88 (dd, *J*=2.0 Hz, 11.5 Hz, 1 H), 7.80 (dd, *J* = 1.7, 8.5 Hz, 1 H), 7.75 (t, *J =* 8.1 Hz, 1 H), 7.71-7.63 (m, 2 H), 7.45 (t, *J* = 8.5 Hz, 1 H), 7.05 (d, *J* =5.5 Hz, 1 H), 6.74 (d, *J* =8.5 Hz, 1 H), 5.31 (s, 2 H), 5.09-5.08 (m, 1 H), 4.91-4.90 (m, 1H), 4.79-4.76 (m, 1 H), 4.65-4.62 (m, 1 H), 4.50-4.47 (m, 1 H), 4.38-4.36 (m, 1 H), 3.94 (d, *J =* 13.5 Hz, 1 H), 3.78 (d, *J =* 13.5 Hz, 1 H), 3.57 (s, 1.5 H), 2.75-2.69 (m, 3 H), 2.50-2.41 (m, 3 H), 1.91-1.89 (m, 2 H), 1.63-1.58 (m, 2 H), 1.11 (s, 4.5 H).

### Example 2-11. Preparation of crystal form 2J-1 of n-heptane solvate of compound II

20 mg of compound II was suspended in 0.5 mL of acetone/*n*-heptane (1:4, v:v). The mixture was stirred at 5 °C for 3 d and filtered. The filter cake was dried in an oven at 50 °C to give crystal form 2J-1 of a n-heptane solvate of compound II. The product was analyzed and characterized by XRPD (FIG. 2-10), DSC (FIG. 2-35), and TGA (FIG. 2-60). ¹H NMR (400 MHz, DMSO-*d₆*) δ 12.75 (br, 1 H), 8.26 (s, 1 H), 7.87 (dd, *J*=2.0 Hz, 11.5 Hz, 1 H), 7.80 (dd, *J =* 1.7, 8.5 Hz, 1 H), 7.73 (t, *J =* 8.1 Hz, 1 H), 7.67-7.63 (m, 2 H), 7.45 (t, *J =* 8.5 Hz, 1 H), 7.05 (d, *J* =5.5 Hz, 1 H), 6.74 (d, *J* =8.5 Hz, 1 H), 5.31 (s, 2 H), 5.09-5.08 (m, 1 H), 4.91-4.90 (m, 1H), 4.79-4.76 (m, 1 H), 4.65-4.61 (m, 1 H), 4.51-4.48 (m, 1 H), 4.38-4.36 (m, 1 H), 3.94 (d, *J=* 13.5 Hz, 1 H), 3.78 (d, *J=* 13.5 Hz, 1 H), 2.70-2.69 (m, 3 H), 2.50-2.43 (m, 3 H), 1.90-1.88 (m, 2 H), 1.61-1.59 (m, 2 H), 1.24 (s, 2 H), 0.86 (s, 1.3 H).

### Example 2-12. Preparation of crystal form 2J-2 of n-heptane solvate of compound II

20 mg of compound II was dissolved in 3.0 mL chloroform/n-heptane (2:1, v:v). The mixture was let stand at room temperature to slowly evaporate the solvent. The resultant solid was dried at 50 °C to give crystal form 2J-2 of a n-heptane solvate of compound II. The product was analyzed and characterized by XRPD (FIG. 2-11), DSC (FIG. 2-36), and TGA (FIG. 2-61). ¹H NMR (400 MHz, DMSO-*d₆*) δ 12.55 (br, 1 H), 8.27 (s, 1 H), 7.88 (dd, *J*=2.0 Hz, 11.5 Hz, 1 H), 7.80 (dd, *J* = 1.7, 8.5 Hz, 1 H), 7.73 (t, *J* = 8.1 Hz, 1 H), 7.67-7.63 (m, 2 H), 7.43 (t, *J* = 8.5 Hz, 1 H), 7.06 (d, *J* =5.5 Hz, 1 H), 6.74 (d, *J* =8.5 Hz, 1 H), 5.31 (s, 2 H), 5.09-5.08 (m, 1 H), 4.91-4.90 (m, 1H), 4.78-4.76 (m, 1 H), 4.67-4.65 (m, 1 H), 4.51-4.49 (m, 1 H), 4.38-4.36 (m, 1 H), 3.92 (d, *J=* 13.5 Hz, 1 H), 3.80 (d, *J= 13.5* Hz, 1 H), 2.70-2.69 (m, 3 H), 2.33-2.30 (m, 3 H), 1.90-1.88 (m, 2 H), 1.61-1.59 (m, 2 H), 1.24 (s, 4 H), 0.87-0.86 (m, 2.5 H).

### Example 2-13. Preparation of crystal form 2K of toluene solvate of compound II

About 20 mg of compound II was suspended in 0.5 mL of toluene. The mixture was stirred at 50 °C for 2 h, cooled to 5 °C, stirred for another 2 h, and filtered. The filter cake was dried in an oven at 50 °C to give crystal form 2K of a toluene solvate of compound II. The product was analyzed and characterized by XRPD (FIG. 2-12), DSC (FIG. 2-37), and TGA (FIG. 2-62). ¹H NMR (400 MHz, DMSO-*d₆*) δ 12.73 (br, 1 H), 8.26 (s, 1 H), 7.88 (dd, *J=2.0* Hz, 11.5 Hz, 1 H), 7.80 (dd, *J* = 1.7, 8.5 Hz, 1 H), 7.73 (t, *J =* 8.1 Hz, 1 H), 7.67-7.63 (m, 2 H), 7.45 (t, J = 8.5 Hz, 1 H), 7.27-7.13 (m, 4 H), 7.06 (d, *J* =5.5 Hz, 1 H), 6.74 (d, *J* =8.5 Hz, 1 H), 5.31 (s, 2 H), 5.09-5.08 (m, 1 H), 4.92-4.90 (m, 1H), 4.81-4.79 (m, 1 H), 4.65-4.62 (m, 1 H), 4.52-4.49 (m, 1 H), 4.40-4.36 (m, 1 H), 3.92 *(d, J=* 13.5 Hz, 1 H), 3.80 (d, *J =* 13.5 Hz, 1 H), 2.75-2.69 (m, 3 H), 2.46-2.41 (m, 3 H), 2.30 (s, 2.5 H), 1.90-1.88 (m, 2 H), 1.63-1.58 (m, 2 H).

### Example 2-14. Preparation of crystal form 2L-1 of methyl isobutyl ketone solvate of compound II

About 20 mg of compound II was suspended in 0.5 mL of methyl isobutyl ketone. The mixture was stirred at room temperature for 3 d and filtered. The filter cake was dried in an oven at 50 °C to give crystal form 2L-1 of a methyl isobutyl ketone solvate of compound II. The product was analyzed and characterized by XRPD (FIG. 2-13), DSC (FIG. 2-38), and TGA (FIG. 2-63). ¹H NMR (400 MHz, DMSO-*d₆*) δ 12.76 (br, 1 H), 8.26 (s, 1 H), 7.88 (dd, *J*=2.0 Hz, 11.5 Hz, 1 H), 7.79 (dd, *J =* 1.7, 8.5 Hz, 1 H), 7.73 (t, *J =* 8.1 Hz, 1 H), 7.67-7.62 (m, 2 H), 7.45 (t, *J =* 8.5 Hz, 1 H), 7.06 (d, *J* =5.5 Hz, 1 H), 6.73 (d, *J* =8.5 Hz, 1 H), 5.31 (s, 2 H), 5.09-5.08 (m, 1 H), 4.92-4.90 (m, 1H), 4.81-4.79 (m, 1 H), 4.65-4.62 (m, 1 H), 4.52-4.49 (m, 1 H), 4.40-4.36 (m, 1 H), 3.93 (d, *J=* 13.5 Hz, 1 H), 3.78 (d, *J*= 13.5 Hz, 1 H), 2.70-2.69 (m, 3 H), 2.43-2.30 (m, 5 H), 2.06 (s, 2 H), 2.00-1.98 (m, 0.6 H), 1.90-1.88 (m, 2 H), 1.61-1.59 (m, 2 H), 0.85 (d, *J=* 5.8 Hz, 4 H).

### Example 2-15. Preparation of crystal form 2L-2 of methyl isobutyl ketone solvate of compound II

About 20 mg of compound II was suspended in 0.5 mL of methyl isobutyl ketone/chloroform (9:1, v:v). The mixture was stirred at 50 °C for 2 h, cooled to 5 °C, stirred for another 2 h, and filtered. The filter cake was dried in an oven at 50 °C to give crystal form 2L-2 of a methyl isobutyl ketone solvate of compound II. The product was analyzed and characterized by XRPD (FIG. 2-14), DSC (FIG. 2-39), and TGA (FIG. 2-64). ¹H NMR (400 MHz, DMSO-*d₆*) δ 12.76 (br, 1 H), 8.26 (s, 1 H), 7.89 (dd, *J*=2.0 Hz, 11.5 Hz, 1 H), 7.79 (dd, *J=* 1.7, 8.5 Hz, 1 H), 7.73 (t, *J =* 8.1 Hz, 1 H), 7.67-7.63 (m, 2 H), 7.45 (t, *J =* 8.5 Hz, 1 H), 7.06 (d, *J* =5.5 Hz, 1 H), 6.73 (d, *J* =8.5 Hz, 1 H), 5.31 (s, 2 H), 5.09-5.08 (m, 1 H), 4.92-4.90 (m, 1H), 4.81-4.79 (m, 1 H), 4.65-4.62 (m, 1 H), 4.51-4.49 (m, 1 H), 4.39-4.36 (m, 1 H), 3.92 (d, *J =* 13.5 Hz, 1 H), 3.78 (d, *J =* 13.5 Hz, 1 H), 2.70-2.69 (m, 3 H), 2.43-2.29 (m, 3.5 H), 2.06 (s, 1 H), 2.00-1.98 (m, 0.3 H), 1.91-1.89 (m, 2 H), 1.61-1.59 (m, 2 H), 0.85 (d, *J =* 5.8 Hz, 2.2 H).

### Example 2-16. Preparation of crystal form 2M-1 of cyclopentyl methyl ether solvate of compound II

About 20 mg of compound II was suspended in 0.5 mL of methanol/cyclopentyl methyl ether (1:6, v:v). The mixture was stirred at room temperature for 3 d and filtered. The filter cake was dried in an oven at 50 °C to give crystal form 2M-1 of a cyclopentyl methyl ether solvate of compound II. The product was analyzed and characterized by XRPD (FIG. 2-15), DSC (FIG. 2-40), and TGA (FIG. 2-65). ¹HNMR (400 MHz, DMSO-*d₆*): δ 12.74 (br, 1 H), 8.26 (s, 1 H), 7.88 (dd, J=2.0 Hz, 11.5 Hz, 1 H), 7.81 (dd, *J* = 1.7, 8.8 Hz, 1 H), 7.73 *(t, J=* 8.0 Hz, 1 H), 7.71-7.63 (m, 2 H), 7.45 (t, *J* = 8.5 Hz, 1 H), 7.06 (d, *J* =7.5 Hz, 1 H), 6.74 (d, *J* =8.6 Hz, 1 H), 5.31 (s, 2 H), 5.09-5.08 (m, 1 H), 4.92-4.90 (m, 1H), 4.81-4.76 (m, 1 H), 4.66-4.62 (m, 1 H), 4.52-4.49 (m, 1 H), 4.38-4.36 (m, 1 H), 3.94 (d, *J =* 13.5 Hz, 1 H), 3.79 (d, *J =* 13.5 Hz, 1 H), 3.75-3.74 (m, 1H), 3.31 (s, 3 H), 2.75-2.69 (m, 3 H), 2.50-2.30 (m, 3 H), 1.91-1.89 (m, 2 H), 1.63-1.46 (m, 10 H).

### Example 2-17. Preparation of crystal form 2M-2 of cyclopentyl methyl ether solvate of compound II

About 20 mg of compound II was dissolved in 0.8 mL of dioxane/toluene (1:1, v:v). The solution was dropwise added to 3.0 mL of cyclopentyl methyl ether, and the mixture was filtered. The filter cake was dried in an oven at 50 °C to give crystal form 2M-2 of a cyclopentyl methyl ether solvate of the compound. The product was analyzed and characterized by XRPD (FIG. 2-16), DSC (FIG. 2-41), and TGA (FIG. 2-66). ¹HNMR (400 MHz, DMSO-*d₆*): δ 12.88 (br, 1 H), 8.26 (s, 1 H), 7.88 (dd, *J*=2.0 Hz, 11.5 Hz, 1 H), 7.80 (dd, *J =* 1.7, 8.8 Hz, 1 H), 7.73 (t, *J =* 8.0 Hz, 1 H), 7.71-7.63 (m, 2 H), 7.45 (t, *J =* 8.5 Hz, 1 H), 7.06 (d, *J* =7.5 Hz, 1 H), 6.74 (d, *J* =8.5 Hz, 1 H), 5.31 (s, 2 H), 5.09-5.08 (m, 1 H), 4.92-4.90 (m, 1H), 4.77-4.76 (m, 1 H), 4.66-4.62 (m, 1 H), 4.50-4.49 (m, 1 H), 4.38-4.36 (m, 1 H), 3.93 (d, *J =* 13.5 Hz, 1 H), 3.77 (d, *J=* 13.5 Hz, 1 H), 3.75-3.74 (m, 0.3 H), 3.32 (s, 1.2 H), 2.75-2.69 (m, 3 H), 2.50-2.30 (m, 3 H), 1.91-1.90 (m, 2 H), 1.63-1.50 (m, 6 H).

### Example 2-18. Preparation of crystal form 2N of methyl ethyl ketone solvate of compound II

About 20 mg of compound II was suspended in 0.5 mL of methyl ethyl ketone/acetonitrile (1:2, v:v). The mixture was stirred at 50 °C for 3 d and filtered. The filter cake was dried in an oven at 50 °C to give crystal form 2N of a methyl ethyl ketone solvate of compound II. The product was analyzed and characterized by XRPD (FIG. 2-17), DSC (FIG. 2-42), and TGA (FIG. 2-67). ¹HNMR (400 MHz, DMSO-*d₆*): δ 12.65 (br, 1 H), 8.26 (s, 1 H), 7.87 (dd, *J*=2.0 Hz, 11.5 Hz, 1 H), 7.80 (dd, *J* = 1.7, 8.5 Hz, 1 H), 7.73 (t, *J =* 8.1 Hz, 1 H), 7.71-7.63 (m, 2 H), 7.45 (t, *J =* 8.6 Hz, 1 H), 7.06 (d, *J* =7.5 Hz, 1 H), 6.74 (d, *J* =8.5 Hz, 1 H), 5.31 (s, 2 H), 5.10-5.08 (m, 1 H), 4.92-4.90 (m, 1H), 4.80-4.78 (m, 1 H), 4.66-4.64 (m, 1 H), 4.50-4.48 (m, 1 H), 4.38-4.36 (m, 1 H), 3.94 (d, *J =* 13.5 Hz, 1 H), 3.79 (d, *J =* 13.5 Hz, 1 H), 2.70-2.69 (m, 3 H), 2.46-2.30 (m, 4 H), 2.07 (s, 1.2 H), 1.91-1.89 (m, 2 H) 1.61-1.59 (m, 2 H), 0.93-0.89 (m, 1.2 H).

### Example 2-19. Preparation of crystal form 2O of methylcyclohexane solvate of compound II

About 20 mg of compound II was suspended in 0.5 mL of acetone/methylcyclohexane (1:4, v:v). The mixture was stirred at room temperature for 3 d and filtered. The filter cake was dried in an oven at 50 °C to give crystal form 2O of a methylcyclohexane solvate of compound II. The product was analyzed and characterized by XRPD (FIG. 2-18), DSC (FIG. 2-43), and TGA (FIG. 2-68). ¹HNMR (400 MHz, DMSO-*d₆*): δ 12.77 (br, 1 H), 8.26 (s, 1 H), 7.87 (dd, *J*=2.0 Hz, 11.5 Hz, 1 H), 7.79 (dd, *J* = 1.7, 8.5 Hz, 1 H), 7.73 (t, *J =* 8.1 Hz, 1 H), 7.71-7.62 (m, 2 H), 7.45 (t, *J =* 8.6 Hz, 1 H), 7.05 (d, *J* =7.5 Hz, 1 H), 6.73 (d, *J* = 8.5 Hz, 1 H), 5.31 (s, 2 H), 5.11-5.08 (m, 1 H), 4.92-4.90 (m, 1H), 4.80-4.77 (m, 1 H), 4.66-4.64 (m, 1 H), 4.50-4.48 (m, 1 H), 4.38-4.36 (m, 1 H), 3.93 (d, *J =* 13.5 Hz, 1 H), 3.78 (d, *J* = 13.5 Hz, 1 H), 2.70-2.69 (m, 3 H), 2.50-2.32 (m, 3 H), 1.90-1.88 (m, 2 H), 1.62-1.60 (m, 7 H), 1.55-1.05 (m, 5 H), 0.84 (d, *J* = 3.9 Hz, 4 H).

### Example 2-20. Preparation of crystal form 2P of DMF solvate of compound II

About 20 mg of compound II was suspended in 0.5 mL of DMF/methyl *tert*-butyl ether (1:9, v:v). The mixture was stirred at 50 °C for 3 d and filtered. The filter cake was dried in an oven at 50 °C to give crystal form 2P of a DMF solvate of compound II. The product was analyzed and characterized by XRPD (FIG. 2-19), DSC (FIG. 2-44), and TGA (FIG. 2-69). ¹HNMR (400 MHz, DMSO-*d₆*): δ 12.72 (br, 1 H), 8.26 (s, 1 H), 7.86 (dd, *J*=2.0 Hz, 11.5 Hz, 1 H), 7.80 (dd, *J =* 1.7, 8.5 Hz, 1 H), 7.73 (t, *J=* 8.1 Hz, 1 H), 7.67-7.63 (m, 2 H), 7.46 (t, *J* = 8.5 Hz, 1 H), 7.05 (d, *J* =7.5 Hz, 1 H), 6.73 (d*, J* =8.5 Hz, 1 H), 5.31 (s, 2 H), 5.11-5.08 (m, 1 H), 4.92-4.90 (m, 1H), 4.80-4.77 (m, 1 H), 4.66-4.64 (m, 1 H), 4.50-4.48 (m, 1 H), 4.39-4.37 (m, 1 H), 3.94 (d, *J=* 13.5 Hz, 1 H), 3.78 (d, *J =* 13.5 Hz, 1 H), 2.89-2.69 (m, 9 H), 2.50-2.30 (m, 3 H), 1.91-1.89 (m, 2 H) 1.61-1.59 (m, 2 H).

### Example 2-21. Preparation of crystal form 2Q-1 of 2-methyltetrahydrofuran solvate of compound II

About 20 mg of compound II was suspended in 0.5 mL of 2-methyltetrahydrofuran. The mixture was stirred at 5 °C for 3 d and filtered. The filter cake was dried in an oven at 50 °C to give crystal form 2Q-1 of a 2-methyltetrahydrofuran solvate of compound II. The product was analyzed and characterized by XRPD (FIG. 2-20), DSC (FIG. 2-45), and TGA (FIG. 2-70). ¹HNMR (400 MHz, DMSO-*d₆*): δ 12.55 (br, 1 H), 8.27 (s, 1 H), 7.88 (dd, *J*=2.1 Hz, 11.5 Hz, 1 H), 7.80 (dd, *J* = 1.7, 8.5 Hz, 1 H), 7.73 (t*, J =* 8.1 Hz, 1 H), 7.68-7.63 (m, 2 H), 7.45 (t, *J =* 8.6 Hz, 1 H), 7.06 (d, *J* =7.5 Hz*,* 1 H), 6.74 (d, *J* =8.5 Hz, 1 H), 5.31 (s, 2 H), 5.10-5.08 (m, 1 H), 4.92-4.90 (m, 1H), 4.80-4.77 (m, 1 H), 4.66-4.64 (m, 1 H), 4.51-4.49 (m, 1 H), 4.38-4.36 (m, 1 H), 3.92 (d, *J =* 13.5 Hz, 1 H), 3.85-3.52 (m, 6 H), 2.70-2.69 (m, 3 H), 2.45-2.30 (m, 3 H), 1.95-1.77 (m, 7 H), 1.62-1.60 (m, 2 H), 1.34-1.29 (m, 2 H), 1.24 (d, *J =* 6.8 Hz, 5H).

### Example 2-22. Preparation of crystal form 2Q-2 of 2-methyltetrahydrofuran solvate of compound II

About 20 mg of compound II was suspended in 0.5 mL of 2-methyltetrahydrofuran/n-hexane (1:2, v:v). The mixture was stirred at 50 °C for 2 h, cooled to 5 °C, stirred for another 2 h, and filtered. The filter cake was dried in an oven at 50 °C to give crystal form 2Q-2 of a 2-methyltetrahydrofuran solvate of compound II. The product was analyzed and characterized by XRPD (FIG. 2-21), DSC (FIG. 2-46), and TGA (FIG. 2-71). ¹HNMR (400 MHz, DMSO-*d₆*): δ 12.75 (br, 1 H), 8.26 (s, 1 H), 7.88 (dd, *J*=2.1 Hz, 11.5 Hz, 1 H), 7.81 (dd, *J* = 1.7, 8.5 Hz, 1 H), 7.73 (t, *J =* 8.1 Hz, 1 H), 7.68-7.62 (m, 2 H), 7.45 (t, *J =* 8.6 Hz, 1 H), 7.06 (d*, J =*6.5 Hz, 1 H), 6.74 (d, *J* =8.5 Hz, 1 H), 5.31 (s, 2 H), 5.09-5.08 (m, 1 H), 4.92-4.90 (m, 1H), 4.77-4.75 (m, 1 H), 4.66-4.64 (m, 1 H), 4.51-4.49 (m, 1 H), 4.38-4.36 (m, 1 H), 3.92 (d, *J* = 13.5 Hz, 1 H), 3.81-3.54 (m, 3 H), 2.70-2.69 (m, 3 H), 2.45-2.30 (m, 3 H), 1.92-1.79 (m, 2 H), 1.63-1.60 (m, 2 H), 1.34-1.29 (m, 2 H), 1.24 (d, *J =* 6.8 Hz, 2H).

### Example 2-23. Preparation of crystal form 2R of N-methylpyrrolidone solvate of compound II

20 mg of compound II was dissolved in 0.5 mL of *N*-methylpyrrolidone/ethyl acetate (1:1, v:v) before 4.0 mL of methyl *tert-*butyl ether was dropwise added. The mixture was stirred at room temperature for 1 h and filtered. The filter cake was dried in an oven at 50 °C to give crystal form 2R of a *N*-methylpyrrolidone solvate of compound II. The product was analyzed and characterized by XRPD (FIG. 2-22), DSC (FIG. 2-47), and TGA (FIG. 2-72). ¹HNMR (400 MHz, DMSO-*d₆*): δ 12.70 (br, 1 H), 8.26 (s, 1 H), 7.87 (dd, *J*=2.0 Hz, 11.5 Hz, 1 H), 7.80 (dd, *J =* 1.7, 8.5 Hz, 1 H), 7.73 (t, *J =* 8.1 Hz, 1 H), 7.67-7.63 (m, 2 H), 7.45 (t, *J =* 8.6 Hz, 1 H), 7.06 (d, *J* =7.5 Hz, 1 H), 6.74 (d, *J*=8.5 Hz, 1 H), 5.31 (s, 2 H), 5.09-5.08 (m, 1 H), 4.92-4.90 (m, 1H), 4.79-4.77 (m, 1 H), 4.66-4.62 (m, 1 H), 4.52-4.47 (m, 1 H), 4.40-4.35 (m, 1 H), 3.94 (d, *J =* 13.5 Hz, 1 H), 3.78 (d, *J =* 13.5 Hz, 1 H), 2.75-2.70 (m, 8 H), 2.50-2.30 (m, 3 H), 2.20-2.16 (m, 3 H), 1.94-1.86 (m, 5 H) 1.61-1.59 (m, 2 H).

### Example 2-24. Preparation of crystal form 2S of trifluoroethanol solvate of compound II

20 mg of compound II was dissolved in 0.2 mL of trifluoroethanol/ethyl acetate (1:1, v:v). The solution was then transferred into a 20-mL vial containing 3.0 mL of methyl *tert-*butyl ether. The 20-mL vial was sealed with a cap, let stand at room temperature for 2 d, and filtered. The filter cake was dried in an oven at 50 °C to give crystal form 2S of a trifluoroethanol solvate of compound II. The product was analyzed and characterized by XRPD (FIG. 2-23), DSC (FIG. 2-48), and TGA (FIG. 2-73). ¹HNMR (400 MHz, DMSO-*d₆*): δ 12.75 (br, 1 H), 8.26 (s, 1 H), 7.88 (dd, *J*=2.0 Hz, 11.5 Hz, 1 H), 7.80 (dd, *J* = 1.7, 8.8 Hz, 1 H), 7.72 (t, *J =* 8.0 Hz, 1 H), 7.71-7.63 (m, 2 H), 7.45 (t, *J =* 8.5 Hz, 1 H), 7.06 (d, *J* =6.5 Hz, 1 H), 6.74 (d, *J* =8.6 Hz, 1 H), 5.31 (s, 2 H), 5.10-5.08 (m, 1 H), 4.92-4.91 (m, 1H), 4.80-4.75 (m, 1 H), 4.66-4.64 (m, 1 H), 4.51-4.49 (m, 1 H), 4.39-4.36 (m, 1 H), 3.94 (d, *J =* 13.5 Hz, 1 H), 3.92-3.86 (m, 1H), 3.78 (d, *J =* 13.5 Hz, 1 H), 2.70-2.69 (m, 3 H), 2.50-2.30 (m, 3 H), 1.90-1.88 (m, 2 H), 1.61-1.59 (m, 2 H).

### Example 2-25. Preparation of crystal form 2T of tetrahydrofuran solvate of compound II

20 mg of compound II was dissolved in 0.5 mL of tetrahydrofuran. The mixture was let stand at room temperature for 2 d to slowly evaporate the solvent and filtered. The filter cake was dried in an oven at 50 °C to give crystal form 2T of a tetrahydrofuran solvate of compound II. The product was analyzed and characterized by XRPD (FIG. 2-24), DSC (FIG. 2-49), and TGA (FIG. 2-74). ¹HNMR (400 MHz, DMSO-*d₆*): δ 12.78 (br, 1 H), 8.26 (s, 1 H), 7.88 (dd, *J*=2.0 Hz, 11.5 Hz, 1 H), 7.80 (dd, *J* = 1.7, 8.8 Hz, 1 H), 7.72 (t, *J* = 8.0 Hz, 1 H), 7.71-7.63 (m, 2 H), 7.45 (t, *J=* 8.5 Hz, 1 H), 7.06 (d, *J=*7.5 Hz, 1 H), 6.74 (d*, J* =8.6 Hz, 1 H), 5.31 (s, 2 H), 5.09-5.08 (m, 1 H), 4.92-4.90 (m, 1H), 4.81-4.76 (m, 1 H), 4.66-4.62 (m, 1 H), 4.51-4.49 (m, 1 H), 4.40-4.36 (m, 1 H), 3.94 (d, *J =* 13.5 Hz, 1 H), 3.78 (d, *J* = 13.5 Hz, 1 H), 3.62-3.59 (m, 1.4 H), 2.75-2.69 (m, 3 H), 2.50-2.30 (m, 3 H), 1.90-1.88 (m, 2 H), 1.78-1.76 (m, 1.4 H), 1.63-1.60 (m, 2 H).

### Example 2-26. Preparation of crystal form 2U of dioxane solvate of compound II

20 mg of compound II was dissolved in 0.3 mL of dioxane. The solution was then transferred into a 20-mL vial containing 3.0 mL of *n*-hexane. The 20-mL vial was sealed with a cap, let stand at room temperature for 11 d, and filtered. The filter cake was dried in an oven at 50 °C to give crystal form 2U of a dioxane solvate of compound II. The product was analyzed and characterized by XRPD (FIG. 2-25), DSC (FIG. 2-50), and TGA (FIG. 2-75). ¹HNMR (400 MHz, DMSO-*d₆*): δ 12.78 (br, 1 H), 8.26 (s, 1 H), 7.88 (dd, *J=*2.0 Hz, 11.6 Hz, 1 H), 7.79 (dd, *J =* 1.6, 8.8 Hz, 1 H), 7.73 (t, *J =* 8.0 Hz, 1 H), 7.68-7.63 (m, 2 H), 7.45 (t, *J =* 8.5 Hz, 1 H), 7.06 (d, *J* =7.5 Hz, 1 H), 6.74 (d, *J* =8.5 Hz, 1 H), 5.31 (s, 2 H), 5.10-5.09 (m, 1 H), 4.92-4.91 (m, 1H), 4.80-4.75 (m, 1 H), 4.66-4.64 (m, 1 H), 4.51-4.49 (m, 1 H), 4.38-4.36 (m, 1 H), 3.94 (d, *J =* 13.5 Hz, 1 H), 3.78 (d, *J =* 13.5 Hz, 1 H), 3.57 (s, 6 H), 2.70-2.69 (m, 3 H), 2.50-2.30 (m, 3 H), 1.90-1.88 (m, 2 H) 1.61-1.59 (m, 2 H).

### Test Example 1. Equilibrium solubility study of polymorph of compound I in biological media

The amorphous solid of compound I and crystal form D of compound I were tested for their equilibrium solubilities in water (H₂O), fasted state simulated gastric fluid (FaSSGF), fasted state simulated intestinal fluid (FaSSIF), and fed state simulated intestinal fluid (FeSSIF). In the tests, the solids were suspended in corresponding buffers to formulate suspensions (~10 mg/mL), and the suspensions were mixed at 37 ± 2 °C. After 24 h, samples of the suspensions were taken. The supernatant was filtered, and the concentration was determined. The solid was tested by XRPD. The test results are shown in the following table:

| Solid form | Preparation method | Solution | Equilibrium solubility | Whether the crystal form is changed |
|---|---|---|---|---|
| Amorphous solid of compound I | Example 1 | H₂O | 0.19 mg/mL | No |
| | | FaSSGF | 0.02 mg/mL | Yes; converted into hydrochloride salt |
| | | FaSSIF | 0.05 mg/mL | Yes; converted into crystal form D |
| | | FeSSIF | 0.16 mg/mL | Yes; converted into crystal form D |
| Crystal form D of compound I | Example 5 | H₂O | 0.17 mg/mL | No |
| | | FaSSGF | 0.02 mg/mL | Yes; converted into hydrochloride salt |
| | | FaSSIF | 0.15 mg/mL | No |
| | | FeSSIF | 0.17 mg/mL | No |

It can be seen from the above results that the stability of crystal form D of compound I in biological media was significantly superior to that of the amorphous solid of compound I. Except that crystal form D of compound I was converted into a hydrochloride salt in the FaSSGF system, no crystal form change was observed in other samples.

The form also exhibited significantly improved solubility in the FaSSIF system, and thus meets the requirement of development of clinical pharmaceutical formulations. Therefore, after salification of the free form of compound I, the solubility and the drug release behavior can be significantly improved.

### Solid state stability study of polymorph of compound I

The amorphous solid of compound I and crystal form D of compound I were preserved in long-term storage (25 °C/60% RH), accelerated (40 °C/75% RH), and high-temperature (60 °C, RH < 30%) conditions for 7 days, and tested for purity and crystal form change by HPLC, so as to examine the stability of the solids. The results are shown in the following table:

| Solid form | Initial purity (%) | 25 °C/60% RH, 7 days | | 40 °C/75% RH, 7 days | | 60 °C, 7 days | |
|---|---|---|---|---|---|---|---|
| | | Crystal form change | Purity (%) | Crystal form change | Purity (%) | Crystal form change | Purity (%) |
| Amorphous solid of compound I | 98.98 | No | 98.94 | No | 95.86 | No | 89.15 |
| Crystal form D of compound I | 99.15 | No | 99.10 | No | 98.98 | No | 98.61 |

It can be seen from the above results that the amorphous solid of compound I exhibited no significant purity changes after standing for 7 days in the long-term storage condition, but significant degradation after standing for 7 days in the accelerated condition and the high-temperature condition. Crystal form D of compound I exhibited no significant purity changes after standing for 7 days in the long-term storage condition and slightly reduced purities after standing for 7 days in the accelerated condition and the high-temperature condition, but no significant crystal form changes. The stability of crystal form D is significantly superior to that of the amorphous solid.

### Hygroscopic behavior test of compound I

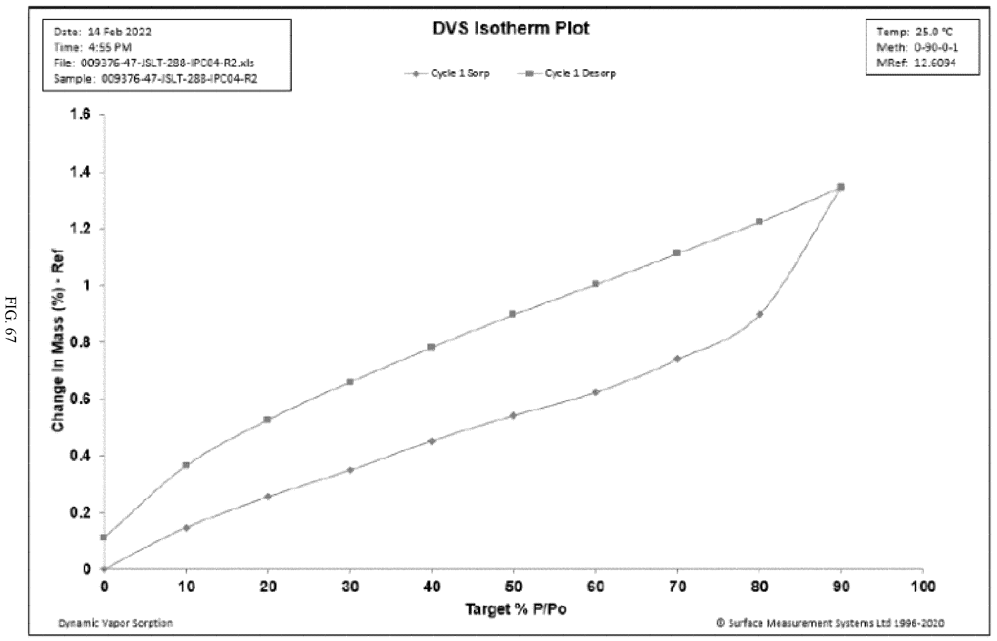

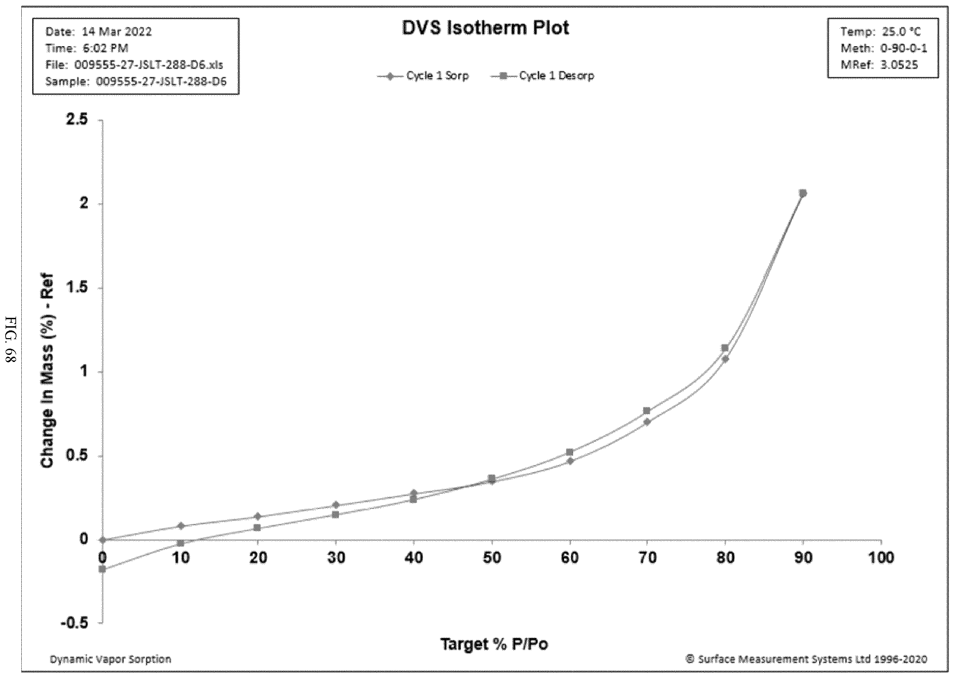

The inventors assessed the stability risk of samples under varying humidity at 25 °C using the dynamic vapor sorption method and conducted the DVS test on the representative crystal form D of compound I to evaluate the hygroscopicity of the crystal form of the compound. The DVS plot of the amorphous solid of the compound is shown in FIG. 67, and the DVS plot of crystal form D of the compound is shown in FIG. 68. The results are shown in the table below:

| Solid form | Sample weight change at 80% RH (%) | Whether the crystal form is changed | Hygroscopicity |
|---|---|---|---|
| Amorphous solid of compound I | 0.90 | No | Slightly hygroscopic |
| Crystal form D of compound I | 1.08 | No | Slightly hygroscopic |

It can be seen from the above results that on the 0-90% RH adsorption curves, at 80% RH, the amorphous solid and crystal form D of compound I were slightly hygroscopic with no significant differences, and no solid form changes were observed.

The thermal analysis of some of the crystal forms of compound I of the present disclosure is summarized in the table below:

| Crystal form | DSC endothermic peak temperature | TGA analysis | Solvent residue/solvate |
|---|---|---|---|
| Crystal form A | 99.81 °C | 0.9286% weight loss at 30-120 °C | N/A |
| Crystal form B | 177.71 °C | 0.0576% weight loss at 30-120 °C | N/A |
| Crystal form C | 104.51 °C | 0.2388% weight loss at 30-120 °C | N/A |
| Crystal form D | 167.48 °C | 0.2692% weight loss at 30-120 °C | N/A |
| Crystal form E | 112.00 °C | 0.2854% weight loss at 30-120 °C | N/A |
| Crystal form F | 169.00 °C | 0.1495% weight loss at 30-120 °C | N/A |
| Crystal form G | 89.95 °C and 104.07 °C | 5.5541% weight loss at 30-120 °C | Dihydrate |
| Crystal form H | 87.76 °C | 7.1770% weight loss at 30-100 °C | Methyl isobutyl ketone 0.5-solvate |
| Crystal form I-1 | 103.27 °C | 12.2415% weight loss at 30-140 °C | Methyl *tert-*butyl ether monosolvate |
| Crystal form I-2 | 97.11 °C | 17.6425% weight loss at 30-150 °C | Methyl *tert-*butyl ether 1.5-solvate |
| Crystal form J | 93.18 °C | 6.0000% weight loss at 30-185 °C | Acetone 0.5-solvate |
| Crystal form K | 91.51 °C | 2.4728% weight loss at 30-100 °C | *n*-Heptane 0.12-solvate |
| Crystal form L | 88.68 °C | 2.6978% weight loss at 30-100 °C | Methylcyclohexane 0.15-solvate |
| Crystal form M | 102.43 °C | 13.2754% weight loss at 30-200 °C | Toluene monosolvate |
| Crystal form N | 116.48 °C | 19.7479% weight loss at 30-180 °C | Dioxane 1.5-solvate |
| Crystal form O | 123.26 °C | 11.1205% weight loss at 30-200 °C | DMF monosolvate |
| Crystal form P | 117.63 °C | 17.7804% weight loss at 30-200 °C | *N*-Methylpyrrolidone monosolvate |
| Crystal form Q | 100.61 °C | 7.2704% weight loss at 30-120 °C | *n*-Butanol 0.5-solvate |
| Crystal form R | 110.90 °C | 9.3140% weight loss at 30-200 °C | *n*-Propanol monosolvate |
| Crystal form S | 96.26 °C | 6.1410% weight loss at 30-120 °C | Tetrahydrofuran 0.5-solvate |
| Crystal form T | 112.95 °C | 12.7701% weight loss at 30-200 °C | 2-Methyltetrahydrofuran monosolvate |

### Test Example 2. Equilibrium solubility study of polymorph of compound II in biological media

Crystal form 2A of compound II was tested for its equilibrium solubility in water (H₂O), fasted state simulated gastric fluid (FaSSGF), fasted state simulated intestinal fluid (FaSSIF), and fed state simulated intestinal fluid (FeSSIF). In the tests, the solids were suspended in corresponding buffers to formulate suspensions (~10 mg/mL), and the suspensions were mixed at 37 ± 2 °C. After 24 h, samples of the suspensions were taken. The supernatant was filtered, and the concentration was determined. The solid was tested by XRPD. The test results are shown in the following table:

| Solid form | Preparation method | Solution | Equilibrium solubility | Whether the crystal form is changed |
|---|---|---|---|---|
| Crystal form 2A of compound II | Example 2-2 | H₂O | 0.09 mg/mL | No |
| | | FaSSGF | 0.18 mg/mL | Yes; converted into hydrochloride salt |
| | | FaSSIF | 0.04 mg/mL | No |
| | | FeSSIF | 0.19 mg/mL | No |

It can be seen from the above results that the stability of crystal form 2A of compound II in biological media was significantly superior to that of the amorphous solid of compound II. Except that crystal form 2A of compound II was converted into a hydrochloride salt in the FaSSGF system, it exhibited good stability in other media and no crystal form changes, thus meeting the requirement for the development of clinical pharmaceutical formulations.

### Solid stability study of polymorph of compound II

Crystal form 2A of compound II was preserved in long-term storage (25 °C/60% RH), accelerated (40 °C/75% RH), and high-temperature (60 °C, RH < 30%) conditions for 7 days, and tested for purity and crystal form change by HPLC, so as to examine the stability of the solids. The results are shown in the following table:

| Solid form | Initial purity (%) | 25 °C/60% RH, 7 days | | 40 °C/75% RH, 7 days | | 60 °C, 7 days | |
|---|---|---|---|---|---|---|---|
| | | Crystal form change | Purity (%) | Crystal form change | Purity (%) | Crystal form change | Purity (%) |
| Crystal form 2A of compound II | 97.71 | No | 97.65 | No | 97.38 | No | 97.63 |

It can be seen from the above results that crystal form 2A of compound II, after standing for 7 days in the long-term storage condition, the accelerated condition, and the high-temperature condition, exhibited no significant purity changes or significant crystal form changes, suggesting that crystal form 2A has good stability.

### Hygroscopic behavior test of compound II

The inventors assessed the stability risk of samples under varying humidity at 25 °C using the dynamic vapor sorption method and conducted the DVS test on the representative crystal form 2A of compound II to evaluate the hygroscopicity of the crystal form of the compound. The DVS plot of crystal form 2A of compound II is shown in FIG. 2-79. The results are shown in the table below:

| Solid form | Sample weight change at 80% RH (%) | Whether the crystal form is changed | Hygroscopicity |
|---|---|---|---|
| Crystal form 2A of compound II | 0.71 | No | Slightly hygroscopic |

It can be seen from the above results that on the 0-90% RH adsorption curves, at 80% RH, crystal form 2A of compound II was slightly hygroscopic, and no solid form changes were observed.

The thermal analysis of some of the crystal forms of compound II of the present disclosure is summarized in the table below:

| Crystal form | DSC endothermic peak temperature | TGA analysis | Solvent residue/solvate |
|---|---|---|---|
| Crystal form 2A | 189.90 °C | 0.7069% weight gain at 30-220 °C | N/A |
| Crystal form 2B | 181.06 °C | 0.4739% weight gain at 30-220 °C | N/A |
| Crystal form 2C | 171.63 °C | 0.2373% weight loss at 30-220 °C | N/A |
| Crystal form 2D | 168.79 °C | 1.9365% weight loss at 30-220 °C | N/A |
| Crystal form 2E | 110.30 °C, 169.27 °C | 0.8772% weight loss at 30-220 °C | N/A |
| Crystal form 2F | 114.41 °C | 9.0819% weight loss at 30-210 °C | Trihydrate |
| Crystal form 2G | 169.22 °C | 6.9565% weight loss at 30-220 °C | Dihydrate |
| Crystal form 2H | 118.06 °C | 5.6617% weight loss at 30-140 °C | Dimethylsulfoxide monosolvate |
| Crystal form 2I | 115.84 °C | 13.0397% weight loss at 30-200 °C | Methyl *tert-butyl* ether 0.5-solvate |
| Crystal form 2J-1 | 103.13 °C | 3.0362% weight loss at 30-120 °C | *n*-Heptane 0.2-solvate |
| Crystal form 2J-2 | 76.82 °C | 7.4737% weight loss at 30-150 °C | *n*-Heptane 0.5-solvate |
| Crystal form 2K | 107.92 °C, 172.19 °C, and 184.68 °C | 9.7222% weight loss at 30-220 °C | Toluene 0.75-solvate |
| Crystal form 2L-1 | 106.45 °C | 9.9209% weight loss at 30-120 °C | Methyl isobutyl ketone 0.75-solvate |
| Crystal form 2L-2 | 102.87 °C | 4.8624% weight loss at 30-120 °C | Methyl isobutyl ketone 0.3-solvate |
| Crystal form 2M-1 | 113.56 °C | 11.6634% weight loss at 30-200 °C | Cyclopentyl methyl ether monosolvate |
| Crystal form 2M-2 | 110.50 °C, 165.01 °C | 15.2574% weight loss at 30-180 °C | Cyclopentyl methyl ether monosolvate |
| Crystal form 2N | 102.66 °C, 113.16 °C | 3.5103% weight loss at 30-220 °C | Methyl ethyl ketone 0.3-solvate |
| Crystal form 2O | 111.80 °C | 11.3122% weight loss at 30-230 °C | Methylcyclohexane monosolvate |
| Crystal form 2P | 105.14 °C | 7.3916% weight loss at 30-230 °C | DMF monosolvate |
| Crystal form 2Q-1 | 111.13 °C | 10.3779% weight loss at 30-230 °C | 2-Methyltetrahydrofuran monosolvate |
| Crystal form 2Q-2 | 114.18 °C | 9.2323% weight loss at 30-200 °C | 2-Methyltetrahydrofuran monosolvate |
| Crystal form 2R | 128.45 °C | 13.9744% weight loss at 30-230 °C | N-methylpyrrolidone monosolvate |
| Crystal form 2S | 123.79 °C, 169.39 °C | 14.6675% weight loss at 30-220 °C | Trifluoroethanol monosolvate |
| Crystal form 2T | 110.50 °C | 2.9438% weight loss at 30-130 °C | Tetrahydrofuran 0.3-solvate |
| Crystal form 2U | 109.16 °C | 6.2753% weight loss at 30-130 °C | Dioxane 0.5-solvate |

The embodiments of the technical solutions of the present disclosure have been described above by way of example. It will be appreciated that the claimed scope of the present disclosure is not limited to the embodiments described above. Any modification, equivalent substitution, improvement, and the like made by those skilled in the art without departing from the spirit and principle of the present disclosure shall fall within the claimed scope of the present application.

## Claims

1. A polymorph of compound III, 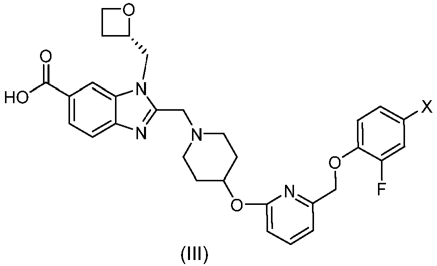 wherein, X is Cl or CN.

2. The polymorph according to claim 1, wherein the polymorph may be a crystal form of a non-solvate, a hydrate, or a solvate of compound III;
preferably, compound III has a structure of compound I or compound II:

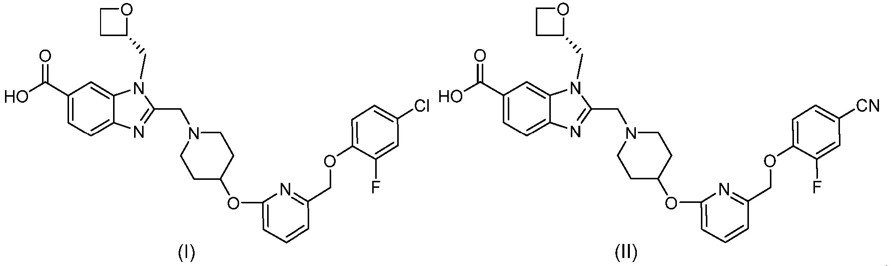

3. The polymorph according to claim 1 or 2, wherein the polymorph is a crystal form of a non-solvate of compound I, including the following crystal forms A, B, C, D, E, and F of non-solvates, wherein:
crystal form A has an X-ray powder diffraction pattern comprising peaks at diffraction angles (2θ) of 19.79 ± 0.2°, 13.13 ± 0.2°, 22.07 ± 0.2°, and 9.48 ± 0.2°;
preferably, further comprising peaks at diffraction angles (2θ) of 7.80 ± 0.2°, 13.59 ± 0.2°, 11.43 ± 0.2°, 18.07 ± 0.2°, and 12.45 ± 0.2°;
and more preferably, further comprising peaks at diffraction angles (2θ) of 14.58 ± 0.2°, 24.66 ± 0.2°, 14.24 ± 0.2°, 4.85 ± 0.2°, 23.70 ± 0.2°, and 26.51 ± 0.2°;
preferably, crystal form A has an X-ray powder diffraction pattern comprising diffraction angles (2θ) shown in Table 1, wherein the 2θ angles have a margin of error of ±0.20°:
**Table 1**
| 2θ (°) | Intensity% | 2θ (°) | Intensity% |
|---|---|---|---|
| 4.85 | 13.1 | 18.07 | 20.8 |
| 7.80 | 37.6 | 19.79 | 100.0 |
| 9.48 | 42.2 | 22.07 | 44.2 |
| 11.43 | 29.1 | 23.70 | 12.5 |
| 12.45 | 18.6 | 24.66 | 14.7 |
| 13.13 | 51.3 | 25.79 | 8.3 |
| 13.59 | 31.1 | 26.51 | 12.5 |
| 14.24 | 13.3 | 31.12 | 8.3 |
| 14.58 | 17.8 | | |
preferably, crystal form A has X-ray powder diffraction intensities shown in Table 1;
preferably, crystal form A has an X-ray powder diffraction pattern substantially shown in FIG. 1;
preferably, crystal form A exhibits an endothermic peak in a DSC analysis when heated to a peak temperature of about 99.81 °C;
preferably, crystal form A has a DSC profile substantially shown in FIG. 22;
preferably, crystal form A has a TGA profile substantially shown in FIG. 43;
crystal form B has an X-ray powder diffraction pattern comprising peaks at diffraction angles (2θ) of 13.79 ± 0.2°, 22.36 ± 0.2°, 17.66 ± 0.2°, and 27.41 ± 0.2°;
preferably, further comprising peaks at diffraction angles (2θ) of 11.31 ± 0.2°, 23.16 ± 0.2°, 25.40 ± 0.2°, 5.59 ± 0.2°, and 8.74 ± 0.2°;
and more preferably, further comprising peaks at diffraction angles (2θ) of 20.20 ± 0.2°, 28.85 ± 0.2°, 11.96 ± 0.2°, 24.19 ± 0.2°, 24.39 ± 0.2°, and 7.28 ± 0.2°;
preferably, crystal form B has an X-ray powder diffraction pattern comprising diffraction angles (2θ) shown in Table 2, wherein the 2θ angles have a margin of error of ±0.20°:
**Table 2**
| 2θ (°) | Intensity% | 2θ (°) | Intensity% |
|---|---|---|---|
| 5.59 | 11.3 | 23.16 | 17.0 |
| 7.28 | 2.9 | 24.19 | 3.3 |
| 8.74 | 8.8 | 24.39 | 3.0 |
| 11.31 | 17.0 | 25.40 | 14.7 |
| 11.96 | 3.7 | 27.41 | 19.7 |
| 13.79 | 100.0 | 28.85 | 5.3 |
| 16.98 | 17.4 | 29.42 | 2.2 |
| 17.66 | 70.5 | 30.66 | 2.7 |
| 20.20 | 6.7 | 31.42 | 2.0 |
| 22.36 | 71.9 | 32.93 | 2.9 |
preferably, crystal form B has X-ray powder diffraction intensities shown in Table 2;
preferably, crystal form B has an X-ray powder diffraction pattern substantially shown in FIG. 2;
preferably, crystal form B exhibits an endothermic peak in a DSC analysis when heated to a peak temperature of about 177.71 °C;
preferably, crystal form B has a DSC profile substantially shown in FIG. 23;
preferably, crystal form B has a TGA profile substantially shown in FIG. 44;
crystal form C has an X-ray powder diffraction pattern comprising peaks at diffraction angles (2θ) of 11.27 ± 0.2°, 19.30 ± 0.2°, 17.92 ± 0.2°, and 20.04 ± 0.2°;
preferably, further comprising peaks at diffraction angles (2θ) of 14.70 ± 0.2°, 22.75 ± 0.2°, 21.21 ± 0.2°, 10.48 ± 0.2°, and 18.83 ± 0.2°;
and more preferably, further comprising peaks at diffraction angles (2θ) of 4.84 ± 0.2°, 10.20 ± 0.2°, 14.16 ± 0.2°, 22.15 ± 0.2°, 20.41 ± 0.2°, and 10.98 ± 0.2°;
preferably, crystal form C has an X-ray powder diffraction pattern comprising diffraction angles (2θ) shown in Table 3, wherein the 2θ angles have a margin of error of ±0.20°:
**Table 3**
| 2θ (°) | Intensity% | 2θ (°) | Intensity% |
|---|---|---|---|
| 4.84 | 36.3 | 20.41 | 18.0 |
| 9.74 | 5.0 | 20.65 | 15.0 |
| 10.20 | 27.6 | 21.21 | 46.9 |
| 10.48 | 46.1 | 22.15 | 23.2 |
| 10.98 | 16.3 | 22.75 | 48.7 |
| 11.27 | 100.0 | 24.41 | 12.2 |
| 12.03 | 6.7 | 25.33 | 8.5 |
| 12.54 | 7.8 | 26.26 | 8.9 |
| 14.16 | 23.4 | 26.88 | 14.3 |
| 14.70 | 53.2 | 28.22 | 12.5 |
| 16.45 | 12.6 | 29.01 | 8.5 |
| 17.27 | 6.1 | 30.74 | 6.5 |
| 17.92 | 63.1 | 31.40 | 7.6 |
| 18.83 | 42.1 | 34.65 | 4.0 |
| 19.30 | 74.2 | 36.35 | 4.8 |
| 20.04 | 56.3 | | |
preferably, crystal form C has X-ray powder diffraction intensities shown in Table 3;
preferably, crystal form C has an X-ray powder diffraction pattern substantially shown in FIG. 3;
preferably, crystal form C exhibits an endothermic peak in a DSC analysis when heated to a peak temperature of about 104.51 °C;
preferably, crystal form C has a DSC profile substantially shown in FIG. 24;
preferably, crystal form C has a TGA profile substantially shown in FIG. 45;
crystal form D has an X-ray powder diffraction pattern comprising peaks at diffraction angles (2θ) of 14.49 ± 0.2°, 16.98 ± 0.2°, 11.51 ± 0.2°, and 18.17 ± 0.2°;
preferably, further comprising peaks at diffraction angles (2θ) of 24.02 ± 0.2°, 21.87 ± 0.2°, 3.58 ± 0.2°, 14.04 ± 0.2°, and 20.68 ± 0.2°;
and more preferably, further comprising peaks at diffraction angles (2θ) of 19.59 ± 0.2°, 25.60 ± 0.2°, 22.30 ± 0.2°, 22.61 ± 0.2°, 23.53 ± 0.2°, and 9.70 ± 0.2°;
preferably, crystal form D has an X-ray powder diffraction pattern comprising diffraction angles (2θ) shown in Table 4, wherein the 2θ angles have a margin of error of ±0.20°:
**Table 4**
| 2θ (°) | Intensity% | 2θ (°) | Intensity% |
|---|---|---|---|
| 3.58 | 33.8 | 22.30 | 20.8 |
| 6.69 | 2.5 | 22.61 | 20.5 |
| 7.24 | 4.3 | 23.16 | 8.2 |
| 9.70 | 17.4 | 23.53 | 20.4 |
| 11.51 | 86.8 | 24.02 | 34.8 |
| 14.04 | 32.8 | 25.11 | 10.2 |
| 14.49 | 100.0 | 25.60 | 27.5 |
| 15.00 | 10.5 | 26.00 | 8.2 |
| 15.91 | 12.1 | 26.92 | 6.4 |
| 16.98 | 61.5 | 28.17 | 3.1 |
| 17.47 | 8.3 | 28.47 | 7.5 |
| 18.17 | 41.4 | 29.12 | 7.2 |
| 18.62 | 15.6 | 30.15 | 6.4 |
| 19.01 | 13.0 | 30.50 | 4.6 |
| 19.59 | 27.7 | 31.36 | 3.4 |
| 20.45 | 16.0 | 33.81 | 2.4 |
| 20.68 | 29.9 | 34.69 | 3.5 |
| 21.87 | 34.4 | | |
preferably, crystal form D has X-ray powder diffraction intensities shown in Table 4;
preferably, crystal form D has an X-ray powder diffraction pattern substantially shown in FIG. 4;
preferably, crystal form D exhibits an endothermic peak in a DSC analysis when heated to a peak temperature of about 167.48 °C;
preferably, crystal form D has a DSC profile substantially shown in FIG. 25;
preferably, crystal form D has a TGA profile substantially shown in FIG. 46;
crystal form E has an X-ray powder diffraction pattern comprising peaks at diffraction angles (2θ) of 11.49 ± 0.2°, 12.17 ± 0.2°, 21.15 ± 0.2°, and 20.16 ± 0.2°;
preferably, further comprising peaks at diffraction angles (2θ) of 14.35 ± 0.2°, 26.49 ± 0.2°, 19.40 ± 0.2°, 4.32 ± 0.2°, and 17.51 ± 0.2°;
and more preferably, further comprising peaks at diffraction angles (2θ) of 10.87 ± 0.2°, 25.13 ± 0.2°, 24.68 ± 0.2°, 18.01 ± 0.2°, 16.83 ± 0.2°, and 15.19 ± 0.2°;
preferably, crystal form E has an X-ray powder diffraction pattern comprising diffraction angles (2θ) shown in Table 5, wherein the 2θ angles have a margin of error of ±0.20°:
**Table 5**
| 2θ (°) | Intensity% | 2θ (°) | Intensity% |
|---|---|---|---|
| 4.32 | 32.3 | 21.87 | 7.1 |
| 10.87 | 26.9 | 22.95 | 4.7 |
| 11.49 | 100.0 | 23.28 | 7.9 |
| 12.17 | 92.0 | 23.92 | 7.6 |
| 13.14 | 15.6 | 24.24 | 5.8 |
| 13.61 | 6.2 | 24.68 | 20.8 |
| 14.35 | 46.1 | 25.13 | 25.5 |
| 15.19 | 16.2 | 26.08 | 11.3 |
| 15.48 | 6.2 | 26.49 | 44.1 |
| 16.83 | 17.2 | 28.30 | 3.1 |
| 17.51 | 30.3 | 28.79 | 4.8 |
| 18.01 | 18.4 | 29.96 | 3.6 |
| 18.85 | 10.4 | 30.21 | 6.1 |
| 19.40 | 43.1 | 31.69 | 4.5 |
| 20.16 | 66.7 | 34.99 | 3.0 |
| 21.15 | 98.3 | 36.05 | 2.8 |
preferably, crystal form E has X-ray powder diffraction intensities shown in Table 5;
preferably, crystal form E has an X-ray powder diffraction pattern substantially shown in FIG. 5;
preferably, crystal form E exhibits an endothermic peak in a DSC analysis when heated to a peak temperature of about 112.00 °C;
preferably, crystal form E has a DSC profile substantially shown in FIG. 26;
preferably, crystal form E has a TGA profile substantially shown in FIG. 47;
crystal form F has an X-ray powder diffraction pattern comprising peaks at diffraction angles (20) of 18.77 ± 0.2°, 25.50 ± 0.2°, 20.98 ± 0.2°, and 23.45 ± 0.2°;
preferably, further comprising peaks at diffraction angles (20) of 10.77 ± 0.2°, 12.70 ± 0.2°, 21.33 ± 0.2°, 24.50 ± 0.2°, and 16.89 ± 0.2°;
and more preferably, further comprising peaks at diffraction angles (20) of 14.72 ± 0.2°, 19.24 ± 0.2°, 9.68 ± 0.2°, 11.7 ± 0.2°, 20.49 ± 0.2°, and 13.26 ± 0.2°;
preferably, crystal form F has an X-ray powder diffraction pattern comprising diffraction angles (20) shown in Table 6, wherein the 2θ angles have a margin of error of ±0.20°:
**Table 6**
| 20 (°) | Intensity% | 20 (°) | Intensity% |
|---|---|---|---|
| 9.68 | 27.1 | 21.33 | 30.0 |
| 10.16 | 14.0 | 22.03 | 4.4 |
| 10.77 | 33.5 | 22.28 | 7.5 |
| 11.45 | 23.0 | 22.94 | 14.5 |
| 11.76 | 26.0 | 23.45 | 50.9 |
| 12.27 | 7.4 | 24.50 | 29.5 |
| 12.70 | 31.0 | 25.50 | 65.5 |
| 13.26 | 23.7 | 26.68 | 23.7 |
| 13.59 | 18.9 | 27.62 | 2.7 |
| 14.72 | 28.2 | 28.15 | 22.2 |
| 15.40 | 1.4 | 28.90 | 5.6 |
| 16.89 | 29.7 | 29.49 | 20.1 |
| 17.91 | 5.9 | 30.14 | 7.5 |
| 18.77 | 100.0 | 31.21 | 3.3 |
| 19.24 | 27.3 | 31.94 | 2.7 |
| 19.54 | 21.3 | 33.50 | 2.3 |
| 20.27 | 12.8 | 34.08 | 2.1 |
| 20.49 | 26.0 | 34.57 | 2.3 |
| 20.98 | 59.3 | | |
preferably, crystal form F has X-ray powder diffraction intensities shown in Table 6;
preferably, crystal form F has an X-ray powder diffraction pattern substantially shown in FIG. 6;
preferably, crystal form F exhibits an endothermic peak in a DSC analysis when heated to a peak temperature of about 169.00 °C;
preferably, crystal form F has a DSC profile substantially shown in FIG. 27;
preferably, crystal form F has a TGA profile substantially shown in FIG. 48;
preferably, the polymorph is crystal form G of a hydrate of compound I;
crystal form G of a hydrate has an X-ray powder diffraction pattern comprising peaks at diffraction angles (20) of 18.66 ± 0.2°, 18.81 ± 0.2°, 3.62 ± 0.2°, and 22.24 ± 0.2°;
preferably, further comprising peaks at diffraction angles (20) of 15.33 ± 0.2°, 17.88 ± 0.2°, 14.76 ± 0.2°, 20.51 ± 0.2°, and 11.08 ± 0.2°;
and more preferably, further comprising peaks at diffraction angles (20) of 19.94 ± 0.2°, 26.57 ± 0.2°, 23.22 ± 0.2°, 24.37 ± 0.2°, 7.32 ± 0.2°, and 23.44 ± 0.2°;
preferably, crystal form G has an X-ray powder diffraction pattern comprising diffraction angles (2θ) shown in Table 7, wherein the 2θ angles have a margin of error of ±0.20°:
**Table 7**
| 2θ (°) | Intensity% | 2θ (°) | Intensity% |
|---|---|---|---|
| 3.62 | 62.0 | 18.81 | 63.5 |
| 7.32 | 21.0 | 19.94 | 33.4 |
| 7.70 | 7.2 | 20.51 | 35.4 |
| 8.72 | 16.8 | 22.24 | 59.6 |
| 10.22 | 12.5 | 23.22 | 25.6 |
| 11.08 | 34.1 | 23.44 | 16.4 |
| 12.48 | 14.6 | 24.37 | 18.8 |
| 14.76 | 40.4 | 26.57 | 27.9 |
| 15.33 | 52.0 | 28.08 | 5.7 |
| 16.45 | 11.6 | 30.21 | 11.1 |
| 17.88 | 50.6 | 31.30 | 7.2 |
| 18.66 | 100.0 | 33.39 | 4.8 |
preferably, crystal form G has X-ray powder diffraction intensities shown in Table 7;
preferably, crystal form G has an X-ray powder diffraction pattern substantially shown in FIG. 7;
preferably, crystal form G exhibits endothermic peaks in a DSC analysis when heated to peak temperatures of about 89.95 °C and about 104.07 °C;
preferably, crystal form G has a DSC profile substantially shown in FIG. 28;
preferably, crystal form G has a TGA profile substantially shown in FIG. 49;
preferably, crystal form G is a dihydrate of compound I;
preferably, the polymorph is a crystal form of a solvate of compound I, including the following crystal forms H, I-1, I-2, J, K, L, M, N, O, P, Q, R, S, and T of solvates, wherein:
crystal form H of a methyl isobutyl ketone solvate has an X-ray powder diffraction pattern comprising peaks at diffraction angles (20) of 10.75 ± 0.2°, 8.61 ± 0.2°, 19.84 ± 0.2°, and 18.48 ± 0.2°;
preferably, further comprising peaks at diffraction angles (20) of 16.79 ± 0.2°, 25.92 ± 0.2°, 9.11 ± 0.2°, 21.15 ± 0.2°, and 15.43 ± 0.2°;
and more preferably, further comprising peaks at diffraction angles (20) of 22.55 ± 0.2°, 3.16 ± 0.2°, 18.95 ± 0.2°, 21.70 ± 0.2°, 12.54 ± 0.2°, and 17.86 ± 0.2°;
preferably, crystal form H has an X-ray powder diffraction pattern comprising diffraction angles (20) shown in Table 8, wherein the 2θ angles have a margin of error of ±0.20°:
**Table 8**
| 2θ (°) | Intensity% | 2θ (°) | Intensity% |
|---|---|---|---|
| 3.16 | 28.1 | 18.95 | 27.4 |
| 4.28 | 12.0 | 19.33 | 9.7 |
| 6.78 | 10.4 | 19.84 | 71.5 |
| 7.69 | 12.5 | 20.51 | 8.3 |
| 8.61 | 86.7 | 21.15 | 32.8 |
| 9.11 | 34.6 | 21.70 | 26.5 |
| 10.75 | 100.0 | 22.55 | 30.4 |
| 12.54 | 24.1 | 23.39 | 12.4 |
| 12.97 | 20.2 | 24.01 | 20.0 |
| 13.53 | 10.7 | 24.52 | 7.0 |
| 14.35 | 16.1 | 25.92 | 43.1 |
| 14.96 | 11.6 | 26.68 | 20.8 |
| 15.43 | 31.2 | 27.29 | 17.2 |
| 16.16 | 9.9 | 29.23 | 6.8 |
| 16.45 | 13.1 | 29.96 | 4.6 |
| 16.79 | 45.1 | 30.06 | 5.6 |
| 17.28 | 10.7 | 31.17 | 7.7 |
| 17.86 | 22.5 | 35.30 | 4.3 |
| 18.48 | 45.3 | | |
preferably, crystal form H has X-ray powder diffraction intensities shown in Table 8;
preferably, crystal form H has an X-ray powder diffraction pattern substantially shown in FIG. 8;
preferably, crystal form H exhibits an endothermic peak in a DSC analysis when heated to a peak temperature of about 87.76 °C;
preferably, crystal form H has a DSC profile substantially shown in FIG. 29;
preferably, crystal form H has a TGA profile substantially shown in FIG. 50;
preferably, crystal form H is a methyl isobutyl ketone 0.5-solvate of compound I;
crystal form I-1 of a methyl *tert-butyl* ether solvate has an X-ray powder diffraction pattern comprising peaks at diffraction angles (20) of 8.53 ± 0.2°, 10.75 ± 0.2°, 4.22 ± 0.2°, and 18.39 ± 0.2°;
preferably, further comprising peaks at diffraction angles (20) of 17.22 ± 0.2°, 21.48 ± 0.2°, 16.85 ± 0.2°, 17.53 ± 0.2°, and 9.11 ± 0.2°;
and more preferably, further comprising peaks at diffraction angles (2θ) of 12.49 ± 0.2°, 19.61 ± 0.2°, 12.87 ± 0.2°, 15.66 ± 0.2°, 26.02 ± 0.2°, and 22.40 ± 0.2°;
preferably, crystal form I-1 has an X-ray powder diffraction pattern comprising diffraction angles (20) shown in Table 9, wherein the 2θ angles have a margin of error of ±0.20°:
**Table 9**
| 20 (°) | Intensity% | 20 (°) | Intensity% |
|---|---|---|---|
| 4.22 | 37.6 | 19.61 | 13.2 |
| 6.84 | 4.8 | 20.57 | 7.0 |
| 8.53 | 100.0 | 21.48 | 23.3 |
| 9.11 | 15.2 | 22.40 | 7.3 |
| 10.75 | 46.0 | 23.41 | 4.4 |
| 12.49 | 13.5 | 23.86 | 3.7 |
| 12.87 | 11.8 | 24.70 | 4.0 |
| 13.69 | 5.1 | 26.02 | 9.1 |
| 14.59 | 3.6 | 26.66 | 2.1 |
| 15.66 | 9.2 | 27.24 | 4.1 |
| 16.40 | 5.1 | 27.69 | 4.0 |
| 16.85 | 21.7 | 28.98 | 4.6 |
| 17.22 | 24.8 | 30.81 | 3.6 |
| 17.53 | 16.1 | 31.13 | 5.1 |
| 18.39 | 30.5 | | 13.2 |
preferably, crystal form I-1 has X-ray powder diffraction intensities shown in Table 9;
preferably, crystal form I-1 has an X-ray powder diffraction pattern substantially shown in FIG. 9;
preferably, crystal form I-1 exhibits an endothermic peak in a DSC analysis when heated to a peak temperature of about 103.27 °C;
preferably, crystal form I-1 has a DSC profile substantially shown in FIG. 30;
preferably, crystal form I-1 has a TGA profile substantially shown in FIG. 51;
preferably, crystal form I-1 is a methyl *tert-*butyl ether monosolvate of compound I;
crystal form I-2 of a methyl *tert*-butyl ether solvate has an X-ray powder diffraction pattern comprising peaks at diffraction angles (20) of 16.88 ± 0.2°, 18.87 ± 0.2°, 20.90 ± 0.2°, and 9.60 ± 0.2°;
preferably, further comprising peaks at diffraction angles (20) of 10.98 ± 0.2°, 8.39 ± 0.2°, 17.68 ± 0.2°, 20.51 ± 0.2°, and 19.97 ± 0.2°;
and more preferably, further comprising peaks at diffraction angles (2θ) of 21.25 ± 0.2°, 17.99 ± 0.2°, 14.10 ± 0.2°, 16.42 ± 0.2°, 7.40 ± 0.2°, and 21.93 ± 0.2°;
preferably, crystal form I-2 has an X-ray powder diffraction pattern comprising diffraction angles (20) shown in Table 10, wherein the 2θ angles have a margin of error of ±0.20°:
**Table 10**
| 20 (°) | Intensity% | 20 (°) | Intensity% |
|---|---|---|---|
| 6.74 | 26.1 | 18.87 | 90.9 |
| 7.04 | 12.7 | 19.32 | 28.4 |
| 7.40 | 36.9 | 19.97 | 49.1 |
| 8.39 | 53.3 | 20.51 | 61.9 |
| 9.60 | 67.1 | 20.90 | 86.5 |
| 10.56 | 35.4 | 21.25 | 47.1 |
| 10.98 | 56.4 | 21.93 | 35.7 |
| 11.55 | 4.8 | 22.64 | 24.6 |
| 12.44 | 11.5 | 23.15 | 24.6 |
| 13.24 | 7.7 | 23.56 | 8.7 |
| 13.62 | 3.4 | 24.03 | 9.0 |
| 14.10 | 47.7 | 24.37 | 19.1 |
| 14.39 | 18.1 | 25.36 | 21.4 |
| 14.92 | 27.1 | 25.75 | 12.9 |
| 15.31 | 4.2 | 26.53 | 29.5 |
| 15.74 | 16.2 | 27.17 | 29.6 |
| 16.00 | 8.5 | 27.83 | 6.1 |
| 16.42 | 38.6 | 28.59 | 10.0 |
| 16.88 | 100.0 | 30.09 | 13.5 |
| 17.20 | 31.0 | 31.62 | 8.0 |
| 17.68 | 63.6 | 36.48 | 6.5 |
| 17.99 | 47.6 | 38.31 | 4.3 |
preferably, crystal form I-2 has X-ray powder diffraction intensities shown in Table 10;
preferably, crystal form I-2 has an X-ray powder diffraction pattern substantially shown in FIG. 10;
preferably, crystal form I-2 exhibits an endothermic peak in a DSC analysis when heated to a peak temperature of about 97.11 °C;
preferably, crystal form I-2 has a DSC profile substantially shown in FIG. 31;
preferably, crystal form I-2 has a TGA profile substantially shown in FIG. 52;
preferably, crystal form I-2 is a methyl *tert-*butyl ether 1.5-solvate of compound I;
crystal form J of an acetone solvate has an X-ray powder diffraction pattern comprising peaks at diffraction angles (20) of 12.02 ± 0.2°, 18.03 ± 0.2°, 8.04 ± 0.2°, and 7.67 ± 0.2°;
preferably, further comprising peaks at diffraction angles (20) of 19.54 ± 0.2°, 23.50 ± 0.2°, 16.05 ± 0.2°, 21.21 ± 0.2°, and 13.83 ± 0.2°;
and more preferably, further comprising peaks at diffraction angles (2θ) of 11.10 ± 0.2°, 17.43 ± 0.2°, 16.54 ± 0.2°, 8.92 ± 0.2°, 22.59 ± 0.2°, and 27.39 ± 0.2°;
preferably, crystal form J has an X-ray powder diffraction pattern comprising diffraction angles (20) shown in Table 11, wherein the 20 angles have a margin of error of ±0.20°:
**Table 11**
| 2θ (°) | Intensity% | 20 (°) | Intensity% |
|---|---|---|---|
| 6.83 | 20.4 | 16.54 | 28.5 |
| 7.67 | 44.9 | 17.43 | 29.8 |
| 8.04 | 56.1 | 18.03 | 61.4 |
| 8.54 | 21.1 | 19.54 | 43.6 |
| 8.92 | 26.1 | 21.21 | 38.6 |
| 10.69 | 7.6 | 22.59 | 24.3 |
| 11.10 | 31.9 | 23.50 | 41.8 |
| 12.02 | 100.0 | 25.83 | 18.5 |
| 13.83 | 34.5 | 27.39 | 21.4 |
| 14.48 | 16.4 | 29.32 | 9.9 |
| 16.05 | 39.4 | 30.93 | 17.0 |
preferably, crystal form J has X-ray powder diffraction intensities shown in Table 11;
preferably, crystal form J has an X-ray powder diffraction pattern substantially shown in FIG. **11;**
preferably, crystal form J exhibits an endothermic peak in a DSC analysis when heated to a peak temperature of about 93.18 °C;
preferably, crystal form J has a DSC profile substantially shown in FIG. 32;
preferably, crystal form J has a TGA profile substantially shown in FIG. 53;
preferably, crystal form J is an acetone 0.5-solvate of compound I;
crystal form K of a n-heptane solvate has an X-ray powder diffraction pattern comprising peaks at diffraction angles (20) of 12.19 ± 0.2°, 18.02 ± 0.2°, 7.69 ± 0.2°, and 19.69 ± 0.2°;
preferably, further comprising peaks at diffraction angles (20) of 21.36 ± 0.2°, 8.98 ± 0.2°, 23.63 ± 0.2°, 16.07 ± 0.2°, and 20.72 ± 0.2°;
and more preferably, further comprising peaks at diffraction angles (2θ) of 6.85 ± 0.2°, 8.12 ± 0.2°, 13.75 ± 0.2°, 6.54 ± 0.2°, 14.53 ± 0.2°, and 13.26 ± 0.2°;
preferably, crystal form K has an X-ray powder diffraction pattern comprising diffraction angles (20) shown in Table 12, wherein the 20 angles have a margin of error of ±0.20°:
**Table 12**
| 2θ (°) | Intensity% | 2θ (°) | Intensity% |
|---|---|---|---|
| 6.54 | 28.2 | 19.69 | 66.0 |
| 6.85 | 33.2 | 20.18 | 14.7 |
| 7.69 | 74.5 | 20.72 | 38.2 |
| 8.12 | 32.8 | 21.36 | 61.4 |
| 8.98 | 53.3 | 23.63 | 47.7 |
| 10.55 | 10.0 | 24.37 | 17.6 |
| 12.19 | 100.0 | 25.37 | 11.2 |
| 13.26 | 21.2 | 26.04 | 20.7 |
| 13.75 | 35.9 | 26.52 | 12.4 |
| 14.53 | 24.7 | 27.44 | 10.6 |
| 15.66 | 21.2 | 27.73 | 14.1 |
| 16.07 | 39.2 | 29.13 | 12.0 |
| 18.02 | 82.0 | 31.20 | 5.4 |
preferably, crystal form K has X-ray powder diffraction intensities shown in Table 12;
preferably, crystal form K has an X-ray powder diffraction pattern substantially shown in FIG. 12;
preferably, crystal form K exhibits an endothermic peak in a DSC analysis when heated to a peak temperature of about 91.51 °C;
preferably, crystal form K has a DSC profile substantially shown in FIG. 33;
preferably, crystal form K has a TGA profile substantially shown in FIG. 54;
preferably, crystal form K is a n-heptane 0.12-solvate of compound I;
crystal form L of a methylcyclohexane solvate has an X-ray powder diffraction pattern comprising peaks at diffraction angles (20) of 10.98 ± 0.2°, 15.54 ± 0.2°, 9.09 ± 0.2°, and 19.01 ± 0.2°;
preferably, further comprising peaks at diffraction angles (20) of 20.39 ± 0.2°, 17.94 ± 0.2°, 8.74 ± 0.2°, 21.03 ± 0.2°, and 13.46 ± 0.2';
and more preferably, further comprising peaks at diffraction angles (2θ) of 22.86 ± 0.2°, 18.25 ± 0.2°, 25.63 ± 0.2°, 16.90 ± 0.2°, 24.74 ± 0.2°, and 25.95 ± 0.2°;
preferably, crystal form L has an X-ray powder diffraction pattern comprising diffraction angles (2θ) shown in Table 13, wherein the 20 angles have a margin of error of ±0.20°:
**Table 13**
| 2θ (°) | Intensity% | 20 (°) | Intensity% |
|---|---|---|---|
| 5.35 | 23.5 | 16.90 | 35.5 |
| 6.25 | 18.5 | 17.94 | 71.0 |
| 6.73 | 17.5 | 18.25 | 44.0 |
| 7.83 | 24.5 | 19.01 | 78.0 |
| 8.74 | 59.5 | 20.39 | 72.5 |
| 9.09 | 84.0 | 21.03 | 57.0 |
| 10.98 | 100.0 | 22.86 | 48.5 |
| 13.46 | 55.0 | 24.74 | 35.0 |
| 15.54 | 100.0 | 25.63 | 41.5 |
| 16.58 | 26.5 | 25.95 | 33.5 |
preferably, crystal form L has X-ray powder diffraction intensities shown in Table 13;
preferably, crystal form L has an X-ray powder diffraction pattern substantially shown in FIG. 13;
preferably, crystal form L exhibits an endothermic peak in a DSC analysis when heated to a peak temperature of about 88.68 °C;
preferably, crystal form L has a DSC profile substantially shown in FIG. 34;
preferably, crystal form L has a TGA profile substantially shown in FIG. 55;
preferably, crystal form L is a methylcyclohexane 0.15-solvate of compound I;
crystal form M of a toluene solvate has an X-ray powder diffraction pattern comprising peaks at diffraction angles (20) of 18.62 ± 0.2°, 9.60 ± 0.2°, 16.34 ± 0.2°, and 21.62 ± 0.2°;
preferably, further comprising peaks at diffraction angles (20) of 14.84 ± 0.2°, 19.05 ± 0.2°, 19.36 ± 0.2°, 13.08 ± 0.2°, and 22.11 ± 0.2°;
and more preferably, further comprising peaks at diffraction angles (2θ) of 26.65 ± 0.2°, 24.48 ± 0.2°, 22.36 ± 0.2°, 11.19 ± 0.2°, 20.47 ± 0.2°, and 18.19 ± 0.2°;
preferably, crystal form M has an X-ray powder diffraction pattern comprising diffraction angles (2θ) shown in Table 14, wherein the 20 angles have a margin of error of ±0.20°:
**Table 14**
| 2θ (°) | Intensity% | 20 (°) | Intensity% |
|---|---|---|---|
| 6.79 | 12.5 | 25.34 | 2.4 |
| 7.79 | 1.8 | 25.58 | 17.3 |
| 9.60 | 96.6 | 26.06 | 4.0 |
| 10.46 | 2.6 | 26.41 | 5.7 |
| 11.19 | 20.0 | 26.65 | 25.7 |
| 11.87 | 6.3 | 27.08 | 4.0 |
| 13.08 | 33.9 | 27.28 | 2.6 |
| 13.67 | 8.3 | 27.63 | 6.9 |
| 14.31 | 1.1 | 28.62 | 1.5 |
| 14.84 | 38.6 | 29.21 | 4.0 |
| 15.78 | 6.4 | 29.43 | 3.1 |
| 16.34 | 51.2 | 29.76 | 7.6 |
| 17.33 | 4.0 | 30.00 | 5.1 |
| 18.19 | 18.0 | 30.23 | 5.4 |
| 18.62 | 100.0 | 30.83 | 1.3 |
| 19.05 | 35.4 | 31.24 | 2.9 |
| 19.36 | 34.8 | 31.77 | 3.3 |
| 20.26 | 17.2 | 32.61 | 4.1 |
| 20.47 | 19.8 | 33.34 | 1.3 |
| 20.67 | 12.9 | 33.84 | 6.9 |
| 21.39 | 10.9 | 34.20 | 2.2 |
| 21.62 | 47.0 | 35.25 | 2.5 |
| 22.11 | 29.5 | 35.64 | 2.2 |
| 22.36 | 21.4 | 37.41 | 2.1 |
| 23.82 | 12.6 | 37.85 | 2.2 |
| 24.48 | 21.8 | 38.83 | 1.0 |
| 24.93 | 4.5 | | |
preferably, crystal form M has X-ray powder diffraction intensities shown in Table 14;
preferably, crystal form M has an X-ray powder diffraction pattern substantially shown in FIG. 14;
preferably, crystal form M exhibits an endothermic peak in a DSC analysis when heated to a peak temperature of about 102.43 °C;
preferably, crystal form M has a DSC profile substantially shown in FIG. 35;
preferably, crystal form M has a TGA profile substantially shown in FIG. 56;
preferably, crystal form M is a toluene monosolvate of compound I;
crystal form N of a dioxane solvate has an X-ray powder diffraction pattern comprising peaks at diffraction angles (20) of 17.10 ± 0.2°, 20.66 ± 0.2°, 22.71 ± 0.2°, and 18.21 ± 0.2°;
preferably, further comprising peaks at diffraction angles (20) of 21.70 ± 0.2°, 15.05 ± 0.2°, 20.27 ± 0.2°, 21.97 ± 0.2°, and 8.53 ± 0.2°;
and more preferably, further comprising peaks at diffraction angles (20) of 19.59 ± 0.2°, 7.52 ± 0.2°, 11.14 ± 0.2°, 16.83 ± 0.2°, 17.47 ± 0.2°, and 23.57 ± 0.2°;
preferably, crystal form N has an X-ray powder diffraction pattern comprising diffraction angles (20) shown in Table 15, wherein the 20 angles have a margin of error of ±0.20°:
**Table 15**
| 2θ (°) | Intensity% | 2θ (°) | Intensity% |
|---|---|---|---|
| 6.85 | 7.6 | 21.70 | 40.1 |
| 7.17 | 5.1 | 21.97 | 35.1 |
| 7.52 | 28.2 | 22.71 | 48.0 |
| 8.53 | 36.8 | 23.57 | 26.5 |
| 8.87 | 2.2 | 23.84 | 14.5 |
| 9.77 | 25.7 | 24.44 | 16.9 |
| 10.75 | 19.5 | 25.12 | 8.2 |
| 11.14 | 29.4 | 26.08 | 13.7 |
| 12.63 | 4.2 | 26.98 | 8.4 |
| 13.38 | 7.1 | 27.27 | 14.0 |
| 13.71 | 11.6 | 27.58 | 13.2 |
| 14.34 | 25.9 | 27.94 | 5.5 |
| 15.05 | 38.1 | 28.50 | 4.9 |
| 15.34 | 6.7 | 28.81 | 6.3 |
| 16.12 | 9.4 | 29.16 | 6.3 |
| 16.83 | 28.4 | 29.57 | 4.7 |
| 17.10 | 100.0 | 30.37 | 3.7 |
| 17.47 | 28.2 | 30.86 | 4.5 |
| 17.67 | 15.4 | 31.61 | 3.2 |
| 18.21 | 44.1 | 32.43 | 4.6 |
| 18.46 | 8.5 | 32.78 | 2.4 |
| 18.91 | 6.8 | 34.46 | 3.0 |
| 19.17 | 15.7 | 35.99 | 3.7 |
| 19.59 | 30.8 | 36.62 | 3.9 |
| 20.27 | 35.3 | 36.99 | 4.3 |
| 20.66 | 49.5 | 38.20 | 3.0 |
| 21.19 | 24.9 | 38.95 | 1.1 |
preferably, crystal form N has X-ray powder diffraction intensities shown in Table 15;
preferably, crystal form N has an X-ray powder diffraction pattern substantially shown in FIG. 15;
preferably, crystal form N exhibits an endothermic peak in a DSC analysis when heated to a peak temperature of about 116.48 °C;
preferably, crystal form N has a DSC profile substantially shown in FIG. 36;
preferably, crystal form N has a TGA profile substantially shown in FIG. 57;
preferably, crystal form N is a dioxane 1.5-solvate of compound I;
crystal form O of a DMF solvate has an X-ray powder diffraction pattern comprising peaks at diffraction angles (20) of 17.72 ± 0.2°, 13.14 ± 0.2°, 15.08 ± 0.2°, and 24.77 ± 0.2°;
preferably, further comprising peaks at diffraction angles (20) of 8.72 ± 0.2°, 21.52 ± 0.2°, 9.70 ± 0.2°, 14.26 ± 0.2°, and 25.46 ± 0.2°;
and more preferably, further comprising peaks at diffraction angles (2θ) of 12.19 ± 0.2°, 20.15 ± 0.2°, 25.90 ± 0.2°, 23.66 ± 0.2°, 28.75 ± 0.2°, and 21.73 ± 0.2°;
preferably, crystal form O has an X-ray powder diffraction pattern comprising diffraction angles (2θ) shown in Table 16, wherein the 20 angles have a margin of error of ±0.20°:
**Table 16**
| 2θ (°) | Intensity% | 20 (°) | Intensity% |
|---|---|---|---|
| 6.66 | 3.7 | 22.09 | 5.4 |
| 7.08 | 3.7 | 22.48 | 4.0 |
| 7.52 | 2.3 | 23.06 | 5.4 |
| 8.72 | 32.4 | 23.66 | 15.5 |
| 9.70 | 23.4 | 23.93 | 2.0 |
| 11.01 | 2.2 | 24.77 | 94.4 |
| 12.195 | 16.8 | 25.46 | 21.0 |
| 13.14 | 87.4 | 25.90 | 14.4 |
| 14.26 | 21.4 | 26.53 | 7.5 |
| 15.08 | 92.5 | 27.35 | 1.9 |
| 16.13 | 2.8 | 28.46 | 6.2 |
| 16.59 | 2.8 | 28.75 | 11.3 |
| 16.79 | 3.0 | 29.70 | 5.7 |
| 17.72 | 100.0 | 30.15 | 1.5 |
| 18.51 | 3.1 | 30.81 | 2.7 |
| 18.86 | 1.0 | 31.03 | 3.8 |
| 19.26 | 4.0 | 32.46 | 1.7 |
| 19.56 | 6.5 | 34.16 | 1.2 |
| 20.15 | 14.4 | 34.67 | 1.1 |
| 20.96 | 1.1 | 35.95 | 1.9 |
| 21.52 | 30.2 | 36.35 | 1.8 |
| 21.73 | 9.1 | 37.92 | 1.2 |
preferably, crystal form O has X-ray powder diffraction intensities shown in Table 16;
preferably, crystal form O has an X-ray powder diffraction pattern substantially shown in FIG. 16;
preferably, crystal form O exhibits an endothermic peak in a DSC analysis when heated to a peak temperature of about 123.26 °C;
preferably, crystal form O has a DSC profile substantially shown in FIG. 37;
preferably, crystal form O has a TGA profile substantially shown in FIG. 58;
preferably, crystal form O is a DMF monosolvate of compound I;
crystal form P of a N-methylpyrrolidone solvate has an X-ray powder diffraction pattern comprising peaks at diffraction angles (20) of 14.63 ± 0.2°, 13.16 ± 0.2°, 16.98 ± 0.2°, and 14.36 ± 0.2°;
preferably, further comprising peaks at diffraction angles (20) of 21.66 ± 0.2°, 23.94 ± 0.2°, 20.22 ± 0.2°, 6.60 ± 0.2°, and 8.41 ± 0.2°;
and more preferably, further comprising peaks at diffraction angles (2θ) of 20.02 ± 0.2°, 11.63 ± 0.2°, 25.14 ± 0.2°, 24.87 ± 0.2°, 21.15 ± 0.2°, and 16.63 ± 0.2°;
preferably, crystal form P has an X-ray powder diffraction pattern comprising diffraction angles (20) shown in Table 17, wherein the 20 angles have a margin of error of ±0.20°:
**Table 17**
| 2θ (°) | Intensity% | 2θ (°) | Intensity% |
|---|---|---|---|
| 6.60 | 18.6 | 25.14 | 11.6 |
| 8.41 | 14.9 | 25.69 | 9.7 |
| 9.70 | 3.6 | 26.20 | 3.8 |
| 11.63 | 13.6 | 26.53 | 10.0 |
| 13.16 | 80.7 | 27.27 | 3.8 |
| 14.36 | 26.8 | 27.48 | 5.1 |
| 14.63 | 100.0 | 28.76 | 3.8 |
| 15.15 | 2.1 | 29.29 | 5.9 |
| 16.63 | 11.0 | 29.51 | 4.2 |
| 16.98 | 47.9 | 29.86 | 2.2 |
| 17.90 | 10.1 | 30.33 | 3.3 |
| 19.54 | 5.1 | 31.16 | 2.3 |
| 20.02 | 14.6 | 31.77 | 3.1 |
| 20.22 | 25.9 | 33.08 | 2.9 |
| 20.86 | 16.6 | 33.66 | 1.3 |
| 21.15 | 11.3 | 34.22 | 1.0 |
| 21.66 | 24.6 | 34.73 | 2.9 |
| 22.32 | 6.9 | 36.49 | 1.4 |
| 22.77 | 2.7 | 38.25 | 0.8 |
| 23.36 | 5.6 | 38.64 | 1.1 |
| 23.94 | 22.3 | 39.17 | 2.1 |
| 24.87 | 11.4 | | |
preferably, crystal form P has X-ray powder diffraction intensities shown in Table 17;
preferably, crystal form P has an X-ray powder diffraction pattern substantially shown in FIG. 17;
preferably, crystal form P exhibits an endothermic peak in a DSC analysis when heated to a peak temperature of about 117.63 °C;
preferably, crystal form P has a DSC profile substantially shown in FIG. 38;
preferably, crystal form P has a TGA profile substantially shown in FIG. 59;
preferably, crystal form P is a N-methylpyrrolidone monosolvate of compound I;
crystal form Q of a n-butanol solvate has an X-ray powder diffraction pattern comprising peaks at diffraction angles (20) of 18.05 ± 0.2°, 10.71 ± 0.2°, 12.54 ± 0.2°, and 18.78 ± 0.2°;
preferably, further comprising peaks at diffraction angles (20) of 20.68 ± 0.2°, 24.89 ± 0.2°, 26.45 ± 0.2°, 22.24 ± 0.2°, and 19.81 ± 0.2°;
and more preferably, further comprising peaks at diffraction angles (2θ) of 15.21 ± 0.2°, 9.00 ± 0.2°, 8.70 ± 0.2°, 23.84 ± 0.2°, 16.55 ± 0.2°, and 24.31 ± 0.2°;
preferably, crystal form Q has an X-ray powder diffraction pattern comprising diffraction angles (2θ) shown in Table 18, wherein the 20 angles have a margin of error of ±0.20°:
**Table 18**
| 2θ (°) | Intensity% | 20 (°) | Intensity% |
|---|---|---|---|
| 4.35 | 5.1 | 21.99 | 19.9 |
| 6.25 | 4.2 | 22.24 | 44.3 |
| 6.54 | 20.3 | 22.81 | 27.0 |
| 7.57 | 24.8 | 23.25 | 19.5 |
| 8.70 | 40.6 | 23.84 | 41.0 |
| 9.00 | 42.5 | 24.31 | 35.9 |
| 10.71 | 99.7 | 24.89 | 46.4 |
| 12.54 | 92.2 | 25.25 | 21.1 |
| 13.13 | 32.2 | 25.96 | 20.7 |
| 14.02 | 26.7 | 26.45 | 46.6 |
| 14.50 | 9.3 | 26.99 | 7.5 |
| 15.21 | 42.6 | 27.29 | 17.7 |
| 15.81 | 12.8 | 27.70 | 8.2 |
| 16.07 | 8.7 | 28.30 | 13.3 |
| 16.55 | 38.2 | 28.67 | 7.4 |
| 16.87 | 14.7 | 29.43 | 9.8 |
| 17.45 | 34.1 | 30.13 | 4.7 |
| 18.05 | 100.0 | 30.62 | 10.5 |
| 18.58 | 33.3 | 31.44 | 7.4 |
| 18.78 | 64.5 | 32.08 | 3.2 |
| 19.19 | 11.3 | 32.60 | 4.1 |
| 19.47 | 9.5 | 33.42 | 5.0 |
| 19.81 | 44.9 | 34.24 | 4.2 |
| 20.14 | 18.4 | 35.10 | 5.4 |
| 20.68 | 62.5 | 36.58 | 6.6 |
| 21.01 | 6.9 | 37.58 | 3.8 |
| 21.31 | 32.2 | 39.31 | 4.3 |
| 21.64 | 17.5 | | |
preferably, crystal form Q has X-ray powder diffraction intensities shown in Table 18;
preferably, crystal form Q has an X-ray powder diffraction pattern substantially shown in FIG. 18;
preferably, crystal form Q exhibits an endothermic peak in a DSC analysis when heated to a peak temperature of about 100.61 °C;
preferably, crystal form Q has a DSC profile substantially shown in FIG. 39;
preferably, crystal form Q has a TGA profile substantially shown in FIG. 60;
preferably, crystal form Q is a n-butanol 0.5-solvate of compound I;
crystal form R of a n-propanol solvate has an X-ray powder diffraction pattern comprising peaks at diffraction angles (20) of 10.67 ± 0.2°, 12.52 ± 0.2°, 18.08 ± 0.2°, and 8.98 ± 0.2°;
preferably, further comprising peaks at diffraction angles (20) of 6.54 ± 0.2°, 20.68 ± 0.2°, 18.84 ± 0.2°, 7.55 ± 0.2°, and 8.67 ± 0.2°;
and more preferably, further comprising peaks at diffraction angles (2θ) of 19.85 ± 0.2°, 26.59 ± 0.2°, 15.19 ± 0.2°, 13.05 ± 0.2°, 14.06 ± 0.2°, and 16.55 ± 0.2°;
preferably, crystal form R has an X-ray powder diffraction pattern comprising diffraction angles (20) shown in Table 19, wherein the 20 angles have a margin of error of ±0.20°:
**Table 19**
| 2θ (°) | Intensity% | 2θ (°) | Intensity% |
|---|---|---|---|
| 6.17 | 7.0 | 21.35 | 8.7 |
| 6.54 | 46.7 | 21.70 | 11.4 |
| 7.55 | 34.4 | 22.13 | 7.9 |
| 8.67 | 35.2 | 22.89 | 8.6 |
| 8.98 | 53.5 | 23.19 | 3.8 |
| 10.67 | 100.0 | 23.72 | 5.3 |
| 12.52 | 75.8 | 24.27 | 10.5 |
| 13.05 | 20.8 | 24.91 | 11.0 |
| 14.06 | 20.6 | 25.49 | 9.4 |
| 14.45 | 8.0 | 25.96 | 6.5 |
| 15.19 | 23.9 | 26.59 | 26.2 |
| 16.55 | 20.0 | 27.12 | 3.2 |
| 16.94 | 4.1 | 28.38 | 9.5 |
| 17.45 | 16.9 | 29.51 | 3.4 |
| 18.08 | 57.5 | 30.79 | 2.4 |
| 18.53 | 17.8 | 31.57 | 3.5 |
| 18.84 | 52.1 | 33.45 | 3.1 |
| 19.24 | 14.9 | 35.46 | 2.8 |
| 19.85 | 34.7 | 38.18 | 2.7 |
| 20.68 | 43.4 | | |
preferably, crystal form R has X-ray powder diffraction intensities shown in Table 19;
preferably, crystal form R has an X-ray powder diffraction pattern substantially shown in FIG. 19;
preferably, crystal form R exhibits an endothermic peak in a DSC analysis when heated to a peak temperature of about 110.90 °C;
preferably, crystal form R has a DSC profile substantially shown in FIG. 40;
preferably, crystal form R has a TGA profile substantially shown in FIG. 61;
preferably, crystal form R is a n-propanol monosolvate of compound I;
crystal form S of a tetrahydrofuran solvate has an X-ray powder diffraction pattern comprising peaks at diffraction angles (20) of 12.17 ± 0.2°, 8.19 ± 0.2°, 7.67 ± 0.2°, and 13.96 ± 0.2°;
preferably, further comprising peaks at diffraction angles (20) of 8.95 ± 0.2°, 18.01 ± 0.2°, 16.50 ± 0.2°, 19.71 ± 0.2°, and 23.70 ± 0.2°;
and more preferably, further comprising peaks at diffraction angles (20) of 13.69 ± 0.2°, 13.22 ± 0.2°, 14.45 ± 0.2°, 20.64 ± 0.2°, 15.83 ± 0.2°, and 6.48 ± 0.2°;
preferably, crystal form S has an X-ray powder diffraction pattern comprising diffraction angles (20) shown in Table 20, wherein the 20 angles have a margin of error of ±0.20°:
**Table 20**
| 2θ (°) | Intensity% | 20 (°) | Intensity% |
|---|---|---|---|
| 4.07 | 8.4 | 20.12 | 4.4 |
| 6.48 | 10.0 | 20.64 | 12.7 |
| 6.81 | 9.6 | 21.11 | 7.1 |
| 7.67 | 42.3 | 21.35 | 6.9 |
| 8.19 | 69.0 | 21.74 | 1.4 |
| 8.95 | 33.3 | 22.26 | 3.1 |
| 10.46 | 6.0 | 22.79 | 5.6 |
| 12.17 | 100.0 | 23.00 | 2.2 |
| 13.22 | 13.4 | 23.70 | 18.4 |
| 13.69 | 15.3 | 24.37 | 5.3 |
| 13.96 | 38.4 | 25.27 | 1.1 |
| 14.45 | 14.2 | 25.82 | 6.2 |
| 15.43 | 3.3 | 26.37 | 4.4 |
| 15.83 | 14.5 | 27.70 | 4.6 |
| 16.50 | 22.3 | 29.10 | 3.1 |
| 17.76 | 7.6 | 30.81 | 1.1 |
| 18.01 | 37.6 | 31.30 | 1.9 |
| 19.71 | 21.4 | 31.94 | 0.8 |
preferably, crystal form S has X-ray powder diffraction intensities shown in Table 20;
preferably, crystal form S has an X-ray powder diffraction pattern substantially shown in FIG. 20;
preferably, crystal form S exhibits an endothermic peak in a DSC analysis when heated to a peak temperature of about 96.26 °C;
preferably, crystal form S has a DSC profile substantially shown in FIG. 41;
preferably, crystal form S has a TGA profile substantially shown in FIG. 62;
preferably, crystal form S is a tetrahydrofuran 0.5-solvate of compound I;
crystal form T of a 2-methyltetrahydrofuran solvate has an X-ray powder diffraction pattern comprising peaks at diffraction angles (20) of 10.79 ± 0.2°, 8.66 ± 0.2°, 9.12 ± 0.2°, and 16.87 ± 0.2°;
preferably, further comprising peaks at diffraction angles (20) of 18.64 ± 0.2°, 15.54 ± 0.2°, 21.27 ± 0.2°, 13.57 ± 0.2°, and 6.72 ± 0.2°;
and more preferably, further comprising peaks at diffraction angles (20) of 4.29 ± 0.2°, 14.41 ± 0.2°, 19.99 ± 0.2°, 7.71 ± 0.2°, 16.57 ± 0.2°, and 19.42 ± 0.2°;
preferably, crystal form T has an X-ray powder diffraction pattern comprising diffraction angles (2θ) shown in Table 21, wherein the 20 angles have a margin of error of ±0.20°:
**Table 21**
| 2θ (°) | Intensity% | 2θ (°) | Intensity% |
|---|---|---|---|
| 4.29 | 15.2 | 19.42 | 10.6 |
| 6.72 | 16.5 | 19.99 | 14.9 |
| 7.32 | 1.7 | 20.47 | 8.1 |
| 7.71 | 12.7 | 20.71 | 5.8 |
| 8.66 | 71.9 | 21.27 | 18.7 |
| 9.12 | 52.0 | 21.87 | 7.7 |
| 9.62 | 1.9 | 22.30 | 5.0 |
| 10.79 | 100.0 | 22.85 | 3.5 |
| 12.62 | 16.1 | 23.62 | 3.4 |
| 13.05 | 9.5 | 24.14 | 1.4 |
| 13.57 | 19.5 | 25.03 | 2.4 |
| 14.41 | 15.3 | 25.95 | 8.9 |
| 15.54 | 24.9 | 26.22 | 5.7 |
| 16.57 | 10.8 | 26.70 | 2.8 |
| 16.87 | 48.4 | 27.44 | 9.1 |
| 17.45 | 8.1 | 28.17 | 1.9 |
| 17.82 | 4.1 | 29.37 | 1.8 |
| 18.64 | 26.4 | 31.50 | 2.9 |
preferably, crystal form T has X-ray powder diffraction intensities shown in Table 21;
preferably, crystal form T has an X-ray powder diffraction pattern substantially shown in FIG. 21;
preferably, crystal form T exhibits an endothermic peak in a DSC analysis when heated to a peak temperature of about 112.95 °C;
preferably, crystal form T has a DSC profile substantially shown in FIG. 42;
preferably, crystal form T has a TGA profile substantially shown in FIG. 63;
preferably, crystal form T is a 2-methyltetrahydrofuran monosolvate of compound I.

4. The polymorph according to claim 1 or 2, wherein the polymorph is a crystal form of a non-solvate of compound II, including the following crystal forms 2A, 2B, 2C, 2D, and 2E of non-solvates, wherein:
crystal form 2A has an X-ray powder diffraction pattern comprising peaks at diffraction angles (20) of 13.94 ± 0.2°, 22.07 ± 0.2°, 17.96 ± 0.2°, and 17.57 ± 0.2°;
preferably, further comprising peaks at diffraction angles (20) of 13.36 ± 0.2°, 5.92 ± 0.2°, 12.33 ± 0.2°, 23.04 ± 0.2°, and 11.02 ± 0.2°;
and more preferably, further comprising peaks at diffraction angles (20) of 22.41 ± 0.2°, 7.42 ± 0.2°, 25.07 ± 0.2°, 27.00 ± 0.2°, 8.71 ± 0.2°, and 16.60 ± 0.2°;
preferably, crystal form 2A has an X-ray powder diffraction pattern comprising diffraction angles (20) shown in Table 2-1, wherein the 20 angles have a margin of error of ±0.20°:
**Table 2-1**
| 20 (°) | Intensity% | 20 (°) | Intensity% |
|---|---|---|---|
| 5.92 | 21.5 | 19.96 | 6.4 |
| 7.42 | 11.0 | 22.07 | 48.6 |
| 8.71 | 7.8 | 22.41 | 11.1 |
| 11.02 | 14.6 | 23.04 | 15.7 |
| 11.92 | 5.2 | 23.78 | 4.8 |
| 12.33 | 17.3 | 25.07 | 9.7 |
| 13.36 | 26.8 | 27.00 | 9.1 |
| 13.94 | 100.0 | 28.22 | 3.8 |
| 16.60 | 6.8 | 29.49 | 2.2 |
| 17.57 | 42.2 | 30.50 | 4.1 |
| 17.96 | 46.1 | 30.99 | 2.2 |
preferably, crystal form 2A has X-ray powder diffraction intensities shown in Table 2-1;
preferably, crystal form 2A has an X-ray powder diffraction pattern substantially shown in FIG. 2-1;
preferably, crystal form 2A exhibits an endothermic peak in a DSC analysis when heated to a peak temperature of about 189.90 °C;
preferably, crystal form 2A has a DSC profile substantially shown in FIG. 2-26;
preferably, crystal form 2A has a TGA profile substantially shown in FIG. 2-51;
crystal form 2B has an X-ray powder diffraction pattern comprising peaks at diffraction angles (20) of 13.16 ± 0.2°, 21.31 ± 0.2°, 25.46 ± 0.2°, and 10.61 ± 0.2°;
preferably, further comprising peaks at diffraction angles (20) of 16.79 ± 0.2°, 20.99 ± 0.2°, 18.76 ± 0.2°, 19.59 ± 0.2°, and 9.71 ± 0.2°;
and more preferably, further comprising peaks at diffraction angles (2θ) of 23.72 ± 0.2°, 11.68 ± 0.2°, 23.92 ± 0.2°, 20.57 ± 0.2°, 16.48 ± 0.2°, and 20.23 ± 0.2°;
preferably, crystal form 2B has an X-ray powder diffraction pattern comprising diffraction angles (20) shown in Table 2-2, wherein the 20 angles have a margin of error of ±0.20°:
**Table 2-2**
| 2θ (°) | Intensity% | 2θ (°) | Intensity% |
|---|---|---|---|
| 9.71 | 59.9 | 19.59 | 62.8 |
| 10.01 | 13.3 | 20.23 | 31.0 |
| 10.61 | 92.7 | 20.57 | 32.2 |
| 11.68 | 35.6 | 20.99 | 81.9 |
| 12.31 | 14.3 | 21.31 | 96.0 |
| 12.71 | 21.4 | 22.16 | 21.4 |
| 13.16 | 100.0 | 23.72 | 47.6 |
| 14.64 | 27.4 | 23.92 | 33.5 |
| 15.87 | 7.1 | 24.56 | 26.4 |
| 16.48 | 31.2 | 25.46 | 95.2 |
| 16.79 | 82.7 | 26.45 | 20.4 |
| 17.15 | 19.1 | 28.30 | 27.7 |
| 17.96 | 25.4 | 29.00 | 10.8 |
| 18.76 | 74.8 | 29.64 | 29.5 |
| 19.02 | 13.9 | 31.51 | 8.7 |
preferably, crystal form 2B has X-ray powder diffraction intensities shown in Table 2-2;
preferably, crystal form 2B has an X-ray powder diffraction pattern substantially shown in FIG. 2-2;
preferably, crystal form 2B exhibits an endothermic peak in a DSC analysis when heated to a peak temperature of about 181.06 °C;
preferably, crystal form 2B has a DSC profile substantially shown in FIG. 2-27;
preferably, crystal form 2B has a TGA profile substantially shown in FIG. 2-52;
crystal form 2C has an X-ray powder diffraction pattern comprising peaks at diffraction angles (20) of 13.63 ± 0.2°, 5.62 ± 0.2°, 17.12 ± 0.2°, and 17.68 ± 0.2°;
preferably, further comprising peaks at diffraction angles (20) of 11.94 ± 0.2°, 13.89 ± 0.2°, 22.65 ± 0.2°, 23.63 ± 0.2°, and 17.97 ± 0.2°;
and more preferably, further comprising peaks at diffraction angles (2θ) of 7.46 ± 0.2°, 27.52 ± 0.2°, 8.80 ± 0.2°, 25.15 ± 0.2°, 19.36 ± 0.2°, and 5.88 ± 0.2°;
preferably, crystal form 2C has an X-ray powder diffraction pattern comprising diffraction angles (20) shown in Table 2-3, wherein the 20 angles have a margin of error of ±0.20°:
**Table 2-3**
| 20 (°) | Intensity% | 20 (°) | Intensity% |
|---|---|---|---|
| 5.62 | 60.9 | 17.97 | 12.8 |
| 5.88 | 5.5 | 19.36 | 6.6 |
| 7.46 | 12.4 | 20.47 | 2.7 |
| 8.80 | 7.2 | 21.17 | 3.0 |
| 11.94 | 33.9 | 22.10 | 3.2 |
| 12.32 | 5.3 | 22.65 | 19.9 |
| 13.63 | 100.0 | 23.63 | 15.9 |
| 13.89 | 33.5 | 25.15 | 7.0 |
| 17.12 | 59.5 | 27.52 | 8.6 |
| 17.68 | 42.9 | 28.13 | 4.7 |
preferably, crystal form 2C has X-ray powder diffraction intensities shown in Table 2-3;
preferably, crystal form 2C has an X-ray powder diffraction pattern substantially shown in FIG. 2-3;
preferably, crystal form 2C exhibits an endothermic peak in a DSC analysis when heated to a peak temperature of about 171.63 °C;
preferably, crystal form 2C has a DSC profile substantially shown in FIG. 2-28;
preferably, crystal form 2C has a TGA profile substantially shown in FIG. 2-53;
crystal form 2D has an X-ray powder diffraction pattern comprising peaks at diffraction angles (2θ) of 5.62 ± 0.2°, 12.01 ± 0.2°, 5.24 ± 0.2°, and 17.72 ± 0.2°;
preferably, further comprising peaks at diffraction angles (20) of 13.65 ± 0.2°, 17.16 ± 0.2°, 23.63 ± 0.2°, 19.30 ± 0.2°, and 16.43 ± 0.2°;
and more preferably, further comprising peaks at diffraction angles (20) of 15.85 ± 0.2°, 11.04 ± 0.2°, 7.50 ± 0.2°, 5.90 ± 0.2°, 22.18 ± 0.2°, and 22.65 ± 0.2°;
preferably, crystal form 2D has an X-ray powder diffraction pattern comprising diffraction angles (20) shown in Table 2-4, wherein the 20 angles have a margin of error of ±0.20°:
**Table 2-4**
| 2θ (°) | Intensity% | 2θ (°) | Intensity% |
|---|---|---|---|
| 5.24 | 88.2 | 17.72 | 87.0 |
| 5.62 | 100.0 | 18.98 | 32.3 |
| 5.90 | 42.9 | 19.30 | 61.5 |
| 7.50 | 43.5 | 20.12 | 21.1 |
| 11.04 | 48.4 | 20.49 | 23.0 |
| 12.01 | 90.1 | 21.63 | 12.4 |
| 13.06 | 24.8 | 22.18 | 41.0 |
| 13.65 | 83.9 | 22.43 | 33.5 |
| 14.32 | 32.3 | 22.65 | 34.2 |
| 15.85 | 53.4 | 22.65 | 34.2 |
| 16.43 | 54.7 | 23.63 | 75.2 |
| 17.16 | 77.6 | 27.59 | 21.7 |
preferably, crystal form 2D has X-ray powder diffraction intensities shown in Table 2-4;
preferably, crystal form 2D has an X-ray powder diffraction pattern substantially shown in FIG. 2-4;
preferably, crystal form 2D exhibits an endothermic peak in a DSC analysis when heated to a peak temperature of about 168.79 °C;
preferably, crystal form 2D has a DSC profile substantially shown in FIG. 2-29;
preferably, crystal form 2D has a TGA profile substantially shown in FIG. 2-54;
crystal form 2E has an X-ray powder diffraction pattern comprising peaks at diffraction angles (20) of 17.96 ± 0.2°, 3.34 ± 0.2°, 5.63 ± 0.2°, and 13.63 ± 0.2°;
preferably, further comprising peaks at diffraction angles (20) of 17.14 ± 0.2°, 11.96 ± 0.2°, 12.19 ± 0.2°, 13.98 ± 0.2°, and 7.46 ± 0.2°;
and more preferably, further comprising peaks at diffraction angles (20) of 19.53 ± 0.2°, 22.14 ± 0.2°, 8.87 ± 0.2°, 19.32 ± 0.2°, 23.90 ± 0.2°, and 19.83 ± 0.2°;
preferably, crystal form 2E has an X-ray powder diffraction pattern comprising diffraction angles (20) shown in Table 2-5, wherein the 20 angles have a margin of error of ±0.20°:
**Table 2-5**
| 2θ (°) | Intensity% | 2θ (°) | Intensity% |
|---|---|---|---|
| 3.34 | 71.0 | 15.86 | 10.5 |
| 5.63 | 51.8 | 17.14 | 47.4 |
| 5.90 | 11.0 | 17.96 | 100.0 |
| 7.46 | 28.7 | 19.32 | 15.6 |
| 8.24 | 12.6 | 19.53 | 18.2 |
| 8.87 | 16.9 | 19.83 | 13.6 |
| 11.07 | 9.2 | 21.09 | 9.2 |
| 11.96 | 46.2 | 22.14 | 18.2 |
| 12.19 | 42.1 | 23.90 | 15.6 |
| 13.63 | 47.9 | 25.87 | 12.1 |
| 13.98 | 41.5 | 27.48 | 7.9 |
preferably, crystal form 2E has X-ray powder diffraction intensities shown in Table 2-5;
preferably, crystal form 2E has an X-ray powder diffraction pattern substantially shown in FIG. 2-5;
preferably, crystal form 2E exhibits endothermic peaks in a DSC analysis when heated to peak temperatures of about 110.30 °C and about 169.27 °C;
preferably, crystal form 2E has a DSC profile substantially shown in FIG. 2-30;
preferably, crystal form 2E has a TGA profile substantially shown in FIG. 2-55;
preferably, the polymorph is a crystal form of a hydrate of compound II, including the following crystal forms 2F and 2G of hydrates, wherein:
crystal form 2F of a hydrate has an X-ray powder diffraction pattern comprising peaks at diffraction angles (20) of 19.51 ± 0.2°, 13.71 ± 0.2°, 14.29 ± 0.2°, and 18.09 ± 0.2°;
preferably, further comprising peaks at diffraction angles (20) of 6.42 ± 0.2°, 12.11 ± 0.2°, 24.93 ± 0.2°, 17.86 ± 0.2°, and 20.57 ± 0.2°;
and more preferably, further comprising peaks at diffraction angles (2θ) of 21.37 ± 0.2°, 13.34 ± 0.2°, 23.39 ± 0.2°, 25.15 ± 0.2°, 30.64 ± 0.2°, and 17.25 ± 0.2°;
preferably, crystal form 2F has an X-ray powder diffraction pattern comprising diffraction angles (20) shown in Table 2-6, wherein the 20 angles have a margin of error of ±0.20°:
**Table 2-6**
| 20 (°) | Intensity% | 2θ (°) | Intensity% |
|---|---|---|---|
| 6.42 | 29.2 | 20.57 | 18.6 |
| 8.72 | 9.8 | 21.37 | 17.8 |
| 9.50 | 6.1 | 22.23 | 3.3 |
| 11.31 | 10.0 | 22.81 | 6.2 |
| 12.11 | 27.9 | 23.39 | 16.2 |
| 12.95 | 7.9 | 24.03 | 3.1 |
| 13.34 | 16.3 | 24.54 | 6.5 |
| 13.71 | 74.6 | 24.93 | 26.5 |
| 14.29 | 42.3 | 25.15 | 14.0 |
| 14.63 | 5.2 | 25.55 | 3.5 |
| 15.23 | 6.5 | 26.14 | 10.9 |
| 16.46 | 3.8 | 26.41 | 7.6 |
| 17.25 | 12.1 | 27.06 | 9.8 |
| 17.86 | 20.4 | 27.54 | 6.5 |
| 18.09 | 36.8 | 30.64 | 12.6 |
| 19.51 | 100.0 | 31.82 | 4.8 |
| 20.13 | 2.8 | | |
preferably, crystal form 2F has X-ray powder diffraction intensities shown in Table 2-6;
preferably, crystal form 2F has an X-ray powder diffraction pattern substantially shown in FIG. 2-6;
preferably, crystal form 2F exhibits an endothermic peak in a DSC analysis when heated to a peak temperature of about 114.41 °C;
preferably, crystal form 2F has a DSC profile substantially shown in FIG. 2-31;
preferably, crystal form 2F has a TGA profile substantially shown in FIG. 2-56;
preferably, crystal form 2F is a trihydrate of compound II;
crystal form 2G of a hydrate has an X-ray powder diffraction pattern comprising peaks at diffraction angles (2θ) of 17.45 ± 0.2°, 13.63 ± 0.2°, 5.61 ± 0.2°, and 5.24 ± 0.2°;
preferably, further comprising peaks at diffraction angles (20) of 13.89 ± 0.2°, 10.69 ± 0.2°, 11.78 ± 0.2°, 23.53 ± 0.2°, and 23.86 ± 0.2°;
and more preferably, further comprising peaks at diffraction angles (2θ) of 20.98 ± 0.2°, 27.54 ± 0.2°, 15.46 ± 0.2°, 22.52 ± 0.2°, 6.89 ± 0.2°, and 22.08 ± 0.2°;
preferably, crystal form 2G has an X-ray powder diffraction pattern comprising diffraction angles (20) shown in Table 2-7, wherein the 20 angles have a margin of error of ±0.20°:
**Table 2-7**
| 20 (°) | Intensity% | 20 (°) | Intensity% |
|---|---|---|---|
| 5.24 | 70.0 | 17.45 | 100.0 |
| 5.61 | 74.8 | 20.98 | 25.2 |
| 6.89 | 14.8 | 22.08 | 14.8 |
| 10.69 | 45.7 | 22.52 | 20.5 |
| 11.78 | 33.3 | 23.53 | 31.4 |
| 13.63 | 88.6 | 23.86 | 31.4 |
| 13.89 | 69.5 | 27.54 | 18.6 |
| 15.46 | 21.4 | | |
preferably, crystal form 2G has X-ray powder diffraction intensities shown in Table 2-7;
preferably, crystal form 2G has an X-ray powder diffraction pattern substantially shown in FIG. 2-7;
preferably, crystal form 2G exhibits an endothermic peak in a DSC analysis when heated to a peak temperature of about 169.22 °C;
preferably, crystal form 2G has a DSC profile substantially shown in FIG. 2-32;
preferably, crystal form 2G has a TGA profile substantially shown in FIG. 2-57;
preferably, crystal form 2G is a dihydrate of compound II;
preferably, the polymorph is a crystal form of a solvate of compound II, including the following crystal forms 2H, 2I, 2J-1, 2J-2, 2K, 2L-1, 2L-2, 2M-1, 2M-2, 2N, 2O, 2P, 2Q-1, 2Q-2, 2R, 2S, 2T, and 2U of solvates, wherein:
crystal form 2H of a dimethylsulfoxide solvate has an X-ray powder diffraction pattern comprising peaks at diffraction angles (20) of 14.84 ± 0.2°, 13.42 ± 0.2°, 24.68 ± 0.2°, and 21.70 ± 0.2°;
preferably, further comprising peaks at diffraction angles (20) of 8.45 ± 0.2°, 24.46 ± 0.2°, 20.22 ± 0.2°, 17.22 ± 0.2°, and 3.23 ± 0.2°;
and more preferably, further comprising peaks at diffraction angles (2θ) of 14.61 ± 0.2°, 25.32 ± 0.2°, 15.56 ± 0.2°, 22.01 ± 0.2°, 18.52 ± 0.2°, and 21.21 ± 0.2°;
preferably, crystal form 2H has an X-ray powder diffraction pattern comprising diffraction angles (20) shown in Table 2-8, wherein the 20 angles have a margin of error of ±0.20°:
**Table 2-8**
| 2θ (°) | Intensity% | 2θ (°) | Intensity% |
|---|---|---|---|
| 3.23 | 23.8 | 19.59 | 7.1 |
| 6.64 | 9.9 | 19.84 | 5.6 |
| 7.36 | 4.9 | 20.22 | 26.8 |
| 8.45 | 29.6 | 20.78 | 7.4 |
| 10.03 | 3.7 | 21.21 | 13.2 |
| 11.51 | 6.5 | 21.70 | 29.9 |
| 13.42 | 51.0 | 22.01 | 15.5 |
| 14.61 | 19.5 | 22.41 | 6.8 |
| 14.84 | 100.0 | 22.61 | 3.7 |
| 15.56 | 16.7 | 23.00 | 8.2 |
| 16.63 | 5.9 | 24.46 | 28.3 |
| 17.22 | 25.6 | 24.68 | 30.4 |
| 17.71 | 8.5 | 25.32 | 19.5 |
| 18.52 | 14.5 | 28.24 | 6.2 |
| 18.72 | 8.7 | 28.90 | 6.2 |
| 19.15 | 8.2 | 31.43 | 4.5 |
preferably, crystal form 2H has X-ray powder diffraction intensities shown in Table 2-8;
preferably, crystal form 2H has an X-ray powder diffraction pattern substantially shown in FIG. 2-8;
preferably, crystal form 2H exhibits an endothermic peak in a DSC analysis when heated to a peak temperature of about 118.06 °C;
preferably, crystal form 2H has a DSC profile substantially shown in FIG. 2-33;
preferably, crystal form 2H has a TGA profile substantially shown in FIG. 2-58;
preferably, crystal form 2H is a dimethylsulfoxide monosolvate of compound II;
crystal form 2I of a methyl *tert*-butyl ether solvate has an X-ray powder diffraction pattern comprising peaks at diffraction angles (20) of 8.74 ± 0.2°, 10.94 ± 0.2°, 4.32 ± 0.2°, and 17.66 ± 0.2°;
preferably, further comprising peaks at diffraction angles (20) of 13.18 ± 0.2°, 9.25 ± 0.2°, 18.79 ± 0.2°, 17.12 ± 0.2°, and 12.79°;
and more preferably, further comprising peaks at diffraction angles (2θ) of 22.13 ± 0.2°, 25.90 ± 0.2°, 19.38 ± 0.2°, 20.90 ± 0.2°, 15.91 ± 0.2°, and 6.91 ± 0.2°;
preferably, crystal form 2I has an X-ray powder diffraction pattern comprising diffraction angles (2θ) shown in Table 2-9, wherein the 20 angles have a margin of error of ±0.20°:
**Table 2-9**
| 2θ (°) | Intensity% | 2θ (°) | Intensity% |
|---|---|---|---|
| 4.32 | 22.4 | 17.12 | 10.7 |
| 6.91 | 4.0 | 17.66 | 20.1 |
| 8.74 | 100.0 | 18.79 | 11.8 |
| 9.25 | 15.7 | 19.38 | 6.5 |
| 10.94 | 33.9 | 20.90 | 5.8 |
| 12.79 | 8.3 | 22.13 | 7.8 |
| 13.18 | 16.3 | 23.90 | 3.9 |
| 15.91 | 5.0 | 25.90 | 6.7 |
preferably, crystal form 2I has X-ray powder diffraction intensities shown in Table 2-9;
preferably, crystal form 2I has an X-ray powder diffraction pattern substantially shown in FIG. 2-9;
preferably, crystal form 2I exhibits an endothermic peak in a DSC analysis when heated to a peak temperature of about 115.84 °C;
preferably, crystal form 2I has a DSC profile substantially shown in FIG. 2-34;
preferably, crystal form 2I has a TGA profile substantially shown in FIG. 2-59;
preferably, crystal form 2I is a methyl *tert-butyl* ether 0.5-solvate of compound II;
crystal form 2J-1 of a n-heptane solvate has an X-ray powder diffraction pattern comprising peaks at diffraction angles (20) of 12.15 ± 0.2°, 8.91 ± 0.2°, 8.18 ± 0.2°, and 7.63 ± 0.2°;
preferably, further comprising peaks at diffraction angles (20) of 13.93 ± 0.2°, 17.94 ± 0.2°, 19.48 ± 0.2°, 23.72 ± 0.2°, and 15.81 ± 0.2°;
and more preferably, further comprising peaks at diffraction angles (20) of 14.37 ± 0.2°, 20.43 ± 0.2°, 6.75 ± 0.2°, 22.68 ± 0.2°, 13.60 ± 0.2°, and 27.41 ± 0.2°;
preferably, crystal form 2J-1 has an X-ray powder diffraction pattern comprising diffraction angles (20) shown in Table 2-10, wherein the 20 angles have a margin of error of ±0.20°:
**Table 2-10**
| 20 (°) | Intensity% | 20 (°) | Intensity% |
|---|---|---|---|
| 6.75 | 18.1 | 17.10 | 10.6 |
| 7.63 | 50.6 | 17.94 | 45.7 |
| 8.18 | 53.7 | 19.48 | 41.6 |
| 8.91 | 81.7 | 20.43 | 20.0 |
| 12.15 | 100.0 | 21.09 | 8.6 |
| 13.07 | 9.9 | 21.39 | 13.8 |
| 13.60 | 16.2 | 22.68 | 17.0 |
| 13.93 | 46.1 | 23.72 | 28.9 |
| 14.37 | 22.8 | 25.68 | 13.6 |
| 15.81 | 24.4 | 27.41 | 14.0 |
| 16.48 | 13.1 | | |
preferably, crystal form 2J-1 has X-ray powder diffraction intensities shown in Table 2-10;
preferably, crystal form 2J-1 has an X-ray powder diffraction pattern substantially shown in FIG. 2-10;
preferably, crystal form 2J-1 exhibits an endothermic peak in a DSC analysis when heated to a peak temperature of about 103.13 °C;
preferably, crystal form 2J-1 has a DSC profile substantially shown in FIG. 2-35;
preferably, crystal form 2J-1 has a TGA profile substantially shown in FIG. 2-60;
preferably, crystal form 2J-1 is a n-heptane 0.2-solvate of compound II;
crystal form 2J-2 of a n-heptane solvate has an X-ray powder diffraction pattern comprising peaks at diffraction angles (20) of 3.52 ± 0.2°, 17.76 ± 0.2°, 14.19 ± 0.2°, and 10.61 ± 0.2°;
preferably, further comprising peaks at diffraction angles (2θ) of 7.05 ± 0.2°, 21.35 ± 0.2°, 16.24 ± 0.2°, and 19.49 ± 0.2°;
preferably, crystal form 2J-2 has an X-ray powder diffraction pattern comprising diffraction angles (20) shown in Table 2-11, wherein the 20 angles have a margin of error of ±0.20°:
**Table 2-11**
| 20 (°) | Intensity% | 20 (°) | Intensity% |
|---|---|---|---|
| 3.52 | 100 | 16.24 | 6.1 |
| 7.05 | 22.5 | 17.76 | 76.6 |
| 10.61 | 30.2 | 19.49 | 4.8 |
| 14.19 | 41.8 | 21.35 | 12.7 |
preferably, crystal form 2J-2 has X-ray powder diffraction intensities shown in Table 2-11;
preferably, crystal form 2J-2 has an X-ray powder diffraction pattern substantially shown in FIG. 2-11;
preferably, crystal form 2J-2 exhibits an endothermic peak in a DSC analysis when heated to a peak temperature of about 76.82 °C;
preferably, crystal form 2J-2 has a DSC profile substantially shown in FIG. 2-36;
preferably, crystal form 2J-2 has a TGA profile substantially shown in FIG. 2-61;
preferably, crystal form 2J-2 is a *n*-heptane 0.5-solvate of compound II;
crystal form 2K of a toluene solvate has an X-ray powder diffraction pattern comprising peaks at diffraction angles (20) of 9.52 ± 0.2°, 18.45 ± 0.2°, 19.09 ± 0.2°, and 21.48 ± 0.2°;
preferably, further comprising peaks at diffraction angles (20) of 16.24 ± 0.2°, 14.76 ± 0.2°, 13.04 ± 0.2°, 22.11 ± 0.2°, and 3.50 ± 0.2°;
and more preferably, further comprising peaks at diffraction angles (20) of 18.23 ± 0.2°, 17.96 ± 0.2°, 26.57 ± 0.2°, 20.31 ± 0.2°, 14.53 ± 0.2°, and 23.76 ± 0.2°;
preferably, crystal form 2K has an X-ray powder diffraction pattern comprising diffraction angles (20) shown in Table 2-12, wherein the 20 angles have a margin of error of ±0.20°:
**Table 2-12**
| 2θ (°) | Intensity% | 2θ (°) | Intensity% |
|---|---|---|---|
| 3.50 | 23.4 | 19.36 | 17.2 |
| 5.62 | 11.7 | 20.31 | 20.5 |
| 7.73 | 8.2 | 20.51 | 16.6 |
| 9.52 | 100.0 | 21.05 | 4.3 |
| 10.46 | 4.9 | 21.48 | 47.9 |
| 11.15 | 16.8 | 22.11 | 31.7 |
| 11.75 | 6.0 | 22.48 | 11.3 |
| 11.94 | 7.3 | 23.76 | 17.3 |
| 13.04 | 31.7 | 24.15 | 15.1 |
| 13.63 | 19.5 | 24.44 | 17.1 |
| 13.85 | 5.5 | 25.36 | 15.6 |
| 14.76 | 35.1 | 25.78 | 3.4 |
| 15.71 | 3.5 | 26.31 | 6.0 |
| 16.24 | 45.5 | 26.57 | 20.8 |
| 17.12 | 11.5 | 27.19 | 7.8 |
| 17.69 | 13.7 | 27.77 | 4.1 |
| 17.96 | 22.0 | 28.96 | 4.1 |
| 18.23 | 23.2 | 29.51 | 6.4 |
| 18.45 | 70.8 | 29.88 | 4.8 |
| 18.80 | 17.0 | 31.76 | 4.7 |
| 19.09 | 49.3 | 33.54 | 5.0 |
preferably, crystal form 2K has X-ray powder diffraction intensities shown in Table 2-12;
preferably, crystal form 2K has an X-ray powder diffraction pattern substantially shown in FIG. 2-12;
preferably, crystal form 2K exhibits endothermic peaks in a DSC analysis when heated to peak temperatures of about 107.92 °C, about 172.19 °C, and about 184.68 °C;
preferably, crystal form 2K has a DSC profile substantially shown in FIG. 2-37;
preferably, crystal form 2K has a TGA profile substantially shown in FIG. 2-62;
preferably, crystal form 2K is a toluene 0.75-solvate of compound II;
crystal form 2L-1 of a methyl isobutyl ketone solvate has an X-ray powder diffraction pattern comprising peaks at diffraction angles (20) of 8.66 ± 0.2°, 10.79 ± 0.2°, 26.00 ± 0.2°, and 19.62 ± 0.2°;
preferably, further comprising peaks at diffraction angles (20) of 9.13 ± 0.2°, 18.60 ± 0.2°, 16.90 ± 0.2°, 15.58 ± 0.2°, and 19.43 ± 0.2°;
and more preferably, further comprising peaks at diffraction angles (20) of 13.03 ± 0.2°, 17.43 ± 0.2°, 6.74 ± 0.2°, 26.72 ± 0.2°, 26.27 ± 0.2°, and 22.40 ± 0.2°;
preferably, crystal form 2L-1 has an X-ray powder diffraction pattern comprising diffraction angles (20) shown in Table 2-13, wherein the 20 angles have a margin of error of ±0.20°:
**Table 2-13**
| 2θ (°) | Intensity% | 2θ (°) | Intensity% |
|---|---|---|---|
| 4.28 | 9.0 | 18.60 | 32.9 |
| 6.40 | 13.3 | 19.43 | 24.2 |
| 6.74 | 21.4 | 19.62 | 40.4 |
| 7.71 | 7.4 | 20.04 | 9.6 |
| 8.66 | 100.0 | 20.71 | 10.6 |
| 9.13 | 39.7 | 20.94 | 11.7 |
| 10.79 | 77.4 | 21.32 | 12.5 |
| 12.64 | 16.5 | 21.62 | 10.6 |
| 13.03 | 23.1 | 21.91 | 11.7 |
| 13.60 | 12.5 | 22.40 | 19.1 |
| 14.04 | 9.2 | 22.88 | 8.1 |
| 14.51 | 12.1 | 23.59 | 14.1 |
| 14.91 | 8.2 | 24.03 | 9.2 |
| 15.58 | 24.7 | 26.00 | 41.4 |
| 16.54 | 8.9 | 26.27 | 19.5 |
| 16.90 | 31.7 | 26.72 | 20.3 |
| 17.43 | 21.7 | 27.44 | 16.6 |
| 17.84 | 6.2 | | |
preferably, crystal form 2L-1 has X-ray powder diffraction intensities shown in Table 2-13;
preferably, crystal form 2L-1 has an X-ray powder diffraction pattern substantially shown in FIG. 2-13;
preferably, crystal form 2L-1 exhibits an endothermic peak in a DSC analysis when heated to a peak temperature of about 106.45 °C;
preferably, crystal form 2L-1 has a DSC profile substantially shown in FIG. 2-38;
preferably, crystal form 2L-1 has a TGA profile substantially shown in FIG. 2-63;
preferably, crystal form 2L-1 is a methyl isobutyl ketone 0.75-solvate of compound II;
crystal form 2L-2 of a methyl isobutyl ketone solvate has an X-ray powder diffraction pattern comprising peaks at diffraction angles (20) of 8.68 ± 0.2°, 10.83 ± 0.2°, 13.10 ± 0.2°, and 11.06 ± 0.2°;
preferably, further comprising peaks at diffraction angles (20) of 17.49 ± 0.2°, 9.15 ± 0.2°, 16.96 ± 0.2°, 18.69 ± 0.2°, and 6.74 ± 0.2°;
and more preferably, further comprising peaks at diffraction angles (2θ) of 12.62 ± 0.2°, 19.63 ± 0.2°, 19.37 ± 0.2°, 22.01 ± 0.2°, 15.65 ± 0.2°, and 21.33 ± 0.2°;
preferably, crystal form 2L-2 has an X-ray powder diffraction pattern comprising diffraction angles (20) shown in Table 2-14, wherein the 20 angles have a margin of error of ±0.20°:
**Table 2-14**
| 20 (°) | Intensity% | 20 (°) | Intensity% |
|---|---|---|---|
| 6.74 | 12.1 | 17.49 | 23.3 |
| 8.68 | 100.0 | 18.69 | 13.8 |
| 9.15 | 17.9 | 19.37 | 8.9 |
| 10.83 | 41.7 | 19.63 | 9.4 |
| 11.06 | 28.2 | 20.90 | 6.2 |
| 12.62 | 11.6 | 21.33 | 8.0 |
| 13.10 | 29.5 | 22.01 | 8.7 |
| 15.65 | 8.5 | 22.41 | 7.2 |
| 16.96 | 14.8 | 27.52 | 5.4 |
preferably, crystal form 2L-2 has X-ray powder diffraction intensities shown in Table 2-14;
preferably, crystal form 2L-2 has an X-ray powder diffraction pattern substantially shown in FIG. 2-14;
preferably, crystal form 2L-2 exhibits an endothermic peak in a DSC analysis when heated to a peak temperature of about 102.87 °C;
preferably, crystal form 2L-2 has a DSC profile substantially shown in FIG. 2-39;
preferably, crystal form 2L-2 has a TGA profile substantially shown in FIG. 2-64;
preferably, crystal form 2L-2 is a methyl isobutyl ketone 0.3-solvate of compound II;
crystal form 2M-1 of a cyclopentyl methyl ether solvate has an X-ray powder diffraction pattern comprising peaks at diffraction angles (20) of 8.78 ± 0.2°, 10.98 ± 0.2°, 17.67 ± 0.2°, and 17.20 ± 0.2°;
preferably, further comprising peaks at diffraction angles (20) of 19.32 ± 0.2°, 25.69 ± 0.2°, 13.23 ± 0.2°, 22.24 ± 0.2°, and 9.29 ± 0.2°;
and more preferably, further comprising peaks at diffraction angles (2θ) of 18.93 ± 0.2°, 26.53 ± 0.2°, 26.04 ± 0.2°, 15.87 ± 0.2°, 21.25 ± 0.2°, and 12.85 ± 0.2°;
preferably, crystal form 2M-1 has an X-ray powder diffraction pattern comprising diffraction angles (2θ) shown in Table 2-15, wherein the 20 angles have a margin of error of ±0.20°:
**Table 2-15**
| 20 (°) | Intensity% | 20 (°) | Intensity% |
|---|---|---|---|
| 5.18 | 6.5 | 17.67 | 28.0 |
| 6.48 | 4.0 | 18.93 | 20.2 |
| 6.85 | 8.9 | 19.32 | 27.7 |
| 8.78 | 100.0 | 20.36 | 4.3 |
| 9.29 | 22.4 | 20.65 | 8.9 |
| 10.98 | 43.6 | 21.25 | 10.9 |
| 12.85 | 9.8 | 22.24 | 22.8 |
| 13.23 | 23.4 | 23.20 | 5.3 |
| 13.63 | 7.1 | 23.84 | 9.1 |
| 13.88 | 7.2 | 25.69 | 27.4 |
| 14.70 | 3.8 | 26.04 | 15.9 |
| 15.42 | 6.6 | 26.53 | 17.2 |
| 15.87 | 12.0 | 27.21 | 9.0 |
| 16.48 | 3.6 | 28.00 | 3.6 |
| 17.20 | 27.7 | | |
preferably, crystal form 2M-1 has X-ray powder diffraction intensities shown in Table 2-15;
preferably, crystal form 2M-1 has an X-ray powder diffraction pattern substantially shown in FIG. 2-15;
preferably, crystal form 2M-1 exhibits an endothermic peak in a DSC analysis when heated to a peak temperature of about 113.56 °C;
preferably, crystal form 2M-1 has a DSC profile substantially shown in FIG. 2-40;
preferably, crystal form 2M-1 has a TGA profile substantially shown in FIG. 2-65;
preferably, crystal form 2M-1 is a cyclopentyl methyl ether monosolvate of compound II;
crystal form 2M-2 of a cyclopentyl methyl ether solvate has an X-ray powder diffraction pattern comprising peaks at diffraction angles (20) of 5.16 ± 0.2°, 10.83 ± 0.2°, 15.66 ± 0.2°, and 21.29 ± 0.2°;
preferably, further comprising peaks at diffraction angles (20) of 17.63 ± 0.2°, 17.90 ± 0.2°, 8.80 ± 0.2°, 13.03 ± 0.2°, and 23.88 ± 0.2°;
and more preferably, further comprising peaks at diffraction angles (2θ) of 25.73 ± 0.2°, 11.99 ± 0.2°, 19.28 ± 0.2°, 20.20 ± 0.2°, 23.57 ± 0.2°, and 22.20 ± 0.2°;
preferably, crystal form 2M-2 has an X-ray powder diffraction pattern comprising diffraction angles (2θ) shown in Table 2-16, wherein the 20 angles have a margin of error of ±0.20°:
**Table 2-16**
| 20 (°) | Intensity% | 20 (°) | Intensity% |
|---|---|---|---|
| 5.16 | 100.0 | 19.28 | 39.3 |
| 7.01 | 23.1 | 19.77 | 13.6 |
| 8.80 | 65.2 | 20.20 | 35.4 |
| 10.83 | 95.3 | 21.29 | 74.1 |
| 11.99 | 42.3 | 21.68 | 16.4 |
| 13.03 | 62.4 | 22.207 | 33.4 |
| 13.26 | 21.4 | 22.50 | 24.0 |
| 14.10 | 27.3 | 23.57 | 34.5 |
| 15.66 | 83.0 | 23.88 | 49.9 |
| 17.23 | 26.2 | 25.73 | 48.5 |
| 17.63 | 73.8 | 26.53 | 15.3 |
| 17.90 | 68.2 | 27.39 | 17.5 |
| 18.95 | 32.6 | 29.93 | 21.7 |
preferably, crystal form 2M-2 has X-ray powder diffraction intensities shown in Table 2-16;
preferably, crystal form 2M-2 has an X-ray powder diffraction pattern substantially shown in FIG. 2-16;
preferably, crystal form 2M-2 exhibits endothermic peaks in a DSC analysis when heated to peak temperatures of about 110.50 °C and about 165.01 °C;
preferably, crystal form 2M-2 has a DSC profile substantially shown in FIG. 2-41;
preferably, crystal form 2M-2 has a TGA profile substantially shown in FIG. 2-66;
preferably, crystal form 2M-2 is a cyclopentyl methyl ether monosolvate of compound II;
crystal form 2N of a methyl ethyl ketone solvate has an X-ray powder diffraction pattern comprising peaks at diffraction angles (20) of 3.34 ± 0.2°, 10.88 ± 0.2°, 9.13 ± 0.2°, and 6.64 ± 0.2°;
preferably, further comprising peaks at diffraction angles (20) of 7.73 ± 0.2°, 19.87 ± 0.2°, 8.78 ± 0.2°, 12.78 ± 0.2°, and 6.25 ± 0.2°;
and more preferably, further comprising peaks at diffraction angles (2θ) of 18.29 ± 0.2°, 19.08 ± 0.2°, 21.19 ± 0.2°, 11.69 ± 0.2°, 19.49 ± 0.2°, and 17.01 ± 0.2°;
preferably, crystal form 2N has an X-ray powder diffraction pattern comprising diffraction angles (20) shown in Table 2-17, wherein the 20 angles have a margin of error of ±0.20°:
**Table 2-17**
| 2θ (°) | Intensity% | 2θ (°) | Intensity% |
|---|---|---|---|
| 3.34 | 100.0 | 12.78 | 27.7 |
| 6.25 | 25.7 | 17.01 | 19.0 |
| 6.64 | 57.7 | 18.29 | 25.3 |
| 7.73 | 45.1 | 19.08 | 24.1 |
| 8.78 | 33.2 | 19.49 | 19.8 |
| 9.13 | 87.7 | 19.87 | 35.2 |
| 10.88 | 97.2 | 21.19 | 21.7 |
| 11.69 | 20.2 | | |
preferably, crystal form 2N has X-ray powder diffraction intensities shown in Table 2-17;
preferably, crystal form 2N has an X-ray powder diffraction pattern substantially shown in FIG. 2-17;
preferably, crystal form 2N exhibits endothermic peaks in a DSC analysis when heated to peak temperatures of about 102.66 °C and about 113.16 °C;
preferably, crystal form 2N has a DSC profile substantially shown in FIG. 2-42;
preferably, crystal form 2N has a TGA profile substantially shown in FIG. 2-67;
preferably, crystal form 2N is a methyl ethyl ketone 0.3-solvate of compound II;
crystal form 2O of a methylcyclohexane solvate has an X-ray powder diffraction pattern comprising peaks at diffraction angles (20) of 8.72 ± 0.2°, 10.92 ± 0.2°, 13.14 ± 0.2°, and 9.23 ± 0.2°;
preferably, further comprising peaks at diffraction angles (20) of 17.10 ± 0.2°, 18.78 ± 0.2°, 17.55 ± 0.2°, 15.79 ± 0.2°, and 6.89 ± 0.2°;
and more preferably, further comprising peaks at diffraction angles (2θ) of 22.05 ± 0.2°, 12.77 ± 0.2°, 19.30 ± 0.2°, 20.84 ± 0.2°, 23.74 ± 0.2°, and 26.45 ± 0.2°;
preferably, crystal form 2O has an X-ray powder diffraction pattern comprising diffraction angles (20) shown in Table 2-18, wherein the 20 angles have a margin of error of ±0.20°:
**Table 2-18**
| 2θ (°) | Intensity% | 2θ (°) | Intensity% |
|---|---|---|---|
| 6.49 | 5.1 | 16.70 | 3.5 |
| 6.89 | 10.9 | 17.10 | 14.8 |
| 7.92 | 4.3 | 17.55 | 12.4 |
| 8.72 | 100.0 | 18.78 | 14.2 |
| 9.23 | 18.1 | 19.30 | 7.8 |
| 10.92 | 36.2 | 20.25 | 3.3 |
| 12.77 | 9.6 | 20.84 | 6.9 |
| 13.14 | 24.8 | 21.65 | 3.1 |
| 13.80 | 4.7 | 22.05 | 10.6 |
| 14.70 | 3.8 | 23.74 | 6.8 |
| 15.79 | 11.4 | 26.45 | 5.5 |
preferably, crystal form 2O has X-ray powder diffraction intensities shown in Table 2-18;
preferably, crystal form 2O has an X-ray powder diffraction pattern substantially shown in FIG. 2-18;
preferably, crystal form 2O exhibits an endothermic peak in a DSC analysis when heated to a peak temperature of about 111.80 °C;
preferably, crystal form 2O has a DSC profile substantially shown in FIG. 2-43;
preferably, crystal form 2O has a TGA profile substantially shown in FIG. 2-68;
preferably, crystal form 2O is a methylcyclohexane monosolvate of compound II;
crystal form 2P of a DMF solvate has an X-ray powder diffraction pattern comprising peaks at diffraction angles (20) of 10.98 ± 0.2°, 9.31 ± 0.2°, 17.68 ± 0.2°, and 25.67 ± 0.2°;
preferably, further comprising peaks at diffraction angles (20) of 19.65 ± 0.2°, 5.78 ± 0.2°, 6.24 ± 0.2°, 16.54 ± 0.2°, and 21.19 ± 0.2°;
and more preferably, further comprising peaks at diffraction angles (2θ) of 24.09 ± 0.2°, 18.25 ± 0.2°, 25.26 ± 0.2°, 20.67 ± 0.2°, 18.84 ± 0.2°, and 22.19 ± 0.2°;
preferably, crystal form 2P has an X-ray powder diffraction pattern comprising diffraction angles (20) shown in Table 2-19, wherein the 20 angles have a margin of error of ±0.20°:
**Table 2-19**
| 20 (°) | Intensity% | 20 (°) | Intensity% |
|---|---|---|---|
| 5.78 | 21 | 18.56 | 9.1 |
| 6.24 | 20.3 | 18.84 | 13.1 |
| 9.31 | 69.8 | 19.65 | 29.4 |
| 10.98 | 100.0 | 20.67 | 13.5 |
| 11.63 | 11.0 | 21.19 | 16.9 |
| 11.88 | 9.4 | 22.19 | 11.1 |
| 12.56 | 8.8 | 24.09 | 16.9 |
| 14.93 | 8.9 | 24.48 | 8.6 |
| 16.54 | 20.2 | 25.26 | 14.6 |
| 17.68 | 49.9 | 25.67 | 31.3 |
| 18.25 | 15.8 | 27.95 | 6.7 |
preferably, crystal form 2P has X-ray powder diffraction intensities shown in Table 2-19;
preferably, crystal form 2P has an X-ray powder diffraction pattern substantially shown in FIG. 2-19;
preferably, crystal form 2P exhibits an endothermic peak in a DSC analysis when heated to a peak temperature of about 105.14 °C;
preferably, crystal form 2P has a DSC profile substantially shown in FIG. 2-44;
preferably, crystal form 2P has a TGA profile substantially shown in FIG. 2-69;
preferably, crystal form 2P is a DMF monosolvate of compound II;
crystal form 2Q-1 of a 2-methyltetrahydrofuran solvate has an X-ray powder diffraction pattern comprising peaks at diffraction angles (20) of 8.74 ± 0.2°, 10.89 ± 0.2°, 3.53 ± 0.2°, and 17.90 ± 0.2°;
preferably, further comprising peaks at diffraction angles (20) of 18.77 ± 0.2°, 17.65 ± 0.2°, 16.93 ± 0.2°, 7.54 ± 0.2°, and 9.19 ± 0.2°;
and more preferably, further comprising peaks at diffraction angles (2θ) of 13.18 ± 0.2°, 8.45 ± 0.2°, 9.63 ± 0.2°, 22.07 ± 0.2°, 6.76 ± 0.2°, and 12.81 ± 0.2°;
preferably, crystal form 2Q-1 has an X-ray powder diffraction pattern comprising diffraction angles (2θ) shown in Table 2-20, wherein the 20 angles have a margin of error of ±0.20°:
**Table 2-20**
| 2θ (°) | Intensity% | 2θ (°) | Intensity% |
|---|---|---|---|
| 3.53 | 40.6 | 13.18 | 16.7 |
| 6.76 | 10.9 | 14.28 | 8.6 |
| 7.11 | 7.9 | 15.69 | 7.9 |
| 7.54 | 17.6 | 16.24 | 9.2 |
| 8.45 | 15.7 | 16.93 | 20.9 |
| 8.74 | 100.0 | 17.65 | 21.3 |
| 9.19 | 17.6 | 17.90 | 28.5 |
| 9.63 | 12.6 | 18.77 | 21.8 |
| 10.89 | 61.5 | 20.83 | 9.2 |
| 12.81 | 10.9 | 22.07 | 12.3 |
preferably, crystal form 2Q-1 has X-ray powder diffraction intensities shown in Table 2-20;
preferably, crystal form 2Q-1 has an X-ray powder diffraction pattern substantially shown in FIG. 2-20;
preferably, crystal form 2Q-1 exhibits an endothermic peak in a DSC analysis when heated to a peak temperature of about 111.13 °C;
preferably, crystal form 2Q-1 has a DSC profile substantially shown in FIG. 2-45;
preferably, crystal form 2Q-1 has a TGA profile substantially shown in FIG. 2-70;
preferably, crystal form 2Q-1 is a 2-methyltetrahydrofuran monosolvate of compound II;
crystal form 2Q-2 of a 2-methyltetrahydrofuran solvate has an X-ray powder diffraction pattern comprising peaks at diffraction angles (20) of 8.76 ± 0.2°, 10.89 ± 0.2°, 9.21 ± 0.2°, and 17.02 ± 0.2°;
preferably, further comprising peaks at diffraction angles (20) of 18.80 ± 0.2°, 6.79 ± 0.2°, 19.65 ± 0.2°, 17.63 ± 0.2°, and 26.08 ± 0.2°;
and more preferably, further comprising peaks at diffraction angles (20) of 13.18 ± 0.2°, 15.68 ± 0.2°, 4.34 ± 0.2°, 13.70 ± 0.2°, 12.74 ± 0.2°, and 21.46 ± 0.2°;
preferably, crystal form 2Q-2 has an X-ray powder diffraction pattern comprising diffraction angles (2θ) shown in Table 2-21, wherein the 20 angles have a margin of error of ±0.20°:
**Table 2-21**
| 2θ (°) | Intensity% | 2θ (°) | Intensity% |
|---|---|---|---|
| 4.34 | 18.6 | 18.58 | 9.0 |
| 6.44 | 11.4 | 18.80 | 30.6 |
| 6.79 | 26.3 | 19.65 | 23.4 |
| 7.79 | 5.7 | 20.21 | 6.6 |
| 8.76 | 100.0 | 20.64 | 5.9 |
| 9.21 | 42.7 | 20.97 | 7.0 |
| 10.89 | 85.3 | 21.46 | 11.7 |
| 12.74 | 12.7 | 22.08 | 11.7 |
| 13.18 | 19.8 | 22.48 | 10.0 |
| 13.70 | 13.6 | 23.066 | 5.4 |
| 14.53 | 8.7 | 24.17 | 4.0 |
| 15.68 | 19.3 | 26.08 | 20.2 |
| 16.75 | 11.3 | 26.81 | 8.0 |
| 17.02 | 36.1 | 27.62 | 9.2 |
| 17.63 | 22.9 | 18.58 | 9.0 |
preferably, crystal form 2Q-2 has X-ray powder diffraction intensities shown in Table 2-21;
preferably, crystal form 2Q-2 has an X-ray powder diffraction pattern substantially shown in FIG. 2-21;
preferably, crystal form 2Q-2 exhibits an endothermic peak in a DSC analysis when heated to a peak temperature of about 114.18 °C;
preferably, crystal form 2Q-2 has a DSC profile substantially shown in FIG. 2-46;
preferably, crystal form 2Q-2 has a TGA profile substantially shown in FIG. 2-71;
preferably, crystal form 2Q-2 is a 2-methyltetrahydrofuran monosolvate of compound II;
crystal form 2R of a N-methylpyrrolidone solvate has an X-ray powder diffraction pattern comprising peaks at diffraction angles (20) of 10.79 ± 0.2°, 9.15 ± 0.2°, 3.44 ± 0.2°, and 17.63 ± 0.2°;
preferably, further comprising peaks at diffraction angles (20) of 25.83 ± 0.2°, 19.26 ± 0.2°, 20.86 ± 0.2°, 16.76 ± 0.2°, and 25.48 ± 0.2°;
and more preferably, further comprising peaks at diffraction angles (20) of 16.51 ± 0.2°, 9.55 ± 0.2°, 17.18 ± 0.2°, 19.54 ± 0.2°, 14.08 ± 0.2°, and 7.34 ± 0.2°;
preferably, crystal form 2R has an X-ray powder diffraction pattern comprising diffraction angles (20) shown in Table 2-22, wherein the 20 angles have a margin of error of ±0.20°:
**Table 2-22**
| 2θ (°) | Intensity% | 2θ (°) | Intensity% |
|---|---|---|---|
| 3.44 | 62.9 | 16.76 | 33.3 |
| 6.11 | 13.9 | 17.18 | 22.9 |
| 6.68 | 12.9 | 17.63 | 59.4 |
| 7.01 | 13.9 | 17.97 | 14.3 |
| 7.34 | 21.4 | 18.41 | 20.4 |
| 8.33 | 13.7 | 19.26 | 41.6 |
| 9.15 | 77.6 | 19.54 | 22.0 |
| 9.55 | 24.3 | 20.47 | 10.2 |
| 10.79 | 100.0 | 20.86 | 37.1 |
| 11.43 | 14.7 | 21.49 | 13.1 |
| 11.66 | 14.7 | 22.21 | 16.1 |
| 12.31 | 11.8 | 23.23 | 18.4 |
| 13.78 | 16.7 | 23.80 | 18.8 |
| 14.08 | 21.6 | 24.09 | 14.5 |
| 14.67 | 17.6 | 25.48 | 28.4 |
| 15.36 | 8.4 | 25.83 | 49.0 |
| 16.51 | 26.1 | 27.19 | 16.9 |
preferably, crystal form 2R has X-ray powder diffraction intensities shown in Table 2-22;
preferably, crystal form 2R has an X-ray powder diffraction pattern substantially shown in FIG. 2-22;
preferably, crystal form 2R exhibits an endothermic peak in a DSC analysis when heated to a peak temperature of about 128.45 °C;
preferably, crystal form 2R has a DSC profile substantially shown in FIG. 2-47;
preferably, crystal form 2R has a TGA profile substantially shown in FIG. 2-72;
preferably, crystal form 2R is a N-methylpyrrolidone monosolvate of compound II;
crystal form 2S of a trifluoroethanol solvate has an X-ray powder diffraction pattern comprising peaks at diffraction angles (20) of 17.64 ± 0.2°, 4.01 ± 0.2°, 8.72 ± 0.2°, and 8.28 ± 0.2°;
preferably, further comprising peaks at diffraction angles (20) of 13.63 ± 0.2°, 10.89 ± 0.2°, 12.17 ± 0.2°, 19.65 ± 0.2°, and 23.65 ± 0.2°;
and more preferably, further comprising peaks at diffraction angles (20) of 26.08 ± 0.2°, 15.8 ± 0.2°, 21.35 ± 0.2°, 7.61 ± 0.2°, 22.49 ± 0.2°, and 5.61 ± 0.2°;
preferably, crystal form 2S has an X-ray powder diffraction pattern comprising diffraction angles (20) shown in Table 2-23, wherein the 20 angles have a margin of error of ±0.20°:
**Table 2-23**
| 2θ (°) | Intensity% | 2θ (°) | Intensity% |
|---|---|---|---|
| 4.01 | 60.0 | 15.81 | 26.3 |
| 5.61 | 16.9 | 17.64 | 100.0 |
| 7.61 | 21.9 | 19.65 | 34.4 |
| 8.28 | 54.7 | 21.35 | 24.4 |
| 8.72 | 57.2 | 22.49 | 17.5 |
| 10.89 | 41.6 | 23.65 | 34.1 |
| 12.17 | 39.4 | 26.08 | 29.4 |
| 13.63 | 42.5 | | |
preferably, crystal form 2S has X-ray powder diffraction intensities shown in Table 2-23;
preferably, crystal form 2S has an X-ray powder diffraction pattern substantially shown in FIG. 2-23;
preferably, crystal form 2S exhibits endothermic peaks in a DSC analysis when heated to peak temperatures of about 123.79 °C and about 169.39 °C;
preferably, crystal form 2S has a DSC profile substantially shown in FIG. 2-48;
preferably, crystal form 2S has a TGA profile substantially shown in FIG. 2-73;
preferably, crystal form 2S is a trifluoroethanol monosolvate of compound II;
crystal form 2T of a tetrahydrofuran solvate has an X-ray powder diffraction pattern comprising peaks at diffraction angles (20) of 12.17 ± 0.2°, 8.92 ± 0.2°, 7.63 ± 0.2°, and 8.22 ± 0.2°;
preferably, further comprising peaks at diffraction angles (20) of 13.99 ± 0.2°, 17.96 ± 0.2°, 9.27 ± 0.2°, 19.51 ± 0.2°, and 13.57 ± 0.2°;
and more preferably, further comprising peaks at diffraction angles (2θ) of 11.10 ± 0.2°, 15.89 ± 0.2°, 14.33 ± 0.2°, 19.77 ± 0.2°, 20.39 ± 0.2°, and 16.61 ± 0.2°;
preferably, crystal form 2T has an X-ray powder diffraction pattern comprising diffraction angles (20) shown in Table 2-24, wherein the 20 angles have a margin of error of ±0.20°:
**Table 2-24**
| 2θ (°) | Intensity% | 2θ (°) | Intensity% |
|---|---|---|---|
| 4.51 | 14.5 | 15.38 | 13.2 |
| 6.37 | 10.5 | 15.89 | 25.4 |
| 6.70 | 16.3 | 16.61 | 19.9 |
| 7.63 | 57.2 | 17.96 | 35.8 |
| 8.22 | 39.2 | 19.51 | 28.9 |
| 8.92 | 75.5 | 19.77 | 22.2 |
| 9.27 | 28.9 | 20.39 | 21.8 |
| 10.51 | 8.6 | 21.15 | 12.2 |
| 11.10 | 26.2 | 21.55 | 10.9 |
| 12.17 | 100.0 | 22.80 | 12.8 |
| 13.05 | 16.3 | 23.78 | 18.5 |
| 13.57 | 28.5 | 25.98 | 10.3 |
| 13.99 | 38.8 | 27.38 | 7.3 |
| 14.33 | 22.4 | | |
preferably, crystal form 2T has X-ray powder diffraction intensities shown in Table 2-24;
preferably, crystal form 2T has an X-ray powder diffraction pattern substantially shown in FIG. 2-24;
preferably, crystal form 2T exhibits an endothermic peak in a DSC analysis when heated to a peak temperature of about 110.50 °C;
preferably, crystal form 2T has a DSC profile substantially shown in FIG. 2-49;
preferably, crystal form 2T has a TGA profile substantially shown in FIG. 2-74;
preferably, crystal form 2T is a tetrahydrofuran 0.3-solvate of compound II;
crystal form 2U of a dioxane solvate has an X-ray powder diffraction pattern comprising peaks at diffraction angles (20) of 8.86 ± 0.2°, 10.98 ± 0.2°, 9.27 ± 0.2°, and 17.12 ± 0.2°;
preferably, further comprising peaks at diffraction angles (20) of 19.05 ± 0.2°, 19.55 ± 0.2°, 15.70 ± 0.2°, 13.36 ± 0.2°, and 6.79 ± 0.2°;
and more preferably, further comprising peaks at diffraction angles (2θ) of 25.85 ± 0.2°, 12.87 ± 0.2°, 22.48 ± 0.2°, 13.69 ± 0.2°, 4.39 ± 0.2°, and 17.86 ± 0.2°;
preferably, crystal form 2U has an X-ray powder diffraction pattern comprising diffraction angles (20) shown in Table 2-25, wherein the 20 angles have a margin of error of ±0.20°:
**Table 2-25**
| 2θ (°) | Intensity% | 2θ (°) | Intensity% |
|---|---|---|---|
| 4.39 | 9.6 | 15.70 | 19.5 |
| 6.35 | 8.3 | 17.12 | 39.0 |
| 6.79 | 16 | 17.86 | 9.1 |
| 7.77 | 6.3 | 19.05 | 21.8 |
| 8.86 | 100.0 | 19.55 | 21.1 |
| 9.27 | 44.9 | 20.36 | 6.8 |
| 10.98 | 71.6 | 20.70 | 8.5 |
| 12.87 | 13.2 | 21.07 | 7.3 |
| 13.36 | 17.0 | 21.48 | 8.0 |
| 13.69 | 12.3 | 22.48 | 12.6 |
| 14.59 | 8.6 | 25.85 | 15.5 |
preferably, crystal form 2U has X-ray powder diffraction intensities shown in Table 2-25;
preferably, crystal form 2U has an X-ray powder diffraction pattern substantially shown in FIG. 2-25;
preferably, crystal form 2U exhibits an endothermic peak in a DSC analysis when heated to a peak temperature of about 109.16 °C;
preferably, crystal form 2U has a DSC profile substantially shown in FIG. 2-50;
preferably, crystal form 2U has a TGA profile substantially shown in FIG. 2-75;
preferably, crystal form 2U is a dioxane 0.5-solvate of compound II.

5. A method for preparing the polymorph according to any one of claims 1-4, including the following methods:
method I, comprising: step 1, dissolving or dispersing compound III in a solvent; and step 2, stirring at 0-50 °C for crystallization, or adding an antisolvent into the clarified solution of the compound for crystallization, or slowly evaporating the clarified solution of the compound;
method II, comprising: dispersing compound III in a solvent and an atmosphere of the solvent to give a crystal; and
method III, comprising: combining method I and method II to give the polymorph of compound III.

6. The method according to claim 5, wherein the solvent is water, an organic solvent, or a mixed solvent thereof, and the organic solvent is selected from alcohols, chloroalkanes, ketones, ethers, cyclic ethers, esters, alkanes, cycloalkanes, benzenoids, amides, sulfoxides, and a mixture thereof; preferably, the organic solvent is selected from methanol, ethanol, n-propanol, isopropanol, n-butanol, trifluoroethanol, acetonitrile, acetone, methyl ethyl ketone, methyl isobutyl ketone, 1,4-dioxane, tetrahydrofuran, 2-methyltetrahydrofuran, *N,N*-dimethylformamide, *N,N*-dimethylacetamide, *N*-methylpyrrolidone, dimethylsulfoxide, ethyl acetate, isopropyl acetate, dichloromethane, trichloroethane, tetrachloromethane, methyl *tert*-butyl ether, cyclopentyl methyl ether, 2-methoxyethyl ether, isopropyl ether, diethyl ether, *n*-heptane, *n*-hexane, isooctane, pentane, cyclohexane, cyclopentane, methylcyclohexane, benzene, toluene, xylene, and a mixture thereof.

7. A pharmaceutical composition, comprising at least one polymorph according to any one of claims 1-4 and a pharmaceutically acceptable carrier.

8. Use of the polymorph according to any one of claims 1-4 for manufacturing a medicament for the treatment of a metabolic disease, tumor, autoimmune disease, or metastatic disease.

9. The use according to claim 8, wherein the metabolic disease, tumor, autoimmune disease, or metastatic disease is selected from T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, kidney disease, diabetic retinopathy, adipocyte dysfunction, visceral adipocyte accumulation, sleep apnea, obesity, eating disorders, weight gain caused by use of other medicaments, excessive sugar craving, dyslipidemia, hyperinsulinemia, NAFLD, NAS, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, osteoporosis, Parkinson's disease, left ventricular hypertrophy, peripheral arterial disease, macular degeneration, cataract, glomerulosclerosis, chronic renal failure, metabolic syndrome, syndrome XI, premenstrual syndrome, angina pectoris, thrombosis, atherosclerosis, transient ischemic attacks, vascular restenosis, impaired glucose metabolism, impaired fasting blood glucose conditions, hyperuricemia, gout, erectile dysfunction, skin and connective tissue abnormalities, psoriasis, foot ulcers, ulcerative colitis, hyperapoB lipoproteinemia, Alzheimer's disease, schizophrenia, impaired cognitive function, inflammatory bowel disease, short bowel syndrome, Crohn's disease, colitis, irritable bowel syndrome, and polycystic ovary syndrome.

10. The use according to claim 8, wherein the metabolic disease, tumor, autoimmune disease, or metastatic disease is selected from T1D, T2DM, prediabetes, idiopathic T1D, LADA, EOD, YOAD, MODY, malnutrition-related diabetes, gestational diabetes, hyperglycemia, insulin resistance, hepatic insulin resistance, glucose intolerance, diabetic neuropathy, diabetic nephropathy, obesity, eating disorders, weight gain caused by use of other medicaments, excessive sugar craving, dyslipidemia, and hyperinsulinemia.
